# EUROPEAN PATENT SPECIFICATION

(11) **EP 1 292 335 B1**
(45) Date of publication and mention of the grant of the patent: **30.05.2007**
(21) Application number: 01965012.6
(22) Date of filing: 20.06.2001
(51) Int. Cl.: A61K 45/06, A61P 35/00

(54) **COMBINATIONS AND COMPOSITIONS WHICH INTERFERE WITH VEGF/VEGF AND ANGIOPOIETIN/TIE RECEPTOR FUNCTION AND THEIR USE (II)**
ZUSAMMENSETZUNGEN, DIE MIT VEGF/VEGF UND ANGIOPOIETIN/TIE REZEPTOR-FUNKTIONEN INTERFERIEREN
COMBINAISONS ET COMPOSITIONS PERTURBANT LA FONCTION DES RECEPTEURS VEGF/VEGF ET ANGIOPOIETINE/TIE ET LEUR UTILISATION (II)

(30) Priority: 23.06.2000 EP 00250194; 28.06.2000 EP 00250214
(43) Date of publication of application: 19.03.2003
(60) Divisional application: 05090011.7
(73) Proprietor: Bayer Schering Pharma Aktiengesellschaft, 13353 Berlin (DE)
(72) Inventor: SIEMEISTER, Gerhard, 13503 Berlin (DE); HABEREY, Martin, 12169 Berlin (DE); THIERAUCH, Karl-Heinz, 14169 Berlin (DE)
(86) International application number: PCT/EP2001/006976
(87) International publication number: WO 2001/097850

(56) References cited:
- WO-A-98/35958
- KOBLIZEK, THOMAS I. ET AL: "Angiopoietin-1 induces sprouting angiogenesis in vitro" CURR. BIOL. (1998), 8(9), 529-532, XP000972727
- SCHLAEPPI, JEAN-MARC ET AL: "Targeting vascular endothelial growth factor (VEGF) for anti-tumor therapy, by anti-VEGF neutralizing monoclonal antibodies or by VEGF receptor tyrosine-kinase inhibitors." CANCER AND METASTASIS REVIEWS, (1999) VOL. 18, NO. 4, PP. 473-481. PRINT., XP000972749

## Description

The present invention provides the combination of substances interfering with the biological activity of Vascular Endothelial Growth Factor (VEGF)/VEGF receptor systems (compound I) and substances interfering with the biological function of Angiopoietin/Tie receptor systems (compound II) for inhibition of vascularization and for cancer treatment.

Protein ligands and receptor tyrosine kinases that specifically regulate endothelial cell function are substantially involved in physiological as well as in disease-related angiogenesis. These ligand/receptor systems include the Vascular Endothelial Growth Factor (VEGF) and the Angiopoietin (Ang) families, and their receptors, the VEGF receptor family and the tyrosine kinase with immunoglobulin-like and epidermal growth factor homology domains (Tie) family. The members of the two families of receptor tyrosine kinases are expressed primarily on endothelial cells. The VEGF receptor family includes Flt1 (VEGF-R1), Flk1/KDR (VEGF-R2), and Flt4 (VEGF-R3). These receptors are recognized by members of the VEGF-related growth factors in that the ligands of Flt1 are VEGF and placenta growth factor (PIGF), whereas Flk1/KDR binds VEGF, VEGF-C and VEGF-D, and the ligands of Flt4 are VEGF-C and VEGF-D (Nicosia, Am. J. Pathol. 153, 11-16, 1998). The second family of endothelial cell specific receptor tyrosine kinases is represented by Tie1 and Tie2 (also kown as Tek). Whereas Tie1 remains an orphan receptor, three secreted glycoprotein ligands of Tie2, Ang1, Ang2, and Ang3/Ang4 have been discovered (Davis et al., Cell 87, 1161-1169, 1996; Maisonpierre et al., Science 277, 55-60, 1997; Valenzuela et al, Proc. Natl. Acad. Sci. USA 96, 1904-1909, 1999; patents: US 5,521,073; US 5,650,490; US 5,814,464).

The pivotal role of VEGF and of its receptors during vascular development was exemplified in studies on targeted gene inactivation. Even the heterozygous disruption of the VEGF gene resulted in fatal deficiencies in vascularization (Carmeliet et al., Nature 380, 435-439, 1996; Ferrara et al., Nature 380, 439-442, 1996). Mice carrying homozygous disruptions in either Flt1 or Flk1/KDR gene die in mid-gestation of acute vascular defects. However, the phenotypes are distinct in that Flkl/KDR knock-out mice lack both endothelial cells and a developing hematopoietic system (Shalaby et al. Nature 376, 62-66, 1995), whereas Flt1 deficient mice have normal hematopoietic progenitors and endothelial cells, which fail to assemble into functional vessels (Fong et al., 376, 66-70, 1995). Disruption of the FIt4 gene, whose extensive embryonic expression becomes restricted to lymphatic vessels in adults, revealed an essential role of Flt4 for the remodeling and maturation of the primary vascular networks into larger blood vessels during early development of the cardiovascular system (Dumont et al., Science 282, 946-949, 1998). Consistent with the lymphatic expression of Flt4 in adults overexpression of VEGF-C in the skin of transgenic mice resulted in lymphatic, but not vascular, endothelial proliferation and vessel enlargement (Jeltsch et al., Science 276, 1423-1425, 1997). Moreover, VEGF-C was reported to induce neovascularization in mouse cornea and chicken embryo chorioallantoic membrane models of angiogenesis (Cao et al., Proc. Natl. Acad. Sci. USA 95, 14389-14394, 1998).

The second class of endothelial cell specific receptor tyrosine kinases has also been found to be critically involved in the formation and integrity of vasculature. Mice deficient in Tie1 die of edema and hemorrhage resulting from poor structural integrity of endothelial cells of the microvasculature (Sato et al., Nature 376, 70-74, 1995; Rodewald & Sato, Oncogene 12, 397-404, 1996). The Tie2 knock-out phenotype is characterized by immature vessels lacking branching networks and lacking periendothelial support cells (Sato et al., Nature 376, 70-74, 1995; Dumont et al., Genes Dev. 8, 1897-1909, 1994). Targeted inactivation of the Tie2 ligand Ang1, as well as overexpression of Ang2, an inhibitory ligand, resulted in phenotypes similar to the Tie2 knock out (Maisonpierre et al., Science 277, 55-60, 1997; Suri et al., cell 87, 1171-1180). Conversely, increased vascularization was observed upon transgenic overexpression of Ang1 (Suri et al., Science 282, 468-471, 1998; Thurstonen et al., Science 286, 2511-2514, 1999).

The results from angiogenic growth factor expression studies in corpus luteum development (Maisonpierre et al., Science 277, 55-60, 1997; Goede et al. Lab.

Invest. 78, 1385-1394, 1998), studies on blood vessel maturation in the retina (Alon et al., Nature Med. 1, 1024-1028, 1995; Benjamin et al, Development 125, 1591-1598, 1998), and gene targeting and transgenic experiments on Tie2, Ang1, and Ang2, suggest a fundamental role of the Angiopoietin/Tie receptor system in mediating interactions between endothelial cells and surrounding pericytes or smooth muscle cells. Ang1, which is expressed by the periendothelial cells and seems to be expressed constitutively in the adult, is thought to stabilize existing mature vessels. Ang2, the natural antagonist of Ang1 which is expressed by endothelial cells at sites of vessel sprouting, seems to mediate loosening of endothelial-periendothelial cell contacts to allow vascular remodeling and sprouting in cooperation with angiogenesis initiators such as VEGF, or vessel regression in the absence of VEGF (Hanahan, Science 277, 48-50, 1997).

In pathological settings associated with aberrant neovascularization elevated expression of angiogenic growth factors and of their receptors has been observed. Most solid tumors express high levels of VEGF and the VEGF receptors appear predominantly in endothelial cells of vessels surrounding or penetrating the malignant tissue (Plate et al., Cancer Res. 53, 5822-5827, 1993). Interference with the VEGFNEGF receptor system by means of VEGF-neutralizing antibodies (Kim et al., Nature 362, 841-844, 1993), retroviral expression of dominant negative VEGF receptor variants (Millauer et al., Nature 367, 576-579, 1994), recombinant VEGF-neutralizing receptor variants (Goldman et al., Proc. Natl. Acad. Sci. USA 95, 8795-8800, 1998), or small molecule inhibitors of VEGF receptor tyrosine kinase (Fong et al., Cancer Res. 59, 99-106, 1999; Wedge et al., Cancer Res. 60, 970-975, 2000; Wood et al. Cancer Res. 60, 2178-2189, 2000), or targeting cytotoxic agents via the VEGFNEGF receptor system (Arora et al., Cancer Res. 59, 183-188, 1999; EP 0696456A2) resulted in reduced tumor growth and tumor vascularization. However, although many tumors were inhibited by interference with the VEGFNEGF receptor system, others were unaffected (Millauer et al., Cancer Res. 56, 1615-1620, 1996). Human tumors as well as experimental tumor xenografts contain a large number of immature blood vessels that have not yet recruited periendothelial cells. The fraction of immature vessels is in the range of 40% in slow growing prostate cancer and 90% in fast growing glioblastoma. A selective obliteration of immature tumor vessels was observed upon withdrawal of VEGF by means of downregulation of VEGF transgene expression in a C6 glioblastoma xenograft model. This result is in accordance with a function of VEGF as endothelial cell survival factor. Similarly, in human prostate cancer shutting off VEGF expression as a consequence of androgen-ablation therapy led to selective apoptotic death of endothelial cells in vessels lacking periendothelial cell coverage. In contrast, the fraction of vessels which resisted VEGF withdrawal showed periendothelial cell coverage (Benjamin et al., J. Clin. Invest. 103, 159-165, 1999).

The observation of elevated expression of Tie receptors in the endothelium of metastatic melanomas (Kaipainen et al., Cancer Res. 54, 6571-6577, 1994), in breast carcinomas (Salvén et al., Br. J. Cancer 74, 69-72, 1996), and in tumor xenografts grown in the presence of dominant-negative VEGF receptors (Millauer et al., Cancer Res. 56, 1615-1620, 1996), as well as elevated expression of Flt 4 receptors in the endothelium of lymphatic vessels surrounding lymphomas and breast carcinomas (Jussila et al., Cancer Res. 58, 1599-1604, 1998), and of VEGF-C in various human tumor samples (Salvén et al., Am. J. Pathol. 153, 103-108, 1998), suggested these endothelium-specific growth factors and receptors as candidate alternative pathways driving tumor neovascularization. The high upregulation of Ang2 expression already in early tumors has been interpreted in terms of a host defense mechanism against initial cooption of existing blood vessels by the developing tumor. In the absence of VEGF, the coopted vessels undergo regression leading to necrosis within the center of the tumor. Contrarily, hypoxic upregulation of VEGF expression in cooperation with elevated Ang2 expression rescues and supports tumor vascularization and tumor growth at the tumor margin (Holash et al., Science 284, 1994-1998, 1999; Holash et al., Oncogene 18, 5356-5362, 1999).

Interference with Tie2 receptor function by means of Angiopoietin-neutralizing Tie2 variants consisting of the extracellular ligand-binding domain has been shown to result in inhibition of growth and vascularization of experimental tumors (Lin et al., J. Clin. Invest. 103, 159-165, 1999; Lin et al. Proc. Natl. Acad. Sci. USA 95, 8829-8834, 1998; Siemeister et al., Cancer Res. 59, 3185-3191, 1999). Comparing the effects of interference with the endothelium-specific receptor tyrosine kinase pathways by means of paracrine expression of the respective extracellular receptor domains on the same cellular background demonstrated inhibition of tumor growth upon blockade of the VEGF receptor system and of the Tie2 receptor system, respectively (Siemeister et al., Cancer Res. 59, 3185-3191, 1999).

It is known that the inhibition of the VEGF/VEGR receptor system by various methods resulted only in slowing down growth of most experimental tumors (Millauer et al., Nature 367, 576-579, 1994; Kim et al., Nature 362, 841-844, 1993; Millauer et al., Cancer Res. 56, 1615-1620, 1996; Goldman et al., Proc. Natl. Acad. Sci. USA 95, 8795-8800, 1998; Fong et al., Cancer Res. 59, 99-106, 1999; Wedge et al., Cancer Res. 60, 970-975, 2000; Wood et al. Cancer Res. 60, 2178-2189, 2000; Siemeister et al., Cancer Res. 59, 3185-3191, 1999). Even by escalation of therapeutic doses a plateau level of therapeutic efficacy was achieved (Kim et al., Nature 362, 841-844, 1993; Wood et al. Cancer Res. 60, 2178-2189, 2000). Similar results were observed upon interference with the Angiopoietin/Tie2 receptor system (Lin et al., J. Clin. Invest. 103, 159-165, 1999; Lin et al., Proc. Natl. Acad. Sci. USA 95, 8829-8834, 1998; Siemeister et al., Cancer Res. 59, 3185-3191, 1999).

However, there is a high demand for methods that enhance the therapeutic efficacy of anti-angiogenous compounds.

Searching for methods that enhance the therapeutic efficacy of anti-angiogenic compounds, superior anti-tumor effects were observed unexpectedly upon combination of inhibition of VEGF/VEGF receptor systems and interference with biological function of Angiopoietin/Tie receptor systems. The mode of action underlying the superior effects observed may be that interference biological function of Angiopoietin/Tie receptor systems destabilizes endothelial cell-periendothelial cell interaction of existing mature tumor vessels and thereby sensitizes the endothelium to compounds directed against VEGF/VEGF receptor systems.

Based on this unexpected finding the present invention provides the combination of functional interference with VEGFNEGF receptor systems and with Angiopoietin/Tie receptor systems for inhibition of vascularization and of tumor growth.

The pharmaceutical composition consists of two components: compound I inhibits the biological activity of one or several of the VEGFNEGF receptor systems or consists of cytotoxic agents which are targeted to the endothelium via recognition of VEGFNEGF receptor systems. Compound II interferes with the biological function of one or several of Angiopoietin/Tie receptor systems or consists of cytotoxic agents which are targeted to the endothelium via recognition of Angiopoietin/Tie receptor systems. Alternatively, compound I inhibits the biological activity of one or several of the VEGFNEGF receptor systems or of the Angiopoietin/Tie receptor systems and coumpound II consists of cytotoxic agents which are targeted to the endothelium via recognition of one or several of the VEGF/VEGF receptor systems or of the Angiopoietin/Tie receptor systems. Targeting or modulation of the biological activities of VEGFNEGF receptor systems and of Angiopietin/Tie receptor systems can be performed by
(a) compounds which inhibit receptor tyrosine kinase activity,
(b) compounds which inhibit ligand binding to receptors,
(c) compounds which inhibit activation of intracellular signal pathways of the receptors,
(d) compounds which inhibit or activate expression of a ligand or of a receptor of the VEGF or Tie receptor system,
(e) delivery systems, such as antibodies, ligands, high-affinity binding oligonucleotides or oligopeptides, or liposomes, which target cytotoxic agents or coagulation-inducing agents to the endothelium via recognition of VEGFNEGF receptor or Angiopoietin/Tie receptor systems,
(f) delivery systems, such as antibodies, ligands, high-affinity binding oligonucleotides or oligopeptides, or liposomes, which are targeted to the endothelium and induce necrosis or apoptosis.

A compound comprised by compositions of the present invention can be a small molecular weight substance, an oligonucleotide, an oligopeptide, a recombinant protein, an antibody, or conjugates or fusionproteins thereof. An example of an inhibitor is a small molecular weight molecule which inactivates a receptor tyrosine kinase by binding to and occupying the catalytic site such that the biological activity of the receptor is decreased. Kinase inhibitors are known in the art (Sugen: SU5416, SU6668; Fong et al. (1999), Cancer Res. 59, 99-106; Vajkoczy et al., Proc. Am. Associ. Cancer Res. San Francisco (2000), Abstract ID 3612; Zeneca: ZD4190, ZD6474; Wedge et al. (2000), Cancer Res. 60, 970-975; Parke-Davis PD0173073, PD0173074; Johnson et al., Proc. Am. Associ. Cancer Res., San Franzisco (2000), Abstract ID 3614; Dimitroff et al. (1999), Invest. New Drugs 17, 121-135). An example of an antagonist is a recombinant protein or an antibody which binds to a ligand such that activation of the receptor by the ligand is prevented. Another example of an antagonist is an antibody which binds to the receptor such that activation of the receptor is prevented. An example of an expression modulator is an antisense RNA or ribozyme which controls expression of a ligand or a receptor. An example of a targeted cytotoxic agent is a fusion protein of a ligand with a bacterial or plant toxin such as Pseudomonas exotoxin A, Diphtheria toxin, or Ricin A. An example of a targeted coagulation-inducing agent is a conjugate of a single chain antibody and tissue factor. Ligand-binding inhibitors such as neutralizing antibodies which are known in the art are described by Genentech (rhuMAbVEGF) and by Presta et al. (1997), Cancer Res. 57, 4593-4599. Ligand-binding receptor domaines are described by Kendall & Thomas (1993), Proc. Natl. Acad. Sci., U.S.A.90, 10705-10709; by Goldman et al. (1998) Proc. Natl. Acad. Sci., U.S.A.95, 8795-8800 and by Lin et al. (1997), J. Clin. Invest. 100, 2072-2078. Further, dominant negative receptors have been described by Millauer et al. (1994), Nature 367, 567-579. Receptor blocking antibodies have been described by Imclone (c-p1C11, US 5,874,542). Further known are antagonistic ligand mutants (Siemeister et al. (1998), Proc. Natl. Acad. Sci., U.S.A.95, 4625-4629). High affinity ligand- or receptor binding oligo nucleotides habe been described by NeXstar (NX-244) and Drolet et al. (1996), Nat. Biotech 14, 1021-1025. Further, small molecules and peptides have been described.

Expression regulators have been described as anti-sense oligo nucleotides and as ribozymes (RPI, Angiozyme^{™}, see RPI Homepage).

Examples for delivery-/Targeting-Systems have been described as ligand/ antibody-toxin-fusion-proteins or conjugates (Arora et al. (1999), Cancer Res. 59, 183-188 and Olson et al. (1997), Int. J. Cancer 73, 865-870), as endothel cell targeting of liposomes (Spragg et al. (1997), Prog. Natl. Acad. Sci, U.S.A94, 8795-8800, and as endothel cell targeting plus coagulation-induction (Ran et al., (1998), Cancer Res. 58, 4646-4653).

Small molecules which inhibit the receptor tyrosine kinase activity are for example molecules of general formula I 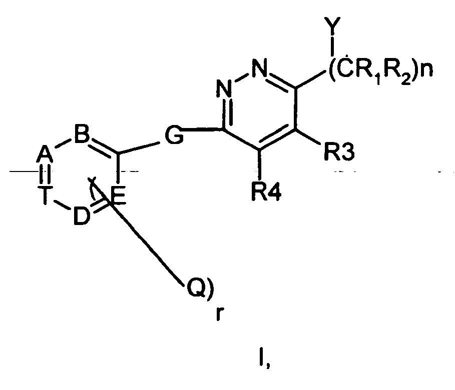 in which
- r: has the meaning of 0 to 2,
- n: has the meaning of 0 to 2;
- R₃ und R₄: a) each independently from each other have the meaning of lower alkyl,
b) together form a bridge of general partial formula II, 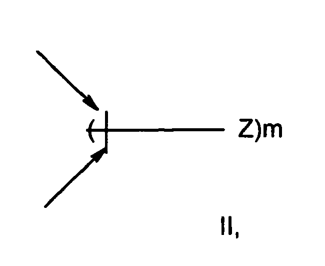 wherein the binding is via the two terminal C- atoms, and
- m: has the meaning of 0 to 4; or
c) together form a bridge of partial formula III 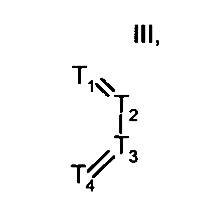 wherein one or two of the ring members T₁,T₂,T₃,T₄ has the meaning of nitrogen, and each others have the meaning of CH, and the binding is via the atoms T₁ and T₄ ;
- G: has the meaning of C₁ -C₆ - alkyl, C₂ - C₆ - alkylene or C₂ - C₆ - alkenylene; or C₂ C₆- alkylene or C₃ -C₆- alkenylene, which are substituted with acyloxy or hydroxy, -CH₂-O-, -CH₂-S-, -CH₂-NH-, -CH₂-O-CH₂-, -CH₂-S-CH₂-, -CH₂-NH-CH₂, oxa (-O-), thia (-S-) or imino (-NH-),
- A, B, D, E and T: independently from each other have the meaning of N or CH , with the provisio that not more than three of these Substituents have the meaning of N,
- Q: has the meaning of lower alkyl, lower alkyloxy or halogene,
- R₁ and R₂: independently from each other have the meaning of H or lower alkyl,
- X: has the meaning of imino, oxa or thia;
- Y: has the meaning of hydrogene, unsubstituted or substituted aryl, heteroaryl, or unsubstituted or substituted cycloalkyl; and
- Z: has the meaning of amino, mono- or disubstituted amino, halogen, alkyl, substituted alkyl, hydroxy, etherificated or esterificated hydroxy, nitro, cyano, carboxy, esterificated carboxy, alkanoyl, carbamoyl, N-mono- or N, N- disubstituted carbamoyl, amidino, guanidino, mercapto, sulfo, phenylthio, phenyl-lower-alkyl-thio, alkyl-phenyl-thio, phenylsulfinyl, phenyl-lower-alkyl-sulfinyl, alkylphenylsulfinyl, phenylsulfonyl, phenyl-lower-alkan-sulfonyl, or alkylphenylsulfonyl, whereas, if more than one rest Z is present (m≥2), the substituents Z are equal or different from each other, and wherein the bonds marked with an arrow are single or double bonds; or an N-oxide of said compound, wherein one ore more N-atoms carry an oxygene atom, or a salt thereof.

A preferred salt is the salt of an organic acid, especially a succinate.

These compounds can preferentially be used as compound I or II in the inventive pharmaceutical composition.

Compounds which stop a tyrosin phosphorylation, or the persistent angiogenese, respectively, which results in a prevention of tumor growth and tumor spread, are for example anthranyl acid derivatives of general formula IV 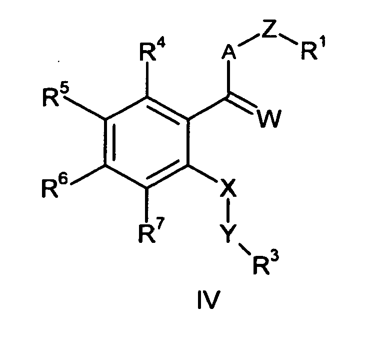 in which
- A: has the meaning of group =NR² ,
- W: has the meaning of oxygen, sulfur, two hydrogen atoms or the group =NR⁸,
- Z: has the meaning of the group =NR¹⁰ or =N-, -N(R¹⁰)-(CH₂)_{q}-, branched or unbranched C₁₋₆-Alkyl or is the group 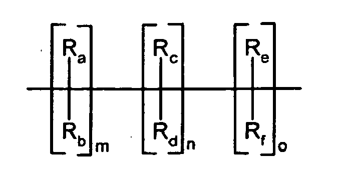 or A, Z and R¹ together form the group 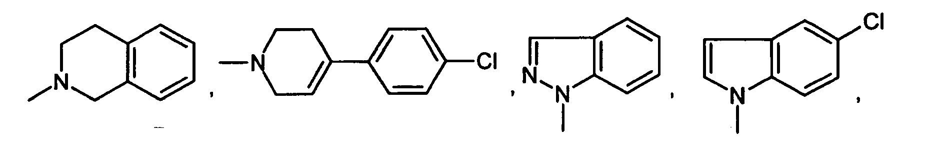 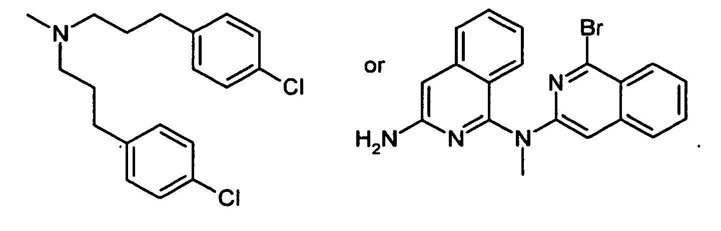
- m, n and o: has the meaning of 0 - 3,
- q: has the meaning of 1 - 6,
- Rₐ, R_{b}, R_{c}, R_{d}, Rₑ, R_{f}: independently from each other have the meaning of hydrogen, C₁₋₄ alkyl or the group =NR¹⁰, and/ or Rₐ and/ or R_{b} together with R_{c} and or R_{d} or R_{c} together with Rₑ and/ or R_{f} form a bound, or up to two of the groups Rₐ-R_{f} form a bridge with each up to 3 C-atoms with R¹ or R²,
- X: has the meaning of group =NR⁹ or =N-,
- Y: has the meaning of group -(CH₂)ₚ,
- p: has the meaning of integer 1-4,
- R¹: has the meaning of unsubstituted or optionally substituted with one or more of halogene, C₁₋₆-alkyl, or C₁₋₆-alkyl or C₁₋₆-alkoxy, which is optionally substituted by one or more of halogen, or is unsubstituted or substituted aryl or heteroaryl,
- R²: has the meaning of hydrogen or C₁-₆-alkyl, or form a bridge with up to 3 ring atoms with Rₐ-R_{f} together with Z or R₁,
- R³: has the meaning of monocyclic or bicyclic aryl or heteroaryl which is unsubstituted or optionally substituted with one or more of für halogen, C₁₋₆-alkyl, C₁₋₆-alkoxy or hydroxy,
- R⁴ ,R⁵, R⁶ and R⁷: independently from each other have the meaning of hydrogen, halogen or C₁₋₆-alkoxy, C₁₋₆-alkyl or C₁₋₆-carboxyalkyl, which are unsubstituted or optionally substituted with one or more of halogen, or R⁵ and R⁶ together form the group 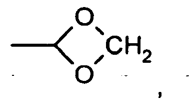
- R⁸, R⁹ and R¹⁰: independently from each other have the meaning of hydrogen or C₁₋₆-alkyl, as well as their isomers and salts.

These compounds can also preferentially be used as compound I or II in the inventive pharmaceutical composition.

More preferentially compounds of general formula V 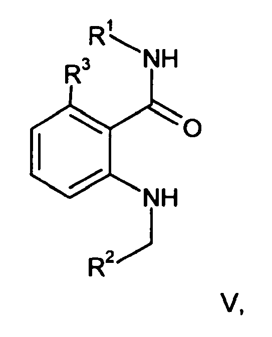 in which
R¹ has the meaning of group 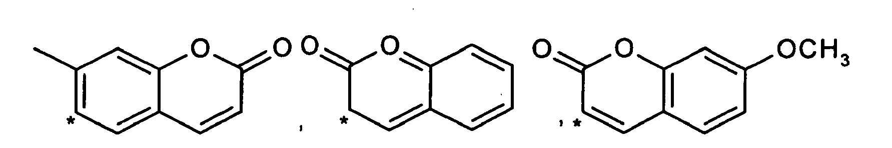 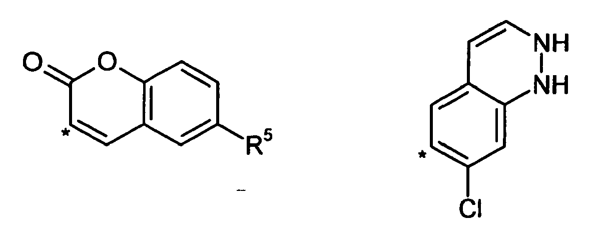 in which R⁵ is chloro, bromo or the group -OCH₃, 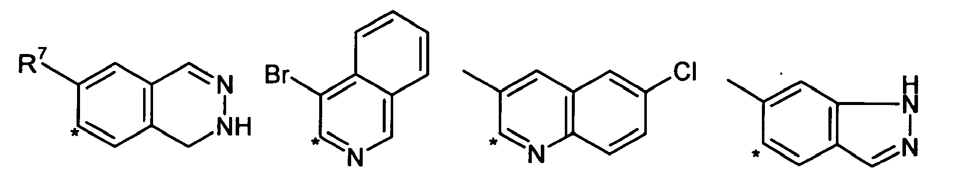 in which R⁷ is -CH₃ or chloro,

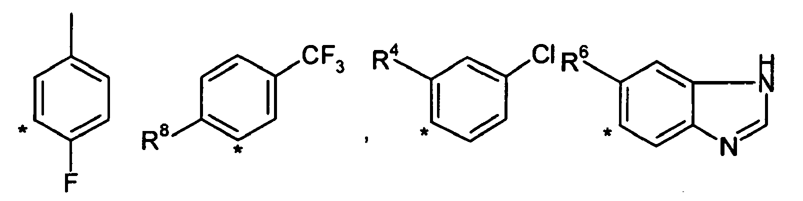

| | | |
|---|---|---|
| in which R⁸ is -CH₃, fluoro, chloro or -CF₃ | in which R⁴ is fluoro, chloro, bromo, -CF₃, -N=C, -CH₃,-OCF₃ or -CH₂OH | in which R⁶ is -CH₃ or chloro |

R² has the meaning of pyridyl or the group 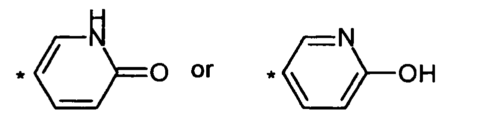 and
R³ has the meaning of hydrogen or fluoro, as well as their isomers and salts can be used as compound I or II in the inventive pharmaceutical composition.

These compounds have the same properties as already mentioned above under compound IV and can be used for the treatment of angiogeneous diseases. Compositions comprise compounds of general formulars I, IV and V, alone or in combination.

The above mentioned compounds are also claimed matter within the inventive combinations.

A further example for ligand binding inhibitors are peptides and DNA sequences coding for such peptides, which are used for the treatment of angiogeneous diseases. Such peptides and DNA sequences are disclosed in Seq. ID No. 1 to 59 of the sequence protocoll. It has been shown that Seq. ID Nos. 34 and 34a are of main interest.

For a sequential therapeutical application the inventive pharmaceutical compositions can be applied simultaneously or separately.

Also claimed matter of the present invention are pharmaceutical compositions which comprise as compound I and/ or II at least one of Seq. ID Nos. 1-59. Of most value are pharmaceutical compositions, which comprise as compound I and/ or II Seq. ID Nos. 34a und pharmaceutical compositions according to claims which comprise as compound I and/ or II at least one of sTie2, mAB 4301-42-35, scFv-tTF and/ or L19 scFv-tTF conjugate.

Further preferred matter of the present invention are pharmaceutical compositions, which comprise as compound I and/ or II at least one small molecule of general formula I, general formula IV and/ or general formula V.

The most preferred compound which can be used as compound I or II in the inventive composition is (4-Chlorophenyl)[4-(4-pyridylmethyl)-phthalazin-1-yl]ammonium hydrogen succinate.

Therefore, claimed matter of the present invention are also pharmaceutical compositions, which comprise as compound I (4-Chlorophenyl)[4-(4-pyridylmethyl)-phthalazin-1-yl]ammonium hydrogen succinate, sTie2, mAB 4301-42-35, scFv-tTF and/ or L19 scFv-tTF conjugate, and as compound II (4-Chlorophenyl)[4-(4-pyridylmethyl)-phthalazin-1-yl]ammonium hydrogen succinate, sTie2, mAB 4301-42-35, scFv-tTF and/ or L19 scFv-tTF conjugate, with the provisio that compound I is not identically to compound II, and most preferred pharmaceutical compositions, which comprise as compound I (4-Chlorophenyl)[4-(4-pyridylmethyl)-phthalazin-1-yl]ammonium hydrogen succinate and as compound II sTie2, mAB 4301-42-35, scFv-tTF and/ or L19 scFv-tTF conjugate; pharmaceutical compositions, which comprise as compound I mAB 4301-42-35 and as compound II sTie2, and/ or scFv-tTF conjugate; pharmaceutical compositions, which comprise as compound I scFv-tTF conjugate and as compound II sTie2 and/ or mAB 4301-42-35; pharmaceutical compositions, which comprise as compound I L19 scFv-tTF conjugate and as compound II sTie2.

The small molecule compounds, proteins and DNA's expressing proteins, as mentioned above can be used as medicament alone, or in form of formulations for the treatment of tumors, cancers, psoriasis, arthritis, such as rheumatoide arthritis, hemangioma, angiofribroma, eye diseases, such as diabetic retinopathy, neovascular glaukoma, kidney diseases, such as glomerulonephritis, diabetic nephropathy, maligneous nephrosclerose, thrombic microangiopatic syndrome, transplantation rejections and glomerulopathy, fibrotic diseases, such as cirrhotic liver, mesangial cell proliferative diseases, artheriosclerosis and damage of nerve tissues.

The treatment of the damaged nerve tissues with the inventive combination hinders the rapid formation of scars at the damaged position. Thus, there is no scar formation before the axons communicate with each other. Therefore a reconstruction of the nerve bindings is much more easier.

Further, the inventive combinations can be used for suppression of the ascites formation in patients. It is also possible to suppress VEGF oedemas. For the use of the inventive combinations as medicament the compounds will be formulated as pharmaceutical composition. Said formulation comprises beside the active compound or compounds acceptable pharmaceutically, organically or inorganically inert carriers, such as water, gelatine, gum arabic, lactose, starch, magnesium stearate, talcum, plant oils, polyalkylene glycols, etc. Said pharmaceutical preparations can be applied in solid form, such as tablets, pills, suppositories, capsules, or can be applied in fluid form, such as solutions, suspensions or emulsions.

If necessary, the compositions additionally contain additives, such as preservatives, stabilizer, detergents or emulgators, salts for alteration of the osmotic pressure and/ or buffer.

These uses are also claimed matter of the instant invention, as well as the formulations of the active compounds

For parenteral application especially injectable solutions or suspensions are suitable, especially hydrous solutions of the active compound in polyhydroxyethoxylated castor-oil are suitable.

As carrier also additives can be used, such as salts of the gallic acid or animal or plant phospholipids, as well as mixtures thereof, and liposomes or ingredients thereof.

For oral application especially suitable are tablets, pills or capsules with talcum and/ or hydrocarbon carriers or binders, such as lactose, maize or potato starch. The oral application can also be in form of a liquid, such as juice, which optionally contains a sweetener.

The dosis of the active compound differs depending on the application of the compound, age and weight of the patient, as well as the form and the progress of the disease.

The daily dosage of the active compound is 0,5-1000 mg, especially 50-200 mg. The dosis can be applied as single dose or as two or more daily dosis.

These formulations and application forms are also part of the instant invention.

Combined functional interference with VEGFNEGF receptor systems and with Angiopoietin/Tie receptor systems can be performed simultaneously, or in sequential order such that the biological response to interference with one ligand/receptor system overlaps with the biological response to interference with a second ligand/receptor system. Alternatively, combined functional interference with VEGFNEGF receptor systems or with Angiopoietin/Tie receptor systems and targeting of cytotoxic agents via VEGFNEGF receptor systems or via Angiopoietin/Tie receptor systems can be performed simultaneously, or in sequential order such that the biological response to functional interference with a ligand/receptor system overlaps in time with targeting of cytotoxic agents.

The invention is also directed to a substance which functional interferes with both VEGFNEGF receptor systems and Angiopoietin/Tie receptor systems, or which are targeted via both VEGFNEGF receptor systems and Angiopoietin/Tie receptor systems.

VEGFNEGF receptor systems include the ligands VEGF-A, VEGF-B, VEGF-C, VEGF-D, PIGF, and the receptor tyrosine kinases VEGF-R1 (Flt1), VEGF-R2 (KDR/FIk1), VEGF-R3 (Flt4), and their co-receptors (i.e. neuropilin-1). Angiopoietin/Tie receptor systems include Ang1, Ang2, Ang3/Ang4, and angiopoietin related polypeptides which bind to Tie1 or to Tie2, and the receptor tyrosine kinases Tie1 and Tie2.

Phamaceutical compositions of the present invention can be used for medicinal purposes. Such diseases are, for example, cancer, cancer metastasis, angiogenesis including retinopathy and psoriasis. Pharmaceutical compositions of the present invention can be applied orally, parenterally, or via gene therapeutic methods.

Therefore the present invention also concerns the use of pharmaceutical compositions for the production of a medicament for the treatment of tumors, cancers, psoriasis, arthritis, such as rheumatoide arthritis, hemangioma, angiofribroma, eye diseases, such as diabetic retinopathy, neovascular glaukoma, kidney diseases, such as glomerulonephritis, diabetic nephropathie, maligneous nephrosclerosis, thrombic microangiopatic syndrome, transplantation rejections and glomerulopathy, fibrotic diseases, such as cirrhotic liver, mesangial cell proliferative diseases, artheriosclerosis, damage of nerve tissues, suppression of the ascites formation in patients and suppression of VEGF oedemas.

The following examples demonstrate the feasability of the disclosed invention, without restricting the inventon to the disclosed examples.

### Example 1

Superior effect on inhibition of tumor growth via combination of inhibition of the VEGF A/VEGF receptor system together with functional interference with the Angiopoietin/Tie2 receptor system over separate modes of intervention was demonstrated in an A375v human melanoma xenograft model.

Human melanoma cell line A375v was stably transfected to overexpress the extracellular ligand-neutralizing domain of human Tie2 receptor tyrosine kinase (sTie2; compound II) (Siemeister et al., Cancer Res. 59, 3185-3191, 1999). For control, A375v cells were stably transfected with the empty expression vector (A375v/pCEP). Swiss *nulnu* mice were s.c. injected with 1x10⁶ transfected A375v/sTie2 or A375v/pCEP tumor cells, respectively. Animals receiving compound I were treated for up to 38 days with daily oral doses of 50 mg/kg of the VEGF receptor tyrosine kinase inhibitor (4-Chlorophenyl)[4-(4-pyridylmethyl)-phthalazin-1-yl]ammonium hydrogen succinate (Wood et al., Cancer Res. 60, 2178-2189, 2000). Various modes of treatment are described in Table 1. Tumor growth was determined by caliper measurement of the largest diameter and its perpendicular.

**Table 1**

| | mode of treatment | |
|---|---|---|
| treatment group | (4-Chlorophenyl)[4-(4-pyridylmethyl)-phthalazin-1-yl]ammonium hydrogen succinate (compound I) | sTie2 (compound II) |
| Group 1: | - | - |
| A375v/pCEP | | |
| Group 2: | + | - |
| A375v/pCEP | | |
| Group 3: | - | + |
| A375v/sTie2 | | |
| Group 4: | + | + |
| A375v/sTie2 | | |

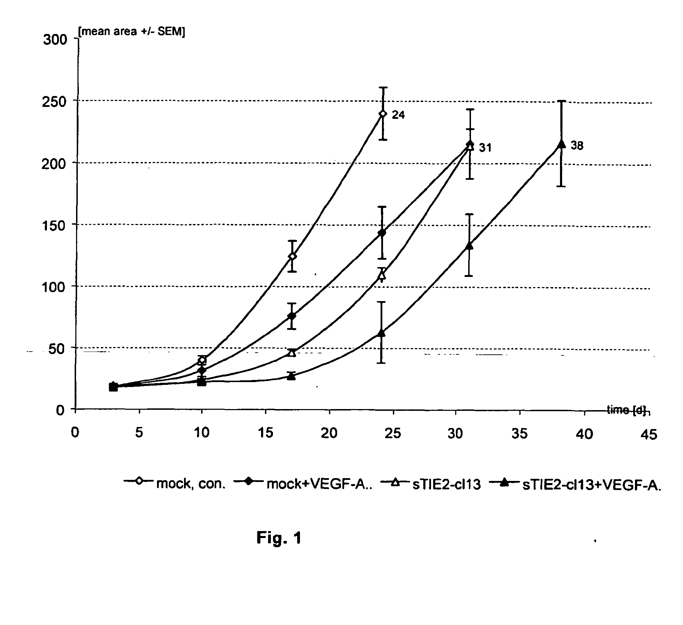

Tumors-derived from A375v/pCEP control cells reached a size of approx. 250 mm² (mean area) within 24 days (Figure 1) without treatment (group 1). Separate treatment with the VEGF receptor inhibitor (4-Chlorophenyl)[4-(4-pyridylmethyl)-phthalazin-1-yl]ammonium hydrogen succinate (compound I, treatment group 2) or separate interference with Angiopoietin/Tie2 receptor system by means of expression of sTie2 (compound II, treatment group 3) delayed growth of tumors to a size of approx. 250 mm² to 31 days, respectively. Combination of interference with the Angiopoietin/Tie2 system by means of expression of sTie2 and of interference with the VEGFNEGF receptor system by means of the kinase inhibitor (4-Chlorophenyl)[4-(4-pyridylmethyl)-phthalazin-1-yl]ammonium hydrogen succinate (compound I + compound II, treatment group 4) delayed growth of the tumors to a size of approx. 250 mm² to 38 days.

This result clearly demonstrates the superior effect of a combination of interference with the VEGF-A/VEGF receptor system and the Angiopoietin/Tie2 receptor system over separate modes of intervention.

### Example 2

Combination of functional interference with the Angiopoietin/Tie2 receptor system and neutralization of VEGF-A is superior to separate modes of intervention in inhibition of tumor growth.

Tumors derived from A375v/sTie2 cells and from A375v/pCEP cells were induced in nude mice as described in example 1. Animals receiving compound I were treated twice weekly over a period of time of 4 weeks with intraperitoneal doses of 200 µg of the VEGF-A-neutralizing monoclonal antibody (mAb) 4301-42-35 (Schlaeppi et al., J. Cancer Res. Clin. Oncol. 125, 336-342, 1999). Various modes of treatment are descibed in Table 2. Animals were sacrificed for ethical reasons when tumors of group 1 exceeded a volume of approx. 1000 mm³. Tumor growth was determined by caliper measurement of the largest diameter and its perpendicular.

**Table 2**

| | mode of treatment | |
|---|---|---|
| treatment group | mAb 4301-42-35 (compound I) | sTie2 (compound II) |
| Group 1: | - | - |
| A375v/pCEP | | |
| Group 2: | + | - |
| A375v/pCEP | | |
| Group 3: | - | + |
| A375v/sTie2 | | |
| Group 4: | + | + |
| A375v/sTie2 | | |

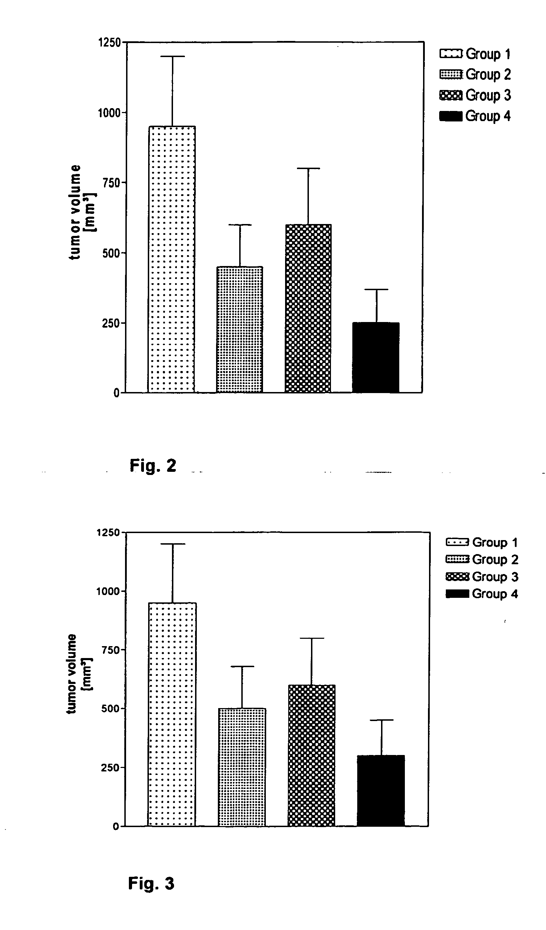

Tumors derived from A375v/pCEP control cells reached a size of approx. 1000 mm³ within 28 days (Figure 2) without treatment (group 1). Tumors treated with the VEGF-A-neutralizing mAb 4301-42-35 (compound 1, treatment group 2) grew to a volume of approx. 450 mm³ within 28 days. Interference with Angiopoietin/Tie2 receptor system by means of expression of sTie2 (compound II, treatment group 3) reduced growth of tumors within 28 day to a volume of approx. 600 mm², respectively. Combination of interference with the Angiopoietin/Tie2 system by means of expression of sTie2 and neutralizing of VEGF-A by means of the mAb 4301-42-35 (compound I + compound II, treatment group 4) resulted in a inhibition of tumor growth to a volume of approx. 250 mm³ within 28 days.

The superior effect of a combination of neutralization of VEGF-A and functional interference with the Angiopoietin/Tie2 receptor system over separate modes of intervention is clearly shown.

### Example 3

Combination of functional interference with the Angiopoietin/Tie2 receptor system and targeting of a coagulation-inducing protein via the VEGF/VEGF receptor system is superior to separate modes of intervention in inhibition of tumor growth.

Tumors derived from A375v/sTie2 cells and from A375v/pCEP cells were induced in nude mice as described in example 1. A single chain antibody (scFv) specifically recognizing the human VEGF-A/VEGF receptor I complex (WO 99/19361) was expressed in E. coli and conjugated to coagulation-inducing recombinant human truncated tissue factor (tTF) by methods descibed by Ran et al. (Cancer Res. 58, 4646-4653, 1998). When tumors reached a size of approx. 200 mm³ animals receiving compound I were treated on day 0 and on day 4 with intravenous doses of 20 µg of the scFv-tTF conjugate. Various modes of treatment are described in Table 3. Animals were sacrificed for ethical reasons when tumors of group 1 exceeded a volume of approx. 1000 mm³. Tumor growth was determined_by caliper measurement of the largest diameter and its perpendicular.

**Table 3**

| | mode of treatment | |
|---|---|---|
| treatment group | scFv-tTF conjugate (compound I) | sTie2 (compound II) |
| Group 1: | - | - |
| A375v/pCEP | | |
| Group 2: | + | - |
| A375v/pCEP | | |
| Group 3: | - | + |
| A375v/sTie2 | | |
| Group 4: | + | + |
| A375v/sTie2 | | |

Tumors derived from A375v/pCEP control cells reached a size of approx. 1000 mm³ within 28 days (Figure 3) without treatment (group 1). Tumors treated with the coagulation-inducting tTF targeted to the VEGF-A/VEGF receptor I complex via the scFv-tTF conjugate (compound I, treatment group 2) grew to a volume of approx. 500 mm³ within 28 days. Interference with Angiopoietin/Tie2 receptor system by means of expression of sTie2 (compound II, treatment group 3) reduced growth of tumors within 28 day to a volume of approx. 600 mm², respectively. Combination of interference with the Angiopoietin/Tie2 system by means of expression of sTie2 and of targeting the VEGF receptor complex (compound I + compound II, treatment group 4) resulted in a inhibition of tumor growth to a volume of approx. 300 mm³ within 28 days.

The superior effect of a combination of targeting of the coagulation-inducting tTF to the VEGF-A/VEGF receptor I complex and functional interference with the Angiopoietin/Tie2 receptor system over separate modes of intervention is clearly shown. Similar effects can be expected upon targeting of cytotoxic agents to VEGF/VEGF receptor systems.

### Example 4

Combination of functional interference with the VEGF/VEGF receptor system and targeting of a coagulation-inducing protein via the VEGF/VEGF receptor system is superior to separate modes of intervention in inhibition of tumor growth.

Tumors derived from A375v/pCEP cells were induced in nude mice as described in example 1. Animals receiving compound I were treated for up to 28 days with daily oral doses of 50 mg/kg of the VEGF receptor tyrosine kinase inhibitor (4-Chlorophenyl)[4-(4-pyridylmethyl)-phthalazin-1-yl]ammonium hydrogen succinate (Wood et al., Cancer Res. 60, 2178-2189, 2000). Compound II consists of a single chain antibody (scFv) specifically recognizing the human VEGF-A/VEGF receptor I complex (WO 99/19361) which was expressed in E. coli and conjugated to coagulation-inducing recombinant human truncated tissue factor (tTF) by methods descibed by Ran et al. (Cancer Res. 58, 4646-4653, 1998). When tumors reached a size of approx. 200 mm³ animals receiving compound II were treated on day 0 and on day 4 with intravenous doses of 20 µg of the scFv-tTF conjugate. Various modes of treatment are described in Table 4. Animals were sacrificed for ethical reasons when tumors of group 1 exceeded a volume of approx. 1000 mm³. Tumor growth was determined by caliper measurement of the largest diameter and its perpendicular.

**Table 4**

| | mode of treatment | |
|---|---|---|
| treatment group | (4-Chlorophenyl)[4-(4-pyridylmethyl)-phthal-azin-1-yl]ammonium hydrogen succinate (compound I) | scFv-tTF conjugate (compound II) |
| Group 1: | - | - |
| A375v/pCEP | | |
| Group 2: | + | - |
| A375v/pCEP | | |
| Group 3: | - | + |
| A375v/pCEP | | |
| Group 4: | + | + |
| A375v/pCEP | | |

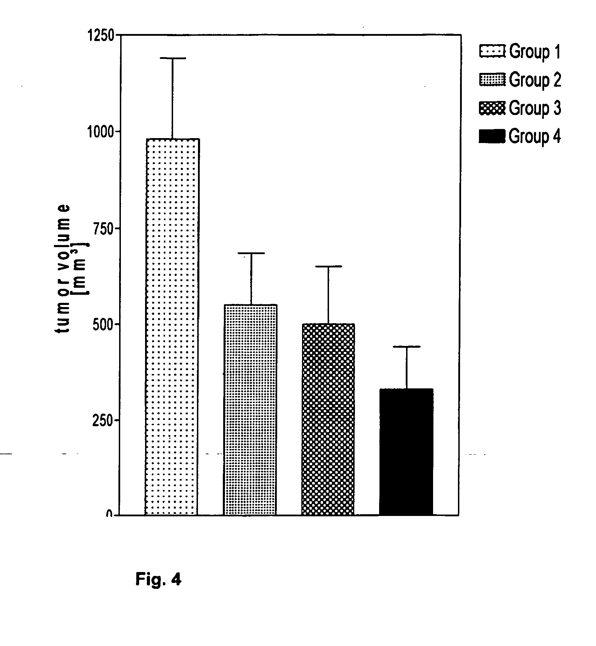

Tumors derived from A375v/pCEP control cells reached a size of approx. 1000 mm³ within 28 days (Figure 4) without treatment (group 1). Separate treatment with the VEGF receptor inhibitor (4-Chlorophenyl)[4-(4-pyridylmethyl)-phthalazin-1-yl]ammonium hydrogen succinate (compound I, treatment group 2) resulted in a reduction of the tumor volumes to approx. 550 mm³. Tumors treated with the coagulation-inducting tTF targeted to the VEGF-A/VEGF receptor I complex via the scFv-tTF conjugate (compound II, treatment group 3) grew to a volume of approx. 500 mm³ within 28 days. Combination of inhibition of VEGF receptor tyrosine kinase by means of (4-Chlorophenyl)[4-(4-pyridylmethyl)-phthalazin-1-yl]ammonium hydrogen succinate and of targeting the VEGF receptor complex (compound I + compound II, treatment group 4) resulted in a inhibition of tumor growth to a volume of approx. 400 mm³ within 28 days.

The superior effect of a combination of targeting of the coagulation-inducting tTF to the VEGF-A/VEGF receptor I complex and functional interference with the VEGFNEGF receptor system over separate modes of intervention is clearly shown. Similar effects can be expected upon targeting of cytotoxic agents to Angiopoietin/Tie receptor systems.

### Example 5

Combination of functional interference with the Angiopoietin/Tie2 receptor system and endothelium-specific targeting of a coagulation-inducing protein is superior to separate modes of intervention in inhibition of tumor growth.

Tumors derived from A375v/sTie2 cells and from A375v/pCEP cells were induced in nude mice as described in example 1. A fusion protein (L19 scFv-tTF) consisting of L19 single chain antibody specifically recognizing the oncofoetal ED-B domain of fibronectin and the extracellular domain of tissue factor was expressed in E. coli as described by Nilsson et al. (Nat. Med., in press). Further, L19 scFv-tTF data have been represented by D. Neri and F. Nilsson (Meeting "Advances in the application of monoclonal antibodies in clinical oncology", Samos, Greece, 31. May-2. June 2000). When tumors reached a size of approx. 200 mm³ animals receiving compound I were treated with a single intravenous dose of 20 µg of L19 scFv-tTF in 200 µl saline. Various modes of treatment are described in Table 5. Animals were sacrificed for ethical reasons when tumors of group 1 exceeded a volume of approx. 1000 mm³. Tumor growth was determined by caliper measurement of the largest diameter and its perpendicular.

**Table 5**

| | mode of treatment | |
|---|---|---|
| treatment group | L19 scFv-tTF (compound I) | sTie2 (compound II) |
| Group 1: | - | |
| A375v/pCEP | | |
| Group 2: | + | - |
| A375v/pCEP | | |
| Group 3: | - | + |
| A375v/sTie2 | | |
| Group 4: | + | + |
| A375v/sTie2 | | |

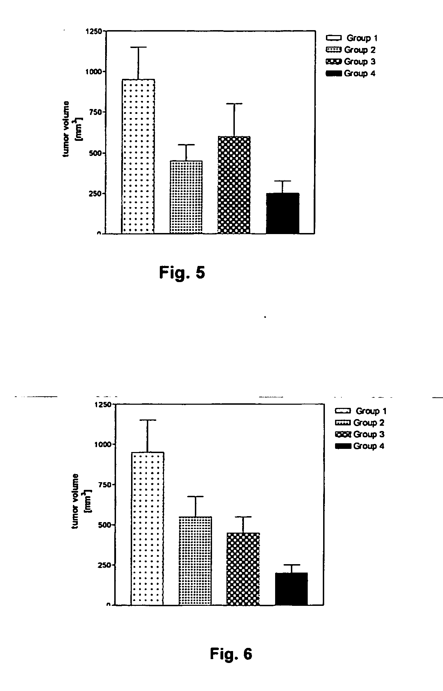

Tumors derived from A375v/pCEP control cells reached a size of approx. 1000 mm³ within 28 days (Figure 5) without treatment (group 1). Tumors treated with the coagulation-inducting L19 scFv-tTF (compound I, treatment group 2) grew to a volume of approx. 450 mm³ within 28 days. Interference with Angiopoietin/Tie2 receptor system by means of expression of sTie2 (compound II, treatment group 3) reduced growth of tumors within 28 day to a volume of approx. 600 mm², respectively. Combination of interference with the Angiopoietin/Tie2 system by means of expression of sTie2 and of targeting the endothelium with L19 scFv-tTF (compound I + compound II, treatment group 4) resulted in a inhibition of tumor growth to a volume of approx. 250 mm³ within 28 days.

The superior effect of a combination of targeting of L19 scFv-tTF to the endothelium and functional interference with the Angiopoietin/Tie2 receptor system over separate modes of intervention is clearly shown.

### Example 6

Combination of functional interference with the VEGFNEGF receptor system and endothelium-specific targeting of a coagulation-inducing protein is superior to separate modes of intervention in inhibition of tumor growth.

Tumors derived from A375v/pCEP cells were induced in nude mice as described in example 1. Animals receiving compound I were treated for up to 28 days with daily oral doses of 50 mg/kg of the VEGF receptor tyrosine kinase inhibitor (4-Chlorophenyl)[4-(4-pyridylmethyl)-phthalazin-1-yl]ammonium hydrogen succinate (Wood et al., Cancer Res. 60, 2178-2189, 2000). Compound 11 consists of L19 scFv-tTF fusion protein as described in example 5. When tumors reached a size of approx. 200 mm³ animals receiving compound II were treated with a single intravenous dose of 20 µg of L19 scFv-tTF in 200 µl saline. Various modes of treatment are described in Table 6. Animals were sacrificed for ethical reasons when tumors of group 1 exceeded a volume of approx. 1000 mm³. Tumor growth was determined by caliper measurement of the largest diameter and its perpendicular.

**Table 6**

| | mode of treatment | |
|---|---|---|
| treatment group | (4-Chlorophenyl)[4-(4-pyridylmethyl)-phthal-azin-1-yl]ammonium hydrogen succinate (compound I) | L19 scFv-tTF (compound II) |
| Group 1: | - | - |
| A375v/pCEP | | |
| Group 2: | + | - |
| A375v/pCEP | | |
| Group 3: | - | + |
| A375v/pCEP | | |
| Group 4: | + | + |
| A375v/pCEP | | |

Tumors derived from A375v/pCEP control cells reached a size of approx. 1000 mm³ within 28 days (Figure 6) without treatment (group 1). Separate treatment with the VEGF receptor inhibitor (4-Chlorophenyl)[4-(4-pyridylmethyl)-phthalazin-1-yl]ammonium hydrogen succinate (compound I, treatment group 2) resulted in a reduction of the tumor volumes to approx. 550 mm³. Tumors treated with the coagulation-inducting L19 scFv-tTF targeted to the endothelium (compound II, treatment group 3) grew to a volume of approx. 450 mm³ within 28 days. Combination of inhibition of VEGF receptor tyrosine kinase by means of (4-Chlorophenyl)[4-(4-pyridylmethyl)-phthalazin-1-yl]ammonium hydrogen succinate and of targeting the VEGF receptor complex (compound I + compound II, treatment group 4) resulted in a inhibition of tumor growth to a volume of approx. 200 mm³ within 28 days.

The superior effect of a combination of targeting of L19 scFv-tTF to the endothelium and functional interference with the VEGFNEGF receptor system over separate modes of intervention is clearly shown.

### Description of the figures

Fig. 1 shows the superior effect of combination of interference with VEGF/VEGF receptor system by means of an specific tyrosine kinase inhibitor and with the Angiopoietin/Tie2 receptor system by means of a soluble receptor domain on inhibition of tumor growth (treatment modes of groups 1-4 are given in Table 1). The abbreviations have the following meaning:

| | | |
|---|---|---|
| mock, con. | = | treatment group 1 |
| mock+VEGF-A | = | treatment group 2 |
| sTIE2-cI13 | = | treatment group 3 |
| sTIE2-cI13+VEGF-A | = | treatment group 4 |

Fig. 2 shows the superior effect on tumor growth inhibition of combination of VEGF-neutralization and functional interference with Angiopoietin/Tie2 receptor system over separate modes of intervention (treatment modes of groups 1-4 are given in Table 2).
Fig. 3 shows the superior effect on tumor growth inhibition of combination of targeting of the coagulation-inducing tTF to the VEGF/VEGF receptor I complex via a scFv-tTF conjugate and functional interference with Angiopoietin/Tie2 receptor system over separate modes of intervention (treatment modes of groups 1-4 are given in Table 3).
Fig. 4 shows the superior effect on tumor growth inhibition of combination of targeting of the coagulation-inducing tTF to the VEGF/VEGF receptor I complex via a scFv-tTF conjugate and functional interference with VEGF/VEGF receptor system by means of the VEGF receptor tyrosine kinase inhibitor (4-Chlorophenyl)[4-(4-pyridylmethyl)-phthalazin-1-yl]ammonium hydrogen succinate over separate modes of intervention (treatment modes of groups 1-4 are given in Table 4).
Fig. 5 shows the superior effect on tumor growth inhibition of combination of targeting of the coagulation-inducing L19 scFv-tTF fusion protein to the endothelium and functional interference with Angiopoietin/Tie2 receptor system over separate modes of intervention (treatment modes of groups 1-4 are given in Table 5).
Fig. 6 shows the superior effect on tumor growth inhibition of combination of targeting of the coagutation-inducing L19 scFv-tTF fusion protein to the endothelium and functional interference with VEGFNEGF receptor system by means of the VEGF receptor tyrosine kinase inhibitor (4-Chlorophenyl)[4-(4-pyridylmethyl)-phthalazin-1-yl]ammonium hydrogen succinate over separate modes of intervention (treatment modes of groups 1-4 are given in Table 6).

### Sequence Identifier

<110> Schering Aktiengesellschaft
<120> Combinations and compositions which interfere with VEGF/ VEGF and angiopoietin/ Tie receptor function and their use II
<130> 51867AEPM1XX00-P
<140>
   <141>
<160> 59
<210> 1
   <211> 1835
   <212> DNA
   <213> Human
<400> 1
   ttttacagtt ttccttttct tcagagttta ttttgaattt tcatttttgg ataaccaagc 60
   agctctttaa gaagaatgca cagaagagtc attctggcac ttttggatag tacataagat 120
   tttctttttt ttttttaaat tttttttaat agtcacattc agctcgcttg ctcaaaccag 180
   actcccacat tgggtgagca agatgagccc ataggattcc agagttaata cgtaaccgta 240
   tatacaaaca gccaaaaaac cataatggtg ccacagggat ggagcaggga agggcatctc 300
   taacgtgtcc tctagtctat cttcgctaaa cagaacccac gttacacatg ataactagag 360
   agcacactgt gttgaaacga ggatgctgac cccaaatggc acttggcagc atgcagttta 420
   aagcaaaaga gacatccttt aataactgta taaaatccag gcagttccat taaaggggtt 480
   aagaaaacca acaacaacaa aaagcgaggg actgtctgtt gtcactgtca aaaaggcact 540
   tggagttaat gggaccagga ttggaggact cttagctgat acagatttca gtacgatttc 600
   attaaaaggc ttggatgtta agagaggaca ctcagcggtt cctgaaggga gacgctgaga 660
   tggaccgctg agaagcggaa cagatgaaca caaaggaatc aaatctttac aaccaaattg 720
   catttaagcg acaacaaaaa aaggcaaacc ccaaaacgca acctaaccaa agcaaaatct 780
   aagcaaaatc agacaacgaa gcagcgatgc atagctttcc tttgagagaa cgcatacctt 840
   gagacgctac gtgccaacct aagttctcaa cgacagcttc acagtaggat tattgtgata 900
   aaaatgactc aagcgatgca aaaagtttca tctgttccca gaatccgagg gagaactgag 960
   gtgatcgtta gagcatagcg acatcacgtg cggtttctta atgtccctgg tggcggatac 1020
   gccgagtcct cggaaggaca tctggacacc actttcagcc acctccttgc aggggcgaca 1080
   tccgccaaag tcatccttta ttccgagtaa taactttaat tcctttctaa catttacacg 1140
   gcaaacagga atgcagtaaa cgtccacgtc cgtcccacgg ctgggctgcc gttccgtttc 1200
   ctccacgaac gggtacgcgc ttccatgaga aaggatattt ggcaatttta tattccacag 1260
   tcaggtgggt ctgcgatagc tcatttaatg ttaaacgcca tcaggggcct ctcctcccgt 1320
   ttctgccagg ggcttttctt gtcttctcct tggcgagctc gtgggcagat cttctctggt 1380
   gggggctggc tgctggctcc gagggggcat ccgcagtccg tctggtcgtc tcctcctgca 1440
   ggctgggcag ctggccacca cttctccgac tcgacccctc caacaagcat cgcagggcac 1500
   tgtcctcggg ggtacagacc gtggtcccac attcgctacc actctgttcc acgtcatcca 1560
   ggtacacgag ctgcgtgtag gccgtgctgt ctggggctcg aggctctttc tgctggtgct 1620
   cttggacggg cgggtagttc tgctgcagag acaaagcatc tccccttccc ttccgggctg 1680
   attttggttc attcatatct acgccagagt ccaaactggc atcattactt ccgttccttc 1740
   cagctctttg gagaatcaat gtatgaatgt ctaacctgac cgttggacct gccatccaag 1800
   gagacgaacc acgcccgggg gtgcggaagc ggcct
<210> 2
   <211> 581
   <212> DNA
   <213> Human
<400> 2
   gttctagatt gttttattca gtaattagct cttaagaccc ctggggcctg tgctacccag 60
   acactaacaa cagtctctat ccagttgctg gttctgggtg acgtgatctc cccatcatga 120
   tcaacttact tcctgtggcc cattagggaa gtggtgacct cgggagctat ttgcctgttg 180
   agtgcacaca cctggaaaca tactgctctc attttttcat ccacatcagt gagaaatgag 240
   tggcccgtta gcaagatata actatgcaat catgcaacaa agctgcctaa taacatttca 300
   tttattacag gactaaaagt tcattattgt ttgtaaagga tgaattcata acctctgcag 360
   agttatagtt catacacagt tgatttccat ttataaaggc agaaagtcct tgttttctct 420
   aaatgtcaag ctttgactga aaactcccgt tttccagtca actggagtgt gtgcgtatga 480
   aagaaaatct ttagcaatta gatgggagag aagggaaata gtacttgaaa tgtaggccct 540
   cacctcccca tgacatcctc catgagcctc ctgatgtagt g
<210> 3
   <211> 516
   <212> DNA
   <213> Human
<400> 3
   tagagatgtt ggttgatgac ccccgggatc tggagcagat gaatgaagag tctctggaag 60
   tcagcccaga catgtgcatc tacatcacag aggacatgct catgtcgcgg aacctgaatg 120
   gacactctgg gttgattgtg aaagaaattg ggtcttccac ctcgagctct tcagaaacag 180
   ttgttaagct tcgtggccag agtactgatt ctcttccaca gactatatgt cggaaaccaa 240
   agacctccac tgatcgacac agcttgagcc tcgatgacat cagactttac cagaaagact 300
   tcctgcgcat tgcaggtctg tgtcaggaca ctgctcagag ttacaccttt ggatgtggcc 360
   atgaactgga tgaggaaggc ctctattgca acagttgctt ggcccagcag tgcatcaaca 420
   tccaagatgc ttttccagtc aaaagaacca gcaaatactt ttctctggat ctcactcatg 480
   atgaagttcc agagtttgtt gtgtaaagtc cgtctg
<210> 4
   <211> 1099
   <212> DNA
   <213> Human
<400> 4
   cccacaacac aggggccctg aaacacgcca gcctctcctc tgtggtcagc ttggcccagt 60
   cctgctcact ggatcacagc ccattgtagg tggggcatgg tggggatcag ggcccctggc 120
   ccacggggag gtagaagaag acctggtccg tgtaagggtc tgagaaggtg ccctgggtcg 180
   ggggtgcgtc ttggccttgc cgtgccctca tcccccggct gaggcagcga cacagcaggt 240
   gcaccaactc cagcaggtta agcaccaggg agatgagtcc aaccaccaac atgaagatga 300
   tgaagatggt cttctccgtg ggcgagagaa caaagcagtc cacgaggtag gggcagggtg 360
   ctcgctggca cacaaacacg ggctccatgg tccagccgta caggcgccac tggccataga 420
   ggaagcctgc ctctagcaca ctcttgcaga gcacactggc gacataggtg cccatcagtg 480
   ctccgcggat gcgcaggcga ccatcttctg ccaccgagat cttggccatc tgacgctcta 540
   cggccgccag cgcccgctcc acctgtgggt ccttggccgg cagtgcccgc agctccccct 600
   ccttctgccg cagccgctct tctcgccgag acaggtaaat gacatggccc aggtagacca 660
   gggtgggtgt gctgacgaag aggaactgca gcacccagta gcggatgtgg gagatgggga 720
   aggcctggtc atagcagacg ttggtgcagc ctggctgggc cgtgttacac tcgaaatctg 780
   actgctcgtc accccacact gactcgccgg ccaggcccag gatgaggatg cggaagatga 840
   agagcaccgt cagccagatc ttacccacca cggtcgagtg ctcctggacc tggtccagca 900
   acttctccac gaagccccag tcacccatgg ctcccgggcc tccgtcggca aggagacaga 960
   gcacgtcagt gtgtcagcat ggcatccttc tcgttcgccc agcaacaagc ctgcagggag 1020
   gtctgccacg cccgttctac cgcctgcctg ccgggcggcc caggtggagg tggggacgat 1080
   ggccggagtg acgcccgcg
<210> 5
   <211> 1015
   <212> DNA
   <213> Human
<400> 5
   gaggataggg agcctggggt caggagtgtg ggagacacag cgagactctg tctccaaaaa 60
   aaaaagtgct ttttgaaaat gttgaggttg aaatgatggg aaccaacatt ctttggattt 120
   agtggggagc ataatagcaa acaccccctt ggttcgcaca tgtacaggaa tgggacccag 180
   ttggggcaca gccatggact tccccgccct ggaatgtgtg gtgcaaagtg gggccagggc 240
   ccagacccaa gaggagaggg tggtccgcag acaccccggg atgtcagcat cccccgacct 300
   gccttctggc ggcacctccc gggtgctgtg ttgagtcagc aggcatgggg tgagagcctg 360
   gtatatgctg ggaacagggt gcaggggcca agcgttcctc cttcagcctt gacttgggcc 420
   atgcaccccc tctcccccaa acacaaacaa gcacttctcc agtatggtgc caggacaggt 480
   gtcccttcag tcctctggtt atgacctcaa gtcctacttg ggccctgcag cccagcctgt 540
   gttgtaacct ctgcgtcctc aagaccacac ctggaagatt cttcttccct ttgaaggaga 600
   atcatcattg ttgctttatc acttctaaga cattttgtac ggcacggaca agttaaacag 660
   aatgtgcttc cctccctggg gtctcacacg ctcccacgag aatgccacag gggccgtgca 720
   ctgggcaggc ttctctgtag aaccccaggg gcttcggccc agaccacagc gtcttgccct 780
   gagcctagag cagggagtcc cgaacttctg cattcacaga ccacctccac aattgttata 840
   accaaaggcc tcctgttctg ttatttcact taaatcaaca tgctattttg ttttcactca 900
   cttctgactt tagcctcgtg ctgagccgtg tatccatgca gtcatgttca cgtgctagtt 960
   acgtttttct tcttacacat gaaaataaat gcataagtgt tagaagaaaa aaaaa
<210> 6
   <211> 2313
   <212> DNA
   <213> Human
<400> 6
   ccagagcagg cctggtggtg agcagggacg gtgcaccgga cggcgggatc gagcaaatgg 60
   gtctggccat ggagcacgga gggtcctacg ctcgggcggg gggcagctct cggggctgct 120
   ggtattacct gcgctacttc ttcctcttcg tctccctcat ccaattcctc atcatcctgg 180
   ggctcgtgct cttcatggtc tatggcaacg tgcacgtgag cacagagtcc aacctgcagg 240
   ccaccgagcg ccgagccgag ggcctataca gtcagctcct agggctcacg gcctcccagt 300
   ccaacttgac caaggagctc aacttcacca cccgcgccaa ggatgccatc atgcagatgt 360
   ggctgaatgc tcgccgcgac ctggaccgca tcaatgccag cttccgccag tgccagggtg 420
   accgggtcat ctacacgaac aatcagaggt acatggctgc catcatcttg agtgagaagc 480
   aatgcagaga tcaattcaag gacatgaaca agagctgcga tgccttgctc ttcatgctga 540
   atcagaaggt gaagacgctg gaggtggaga tagccaagga gaagaccatt tgcactaagg 600
   ataaggaaag cgtgctgctg aacaaacgcg tggcggagga acagctggtt gaatgcgtga 660
   aaacccggga gctgcagcac caagagcgcc actggccaag gagcaactgc aaaaggtgca 720
   agccctctgc ctgcccctgg acaaggacaa gtttgagatg gaccttcgta acctgtggag 780
   ggactccatt atcccacgca gcctggacaa cctgggttac aacctctacc atcccctggg 840
   ctcggaattg gcctccatcc gcagagcctg cgaccacatg cccagcctca tgagctccaa 900
   ggtggaggag ctggcccgga gcctccgggc ggatatcgaa cgcgtggccc gcgagaactc 960
   agacctccaa cgccagaagc tggaagccca gcagggcctg cgggccagtc aggaggcgaa 1020
   acagaaggtg gagaaggagg ctcaggcccg ggaggccaag ctccaagctg aatgctcccg 1080
   gcagacccag ctagcgctgg aggagaaggc ggtgctgcgg aaggaacgag acaacctggc 1140
   caaggagctg gaagagaaga agagggaggc ggagcagctc aggatggagc tggccatcag 1200
   aaactcagcc ctggacacct gcatcaagac caagtcgcag ccgatgatgc cagtgtcaag 1260
   gcccatgggc cctgtcccca acccccagcc catcgaccca gctagcctgg aggagttcaa 1320
   gaggaagatc ctggagtccc agaggccccc tgcaggcatc cctgtagccc catccagtgg 1380
   ctgaggaggc tccaggcctg aggaccaagg gatggcccga ctcggcggtt tgcggaggat 1440
   gcagggatat gctcacagcg cccgacacaa ccccctcccg ccgcccccaa ccacccaggg 1500
   ccaccatcag acaactccct gcatgcaaac ccctagtacc ctctcacacc cgcacccgcg 1560
   cctcacgatc cctcacccag agcacacggc cgcggagatg acgtcacgca agcaacggcg 1620
   ctgacgtcac atatcaccgt ggtgatggcg tcacgtggcc atgtagacgt cacgaagaga 1680
   tatagcgatg gcgtcgtgca gatgcagcac gtcgcacaca gacatgggga acttggcatg 1740
   acgtcacacc gagatgcagc aacgacgtca cgggccatgt cgacgtcaca catattaatg 1800
   tcacacagac gcggcgatgg catcacacag acggtgatga tgtcacacac agacacagtg 1860
   acaacacaca ccatgacaac gacacctata gatatggcac caacatcaca tgcacgcatg 1920
   ccctttcaca cacactttct acccaattct cacctagtgt cacgttcccc cgaccctggc 1980
   acacgggcca aggtacccac aggatcccat cccctcccgc acagccctgg gccccagcac 2040
   ctcccctcct ccagcttcct ggcctcccag ccacttcctc acccccagtg cctggacccg 2100
   gaggtgagaa caggaagcca ttcacctccg ctccttgagc gtgagtgttt ccaggacccc 2160
   ctcggggccc tgagccgggg ggtgaggtca cctgttgtcg ggaggggagc cactccttct 2220
   cccccaactc ccagccctgc ctgtggcccg ttgaaatgtt ggtggcactt aataaatatt 2280
   agtaaatcct taaaaaaaaa aaaaaaaaaa aaa
<210> 7
   <211> 389
   <212> DNA
   <213> Human
<400> 7
   gccaaaaaga tggcttcaaa agtaagaatg aaacatttga tccattcagc tttaggctat 60
   gccactggat tcatgtctag aaaagatagg ataatttctg taaagaaatg aagaccttgc 120
   tattctaaaa tcagatcctt acagatccag atttcaggaa acaaatacat aggggactaa 180
   ctttccttgt tcagattagt ttttctcctt tgcacccagc tatataatat gaggaagtat 240
   tgacttttta aaagtgtttt agttttccat ttctttgata tgaaaagtaa tatttcggga 300
   gaaccctgag ctattaataa tctatgtggc tagtgcgtat atattggtct gaatttgttc 360
   tccttttgtg gtgtccagtg ggtaacatc
<210> 8
   <211> 157
   <212> DNA
   <213> Human
<400> 8
   tgctttaaac agctgtgtca aaaactgaca tcagagagta aattgaattt ggttttgtag 60
   gaagcaggaa gcaagcccac tcaaacgtga aatttggcat gagggatcca gtaactttct 120
   cctcaatctg tgaactatat gtgagtttga tattttg
<210> 9
   <211> 561
   <212> DNA
   <213> Human
<400> 9
   aatagtcaaa acataaacaa aagctaatta actggcactg ttgtcacctg agactaagtg 60
   gatgttgttg gctgacatac aggctcagcc agcagagaaa gaattctgaa ttccccttgc 120
   tgaactgaac tattctgtta catatggttg acaaatctgt gtgttatttc ttttctacct 180
   accatattta aatttatgag tatcaaccga ggacatagtc aaaccttcga tgatgaacat 240
   tcctgatttt ttgcctgatt aatctctgtt gagctctact tgtggtcatt caagatttta 300
   tgatgttgaa aggaaaagtg aatatgacct ttaaaaattg tattttgggt gatgatagtc 360
   tcaccactat aaaactgtca attattgcct aatgttaaag atatccatca ttgtgattaa 420
   ttaaacctat aatgagtatt cttaatggag aattcttaat ggatggatta tcccctgatc 480
   ttttctttaa aatttctctg cacacacagg acttctcatt ttccaataaa tgggtgtact 540
   ctgccccaat ttctaggaaa a
<210> 10
   <211> 1508
   <212> DNA
   <213> Human
<400> 10
   cacaaacacg agagactcca cggtctgcct gagcaccgcc agcctcctag gctccagcac 60
   tcgcaggtcc attcttctgc acgagcctct ctgtccagat ccataagcac ggtcagctca 120
   gggtcgcgga gcagtacgag gacaagtacc agcagcagct cctctgaaca gagactgcta 180
   ggatcatcct tctcctccgg gcctgttgct gatggcataa tccgggtgca acccaaatct 240
   gagctcaagc caggtgagct taagccactg agcaaggaag atttgggcct gcacgcctac 300
   aggtgtgagg actgtggcaa gtgcaaatgt aaggagtgca cctacccaag gcctctgcca 360
   tcagactgga tctgcgacaa gcagtgcctt tgctcggccc agaacgtgat tgactatggg 420
   acttgtgtat gctgtgtgaa aggtctcttc tatcactgtt ctaatgatga tgaggacaac 480
   tgtgctgaca acccatgttc ttgcagccag tctcactgtt gtacacgatg gtcagccatg 540
   ggtgtcatgt ccctcttttt gccttgttta tggtgttacc ttccagccaa gggttgcctt 600
   aaattgtgcc aggggtgtta tgaccgggtt aacaggcctg gttgccgctg taaaaactca 660
   aacacagttt gctgcaaagt tcccactgtc ccccctagga actttgaaaa accaacatag 720
   catcattaat caggaatatt acagtaatga ggattttttc tttctttttt taatacacat 780
   atgcaaccaa ctaaacagtt ataatcttgg cactgttaat agaaagttgg gatagtcttt 840
   gctgtttgcg gtgaaatgct ttttgtccat gtgccgtttt aactgatatg cttgttagaa 900
   ctcagctaat ggagctcaaa gtatgagata cagaacttgg tgacccatgt attgcataag 960
   ctaaagcaac acagacactc ctaggcaaag tttttgtttg tgaatagtac ttgcaaaact 1020
   tgtaaattag cagatgactt ttttccattg ttttctccag agagaatgtg ctatattttt 1080
   gtatatacaa taatatttgc aactgtgaaa aacaagtggt gccatactac atggcacaga 1140
   cacaaaatat tatactaata tgttgtacat tcggaagaat gtgaatcaat cagtatgttt 1200
   ttagattgta ttttgcctta cagaaagcct ttattgtaag actctgattt ccctttggac 1260
   ttcatgtata ttgtacagtt acagtaaaat tcaaccttta ttttctaatt ttttcaacat 1320
   attgtttagt gtaaagaata tttatttgaa gttttattat tttataaaaa agaatattta 1380
   ttttaagagg catcttacaa attttgcccc ttttatgagg atgtgatagt tgctgcaaat 1440
   gaggggttac agatgcatat gtccaatata aaatagaaaa tatattaacg tttgaaatta 1500
   aaaaaaaa
<210> 11
   <211> 389
   <212> DNA
   <213> Human
<400> 11
   gggcaggtga tcagggcaca catttcccgt ccattgagac agtagcattc ccggcaccca 60
   tcgtgccagc tctcctcatt tttatgatga tgaccatcca cggtgagaca agtgcccgac 120
   aggatgggtg gcccagctga agcacaggcc gctctgcact tgcagataag acagccgtga 180
   ctgtcctgct ggaaacccaa ggggcagatc ttactgcatg agagctctgg acatttctta 240
   cagcgacaga tgtcacagcc gtgcttattc ttcagcaatc caagtggaca atacttgtca 300
   cagattatgg gtctgcactt cttgggcctt gggcggcact cacagatctc acagttttgg 360
   acctcggccg cgaccacgct gggtaccga
<210> 12
   <211> 981
   <212> DNA
   <213> Human
<400> 12
   tttttttttt ttggattgca aaaatttatt aaaattggag acactgtttt aatcttcttg 60
   tgccatgaga ctccatcagg cagtctacaa agaccactgg gaggctgagg atcacttgag 120
   cccagaagtt tgaggctgta gtaagcttca aaggccactg cactctagct tgggtgaggc 180
   aagacccttt caagcagtaa gctgcatgct tgcttgttgt ggtcattaaa aaccctagtt 240
   taggataaca acatattaat cagggcaaaa tacaaatgtg tgatgcttgt tagtagagta 300
   acctcagaat caaaatggaa cggttttaca gtgatatcat tatatttcat ttggcagaat 360
   cattacatca ttggttacac tgaaaatcat cacatgtacc aaaagctgac tcacctagtt 420
   taggataaca ggtctgcctg tttgaagatg aaaaataata cccatttaaa atttgcccta 480
   ctcaatttcc ttctcagtca cattttaact tttaaacagc taatcactcc catctacaga 540
   ttaaggtgta tatgccacca aaaccttttg ccaccttaaa aatttccttc aaagtttaaa 600
   ctaatgcctg catttcttca atcatgaatt ctgagtcctt tgcttcttta aaacttgctc 660
   cacacagtgt agtcaagccg actctccata cccaagcaag tcatccatgg ataaaaacgt 720
   taccaggagc agaaccatta agctggtcca ggcaagttgg actccaccat ttcaacttcc 780
   agctttctgt ctaatgcctg tgtgccaatg gcttgagtta ggcttgctct ttaggacttc 840
   agtagctatt ctcatccttc cttggggaca caactgtcca taaggtgcta tccagagcca 900
   cactgcatct gcacccagca ccatacctca caggagtcga ctcccacgag ccgcctgtat 960
   ataagagttc ttttgatgac g
<210> 13
   <211> 401
   <212> DNA
   <213> Human
<400> 13
   ataactacag cttcagcaga caactaaaga gactgcatta aggtgatttc tctggctata 60
   aagagagccc ggccgcagag catgtgactg ctgggacctc tgggataggc aacactgccc 120
   tctctccccc agagcgaccc cccgggcagg tcggggccca aggaatgacc cagcaactgc 180
   tccctaccca gcacactctc tttactgcca cctgcaatta tgctgtgaag atgactgggt 240
   gtggtcatca cgattcagag aaatcaagat ctatgaccat tttaggcaaa gagagaaact 300
   tggagaattg ctgaggacta ctgaaccttg ttttgctttt ttaaaaaata ctaaatcctc 360
   acttcagcat atttagttgt cattaaaatt aagctgatat t
<210> 14
   <211> 1002
   <212> DNA
   <213> Human
<400> 14
   gacaatataa aaagtggaaa caagcataaa ttgcagacat aaaataatct tctggtagaa 60
   acagttgtgg agaacaggtt gagtagagca acaacaacaa aagcttatgc agtcaccttc 120
   tttgaaaatg ttaaatacaa gtcctattct ctttgtccag ctgggtttag ctagaggtag 180
   ccaattactt ctcttaaggt ccatggcatt cgccaggatt ctataaaagc caagttaact 240
   gaagtaaata tctggggccc atcgcacccc cactaagtac tttgtcacca tgttgtatct 300
   taaaagtcat ttttcactgt ttgactcaga atttgggact tcagagtcaa acttcattgc 360
   ttactccaaa cccagtttaa ttccccactt ttttaagtag gcttagcttt gagtgatttt 420
   tggctataac cgaaatgtaa atccaccttc aaacaacaaa gtttgacaag actgaaatgt 480
   tactgaaaac aatggtgcca tatgctccaa agacatttcc ccaagataac tgccaaagag 540
   tttttgagga ggacaatgat catttattat gtaggagcct tgatatctct gcaaaataga 600
   attaatacag ctcaaatgga gtagtaacca agcttttctg cccaggaagt aacaaacatc 660
   actacgaaca tgagagtaca agaggaaact ttcataatgc attttttcat tcatacattc 720
   attcaataaa cattagccaa gctaatgtcc caagccactg tgccaggtat taacaatata 780
   acaacaataa aagacacagt ccttcctctc aaggtgttca gtctagtagg gaagatgatt 840
   attcattaaa atttttggtg catcagaatc atgaggagct tgtcaaaaat gtaaattcct 900
   gcctatgttc tcagatattc tggttaggtc aggagtggga acccaaaatc aattctttta 960
   acaaacacta aaggtgattc taacacaggc ggtgtgagga cc
<210> 15
   <211> 280
   <212> DNA
   <213> Human
<400> 15
   cgaggtgggc cacccgtgtc tggtctgaga tttttaaatg aggattacat tatcctattt 60
   ataatattcc tattctaatc tattgtattc ttacaattaa atgtatcaaa taattcttaa 120
   aaacattatt agaaacaaac tgcctaatac cttataagac taaaaaaatc accaagatga 180
   aactgtatta tgactctcaa tatttaaaca tttaaaaaaa tgttagtgtt tgttaagcac 240
   caatcttaac tatttcacct gcccgggcgg ccgctcgagg
<210> 16
   <211> 2041
   <212> DNA
   <213> Human
<400> 16
   ccccccgcag aactcccccc tggaatagga tttttaaaac ccttgacaat tagaaatcct 60
   atagaggtta gcatttttta ggtaaaaata tggttgcccc tacagggatc atgcaacttc 120
   cttaaaacca attcagcaca tatgtataaa gaaccctttt taaaaacatt tgtacttgaa 180
   atacagacac agtgatgctg aagacactaa acaaaaactg aaaagtacta taccttgata 240
   aattttgtta ttgccttctt tagagacttt ataatctcta gttgatttcc aaggacttga 300
   atttaataat ggggtaatta cacaagacgt aaaggatttt ttaaaaacaa gtattttttt 360
   ttacctctag catcaattct tttataaaga atgctaaata aattacattt tttgttcagt 420
   aaaactgaag atagaccatt taaatgcttc taccaaattt aacgcagctt aattagggac 480
   caggtacata ttttcttctg aacatttttg gtcaagcatg tctaaccata aaagcaaatg 540
   gaattttaag aggtagattt tttttccatg atgcattttg ttaataaatg tgtcaagaaa 600
   ataaaaacaa gcactgagtg tgttctcttg aagtataagg gtctaatgaa aaataaaaga 660
   tagatatttg ttatagtctg acattttaac agtcatagta ttagacgttt cgtgaccagt 720
   gcattttgga ctctctcagg atcaaaatac gagtctgcca actgtattaa atcctcctcc 780
   accccctcca ccagttggtc cacagcttcc tggtgggtcg ttgtcatcaa atccattggg 840
   ccgaaatgaa catgaagcag atgcagcttg gagggcccgg gctcgagcat tcaactcttg 900
   ttcctgtaaa tatagtttat tgtcttttgt tatagcatcc ataagttctt tctgtagagg 960
   tgggtctcca tttatccaga gtccactggt tgggttatta ccacttaaac cattagtact 1020
   atgctgtttt ttatacaaaa gcacataagc tgtgtccttt ggaaacctgc tcgtaatttt 1080
   ctggactgac tgaaatgaag taaatgtcac tctactgtca ttaaataaaa acccattctt 1140
   ttgacatttc cttattttcc aaatcctgtt caaaaactgc actgggacta tctctcccta 1200
   gtaaatgact ctgggaggat gctaatgcca gagcctcaga ctggtggtac atctgatatg 1260
   aagagtctgt acttgtgata tttctggcat aagaatagta atgcccactt tcagaggata 1320
   taccagagtg aaccacaacg gaacttaata gatagggcac caattttgtg caggaagctt 1380
   catcagtccc tgaaggcttt aattttttag caaggttctc actaagatca gtgaagtcaa 1440
   catctacaga ccaactttct gacaatgaag agaaagaagt aattcttcta actggcaact 1500
   ccaaaaccag tggccagtga tacattgtct aaaattttcc ttctcacatg atacttctga 1560
   tcatatgaaa atctcaggag agtaagaata aggtattcag gttcctccgt gatttgcata 1620
   gttttctcag cattttgcag agaggcacag ttttcacaat aatattggtt atcaccagta 1680
   agaatctctg gagcccaaaa aataatttag taagtcagtt actgaaggtg tggtttcacc 1740
   tcccggtttc tgaggtacat ctttattaac aagaatcttg ttagattcgt tagggacaga 1800
   agtgttttca gaacagtaaa actcattagg aggactgcct atggtttttt cattcacaag 1860
   tgagtcacag atgaaggcag ctgttgttgg attataaact actggctctt ctgaaggacc 1920
   gggtacagac gcttgcatta gaccaccatc ttgtatactg ggtgatgatg ctggatcttg 1980
   gacagacatg ttttccaaag aagaggaagc acaaaacgca agcgaaagat ctgtaaaggc 2040
   t
<210> 17
   <211> 235
   <212> DNA
   <213> Human
<400> 17
   cgccccgggc aggtgtcagg ggttccaaac cagcctgggg aaacacagcg tagacccctc 60
   acctctacaa ataaaaaatt aaaaaattag ccaggtgtgg cagcgaacaa ctgtagtctc 120
   agatactcag gagactgagc tggaaaggat cacttgagcc caagaagttc aaggttacag 180
   tgggccacga tcatgtcatt acactccagc ttgggtgaca aaatgagact gtcta
<210> 18
   <211> 2732
   <212> DNA
   <213> Human
<400> 18
   gtgtggagtt tcagctgcta ttgactataa gagctatgga acagaaaaag cttgctggct 60
   tcatgttgat aactacttta tatggagctt cattggacct gttaccttca ttattctgct 120
   aaatattatc ttcttggtga tcacattgtg caaaatggtg aagcattcaa acactttgaa 180
   accagattct agcaggttgg aaaacattaa gtcttgggtg cttggcgctt tcgctcttct 240
   gtgtcttctt ggcctcacct ggtcctttgg gttgcttttt attaatgagg agactattgt 300
   gatggcatat ctcttcacta tatttaatgc tttccaggga gtgttcattt tcatctttca 360
   ctgtgctctc caaaagaaag tacgaaaaga atatggcaag tgcttcagac actcatactg 420
   ctgtggaggc ctcccaactg agagtcccca cagttcagtg aaggcatcaa ccaccagaac 480
   cagtgctcgc tattcctctg gcacacagag tcgtataaga agaatgtgga atgatactgt 540
   gagaaaacaa tcagaatctt cttttatctc aggtgacatc aatagcactt caacacttaa 600
   tcaaggtggc ataaatctta atatattatt acaggactga catcacatgg tctgagagcc 660
   catcttcaag atttatatca tttagaggac attcactgaa caatgccagg gatacaagtg 720
   ccatggatac tctaccgcta aatggtaatt ttaacaacag ctactcgctg cacaagggtg 780
   actataatga cagcgtgcaa gttgtggact gtggactaag tctgaatgat actgcttttg 840
   agaaaatgat catttcagaa ttagtgcaca acaacttacg gggcagcagc aagactcaca 900
   acctcgagct cacgctacca gtcaaacctg tgattggagg tagcagcagt gaagatgatg 960
   ctattgtggc agatgcttca tctttaatgc acagcgacaa cccagggctg gagctccatc 1020
   acaaagaact cgaggcacca cttattcctc agcggactca ctcccttctg taccaacccc 1080
   agaagaaagt gaagtccgag ggaactgaca gctatgtctc ccaactgaca gcagaggctg 1140
   aagatcacct acagtccccc aacagagact ctctttatac aagcatgccc aatcttagag 1200
   actctcccta tccggagagc agccctgaca tggaagaaga cctctctccc tccaggagga 1260
   gtgagaatga ggacatttac tataaaagca tgccaaatct tggagctggc catcagcttc 1320
   agatgtgcta ccagatcagc aggggcaata gtgatggtta tataatcccc attaacaaag 1380
   aagggtgtat tccagaagga gatgttagag aaggacaaat gcagctggtt acaagtcttt 1440
   aatcatacag ctaaggaatt ccaagggcca catgcgagta ttaataaata aagacaccat 1500
   tggcctgacg cagctccctc aaactctgct tgaagagatg actcttgacc tgtggttctc 1560
   tggtgtaaaa aagatgactg aaccttgcag ttctgtgaat ttttataaaa catacaaaaa 1620
   ctttgtatat acacagagta tactaaagtg aattatttgt tacaaagaaa agagatgcca 1680
   gccaggtatt ttaagattct gctgctgttt agagaaattg tgaaacaagc aaaacaaaac 1740
   tttccagcca ttttactgca gcagtctgtg aactaaattt gtaaatatgg ctgcaccatt 1800
   tttgtaggcc tgcattgtat tatatacaag acgtaggctt taaaatcctg tgggacaaat 1860
   ttactgtacc ttactattcc tgacaagact tggaaaagca ggagagatat tctgcatcag 1920
   tttgcagttc actgcaaatc ttttacatta aggcaaagat tgaaaacatg cttaaccact 1980
   agcaatcaag ccacaggcct tatttcatat gtttcctcaa ctgtacaatg aactattctc 2040
   atgaaaaatg gctaaagaaa ttatattttg ttctattgct agggtaaaat aaatacattt 2100
   gtgtccaact gaaatataat tgtcattaaa ataattttaa agagtgaaga aaatattgtg 2160
   aaaagctctt ggttgcacat gttatgaaat gttttttctt acactttgtc atggtaagtt 2220
   ctactcattt tcacttcttt tccactgtat acagtgttct gctttgacaa agttagtctt 2280
   tattacttac atttaaattt cttattgcca aaagaacgtg ttttatgggg agaaacaaac 2340
   tctttgaagc cagttatgtc atgccttgca caaaagtgat gaaatctaga aaagattgtg 2400
   tgtcacccct gtttattctt gaacagaggg caaagagggc actgggcact tctcacaaac 2460
   tttctagtga acaaaaggtg cctattcttt tttaaaaaaa taaaataaaa cataaatatt 2520
   actcttccat attccttctg cctatattta gtaattaatt tattttatga taaagttcta 2580
   atgaaatgta aattgtttca gcaaaattct gctttttttt catccctttg tgtaaacctg 2640
   ttaataatga gcccatcact aatatccagt gtaaagttta acacggtttg acagtaaata 2700
   aatgtgaatt ttttcaagtt aaaaaaaaaa aa
<210> 19
   <211> 276
   <212> DNA
   <213> Human
<400> 19
   ctccctaaat gattttaaaa taaattggat aaacatatga tataaagtgg gtactttaga 60
   aaccgccttt gcatattttt tatgtacaaa tctttgtata caattccgat gttccttata 120
   tattccctat atagcaaacc aaaaccagga cctcccaact gcatgcctca agtccctgtg 180
   gagcactctg gcaactggat ggccctactt gctttctgac aaaatagctg gaaaggagga 240
   gggaccaatt aaatacctcg gccgcgacca cgctgg
<210> 20
   <211> 2361
   <212> DNA
   <213> Human
<400> 20
   attgtaccag ccttgatgaa cgtgggccct gcttcgcttt tgagggccat aagctcattg 60
   cccactggtt tagaggctac cttatcattg tctcccgtga ccggaaggtt tctcccaagt 120
   cagagtttac cagcagggat tcacagagct ccgacaagca gattctaaac atctatgacc 180
   tgtgcaacaa gttcatagcc tatagcaccg tctttgagga tgtagtggat gtgcttgctg 240
   agtggggctc cctgtacgtg ctgacgcggg atgggcgggt ccacgcactg caggagaagg 300
   acacacagac caaactggag atgctgttta agaagaacct atttgagatg gcgattaacc 360
   ttgccaagag ccagcatctg gacagtgatg ggctggccca gattttcatg cagtatggag 420
   accatctcta cagcaagggc aaccacgatg gggctgtcca gcaatatatc cgaaccattg 480
   gaaagttgga gccatcctac gtgatccgca agtttctgga tgcccagcgc attcacaacc 540
   tgactgccta cctgcagacc ctgcaccgac aatccctggc caatgccgac cataccaccc 600
   tgctcctcaa ctgctatacc aagctcaagg acagctcgaa gctggaggag ttcatcaaga 660
   aaaagagtga gagtgaagtc cactttgatg tggagacagc catcaaggtc ctccggcagg 720
   ctggctacta ctcccatgcc ctgtatctgg cggagaacca tgcacatcat gagtggtacc 780
   tgaagatcca gctagaagac attaagaatt atcaggaagc ccttcgatac atcggcaagc 840
   tgccttttga gcaggcagag agcaacatga agcgctacgg caagatcctc atgcaccaca 900
   taccagagca gacaactcag ttgctgaagg gactttgtac tgattatcgg cccagcctcg 960
   aaggccgcag cgatagggag gccccaggct gcagggccaa ctctgaggag ttcatcccca 1020
   tctttgccaa taacccgcga gagctgaaag ccttcctaga gcacatgagt gaagtgcagc 1080
   cagactcacc ccaggggatc tacgacacac tccttgagct gcgactgcag aactgggccc 1140
   acgagaagga tccacaggtc aaagagaagc ttcacgcaga ggccatttcc ctgctgaaga 1200
   gtggtcgctt ctgcgacgtc tttgacaagg ccctggtcct gtgccagatg cacgacttcc 1260
   aggatggtgt cctttacctt tatgagcagg ggaagctgtt ccagcagatc atgcactacc 1320
   acatgcagca cgagcagtac cggcaggtca tcagcgtgtg tgagcgccat ggggagcagg 1380
   acccctcctt gtgggagcag gccctcagct acttcgctcg caaggaggag gactgcaagg 1440
   agtatgtggc agctgtcctc aagcatatcg agaacaagaa cctcatgcca cctcttctag 1500
   tggtgcagac cctggcccac aactccacag ccacactctc cgtcatcagg gactacctgg 1560
   tccaaaaact acagaaacag agccagcaga ttgcacagga tgagctgcgg gtgcggcggt 1620
   accgagagga gaccacccgt atccgccagg agatccaaga gctcaaggcc agtcctaaga 1680
   ttttccaaaa gaccaagtgc agcatctgta acagtgcctt ggagttgccc tcagtccact 1740
   tcctgtgtgg ccactccttc caccaacact gctttgagag ttactcggaa agtgatgctg 1800
   actgccccac ctgcctccct gaaaaccgga aggtcatgga tatgatccgg gcccaggaac 1860
   agaaacgaga tctccatgat caattccagc atcagctcaa gtgctccaat gacagctttt 1920
   ctgtgattgc tgactacttt ggcagaggtg ttttcaacaa attgactctg ctgaccgacc 1980
   ctcccacagc cagactgacc tccagcctgg aggctgggct gcaacgcgac ctactcatgc 2040
   actccaggag gggcacttaa gcagcctgga ggaagatgtg ggcaacagtg gaggaccaag 2100
   agaacagaca caatgggacc tgggcgggcg ttacacagaa ggctggctga catgcccagg 2160
   gctccactct catctaatgt cacagccctc acaagactaa agcggaactt tttcttttcc 2220
   ctggccttcc ttaattttaa gtcaagcttg gcaatccctt cctctttaac taggcaggtg 2280
   ttagaatcat ttccagatta atggggggga aggggaacct caggcaaacc tcctgaagtt 2340
   ttggaaaaaa aagctggttt c
<210> 21
   <211> 179
   <212> DNA
   <213> Human
<400> 21
   aggtgttaga tgctcttgaa aaagaaactg catctaagct gtcagaaatg gattctttta 60
   acaatcaact aaaggaactg agagaaacct acaacacaca gcagttagcc cttgaacagc 120
   tttataagat caacgtgaca agttgaagga aattgaaagg aaaaaattag aactaatgc
<210> 22
   <211> 905
   <212> DNA
   <213> Human
<400> 22
   tttttttttt ttctttaacc gtgtggtctt tatttcagtg ccagtgttac agatacaaca 60
   caaatgttcc agttagaagg aattcaaacg gaatgccaag gtccaagcca ggctcaagaa 120
   ataaaaaggg aggtttggag taatagataa gatgactcca atactcactc ttcctaaggg 180
   caaaggtact tttgatacag agtctgatct ttgaaactgg tgaactcctc ttccacccat 240
   taccatagtt caaacaggca agttatgggc ttaggagcac tttaaaattt gtggtgggaa 300
   tagggtcatt aataactatg aatatatctt ttagaaggtg accattttgc actttaaagg 360
   gaatcaattt tgaaaatcat ggagactatt catgactaca gctaaagaat ggcgagaaag 420
   gggagctgga agagccttgg aagtttctat tacaaataga gcaccatatc cttcatgcca 480
   aatctcaaca aaagctcttt ttaactccat ctgtccagtg tttacaaata aactcgcaag 540
   gtctgaccag ttcttggtaa caaacataca tgtgtgtgtc tgtgtgtata cagcaatgca 600
   cagaaaaggc taccaggagc ctaatgcctc tttcaaacat tgggggaacc agtagaaaaa 660
   ggcagggctc cctaatgtcc attattacat ttccattccg aatgccagat gttaaaagtg 720
   cctgaagatg gtaacccagc tagtgaggaa taaatacccc accttgccca gtccacagag 780
   aaacaacagt agaaagaagg ggcaactctt tgctgcagag acaaagtgag tgttttttcg 840
   ccatggattg cagtcctctc ctccagacca gctgcttatt tcctcagggg cccagggaat 900
   gttga
<210> 23
   <211> 2134
   <212> DNA
   <213> Human
<400> 23
   ggtctcttct ttcctttttt tttttccaaa agtgttcttt tatttctagt aacatatatt 60
   gtataaatac tctattttat atgcacttcc acaaaagcga tataatttaa aagttttttt 120
   cattagaaat aaatgtataa aaataaatat gttattatag gcatttatta ctaactatag 180
   tccttcttgg aaggaacacc caaaccaata cttataaagt acatgtaatt tatagtaaca 240
   tattttacta tatacatatg gaaaaaatca tattctcaca gaagagctga acagacattc 300
   accaggatac gactgttgga ccagctgctg gagatggacc tgctacccct cagcagcctc 360
   cccaccacaa gacaagtgat ctcaatgtcc ccaaacctgt gggaccctgt tctacacacc 420
   tcatttttgt tccggcgttt catcctcctt gtgtgattgt actgattttc atgagacaca 480
   agttacttct ttacatccat attcccaaag cagggttaca tggtaggaaa gaaaggaagt 540
   tggaggtact aagctcattg tgtctcctct agcttttacc agcatctaat gcttcactgc 600
   tttttttcca ttgtagactt taatgcactt gaataaatac atggagttgt tttttcctca 660
   aaatgaatta cacaaataaa gactgagatg gtccaaaaaa ggaaagagga agccatttgc 720
   gttatttcac gttgctgagc ctttctctca tgttgaacaa tctgaagttt taattctcgg 780
   tagaaataat gtataaacat tctctgaaac catagcagcc ataaacagtg ctggtcaaag 840
   atcctatttg tactcctttc tccccccatt gttagtgagg taaagtaaaa caggtcttag 900
   taaaatctca cttttctcct acttttcatt tcccaacccc catgatacta agtatttgat 960
   aagtaccagg aaacaggggt tgtaatagtt ctaacttttt ttgacaattg ctttgttttt 1020
   tctaaacttg taatagatgt aacaaaagaa ataataataa taatgcccgg ggctttatta 1080
   tgctatatca ctgctcagag gttaataatc ctcactaact atcctatcaa atttgcaact 1140
   ggcagtttac tctgatgatt caactccttt tctatctacc cccataatcc caccttactg 1200
   atacacctca ctggttactg gcaagatacg ctggatccct ccagccttct tgctttccct 1260
   gcaccagccc ttcctcactt tgccttgccc tcaaagctaa caccacttaa accacttaac 1320
   tgcattctgc cattgtgcaa aagtctatga aatgtttagg tttctttaaa ggatcacagc 1380
   tctcatgaga taacacccct ccatcatggg acagacactt caagcttctt tttttgtaac 1440
   ccttcccaca ggtcttagaa catgatgacc actcccccag ctgccactgg gggcagggat 1500
   ggtctgcaca aggtctggtg ctggctggct tcacttcctt tgcacactcg gaagcaggct 1560
   gtccattaat gtctcggcat tctaccagtc ttctctgcca acccaattca catgacttag 1620
   aacattcgcc ccactcttca atgacccatg ctgaaaaagt ggggatagca ttgaaagatt 1680
   ccttcttctt ctttacgaag taggtgtatt taattttagg tcgaagggca ttgcccacag 1740
   taagaacctg gatggtcaag ggctctttga gagggctaaa gctgcgaatt ctttccaatg 1800
   ccgcagagga gccgctgtac ctcaagacaa cacctttgta cataatgtct tgctctaagg 1860
   tggacaaagt gtagtcacca ttaagaatat atgtgccatc agcagctttg atggcaagaa 1920
   agctgccatt gttcctggat cccctctggt tccgctgttt cacttcgatg ttggtggctc 1980
   cagttggaat tgtgatgata tcatgatatc caggttttgc actagtaact gatcctgata 2040
   tttttttaca agtagatcca tttcccccgc aaacaccaca tttatcaaac ttctttttgg 2100
   agtctatgat gcgatcacaa ccagctttta caca
<210> 24
   <211> 1626
   <212> DNA
   <213> Human
<400> 24
   ggacaatttc tagaatctat agtagtatca ggatatattt tgctttaaaa tatattttgg 60
   ttattttgaa tacagacatt ggctccaaat tttcatcttt gcacaatagt atgacttttc 120
   actagaactt ctcaacattt gggaactttg caaatatgag catcatatgt gttaaggctg 180
   tatcatttaa tgctatgaga tacattgttt tctccctatg ccaaacaggt gaacaaacgt 240
   agttgttttt tactgatact aaatgttggc tacctgtgat tttatagtat gcacatgtca 300
   gaaaaaggca agacaaatgg cctcttgtac tgaatacttc ggcaaactta ttgggtcttc 360
   attttctgac agacaggatt tgactcaata tttgtagagc ttgcgtagaa tggattacat 420
   ggtagtgatg cactggtaga aatggttttt agttattgac tcagaattca tctcaggatg 480
   aatcttttat gtctttttat tgtaagcata tctgaattta ctttataaag atggttttag 540
   aaagctttgt ctaaaaattt ggcctaggaa tggtaacttc attttcagtt gccaaggggt 600
   agaaaaataa tatgtgtgtt gttatgttta tgttaacata ttattaggta ctatctatga 660
   atgtatttaa atatttttca tattctgtga caagcattta taatttgcaa caagtggagt 720
   ccatttagcc cagtgggaaa gtcttggaac tcaggttacc cttgaaggat atgctggcag 780
   ccatctcttt gatctgtgct taaactgtaa tttatagacc agctaaatcc ctaacttgga 840
   tctggaatgc attagttatg ccttgtacca ttcccagaat ttcaggggca tcgtgggttt 900
   ggtctagtga ttgaaaacac aagaacagag agatccagct gaaaaagagt gatcctcaat 960
   atcctaacta actggtcctc aactcaagca gagtttcttc actctggcac tgtgatcatg 1020
   aaacttagta gaggggattg tgtgtatttt atacaaattt aatacaatgt cttacattga 1080
   taaaattctt aaagagcaaa actgcatttt atttctgcat ccacattcca atcatattag 1140
   aactaagata tttatctatg aagatataaa tggtgcagag agactttcat ctgtggattg 1200
   cgttgtttct tagggttcct agcactgatg cctgcacaag catgtgatat gtgaaataaa 1260
   atggattctt ctatagctaa atgagttccc tctggggaga gttctggtac tgcaatcaca 1320
   atgccagatg gtgtttatgg gctatttgtg taagtaagtg gtaagatgct atgaagtaag 1380
   tgtgtttgtt ttcatcttat ggaaactctt gatgcatgtg cttttgtatg gaataaattt 1440
   tggtgcaata tgatgtcatt caactttgca ttgaattgaa ttttggttgt atttatatgt 1500
   attatacctg tcacgcttct agttgcttca accattttat aaccattttt gtacatattt 1560
   tacttgaaaa tattttaaat ggaaatttaa ataaacattt gatagtttac ataataaaaa 1620
   aaaaaa
<210> 25
   <211> 1420
   <212> DNA
   <213> Human
<400> 25
   gttcagcatt gtttctgctt ctgaaatctg tatagtacac tggtttgtaa tcattatgtc 60
   ttcattgaaa tccttgctac ttctcttcct cctcaatgaa agacacgaga gacaagagcg 120
   acacaagctt aagaaaaacg agcaaggaag agtatcttca ttattctcat tttctctgag 180
   ttggaaacaa aaacatgaag gactccaact agaagacaga tatttacatt taaatagatt 240
   agtgggaaaa ctttaagagt ttccacatat tagttttcat tttttgagtc aagagactgc 300
   tccttgtact gggagacact agtagtatat gtttgtaatg ttactttaaa attatctttt 360
   tattttataa ggcccataaa tactggttaa actctgttaa aagtgggcct tctatcttgg 420
   atggtttcac tgccatcagc catgctgata tattagaaat ggcatcccta tctacttact 480
   ttaatgctta aaattataca taaaatgctt tatttagaaa acctacatga tacagtggtg 540
   tcagccttgc catgtatcag tttcacttga aatttgagac caattaaatt tcaactgttt 600
   agggtggaga aagaggtact ggaaaacatg cagatgagga tatcttttat gtgcaacagt 660
   atcctttgca tgggaggaga gttactcttg aaaggcaggc agcttaagtg gacaatgttt 720
   tgtatatagt tgagaatttt acgacacttt taaaaattgt gtaattgtta aatgtccagt 780
   tttgctctgt tttgcctgaa gttttagtat ttgttttcta ggtggacctc tgaaaaccaa 840
   accagtacct ggggaggtta gatgtgtgtt tcaggcttgg agtgtatgag tggttttgct 900
   tgtattttcc tccagagatt ttgaacttta ataattgcgt gtgtgttttt ttttttttaa 960
   gtggctttgt ttttttttct caagtaaaat tgtgaacata tttcctttat aggggcaggg 1020
   catgagttag ggagactgaa gagtattgta gactgtacat gtgccttctt aatgtgtttc 1080
   tcgacacatt ttttttcagt aacttgaaaa ttcaaaaggg acatttggtt aggttactgt 1140
   acatcaatct atgcataaat ggcagcttgt tttcttgagc cactgtctaa attttgtttt 1200
   tatagaaatt ttttatactg attggttcat agatggtcag ttttgtacac agactgaaca 1260
   atacagcact ttgccaaaaa tgagtgtagc attgtttaaa cattgtgtgt taacacctgt 1320
   tctttgtaat tgggttgtgg tgcattttgc actacctgga gttacagttt tcaatctgtc 1380
   agtaaataaa gtgtccttta acttcaaaaa aaaaaaaaaa
<210> 26
   <211> 689
   <212> DNA
   <213> Human
<400> 26
   aaacaaacaa aaaaaaagtt agtactgtat atgtaaatac tagcttttca atgtgctata 60
   caaacaatta tagcacatcc ttccttttac tctgtctcac ctcctttagg tgagtacttc 120
   cttaaataag tgctaaacat acatatacgg aacttgaaag ctttggttag ccttgcctta 180
   ggtaatcagc ctagtttaca ctgtttccag ggagtagttg aattactata aaccattagc 240
   cacttgtctc tgcaccattt atcacaccag gacagggtct ctcaacctgg gcgctactgt 300
   catttggggc caggtgattc ttccttgcaa gggctgtcct gtacctgccc gggcggccgc 360
   tcgaagcgtg gtcgcggccg aggtactgaa aggaccaagg agctctggct gccctcagga 420
   attccaaatg accgaaggaa caaagcttca gggctctggg tggtgtctcc cactattcag 480
   gaggtggtcg gaggtaacgc agcttcattt cgtccagtcc tttccagtat ttaaagttgt 540
   tgtcaagatg ctgcattaaa tcaggcaggt ctacaaaggc atcccaagca tcaaacatgt 600
   ctgtgatgaa gtaatcaatg aaacaccgga acctccgacc acctcctgaa tagtgggaga 660
   cacacccaga gcctgaagtt tgtccttcg
<210> 27
   <211> 471
   <212> DNA
   <213> Human
<400> 27
   tcccagcggc atgaagtttg agattggcca ggccctgtac ctgggcttca tctccttcgt 60
   ccctctcgct cattggtggc accctgcttt gcctgtcctg ccaggacgag gcaccctaca 120
   agccctaacc caggccccgc ccagggccac cacgaccact gcaaacaccg cacctgccta 180
   ccagccacca gctgcctaca aagacaatcg ggccccctca gtgacctcgg ccaccacagc 240
   gggtacaggc tgaacgacta cgtgtgagtc cccacagcct gcttctcccc tgggctgctg 300
   tgggctggtt cccggcggga ctgtcaatgg aggcaggggt tccagcacaa agtttacttc 360
   tgggcaattt ttgtatccaa ggaaataatg tgaatgcgag gaaatgtctt tagagcacag 420
   ggacagaggg ggaaataaga ggaggagaaa gctctctata ccaaagactg a
<210> 28
   <211> 929
   <212> DNA
   <213> Human
<400> 28
   ggtgaactca gtgcattggg ccaatggttc gacacaggct ctgccagcca caaccatcct 60
   gctgcttctg acggtttggc tgctggtggg ctttcccctc actgtcattg gaggcatctt 120
   tgggaagaac aacgccagcc cctttgatgc accctgtcgc accaagaaca tcgcccggga 180
   gattccaccc cagccctggt acaagtctac tgtcatccac atgactgttg gaggcttcct 240
   gcctttcagt gccatctctg tggagctgta ctacatcttt gccacagtat ggggtcggga 300
   gcagtacact ttgtacggca tcctcttctt tgtcttcgcc atcctgctga gtgtgggggc 360
   ttgcatctcc attgcactca cctacttcca gttgtctggg gaggattacc gctggtggtg 420
   gcgatctgtg ctgagtgttg gctccaccgg cctcttcatc ttcctctact cagttttcta 480
   ttatgcccgg cgctccaaca tgtctggggc agtacagaca gtagagttct tcggctactc 540
   cttactcact ggttatgtct tcttcctcat gctgggcacc atctcctttt tttcttccct 600
   aaagttcatc cggtatatct atgttaacct caagatggac tgagttctgt atggcagaac 660
   tattgctgtt ctctcccttt cttcatgccc tgttgaactc tcctaccagc ttctcttctg 720
   attgactgaa ttgtgtgatg gcattgttgc cttccctttt tccctttggg cattccttcc 780
   ccagagaggg cctggaaatt ataaatctct atcacataag gattatatat ttgaactttt 840
   taagttgcct ttagttttgg tcctgatttt tctttttaca attaccaaaa taaaatttat 900
   taagaaaaag aaaaaaaaaa aaaaaaaaa
<210> 29
   <211> 1775
   <212> DNA
   <213> Human
<400> 29
   gaacgtgatg ggaactttgg gaggatgtct gagaaaatgt ccgaagggat tttggccaac 60
   accagaaaac gccaatgtcc taggaattcc ctcccaaaat gcttcccaaa aaattactca 120
   ttgacaattc aaattgcact tggctggcgg cagcccgggc ggccttcagt ccgtgtgggg 180
   cgcccgcgtg gccttctcct cgtaggactc cccaaactcg ttcactctgc gtttatccac 240
   aggataaagc caccgctggt acaggtagac cagaaacacc acgtcgtccc ggaagcaggc 300
   cagccggtga gacgtgggca tggtgatgat gaaggcaaag acgtcatcaa tgaaggtgtt 360
   gaaagccttg taggtgaagg ccttccaggg cagatgtgcc actgacttca acttgtagtt 420
   cacaaagagc tggggcagca tgaagaggaa accaaaggca tagaccccgt tgacgaagct 480
   gttgattaac caggagtacc agctcttata tttgatattc aggagtgaat agacagcacc 540
   cccgacacag agagggtaca gcaggtatga caagtacttc atggcctgag tatcgtactc 600
   ctcggttttc ctctcagatt cgctgtaagt gccaaactga aattcgggca tcaggcctct 660
   ccaaaaaata gtcatcttca atgccttctt cactttccac agctcaatgg cggctccaac 720
   acccgccggg accagcacca gcaggctcgt ctgctcgtcc agcaggaaca gaaagatgac 780
   cacggtgctg aagcagcgcc agagcactgc cttggtggac atgccgatca tgctcttctt 840
   cttcttccag aaactgatgt catttttaaa ggccaggaaa tcaaagagaa gatggaacgc 900
   tgcgacaaag aaggtcagcg ccaggaagta taagttggta tctacaaaaa ttcctttcac 960
   ctcatcagca tctttctctg aaaacccgaa ctgctgcagg gagtacacgg cgtcctgcat 1020
   gtggatccag aagcgcagcc gccccagtga gaccttgtcg taggacacgg tgaggggcag 1080
   ctcggtggtg gagcggttta tgaccatcag gtccttcacg cggttgctga gctggtcgat 1140
   gaacaggatg ggcaggtaat gcacggtttt ccccagctgg atcatcttca tgtaccgatg 1200
   cacatcggca ggcagggagg acccgtcaaa gacaaagttg tccgccatca cgttcagcgc 1260
   cagccgcggt cgccagtggg acactggctc atccagggca ctcgtcggct tcttctccgc 1320
   ctcgatctgc tgtgtatcag actccccggt gagcaggttg atttcttctg gcttggggac 1380
   catgtaggtg gtcagaggac tgaccaggtg cacctgcttc ccgtcgtgcc acggcaggac 1440
   cccagcgtga tggaggaaga tgtaggcata cagcgtccca ttgtttctcg ttttctttgg 1500
   tacagaaaca ttaactgtcc tttcaaattt ggactccaca tcaaagtctt ccacattcaa 1560
   gaccaggtcg atgttgttct cagcacccag gtgggacctc gtcgtggtgt acacgctcag 1620
   ctgcagcttg ggccgccgcg ccaggtaggg ctggatgcag ttggcgtcgc cggagcacgg 1680
   gcgggtgtag acgatgccgt acatgaccca gcaggtgtgc accacgtaga ccacgaacac 1740
   gcccaccacc aagctggtga aggagctgcg gcccc
<210> 30
   <211> 1546
   <212> DNA
   <213> Human
<400> 30
   aaaataagta ggaatgggca gtgggtattc acattcacta caccttttcc atttgctaat 60
   aaggccctgc caggctggga gggaattgtc cctgcctgct tctggagaaa gaagatattg 120
   acaccatcta cgggcaccat ggaactgctt caagtgacca ttctttttct tctgcccagt 180
   atttgcagca gtaacagcac aggtgtttta gaggcagcta ataattcact tgttgttact 240
   acaacaaaac catctataac aacaccaaac acagaatcat tacagaaaaa tgttgtcaca 300
   ccaacaactg gaacaactcc taaaggaaca atcaccaatg aattacttaa aatgtctctg 360
   atgtcaacag ctactttttt aacaagtaaa gatgaaggat tgaaagccac aaccactgat 420
   gtcaggaaga atgactccat catttcaaac gtaacagtaa caagtgttac acttccaaat 480
   gctgtttcaa cattacaaag ttccaaaccc aagactgaaa ctcagagttc aattaaaaca 540
   acagaaatac caggtagtgt tctacaacca gatgcatcac cttctaaaac tggtacatta 600
   acctcaatac cagttacaat tccagaaaac acctcacagt ctcaagtaat aggcactgag 660
   ggtggaaaaa atgcaagcac ttcagcaacc agccggtctt attccagtat tattttgccg 720
   gtggttattg ctttgattgt aataacactt tcagtatttg ttctggtggg tttgtaccga 780
   atgtgctgga aggcagatcc gggcacacca gaaaatggaa atgatcaacc tcagtctgat 840
   aaagagagcg tgaagcttct taccgttaag acaatttctc atgagtctgg tgagcactct 900
   gcacaaggaa aaaccaagaa ctgacagctt gaggaattct ctccacacct aggcaataat 960
   tacgcttaat cttcagcttc tatgcaccaa gcgtggaaaa ggagaaagtc ctgcagaatc 1020
   aatcccgact tccatacctg ctgctggact gtaccagacg tctgtcccag taaagtgatg 1080
   tccagctgac atgcaataat ttgatggaat caaaaagaac cccggggctc tcctgttctc 1140
   tcacatttaa aaattccatt actccattta caggagcgtt cctaggaaaa ggaattttag 1200
   gaggagaatt tgtgagcagt gaatctgaca gcccaggagg tgggctcgct gataggcatg 1260
   actttcctta atgtttaaag ttttccgggc caagaatttt tatccatgaa gactttccta 1320
   cttttctcgg tgttcttata ttacctactg ttagtattta ttgtttacca ctatgttaat 1380
   gcagggaaaa gttgcacgtg tattattaaa tattaggtag aaatcatacc atgctacttt 1440
   gtacatataa gtattttatt cctgctttcg tgttactttt aataaataac tactgtactc 1500
   aatactctaa aaatactata acatgactgt gaaaatggca aaaaaa
<210> 31
   <211> 750
   <212> DNA
   <213> Human
<400> 31
   cacttgggca cccccatttt ctaaaaaaat ggaaatctgg agggcaaaaa aggtgtgctg 60
   aagggaagtg cctctgatgg cccaaaaacc ttcttccaaa ctagtgtagg aatggaatgg 120
   atagcaaatg gatccttttt ggcctccttt ggagcatgcc ttccctatct tatccttggc 180
   cccactaaag cagaacgtta cggatatttc tgtttttgcc attggatgcc tatctggcca 240
   aacagccttt ccctaattgg aaaatgcagt cctgtttaaa acctttgatt tacgactact 300
   tgtacatgct tgctcattac aattttgaca ttttttacat agtgaagacc ccaaacatat 360
   cagtgaaaca tgacaagatc ataaagaaca gtatcatatt attatttagt cgcttttaca 420
   gtggcaagcc aattttgaaa tatctcattt aaaactcaga cccaattcac tgagttatac 480
   ttttaatagc ttcctcagca cactatttcc catgcattaa atatgataaa ataatctatc 540
   actgcccatc ggtcttgtaa aaaggaagtc tgaatacaga gcccacaaca ctaaaattgt 600
   ttttctagct acaaagtata gcatcatcaa cacagacacg atttggactc cctgacaggt 660
   ggattggaaa acggtgttta aagagaagag aacattttaa cataaatgtc attaagaatc 720
   ccaaaggcct tatttgtcac caccgtcccg
<210> 32
   <211> 1620
   <212> DNA
   <213> Human
<400> 32
   gcaattcccc cctcccacta aacgactccc agtaattatg tttacaaccc attggatgca 60
   gtgcagccat tcataagaac cttggtgccc cagaaaaatc tgtccttttt ggtaccaaac 120
   ctgaggtctt ttggaagata atgtagaaaa ccactaccta ttgaaggcct gttttggcta 180
   atctgtgcaa actctgatga tacctgcctt atgtggattc ttttccacac tgctttcatt 240
   tttaagtata aagacttaga aaactagaat aatgctttta caaataatta aaagtatgtg 300
   atgttctggg ttttttcctt ctttttagaa ccccgcctcc atttaaaaaa ttaaaaaaaa 360
   aaaaaaaact tttaacattt aaaaaataaa aattaacaaa atttcactta ttccaggaca 420
   cgctggcatt tggactcaat gaaaagggca cctaaagaaa ataaggctga ctgaatgttt 480
   tccataattt tcacacaata acagtccctt tctatccagc ttgccttcca tttatctcta 540
   gggttagctt ttcaggcaac atccttggtc attgcccaga aagtacctga gctatcagtg 600
   attggaatgg cacaggaaac cgaatcacat gggtgccctc cccttggttt tcaagtatct 660
   tggagttgtg cacaaaaatt aggtcatgcc ttcagtgtct tgttctttaa acctaccctt 720
   tgacaatcag gtgctaatga ttgtatacta ttaaaaccag cacataagta ttgtaaatgt 780
   gtgttcctcc taggttggaa gaaatgtctt tccttctatc tgggtcctgt taaagcgggt 840
   gtcagttgtg tcttttcacc tcgatttgtg aattaataga attgggggga gaggaaatga 900
   tgatgtcaat taagtttcag gtttggcatg atcatcattc tcgatgatat tctcactttg 960
   tcgcaaatct gcccttatcg taagaacaag tttcagaatt ttccctccac tatacgactc 1020
   cagtattatg tttacaatcc attggatgag tgcagcatta taagaccttg gtgcccagaa 1080
   aaatctgtcc tttttggtac caaacctgag gtcttttgga agataatgta gaaaaccact 1140
   acctattgaa ggcctgtttt ggctaatctg tgcaaactct gatgatacct gcttatgtgg 1200
   attcttttcc acactgcttt catttttaag tataaagact tagaaaacta gaataatgct 1260
   tttacaaata attaaaagta tgtgatgttc tgggtttttt ccttcttttt agaaccctgt 1320
   atttaaacaa gccttctttt taagtcttgt ttgaaattta agtctcagat cttctggata 1380
   ccaaatcaaa aacccaacgc gtaaaacagg gcagtatttg tgttcctaat tttaaaaagc 1440
   tttatgtata ctctataaat atagatgcat aaacaacact tccccttgag tagcacatca 1500
   acatacagca ttgtacatta caatgaaaat gtgtaactta agggtattat atatataaat 1560
   acatatatac ctttgtaacc tttatactgt aaataaaaaa gttgctttag tcaaaaaaaa 1620
<210> 33
   <211> 2968
   <212> DNA
   <213> Human
<400> 33
   gaaaaagtag aaggaaacac agttcatata gaagtaaaag aaaaccctga agaggaggag 60
   gaggaggaag aagaggaaga agaagatgaa gaaagtgaag aggaggagga agaggaggga 120
   gaaagtgaag gcagtgaagg tgatgaggaa gatgaaaagg tgtcagatga gaaggattca 180
   gggaagacat tagataaaaa gccaagtaaa gaaatgagct cagattctga atatgactct 240
   gatgatgatc ggactaaaga agaaagggct tatgacaaag caaaacggag gattgagaaa 300
   cggcgacttg aacatagtaa aaatgtaaac accgaaaagc taagagcccc tattatctgc 360
   gtacttgggc atgtggacac agggaagaca aaaattctag ataagctccg tcacacacat 420
   gtacaagatg gtgaagcagg tggtatcaca caacaaattg gggccaccaa tgttcctctt 480
   gaagctatta atgaacagac taagatgatt aaaaattttg atagagagaa tgtacggatt 540
   ccaggaatgc taattattga tactcctggg catgaatctt tcagtaatct gagaaataga 600
   ggaagctctc tttgtgacat tgccatttta gttgttgata ttatgcatgg tttggagccc 660
   cagacaattg agtctatcaa ccttctcaaa tctaaaaaat gtcccttcat tgttgcactc 720
   aataagattg ataggttata tgattggaaa aagagtcctg actctgatgt ggctgctact 780
   ttaaagaagc agaaaaagaa tacaaaagat gaatttgagg agcgagcaaa ggctattatt 840
   gtagaatttg cacagcaggg tttgaatgct gctttgtttt atgagaataa agatccccgc 900
   acttttgtgt ctttggtacc tacctctgca catactggtg atggcatggg aagtctgatc 960
   taccttcttg tagagttaac tcagaccatg ttgagcaaga gacttgcaca ctgtgaagag 1020
   ctgagagcac aggtgatgga ggttaaagct ctcccgggga tgggcaccac tatagatgtc 1080
   atcttgatca atgggcgttt gaaggaagga gatacaatca ttgttcctgg agtagaaggg 1140
   cccattgtaa ctcagattcg aggcctcctg ttacctcctc ctatgaagga attacgagtg 1200
   aagaaccagt atgaaaagca taaagaagta gaagcagctc agggggtaaa gattcttgga 1260
   aaagacctgg agaaaacatt ggctggttta cccctccttg tggcttataa agaagatgaa 1320
   atccctgttc ttaaagatga attgatccat gagttaaagc agacactaaa tgctatcaaa 1380
   ttagaagaaa aaggagtcta tgtccaggca tctacactgg gttctttgga agctctactg 1440
   gaatttctga aaacatcaga agtgccctat gcaggaatta acattggccc agtgcataaa 1500
   aaagatgtta tgaaggcttc agtgatgttg gaacatgacc ctcagtatgc agtaattttg 1560
   gccttcgatg tgagaattga acgagatgca caagaaatgg ctgatagttt aggagttaga 1620
   atttttagtg cagaaattat ttatcattta tttgatgcct ttacaaaata tagacaagac 1680
   tacaagaaac agaaacaaga agaatttaag cacatagcag tatttccctg caagataaaa 1740
   atcctccctc agtacatttt taattctcga gatccgatag tgatgggggt gacggtggaa 1800
   gcaggtcagg tgaaacaggg gacacccatg tgtgtcccaa gcaaaaattt tgttgacatc 1860
   ggaatagtaa caagtattga aataaaccat aaacaagtgg atgttgcaaa aaaaggacaa 1920
   gaagtttgtg taaaaataga acctatccct ggtgagtcac ccaaaatgtt tggaagacat 1980
   tttgaagcta cagatattct tgttagtaag atcagccggc agtccattga tgcactcaaa 2040
   gactggttca gagatgaaat gcagaagagt gactggcagc ttattgtgga gctgaagaaa 2100
   gtatttgaaa tcatctaatt ttttcacatg gagcaggaac tggagtaaat gcaatactgt 2160
   gttgtaatat cccaacaaaa atcagacaaa aaatggaaca gacgtatttg gacactgatg 2220
   gacttaagta tggaaggaag aaaaataggt gtataaaatg ttttccatga gaaaccaaga 2280
   aacttacact ggtttgacag tggtcagtta catgtcccca cagttccaat gtgcctgttc 2340
   actcacctct cccttcccca acccttctct acttggctgc tgttttaaag tttgcccttc 2400
   cccaaatttg gatttttatt acagatctaa agctctttcg attttatact gattaaatca 2460
   gtactgcagt atttgattaa aaaaaaaaaa gcagattttg tgattcttgg gacttttttg 2520
   acgtaagaaa tacttcttta tttatgcata ttcttcccac agtgattttt ccagcattct 2580
   tctgccatat gcctttaggg cttttataaa atagaaaatt aggcattctg atatttcttt 2640
   agctgctttg tgtgaaacca tggtgtaaaa gcacagctgg ctgcttttta ctgcttgtgt 2700
   agtcacgagt ccattgtaat catcacaatt ctaaaccaaa ctaccaataa agaaaacaga 2760
   catccaccag taagcaagct ctgttaggct tccatggtta gtggtagctt ctctcccaca 2820
   agttgtcctc ctaggacaag gaattatctt aacaaactaa actatccatc acactacctt 2880
   ggtatgccag cacctgggta acagtaggag attttataca ttaatctgat ctgtttaatc 2940
   tgatcggttt agtagagatt ttatacat
<210> 34
   <211> 6011
   <212> DNA
   <213> Human
<400> 34
   acggggcgcc ggacgacccg cacatcttat cctccacgcc ccactcgcac tcggagcggg 60
   accgccccgg actccccctc gggccggcca ctcgaggagt gaggagagag gccgccggcc 120
   cggcttgagc cgagcgcagc accccccgcg ccccgcgcca gaagtttggt tgaaccgggc 180
   tgccgggaga aacttttttc ttttttcccc ctctcccggg agagtctctg gaggaggagg 240
   ggaactcccc cggcccaagg ctcgtgggct cggggtcgcg cggccgcaga aggggcgggg 300
   tccgcccgcg aggggaggcg cccccgggga cccgagaggg gggtgaggac cgcgggctgc 360
   tggtgcggcg gcggcagcgt gtgccccgcg caggggaggc gccgccccgc tcccggcccg 420
   gctgcgagga ggaggcggcg gcggcgcagg aggatgtact tggtggcggg ggacaggggg 480
   ttggccggct gcgggcacct cctggtctcg ctgctggggc tgctgctgct gccggcgcgc 540
   tccggcaccc gggcgctggt ctgcctgccc tgtgacgagt ccaagtgcga ggagcccagg 600
   aaccgcccgg ggagcatcgt gcagggcgtc tgcggctgct gctacacgtg cgccagccag 660
   gggaacgaga gctgcggcgg caccttcggg atttacggaa cctgcgaccg gggctgcgtt 720
   tgtgtcatcc gccccccgct caatggcgac tccctcaccg agtacgaagc gggcgtttgc 780
   gaagatgaga actggactga tgaccaactg cttggtttta aaccatgcaa tgaaaacctt 840
   attgctggct gcaatataat caatgggaaa tgtgaatgta acaccattcg aacctgcagc 900
   aatccctttg agtttccaag tcaggatatg tgcctttcag ctttaaagag aattgaagaa 960
   gagaagccag attgctccaa ggcccgctgt gaagtccagt tctctccacg ttgtcctgaa 1020
   gattctgttc tgatcgaggg ttatgctcct cctggggagt gctgtccctt acccagccgc 1080
   tgcgtgtgca accccgcagg ctgtctgcgc aaagtctgcc agccgggaaa cctgaacata 1140
   ctagtgtcaa aagcctcagg gaagccggga gagtgctgtg acctctatga gtgcaaacca 1200
   gttttcggcg tggactgcag gactgtggaa tgccctactg ttcagcagac cgcgtgtccc 1260
   ccggacagct atgaaactca agtcagacta actgcagatg gttgctgtac tttgccaaca 1320
   agatgcgagt gtctctctgg cttatgtggt ttccccgtgt gtgaggtggg atccactccc 1380
   cgcatagtct ctcgtggcga tgggacacct ggaaagtgct gtgatgtctt tgaatgtgtt 1440
   aatgatacaa agccagcctg cgtatttaac aatgtggaat attatgatgg agacatgttt 1500
   cgaatggaca actgtcggtt ctgtcgatgc caagggggcg ttgccatctg cttcaccgcc 1560
   cagtgtggtg agataaactg cgagaggtac tacgtgcccg aaggagagtg ctgcccagtg 1620
   tgtgaagatc cagtgtatcc ttttaataat cccgctggct gctatgccaa tggcctgatc 1680
   cttgcccacg gagaccggtg gcgggaagac gactgcacat tctgccagtg cgtcaacggt 1740
   gaacgccact gcgttgcgac cgtctgcgga cagacctgca caaaccctgt gaaagtgcct 1800
   ggggagtgtt gccctgtgtg cgaagaacca accatcatca cagttgatcc acctgcatgt 1860
   ggggagttat caaactgcac tctgacacgg aaggactgca ttaatggttt caaacgcgat 1920
   cacaatggtt gtcggacctg tcagtgcata aacacccagg aactatgttc agaacgtaaa 1980
   caaggctgca ccttgaactg tcccttcggt ttccttactg atgcccaaaa ctgtgagatc 2040
   tgtgagtgcc gcccaaggcc caagaagtgc agacccataa tctgtgacaa gtattgtcca 2100
   cttggattgc tgaagaataa gcacggctgt gacatctgtc gctgtaagaa atgtccagag 2160
   ctctcatgca gtaagatctg ccccttgggt ttccagcagg acagtcacgg ctgtcttatc 2220
   tgcaagtgca gagaggcctc tgcttcagct gggccaccca tcctgtcggg cacttgtctc 2280
   accgtggatg gtcatcatca taaaaatgag gagagctggc acgatgggtg ccgggaatgc 2340
   tactgtctca atggacggga aatgtgtgcc ctgatcacct gcccggtgcc tgcctgtggc 2400
   aaccccacca ttcaccctgg acagtgctgc ccatcatgtg cagatgactt tgtggtgcag 2460
   aagccagagc tcagtactcc ctccatttgc cacgcccctg gaggagaata ctttgtggaa 2520
   ggagaaacgt ggaacattga ctcctgtact cagtgcacct gccacagcgg acgggtgctg 2580
   tgtgagacag aggtgtgccc accgctgctc tgccagaacc cctcacgcac ccaggattcc 2640
   tgctgcccac agtgtacaga tcaacctttt cggccttcct tgtcccgcaa taacagcgta 2700
   cctaattact gcaaaaatga tgaaggggat atattcctgg cagctgagtc ctggaagcct 2760
   gacgtttgta ccagctgcat ctgcattgat agcgtaatta gctgtttctc tgagtcctgc 2820
   ccttctgtat cctgtgaaag acctgtcttg agaaaaggcc agtgttgtcc ctactgcata 2880
   aaagacacaa ttccaaagaa ggtggtgtgc cacttcagtg ggaaggccta tgccgacgag 2940
   gagcggtggg accttgacag ctgcacccac tgctactgcc tgcagggcca gaccctctgc 3000
   tcgaccgtca gctgcccccc tctgccctgt gttgagccca tcaacgtgga aggaagttgc 3060
   tgcccaatgt gtccagaaat gtatgtccca gaaccaacca atatacccat tgagaagaca 3120
   aaccatcgag gagaggttga cctggaggtt cccctgtggc ccacgcctag tgaaaatgat 3180
   atcgtccatc tccctagaga tatgggtcac ctccaggtag attacagaga taacaggctg 3240
   cacccaagtg aagattcttc actggactcc attgcctcag ttgtggttcc cataattata 3300
   tgcctctcta ttataatagc attcctattc atcaatcaga agaaacagtg gataccactg 3360
   ctttgctggt atcgaacacc aactaagcct tcttccttaa ataatcagct agtatctgtg 3420
   gactgcaaga aaggaaccag agtccaggtg gacagttccc agagaatgct aagaattgca 3480
   gaaccagatg caagattcag tggcttctac agcatgcaaa aacagaacca tctacaggca 3540
   gacaatttct accaaacagt gtgaagaaag gcaactagga tgaggtttca aaagacggaa 3600
   gacgactaaa tctgctctaa aaagtaaact agaatttgtg cacttgctta gtggattgta 3660
   ttggattgtg acttgatgta cagcgctaag accttactgg gatgggctct gtctacagca 3720
   atgtgcagaa caagcattcc cacttttcct caagataact gaccaagtgt tttcttagaa 3780
   ccaaagtttt taaagttgct aagatatatt tgcctgtaag atagctgtag agatatttgg 3840
   ggtggggaca gtgagtttgg atggggaaag gggtgggagg gtggtgttgg gaagaaaaat 3900
   tggtcagctt ggctcgggga gaaacctggt aacataaaag cagttcagtg gcccagaggt 3960
   tatttttttc ctattgctct gaagactgca ctggttgctg caaagctcag gcctgaatga 4020
   gcaggaaaca aaaaaggcct tgcgacccag ctgccataac caccttagaa ctaccagacg 4080
   agcacatcag aaccctttga cagccatccc aggtctaaag ccacaagttt cttttctata 4140
   cagtcacaac tgcagtaggc agtgaggaag ccagagaaat gcgatagcgg catttctcta 4200
   aagcgggtta ttaaggatat atacagttac actttttgct gcttttattt tcttccaagc 4260
   caatcaatca gccagttcct agcagagtca gcacatgaac aagatctaag tcatttcttg 4320
   atgtgagcac tggagctttt tttttttaca acgtgacagg aagaggaggg agagggtgac 4380
   gaacaccagg catttccagg ggctatattt cactgtttgt tgttgctttg ttctgttata 4440
   ttgttggttg ttcatagttt ttgttgaagc tctagcttaa gaagaaactt tttttaaaaa 4500
   gactgtttgg ggattctttt tccttattat atactgattc tacaaaatag aaactacttc 4560
   attttaattg tatattattc aagcaccttt gttgaagctc aaaaaaaatg atgcctcttt 4620
   aaactttagc aattatagga gtatttatgt aactatctta tgcttcaaaa aacaaaagta 4680
   tttgtgtgca tgtgtatata atatatatat atacatatat atttatacac atacaattta 4740
   tgttttcctg ttgaatgtat ttttatgaga ttttaaccag aacaaaggca gataaacagg 4800
   cattccatag cagtgctttt gatcacttac aaattttttg aataacacaa aatctcattc 4860
   tacctgcagt ttaattggaa agatgtgtgt gtgagagtat gtatgtgtgt gtgtgtgtgt 4920
   gtgtgtgcgc gcgcacgcac gccttgagca gtcagcattg cacctgctat ggagaagggt 4980
   attcctttat taaaatcttc ctcatttgga tttgctttca gttggttttc aatttgctca 5040
   ctggccagag acattgatgg cagttcttat ctgcatcact aatcagctcc tggatttttt 5100
   tttttttttt tcaaacaatg gtttgaaaca actactggaa tattgtccac aataagctgg 5160
   aagtttgttg tagtatgcct caaatataac tgactgtata ctatagtggt aacttttcaa 5220
   acagccctta gcacttttat actaattaac ccatttgtgc attgagtttt cttttaaaaa 5280
   tgcttgttgt gaaagacaca gatacccagt atgcttaacg tgaaaagaaa atgtgttctg 5340
   ttttgtaaag gaactttcaa gtattgttgt aaatacttgg acagaggttg ctgaacttta 5400
   aaaaaaatta atttattatt ataatgacct aatttattaa tctgaagatt aaccattttt 5460
   ttgtcttaga atatcaaaaa gaaaaagaaa aaggtgttct agctgtttgc atcaaaggaa 5520
   aaaaagattt attatcaagg ggcaatattt ttatcttttc caaaataaat ttgttaatga 5580
   tacattacaa aaatagattg acatcagcct gattagtata aattttgttg gtaattaatc 5640
   cattcctggc ataaaaagtc tttatcaaaa aaaattgtag atgcttgctt tttgtttttt 5700
   caatcatggc catattatga aaatactaac aggatatagg acaaggtgta aattttttta 5760
   ttattatttt aaagatatga tttatcctga gtgctgtatc tattactctt ttactttggt 5820
   tcctgttgtg ctcttgtaaa agaaaaatat aatttcctga agaataaaat agatatatgg 5880
   cacttggagt gcatcatagt tctacagttt gtttttgttt tcttcaaaaa agctgtaaga 5940
   gaattatctg caacttgatt cttggcagga aataaacatt ttgagttgaa atcaaaaaaa 6000
   aaaaaaaaaa a
<210> 34a
   <211> 1036
   <212> DNA
   <213> Human
<400> 34a
<210> 35
   <211> 716
   <212> DNA
   <213> Human
<400> 35
<210> 36
   <211> 395
   <212> DNA
   <213> Human
<400> 36
<210> 37
   <211> 134
   <212> DNA
   <213> Human
<400> 37
   ccctcgagcg gccgcccggg caggtacttt taccaccgaa ttgttcactt gactttaaga 60
   aacccataaa gctgcctggc tttcagcaac aggcctatca acaccatggt gagtctccat 120
   aagggacacc gtgt
<210> 38
   <211> 644
   <212> DNA
   <213> Human
<400> 38
   aagcctgttg tcatggggga ggtggtggcg cttggtggcc actggcggcc gaggtagagg 60
   cagtggcgct tgagttggtc gggggcagcg gcagatttga ggcttaagca acttcttccg 120
   gggaagagtg ccagtgcagc cactgttaca attcaagatc ttgatctata tccatagatt 180
   ggaatattgg tgggccagca atcctcagac gcctcactta ggacaaatga ggaaactgag 240
   gcttggtgaa gttacgaaac ttgtccaaaa tcacacaact tgtaaagggc acagccaaga 300
   ttcagagcca ggctgtaaaa attaaaatga acaaattacg gcaaagtttt aggagaaaga 360
   aggatgttta tgttccagag gccagtcgtc cacatcagtg gcagacagat gaagaaggcg 420
   ttcgcaccgg aaaatgtagc ttcccggtta agtaccttgg ccatgtagaa gttgatgaat 480
   caagaggaat gcacatctgt gaagatgctg taaaaagatt gaaagctgaa aggaagttct 540
   tcaaaggctt ctttggaaaa actggaaaga aagcagttaa agcagtttct gtgggtctaa 600
   gcagatggac tcagaggttg tggatgaaaa actaaggacc tcat
<210> 39
   <211> 657
   <212> DNA
   <213> Human
<400> 39
   ctttttgttt gggttttcca atgtagatgt ctcagtgaaa tgtgcagata tactttgttc 60
   cttatatggt caccagtgtt aattatggac aaatacatta aaacaagggt tcctggccca 120
   gcctcccatc taatctcttt gatactcttg gaatctaagt ctgaggagcg atttctgaat 180
   tagccagtgt tgtaccaact ttctgttagg aattgtatta gaataacctt tctttttcag 240
   acctgctcag tgagacatct tggggaatga agtaggaaaa tagacatttg gtggaaaaac 300
   agcaaaatga gaacattaaa aagactcatt caagtatgag tataaagggc atggaaattc 360
   tggtcctttg agcaaaatga gaagaaaaaa ttctgctcag cagtattcac tgtgttaaga 420
   ttttttgttt tttacacgaa tggaaaaatg atgtgtaagt ggtatagatt ttaatcagct 480
   aacagtcact ccagagattt tgatcagcac caattcctat agtagtaagt atttaaaagt 540
   taagaaatac tactacattt aacattataa agtagagttc tggacataac tgaaaattag 600
   atgtttgctt caatagaaat ttgttcccac ttgtattttc aacaaaatta tcggaac
<210> 40
   <211> 1328
   <212> DNA
   <213> Human
<400> 40
   acaattttaa aataactagc aattaatcac agcatatcag gaaaaagtac acagtgagtt 60
   ctggttagtt tttgtaggct cattatggtt agggtcgtta agatgtatat aagaacctac 120
   ctatcatgct gtatgtatca ctcattccat tttcatgttc catgcatact cgggcatcat 180
   gctaatatgt atccttttaa gcactctcaa ggaaacaaaa gggcctttta tttttataaa 240
   ggtaaaaaaa attccccaaa tattttgcac tgaatgtacc aaaggtgaag ggacattaca 300
   atatgactaa cagcaactcc atcacttgag aagtataata gaaaatagct tctaaatcaa 360
   acttccttca cagtgccgtg tctaccacta caaggactgt gcatctaagt aataattttt 420
   taagattcac tatatgtgat agtatgatat gcatttattt aaaatgcatt agactctctt 480
   ccatccatca aatactttac aggatggcat ttaatacaga tatttcgtat ttcccccact 540
   gctttttatt tgtacagcat cattaaacac taagctcagt taaggagcca tcagcaacac 600
   tgaagagatc agtagtaaga attccatttt ccctcatcag tgaagacacc acaaattgaa 660
   actcagaact atatttctaa gcctgcattt tcactgatgc ataattttct tagtaatatt 720
   aagagacagt ttttctatgg catctccaaa actgcatgac atcactagtc ttacttctgc 780
   ttaattttat gagaaggtat tcttcatttt aattgctttt gggattactc cacatctttg 840
   tttatttctt gactaatcag attttcaata gagtgaagtt aaattggggg tcataaaagc 900
   attggattga catatggttt gccagcctat gggtttacag gcattgccca aacatttctt 960
   tgagatctat atttataagc agccatggaa ttcctattat gggatgttgg caatcttaca 1020
   ttttatagag gtcatatgca tagttttcat aggtgttttg taagaactga ttgctctcct 1080
   gtgagttaag ctatgtttac tactgggacc ctcaagagga ataccactta tgttacactc 1140
   ctgcactaaa ggcacgtact gcagtgtgaa gaaatgttct gaaaaagggt tatagaaatc 1200
   tggaaataag aaaggaagag ctctctgtat tctataattg gaagagaaaa aaagaaaaac 1260
   ttttaactgg aaatgttagt ttgtacttat tgatcatgaa tacaagtata tatttaattt 1320
   tgaaaaaa
<210> 41
   <211> 987
   <212> DNA
   <213> Human
<400> 41
   aacagagact ggcacaggac ctcttcattg caggaagatg gtagtgtagg caggtaacat 60
   tgagctcttt tcaaaaaagg agagctcttc ttcaagataa ggaagtggta gttatggtgg 120
   taacccccgg ctatcagtcc ggatggttgc cacccctcct gctgtaggat ggaagcagcc 180
   atggagtggg agggaggcgc aataagacac ccctccacag agcttggcat catgggaagc 240
   tggttctacc tcttcctggc tcctttgttt aaaggcctgg ctgggagcct tccttttggg 300
   tgtctttctc ttctccaacc aacagaaaag actgctcttc aaaggtggag ggtcttcatg 360
   aaacacagct gccaggagcc caggcacagg gctgggggcc tggaaaaagg agggcacaca 420
   ggaggaggga ggagctggta gggagatgct ggctttacct aaggtctcga aacaaggagg 480
   gcagaatagg cagaggcctc tccgtcccag gcccattttt gacagatggc gggacggaaa 540
   tgcaatagac cagcctgcaa gaaagacatg tgttttgatg acaggcagtg tggccgggtg 600
   gaacaagcac aggccttgga atccaatgga ctgaatcaga accctaggcc tgccatctgt 660
   cagccgggtg acctgggtca attttagcct ctaaaagcct cagtctcctt atctgcaaaa 720
   tgaggcttgt gatacctgtt ttgaagggtt gctgagaaaa ttaaagataa gggtatccaa 780
   aatagtctac ggccatacca ccctgaacgt gcctaatctc gtaagctaag cagggtcagg 840
   cctggttagt acctggatgg ggagagtatg gaaaacatac ctgcccgcag ttggagttgg 900
   actctgtctt aacagtagcg tggcacacag aaggcactca gtaaatactt gttgaataaa 960
   tgaagtagcg atttggtgtg aaaaaaa
<210> 42
   <211> 956
   <212> DNA
   <213> Human
<400> 42
   cggacggtgg ggcggacgcg tgggtgcagg agcagggcgg ctgccgactg ccccaaccaa 60
   ggaaggagcc cctgagtccg cctgcgcctc catccatctg tccggccaga gccggcatcc 120
   ttgcctgtct aaagccttaa ctaagactcc cgccccgggc tggccctgtg cagaccttac 180
   tcaggggatg tttacctggt gctcgggaag ggaggggaag gggccgggga gggggcacgg 240
   caggcgtgtg gcagccacac gcaggcggcc agggcggcca gggacccaaa gcaggatgac 300
   cacgcacctc cacgccactg cctcccccga atgcatttgg aaccaaagtc taaactgagc 360
   tcgcagcccc cgcgccctcc ctccgcctcc catcccgctt agcgctctgg acagatggac 420
   gcaggccctg tccagccccc agtgcgctcg ttccggtccc cacagactgc cccagccaac 480
   gagattgctg gaaaccaagt caggccaggt gggcggacaa aagggccagg tgcggcctgg 540
   ggggaacgga tgctccgagg actggactgt ttttttcaca catcgttgcc gcagcggtgg 600
   gaaggaaagg cagatgtaaa tgatgtgttg gtttacaggg tatatttttg ataccttcaa 660
   tgaattaatt cagatgtttt acgcaaggaa ggacttaccc agtattactg ctgctgtgct 720
   tttgatctct gcttaccgtt caagaggcgt gtgcaggccg acagtcggtg accccatcac 780
   tcgcaggacc aagggggcgg ggactgctgg ctcacgcccc gctgtgtcct ccctcccctc 840
   ccttccttgg gcagaatgaa ttcgatgcgt attctgtggc cgccatctgc gcagggtggt 900
   ggtattctgt catttacaca cgtcgttcta attaaaaagc gaattatact ccaaaa
<210> 43
   <211> 536
   <212> DNA
   <213> Human
<400> 43
   aaataaacac ttccataaca ttttgttttc gaagtctatt aatgcaatcc cacttttttc 60
   cccctagttt ctaaatgtta aagagagggg aaaaaaggct caggatagtt ttcacctcac 120
   agtgttagct gtcttttatt ttactcttgg aaatagagac tccattaggg ttttgacatt 180
   ttgggaaccc agttttacca ttgtgtcagt aaaacaataa gatagtttga gagcatatga 240
   tctaaataaa gacatttgaa gggttagttt gaattctaaa agtaggtaat agccaaatag 300
   cattctcatc ccttaacaga caaaaactta tttgtcaaaa gaattagaaa aggtgaaaat 360
   attttttcca gatgaaactt gtgccacttc caattgacta atgaaataca aggagacaga 420
   ctggaaaaag tgggttatgc cacctttaaa accctttctg gtaaatatta tggtagctaa 480
   agggtggttt ccccggcacc tggacctgga caggtagggt tccgtggtta accagt
<210> 44
   <211> 1630
   <212> DNA
   <213> Human
<400> 44
   ggggagggac gagtatggaa ccctgaaggt agcaagtcca ggcactggcc tgaccatccg 60
   gctccctggg caccaagtcc caggcaggag cagctgtttt ccatcccttc ccagacaagc 120
   tctattttta tcacaatgac ctttagagag gtctcccagg ccagctcaag gtgtcccact 180
   atcccctctg gagggaagag gcaggaaaat tctccccggg tccctgtcat gctactttct 240
   ccatcccagt tcagactgtc caggacatct tatctgcagc cataagagaa ttataaggca 300
   gtgatttccc ttaggcccag gacttgggcc tccagctcat ctgttccttc tgggcccatt 360
   catggcaggt tctgggctca aagctgaact ggggagagaa gagatacaga gctaccatgt 420
   gactttacct gattgccctc agtttggggt tgcttattgg gaaagagaga gacaaagagt 480
   tacttgttac gggaaatatg aaaagcatgg ccaggatgca tagaggagat tctagcaggg 540
   gacaggattg gctcagatga cccctgaggg ctcttccagt cttgaaatgc attccatgat 600
   attaggaagt cgggggtggg tggtggtggt gggctagttg ggtttgaatt taggggccga 660
   tgagcttggg tacgtgagca gggtgttaag ttagggtctg cctgtatttc tggtcccctt 720
   ggaaatgtcc ccttcttcag tgtcagacct cagtcccagt gtccatatcg tgcccagaaa 780
   agtagacatt atcctgcccc atcccttccc cagtgcactc tgacctagct agtgcctggt 840
   gcccagtgac ctgggggagc ctggctgcag gccctcactg gttccctaaa ccttggtggc 900
   tgtgattcag gtccccaggg gggactcagg gaggaatatg gctgagttct gtagtttcca 960
   gagttggctg gtagagcctt ctagaggttc agaatattag cttcaggatc agctgggggt 1020
   atggaattgg ctgaggatca aacgtatgta ggtgaaagga taccaggatg ttgctaaagg 1080
   tgagggacag tttgggtttg ggacttacca gggtgatgtt agatctggaa cccccaagtg 1140
   aggctggagg gagttaaggt cagtatggaa gatagggttg ggacagggtg ctttggaatg 1200
   aaagagtgac cttagagggc tccttgggcc tcaggaatgc tcctgctgct gtgaagatga 1260
   gaaggtgctc ttactcagtt aatgatgagt gactatattt accaaagccc ctacctgctg 1320
   ctgggtccct tgtagcacag gagactgggg ctaagggccc ctcccaggga agggacacca 1380
   tcaggcctct ggctgaggca gtagcataga ggatccattt ctacctgcat ttcccagagg 1440
   actagcagga ggcagccttg agaaaccggc agttcccaag ccagcgcctg gctgttctct 1500
   cattgtcact gccctctccc caacctctcc tctaacccac tagagattgc ctgtgtcctg 1560
   cctcttgcct cttgtagaat gcagctctgg ccctcaataa atgcttcctg cattcatctg 1620
   caaaaaaaaa
<210> 45
   <211> 169
   <212> DNA
   <213> Human
<400> 45
   tcttttgctt ttagcttttt atttttgtat taacaggagt cttattacac ataggtctga 60
   taaaactggt ttatgatctt cagtctgatt ccagtgctgc ataactagat aacgtatgaa 120
   ggaaaaacga cgacgaacaa aaaagtaagt gcttggaaga cttagttga
<210> 46
   <211> 769
   <212> DNA
   <213> Human
<400> 46
   tgcaggtcat atttactatc ggcaataaaa ggaagcaaag cagtattaag cagcggtgga 60
   atttgtcgct ttcacttttt ataaagtgct acataaaatg tcatatttcc aaatttaaaa 120
   acataactcc agttcttacc atgagaacag catggtgatc acgaaggatc ttcttgaaaa 180
   aaacaaaaac aaaaacaaaa aacaatgatc tcttctgggt atcacatcaa atgagataca 240
   aaggtgtact aggcaatctt agagatctgg caacttattt tatatataag gcatctgtga 300
   ccaagagacg ttatgaatta aatgtacaaa tgtattatgt ataaatgtat taaatgcaag 360
   cttcatataa tgacaccaat gtctctaagt tgctcagaga tcttgactgg ctgtggccct 420
   ggccagctcc tttcctgata gtctgattct gccttcatat ataggcagct cctgatcatc 480
   catgccagtg aatgagaaaa caagcatgga atatataaac tttaacatta aaaaatgttt 540
   tattttgtaa taaaatcaaa tttcccattg aaaccttcaa aaactttgca gaatgaggtt 600
   ttgatatatg tgtacaagta gtaccttctt agtgcaagaa aacatcatta tttctgtctg 660
   cctgcctttt tgtttttaaa aatgaagact atcattgaaa caagtttgtc ttcagtatca 720
   ggacatgttg acggagagga aaggtaggaa agggttaggg atagaagcc
<210> 47
   <211> 2529
   <212> DNA
   <213> Human
<400> 47
   tttagttcat agtaatgtaa aaccatttgt ttaattctaa atcaaatcac tttcacaaca 60
   gtgaaaatta gtgactggtt aaggtgtgcc actgtacata tcatcatttt ctgactgggg 120
   tcaggacctg gtcctagtcc acaagggtgg caggaggagg gtggaggcta agaacacaga 180
   aaacacacaa aagaaaggaa agctgccttg gcagaaggat gaggtggtga gcttgccgag 240
   ggatggtggg aagggggctc cctgttgggg ccgagccagg agtcccaagt cagctctcct 300
   gccttactta gctcctggca gagggtgagt ggggacctac gaggttcaaa atcaaatggc 360
   atttggccag cctggcttta ctaacaggtt cccagagtgc ctctgttggc tgagctctcc 420
   tgggctcact ccatttcatt gaagagtcca aatgattcat tttcctaccc acaacttttc 480
   attattcttc tggaaaccca tttctgttga gtccatctga cttaagtcct ctctccctcc 540
   actagttggg gccactgcac tgaggggggt cccaccaatt ctctctagag aagagacact 600
   ccagaggccc ctgcaacttt gcggatttcc agaaggtgat aaaaagagca ctcttgagtg 660
   ggtgcccagg aatgtttaaa atctatcagg cacactataa agctggtggt ttcttcctac 720
   caagtggatt cggcatatga accacctact caatacttta tattttgtct gtttaaacac 780
   tgaactctgg tgttgacagg tacaaaggag aagagatggg gactgtgaag aggggagggc 840
   ttccctcatc ttcctcaaga tctttgtttc cataaactat gcagtcataa ttgagaaaaa 900
   gcaatagatg gggcttccta ccatttgttg gttattgctg gggttagcca ggagcagtgt 960
   ggatggcaaa gtaggagaga ggcccagagg aaagcccatc tccctccagc tttggggtct 1020
   ccagaaagag gctggatttc tgggatgaag cctagaaggc agagcaagaa ctgttccacc 1080
   aggtgaacag tcctacctgc ttggtaccat agtccctcaa taagattcag aggaagaagc 1140
   ttatgaaact gaaaatcaaa tcaaggtatt gggaagaata atttcccctc gattccacag 1200
   gagggaagac cacacaatat cattgtgctg gggctcccca aggccctgcc acctggcttt 1260
   acaaatcatc aggggttgcc tgcttggcag tcacatgctt ccctggtttt agcacacata 1320
   caaggagttt tcagggaact ctatcaagcc ataccaaaat cagggtcaca tgtgggtttc 1380
   ccctttcctt gcctcttcat aaaagacaac ttggcttctg aggatggtgg tcttttgcat 1440
   gcagttgggc tgacctgaca aagcccccag tttcctgtgg caggttctgg gagaggatgc 1500
   attcaagctt ctgcagccta ggggacaggg ctgcttgttc agttattact gcctcggagc 1560
   tccaaatccc accaaagtcc tgactccagg tctttcctaa tgcacagtag tcagtctcag 1620
   cttcggcagt attctcggct gtatgttctc tggcagagag aggcagatga acatagtttt 1680
   agggagaaag ctgatgggaa acctgtgagt taagccacat gtctcaccag gaataattta 1740
   tgccaggaaa ccaggaagtc attcaagttg ttctctgagg ccaaagacac tgagcacagc 1800
   ccagagccaa taaaagatct ttgagtctct ggtgaattca cgaagtgacc ccagctttag 1860
   ctactgcaat tatgattttt atgggacagc aatttcttgc atctctacag aggaagaaga 1920
   gggggagtgg gaggggaagg aaagagaaca gagcggcact gggatttgaa aggggaacct 1980
   ctctatctga ggagccccca ctggcttcag aagcaactta ccaaggggta tttaaagaca 2040
   tgaaaatttc cagaaatacc atttggtgca tccctttgtt tctgtaatat taaactcagg 2100
   tgaaattata ctctgacagt ttctctcttt ctgcctcttc cctctgcaga gtcaggacct 2160
   gcagaactgg ctgaaacaag atttcatggt gtcacccatg agagatgact caatgccaag 2220
   gcctgaagtt atagagtgtt tacagcggtg gcgatattca ggggtcatcg ccaactggtc 2280
   tcgagttcca aagctctgat gaagaaacaa gactccttga tgtgttactg atcccactga 2340
   ttccaggagt caagattagc caggaagcca aacaccagga gttggggtgg cacgtcacca 2400
   gtccagagcc ctgccacgga tgtacgcagg agcccagcat taggcaatca ggagccagaa 2460
   catgatcacc agggccacaa ataggaagag gcgtgacagg aactgctcgt ccacatacct 2520
   ggggtgtcc
<210> 48
   <211> 1553
   <212> DNA
   <213> Human
<400> 48
<210> 49
   <211> 921
   <212> DNA
   <213> Human
<400> 49
   ctgtggtccc agctactcag gaggctgagg cgggaggatt gcttgagccc aggagttgga 60
   tgttgcagtg agccaagatc gcaccattgc cctccactct gggccacgga gcaataccct 120
   gtctcagaaa acaaacaaca aaaagcagaa acgctgaagg ggtcggttta cgggaaaacc 180
   gcctgtcaga acacttggct actcctaccc cagatcagtg gacctgggaa tgagggttgg 240
   tcccgggagg cttttctcca agctgttgcc accagacccg ccatgggaac cctggccaca 300
   gaagcctccc ggggagtgag ccagagcctg gaccgctgtg ctgatgtgtc tggggtggag 360
   ggagggtggg gagtgtgcaa gggtgtgtgt gtgcccgggg ggtgttcatg ggcaagcatg 420
   tgcgtgcctg tgtgtgtgcg tgcccctccc ctgcagccgt cggtggtatc tccctccagc 480
   cccttcgcca ccttctgagc attgtctgtc cacgtgagac tgcccagaga cagcagagct 540
   ccacgtggtt ttaaggggag acctttccct ggacctgggg gtctcgccgt atctcatgac 600
   caggtgctaa atgacccgac atgcatcacc tgcctttcga tgaccaacct ccctgtcccc 660
   gtcccgctga cctgcccccg tggcgtctca cggtgatgcc tgctcctgac attggtgttc 720
   actgtagcaa actacattct ggatgggaat tttcatgtac atgtgtggca tgtggaaaat 780
   ttcaaataaa atggacttga tttagaaagc caaaaagctg tgtggtcctt ccagcacgga 840
   tactttgacc tcttgcctac aaccccttcc ttgggtccga ggctggtagc tttgttcact 900
   tcagatggtt gggggcgggt g
<210> 50
   <211> 338
   <212> DNA
   <213> Human
<400> 50
   atgatctatc tagatgccct accgtaaaat caaaacacaa aaccctactg actcattccc 60
   tcccttccag atattacccc atttctctac ttcccattgt agccaaactt tccaaaaatt 120
   catgttctgt cttcatttcc tcatgttcaa cccaccctgt cttagctacc acccctcagt 180
   aacgacctag cctgggtaga aacaaatgtc agcatgatac catactcaat gatccttcgt 240
   cactgttgtc attgtcatca ttccatggcc ttactttccc tctcagcgcc atttgctaca 300
   gtaagaaact ttctttcttg aattcttggt tctcttgg
<210> 51
   <211> 1191
   <212> DNA
   <213> Human
<400> 51
   ctagcaagca ggtaaacgag ctttgtacaa acacacacag accaacacat ccggggatgg 60
   ctgtgtgttg ctagagcaga ggctgattaa acactcagtg tgttggctct ctgtgccact 120
   cctggaaaat aatgaattgg gtaaggaaca gttaataaga aaatgtgcct tgctaactgt 180
   gcacattaca acaaagagct ggcagctcct gaaggaaaag ggcttgtgcc gctgccgttc 240
   aaacttgtca gtcaactcat gccagcagcc tcagcgtctg cctccccagc acaccctcat 300
   tacatgtgtc tgtctggcct gatctgtgca tctgctcgga gacgctcctg acaagtcggg 360
   aatttctcta tttctccact ggtgcaaaga gcggatttct ccctgcttct cttctgtcac 420
   ccccgctcct ctcccccagg aggctccttg atttatggta gctttggact tgcttccccg 480
   tctgactgtc cttgacttct agaatggaag aagctgagct ggtgaaggga agactccagg 540
   ccatcacaga taaaagaaaa atacaggaag aaatctcaca gaagcgtctg aaaatagagg 600
   aagacaaact aaagcaccag catttgaaga aaaaggcctt gagggagaaa tggcttctag 660
   atggaatcag cagcggaaaa gaacaggaag agatgaagaa gcaaaatcaa caagaccagc 720
   accagatcca ggttctagaa caaagtatcc tcaggcttga gaaagagatc caagatcttg 780
   aaaaagctga actgcaaatc tcaacgaagg aagaggccat tttaaagaaa ctaaagtcaa 840
   ttgagcggac aacagaagac attataagat ctgtgaaagt ggaaagagaa gaaagagcag 900
   aagagtcaat tgaggacatc tatgctaata tccctgacct tccaaagtcc tacatacctt 960
   ctaggttaag gaaggagata aatgaagaaa aagaagatga tgaacaaaat aggaaagctt 1020
   tatatgccat ggaaattaaa gttgaaaaag acttgaagac tggagaaagt acagttctgt 1080
   cttccaatac ctctggccat cagatgactt taaaaggtac aggagtaaaa gtttaagatg 1140
   atgggcaaaa gtccagtgta ttcagtaaag tgctaatcac aagttggagg t
<210> 52
   <211> 1200
   <212> DNA
   <213> Human
<400> 52
   aacagggact ctcactctat caaccccagg ctggagtccg gtgcgcccac cctggctccc 60
   tgcaacctcc gcctcccagg ctcaagcaac tctcctgcct cagtcgctct agtagctggg 120
   actacaggca cacaccacca tgcccagcca atttttgcat tttttgtaga gacagggttt 180
   cgccttctgt ccaggccggc atcatatact ttaaatcatg cccagatgac tttaatacct 240
   aatacaatat atcaggttgg tttaaaaata attgcttttt tattattttt gcatttttgc 300
   accaacctta atgctatgta aatagttgtt atactgttgc ttaacaacag tatgacaatt 360
   ttggcttttt ctttgtatta ttttgtattt ttttttttta ttgtgtggtc tttttttttt 420
   ttctcagtgt tttcaattcc tccttggttg aatccatgga tgcaaaaccc acagatatga 480
   agggctggct atatatgcat tgatgattgt cctattatat tagttataaa gtgtcattta 540
   atatgtagtg aaagttatgg tacagtggaa agagtagttg aaaacataaa catttggacc 600
   tttcaagaaa ggtagcttgg tgaagttttt caccttcaaa ctatgtccca gtcagggctc 660
   tgctactaat tagctataat ctttgcacaa attacatcac ctttgagtct cagttgcctc 720
   acctgtaaaa tgaaagaact ggatactctc taaggtcact tccagccctg tcattctata 780
   actctgttat gctgaggaag aaattcacat tgtgttaact gtatgagtca aactgaaaat 840
   gattattaaa gtgggaaaaa gccaattgct tctcttagaa agctcaacta aatttgagaa 900
   gaataatctt ttcaattttt taagaattta aatattttta agggtttgac ctatttattt 960
   agagatgggg tctcactctg tcacccagac tggagtacag tggcacaatc atagctcact 1020
   gctgcctcaa attcatgggc tcaagtgatc ctcctgcctc tgcctccaga gtagctgcga 1080
   ctatgggcat gtgccaccac gcctggctaa catttgtatt gacctattta tttattgtga 1140
   tttatatctt tttttttttt tctttttttt ttttttacaa aatcagaaat acttattttg 1200
<210> 53
   <211> 989
   <212> DNA
   <213> Human
<400> 53
   aagccaccac tcaaaacttc ctatacattt tcacagcaga gacaagtgaa catttatttt 60
   tatgcctttc ttcctatgtg tatttcaagt ctttttcaaa acaaggcccc aggactctcc 120
   gattcaatta gtccttgggc tggtcgactg tgcaggagtc cagggagcct ctacaaatgc 180
   agagtgactc tttaccaaca taaaccctag atacatgcaa aaagcaggac ccttcctcca 240
   ggaatgtgcc atttcagatg cacagcaccc atgcagaaaa gctggaattt tccttggaac 300
   cgactgtgat agaggtgctt acatgaacat tgctactgtc tttctttttt tttgagacag 360
   gtttcgcttg tgcccaggct gagtgcaatg cgtgatctca ctcactgcaa ttccacctcc 420
   aggttcaagc attctcctgc tcagcctcct agtagctggg ttacaggcac tgccaccatg 480
   ccggctaatt ttgtattttt gtagagatgg atttctccat ttggtcaggc ggtctcgaac 540
   cccaacctca gtgatctgcc acctcagcct cctaagtgtt ggattacagg atgagccacc 600
   cgaccggcca ctactgtctt tctttgaccc ttccagtttc gaagataaag aggaaataat 660
   ttctctgaag tacttgataa aatttccaaa caaaacacat gtccacttca ctgataaaaa 720
   atttaccgca gtttggcacc taagagtatg acaacagcaa taaaaagtaa tttcaaagag 780
   ttaagatttc ttcagcaaaa tagatgattc acatcttcaa gtcctttttg aaatcagtta 840
   ttaatattat tctttcctca tttccatctg aatgactgca gcaatagttt tttttttttt 900
   tttttttttt ttgcgagatg gaatctcgct ctgtcgccca gcgggagtgc actggcgcaa 960
   gcccggctca ccgcaatctc tgccacccg
<210> 54
   <211> 250
   <212> DNA
   <213>.Human
<400> 54
   catttcccca ttggtcctga tgttgaagat ttagttaaag aggctgtaag tcaggttcga 60
   gcagaggcta ctacaagaag tagggaatca agtccctcac atgggctatt aaaactaggt 120
   agtggtggag tagtgaaaaa gaaatctgag caacttcata acgtaactgc ctttcaggga 180
   aaagggcatt ctttaggaac tgcatctggt aacccacacc ttgatccaag agctagggaa 240
   acttcagttg
<210> 55
   <211> 2270
   <212> DNA
   <213> Human
<400> 55
   gcgcccccga gcagcgcccg cgccctccgc gccttctccg ccgggacctc gagcgaaaga 60
   ggcccgcgcg ccgcccagcc ctcgcctccc tgcccaccgg gcacaccgcg ccgccacccc 120
   gaccccgctg cgcacggcct gtccgctgca caccagcttg ttggcgtctt cgtcgccgcg 180
   ctcgccccgg gctactcctg cgcgccacaa tgagctcccg catcgccagg gcgctcgcct 240
   tagtcgtcac ccttctccac ttgaccaggc tggcgctctc cacctgcccc gctgcctgcc 300
   actgccccct ggaggcgccc aagtgcgcgc cgggagtcgg gctggtccgg gacggctgcg 360
   gctgctgtaa ggtctgcgcc aagcagctca acgaggactg cagcaaaacg cagccctgcg 420
   accacaccaa ggggctggaa tgcaacttcg gcgccaagtc caccgctctg aaggggatct 480
   gcagagctca gtcagagggc agaccctgtg aatataactc cagaatctac caaaacgggg 540
   aaagtttcca gcccaactgt aaacatcagt gcacatgtat tgatggcgcc gtgggctgca 600
   ttcctctgtg tccccaagaa ctatctctcc ccaacttggg ctgtcccaac cctcggctgg 660
   tcaaagttac cgggcagtgc tgcgaggagt gggtctgtga cgaggatagt atcaaggacc 720
   ccatggagga ccaggacggc ctccttggca aggagctggg attcgatgcc tccgaggtgg 780
   agttgacgag aaacaatgaa ttgattgcag ttggaaaagg cagctcactg aagcggctcc 840
   ctgtttttgg aatggagcct cgcatcctat acaacccttt acaaggccag aaatgtattg 900
   ttcaaacaac ttcatggtcc cagtgctcaa agacctgtgg aactggtatc tccacacgag 960
   ttaccaatga caaccctgag tgccgccttg tgaaagaaac ccggatttgt gaggtgcggc 1020
   cttgtggaca gccagtgtac agcagcctga aaaagggcaa gaaatgcagc aagaccaaga 1080
   aatcccccga accagtcagg tttacttacg ctggatgttt gagtgtgaag aaataccggc 1140
   ccaagtactg cggttcctgc gtggacggcc gatgctgcac gccccagctg accaggactg 1200
   tgaagatgcg gttccgctgc gaagatgggg agacattttc caagaacgtc atgatgatcc 1260
   agtcctgcaa atgcaactac aactgcccgc atgccaatga agcagcgttt cccttctaca 1320
   ggctgttcaa tgacattcac aaatttaggg actaaatgct acctgggttt ccagggcaca 1380
   cctagacaaa caagggagaa gagtgtcaga atcagaatca tggagaaaat gggcgggggt 1440
   ggtgtgggtg atgggactca ttgtagaaag gaagccttgc tcattcttga ggagcattaa 1500
   ggtatttcga aactgccaag ggtgctggtg cggatggaca ctaatgcagc cacgattgga 1560
   gaatactttg cttcatagta ttggagcaca tgttactgct tcattttgga gcttgtggag 1620
   ttgatgactt tctgttttct gtttgtaaat tatttgctaa gcatattttc tctaggcttt 1680
   tttccttttg gggttctaca gtcgtaaaag agataataag attagttgga cagtttaaag 1740
   cttttattcg tcctttgaca aaagtaaatg ggagggcatt ccatcccttc ctgaaggggg 1800
   acactccatg agtgtctgtg agaggcagct atctgcactc taaactgcaa acagaaatca 1860
   ggtgttttaa gactgaatgt tttatttatc aaaatgtagc ttttggggag ggaggggaaa 1920
   tgtaatactg gaataatttg taaatgattt taattttata ttcagtgaaa agattttatt 1980
   tatggaatta accatttaat aaagaaatat ttacctaata tctgagtgta tgccattcgg 2040
   tatttttaga ggtgctccaa agtcattagg aacaacctag ctcacgtact caattattca 2100
   aacaggactt attgggatac agcagtgaat taagctatta aaataagata atgattgctt 2160
   ttataccttc agtagagaaa agtctttgca tataaagtaa tgtttaaaaa acatgtattg 2220
   aacacgacat tgtatgaagc acaataaaga ttctgaagct aaaaaaaaaa
<210> 56
   <211> 1636
   <212> DNA
   <213> Human
<400> 56
   cttgaatgaa gctgacacca agaaccgcgg gaagagcttg ggcccaaagc aggaaaggga 60
   agcgctcgag ttggaaagga accgctgctg ctggccgaac tcaagcccgg gcgcccccac 120
   cagtttgatt ggaagtccag ctgtgaaacc tggagcgtcg ccttctcccc agatggctcc 180
   tggtttgctt ggtctcaagg acactgcatc gtcaaactga tcccctggcc gttggaggag 240
   cagttcatcc ctaaagggtt tgaagccaaa agccgaagta gcaaaaatga gacgaaaggg 300
   cggggcagcc caaaagagaa gacgctggac tgtggtcaga ttgtctgggg gctggccttc 360
   agcccgtggc cttccccacc cagcaggaag ctctgggcac gccaccaccc ccaagtgccc 420
   gatgtctctt gcctggttct tgctacggga ctcaacgatg ggcagatcaa gatctgggag 480
   gtgcagacag ggctcctgct tttgaatctt tccggccacc aagatgtcgt gagagatctg 540
   agcttcacac ccagtggcag tttgattttg gtctccgcgt cacgggataa gactcttcgc 600
   atctgggacc tgaataaaca cggtaaacag attcaagtgt tatcgggcca cctgcagtgg 660
   gtttactgct gttccatctc cccagactgc agcatgctgt gctctgcagc tggagagaag 720
   tcggtctttc tatggagcat gaggtcctac acgttaattc ggaagctaga gggccatcaa 780
   agcagtgttg tctcttgtga cttctccccc gactctgccc tgcttgtcac ggcttcttac 840
   gataccaatg tgattatgtg ggacccctac accggcgaaa ggctgaggtc actccaccac 900
   acccaggttg accccgccat ggatgacagt gacgtccaca ttagctcact gagatctgtg 960
   tgcttctctc cagaaggctt gtaccttgcc acggtggcag atgacagact cctcaggatc 1020
   tgggccctgg aactgaaaac tcccattgca tttgctccta tgaccaatgg gctttgctgc 1080
   acattttttc cacatggtgg agtcattgcc acagggacaa gagatggcca cgtccagttc 1140
   tggacagctc ctagggtcct gtcctcactg aagcacttat gccggaaagc ccttcgaagt 1200
   ttcctaacaa cttaccaagt cctagcactg ccaatcccca agaaaatgaa agagttcctc 1260
   acatacagga ctttttaagc aacaccacat cttgtgcttc tttgtagcag ggtaaatcgt 1320
   cctgtcaaag ggagttgctg gaataatggg ccaaacatct ggtcttgcat tgaaatagca 1380
   tttctttggg attgtgaata gaatgtagca aaaccagatt ccagtgtaca taaaagaatt 1440
   tttttgtctt taaatagata caaatgtcta tcaactttaa tcaagttgta acttatattg 1500
   aagacaattt gatacataat aaaaaattat gacaatgtcc tgggaaaaaa aaaatgtaga 1560
   aagatggtga agggtgggat ggatgaggag cgtggtgacg ggggcctgca gcgggttggg 1620
   gaccctgtgc tgcgtt
<210> 57
   <211> 460
   <212> DNA
   <213> Human
<400> 57
   ccatgtgtgt atgagagaga gagagattgg gagggagagg gagctcacta gcgcatatgt 60
   gcctccaggg ggctgcagat gtgtctgagg gtgagcctgg tgaaagagaa gacaaaagaa 120
   tggaatgagc taaagcagcc gcctggggtg ggaggccgag cccatttgta tgcagcaggg 180
   ggcaggagcc cagcaaggga gcctccattc ccaggactct ggagggagct gagaccatcc 240
   atgcccgcag agccctccct cacactccat cctgtccagc cctaattgtg caggtgggga 300
   aactgaggct gggaagtcac atagcaagtg actggcagag ctgggactgg aacccaacca 360
   gcctcctaga ccacggttct tcccatcaat ggaatgctag agactccagc caggtgggta 420
   ccgagctcga attcgtaatc atggtcatag ctgtttcctg
<210> 58
   <211> 1049
   <212> DNA
   <213> Human
<400> 58
   atctgatcaa gaatacctgc cctggtcact ctgcggatgt ttctgtccac ttgttcacat 60
   tgaggaccaa gatatccttt tttacagagg cacttgttcg gtctaacaca gacacctcca 120
   tgacgacatg ctggctcaca ttttgcagtt ctgcagaagt ccccctccca gcctggacta 180
   cagcagcact ttcccgtggg ggtgcagtag ccgtttcgac agagcctgga gcactctgaa 240
   gtcagtgtct gtgcaggttg taccgtggct ctgcattcct caggcattaa aggtcttttg 300
   ggatctacaa ttttgtagag ttttccattg tgagtctggg tcatactttt actgcttgat 360
   aaaatgtaaa cttcacctag ttcatcttct ccaaatccca agatgtgacc ggaaaagtag 420
   cctctacagg acccactagt gccgacacag agtggttttt cttgccactg ctttgtcaca 480
   ggactttgct ggagagttag gaaattccca ttacgatctc caaacacgta gcttccatac 540
   aatctttctg actggcagcc ccggtataca aatccaccaa ccaaaggacc attactgaat 600
   ggcttgaatt ctaaaagtga tggctcactt tcataatctt tcccctttat tatctgtaga 660
   attctggctg atgatctgtt ttttccattg gagtctgaac acagtatcgt taaattgatg 720
   tttatatcag tgggatgtct atccacagca catctgcctg gatcgtggag cccatgagca 780
   aacacttcgg ggggctggtt ggtgctgttg aagtgtgggt tgctccttgg tatggaataa 840
   ggcacgttgc acatgtctgt gtccacatcc agccgtagca ctgagcctgt gaaatcactt 900
   aacccatcca tttcttccat atcatccagt gtaatcatcc catcaccaag aatgatgtac 960
   aaaaacccgt cagggccaaa gagcagttgc cctcccagat gctttctgtg gagttctgca 1020
   acttcaagaa agactctggc tgttctcaa
<210> 59
   <211> 747
   <212> DNA
   <213> Human
<400> 59

### Sequence Identifier

<110> Schering Aktiengesellschaft
<120> Combinations and compositions which interfere with VEGF/ VEGF and angiopoietin/ Tie receptor function and their use II
<130> 51867AEPM1XX00-P
<140>
   <141>
<160> 59
<210> 1
   <211> 1835
   <212> DNA
   <213> Human
<400> 1
   ttttacagtt ttccttttct tcagagttta ttttgaattt tcatttttgg ataaccaagc 60
   agctctttaa gaagaatgca cagaagagtc attctggcac ttttggatag tacataagat 120
   tttctttttt ttttttaaat tttttttaat agtcacattc agctcgcttg ctcaaaccag 180
   actcccacat tgggtgagca agatgagccc ataggattcc agagttaata cgtaaccgta 240
   tatacaaaca gccaaaaaac cataatggtg ccacagggat ggagcaggga agggcatctc 300
   taacgtgtcc tctagtctat cttcgctaaa cagaacccac gttacacatg ataactagag 360
   agcacactgt gttgaaacga ggatgctgac cccaaatggc acttggcagc atgcagttta 420
   aagcaaaaga gacatccttt aataactgta taaaatccag gcagttccat taaaggggtt 480
   aagaaaacca acaacaacaa aaagcgaggg actgtctgtt gtcactgtca aaaaggcact 540
   tggagttaat gggaccagga ttggaggact cttagctgat acagatttca gtacgatttc 600
   attaaaaggc ttggatgtta agagaggaca ctcagcggtt cctgaaggga gacgctgaga 660
   tggaccgctg agaagcggaa cagatgaaca caaaggaatc aaatctttac aaccaaattg 720
   catttaagcg acaacaaaaa aaggcaaacc ccaaaacgca acctaaccaa agcaaaatct 780
   aagcaaaatc agacaacgaa gcagcgatgc atagctttcc tttgagagaa cgcatacctt 840
   gagacgctac gtgccaacct aagttctcaa cgacagcttc acagtaggat tattgtgata 900
   aaaatgactc aagcgatgca aaaagtttca tctgttccca gaatccgagg gagaactgag 960
   gtgatcgtta gagcatagcg acatcacgtg cggtttctta atgtccctgg tggcggatac 1020
   gccgagtcct cggaaggaca tctggacacc actttcagcc acctccttgc aggggcgaca 1080
   tccgccaaag tcatccttta ttccgagtaa taactttaat tcctttctaa catttacacg 1140
   gcaaacagga atgcagtaaa cgtccacgtc cgtcccacgg ctgggctgcc gttccgtttc 1200
   ctccacgaac gggtacgcgc ttccatgaga aaggatattt ggcaatttta tattccacag 1260
   tcaggtgggt ctgcgatagc tcatttaatg ttaaacgcca tcaggggcct ctcctcccgt 1320
   ttctgccagg ggcttttctt gtcttctcct tggcgagctc gtgggcagat cttctctggt 1380
   gggggctggc tgctggctcc gagggggcat ccgcagtccg tctggtcgtc tcctcctgca 1440
   ggctgggcag ctggccacca cttctccgac tcgacccctc caacaagcat cgcagggcac 1500
   tgtcctcggg ggtacagacc gtggtcccac attcgctacc actctgttcc acgtcatcca 1560
   ggtacacgag ctgcgtgtag gccgtgctgt ctggggctcg aggctctttc tgctggtgct 1620
   cttggacggg cgggtagttc tgctgcagag acaaagcatc tccccttccc ttccgggctg 1680
   attttggttc attcatatct acgccagagt ccaaactggc atcattactt ccgttccttc 1740
   cagctctttg gagaatcaat gtatgaatgt ctaacctgac cgttggacct gccatccaag 1800
   gagacgaacc acgcccgggg gtgcggaagc ggcct
<210> 2
   <211> 581
   <212> DNA
   <213> Human
<400> 2
   gttctagatt gttttattca gtaattagct cttaagaccc ctggggcctg tgctacccag 60
   acactaacaa cagtctctat ccagttgctg gttctgggtg acgtgatctc cccatcatga 120
   tcaacttact tcctgtggcc cattagggaa gtggtgacct cgggagctat ttgcctgttg 180
   agtgcacaca cctggaaaca tactgctctc attttttcat ccacatcagt gagaaatgag 240
   tggcccgtta gcaagatata actatgcaat catgcaacaa agctgcctaa taacatttca 300
   tttattacag gactaaaagt tcattattgt ttgtaaagga tgaattcata acctctgcag 360
   agttatagtt catacacagt tgatttccat ttataaaggc agaaagtcct tgttttctct 420
   aaatgtcaag ctttgactga aaactcccgt ttttccagtc actggagtgt gtgcgtatga 480
   aagaaaatct ttagcaatta gatgggagag aagggaaata gtacttgaaa tgtaggccct 540
   cacctcccca tgacatcctc catgagcctc ctgatgtagt g
<210> 3
   <211> 516
   <212> DNA
   <213> Human
<400> 3
   tagagatgtt ggttgatgac ccccgggatc tggagcagat gaatgaagag tctctggaag 60
   tcagcccaga catgtgcatc tacatcacag aggacatgct catgtcgcgg aacctgaatg 120
   gacactctgg gttgattgtg aaagaaattg ggtcttccac ctcgagctct tcagaaacag 180
   ttgttaagct tcgtggccag agtactgatt ctcttccaca gactatatgt cggaaaccaa 240
   agacctccac tgatcgacac agcttgagcc tcgatgacat cagactttac cagaaagact 300
   tcctgcgcat tgcaggtctg tgtcaggaca ctgctcagag ttacaccttt ggatgtggcc 360
   atgaactgga tgaggaaggc ctctattgca acagttgctt ggcccagcag tgcatcaaca 420
   tccaagatgc ttttccagtc aaaagaacca gcaaatactt ttctctggat ctcactcatg 480
   atgaagttcc agagtttgtt gtgtaaagtc cgtctg
<210> 4
   <211> 1099
   <212> DNA
   <213> Human
<400> 4
   cccacaacac aggggccctg aaacacgcca gcctctcctc tgtggtcagc ttggcccagt 60
   cctgctcact ggatcacagc ccattgtagg tggggcatgg tggggatcag ggcccctggc 120
   ccacggggag gtagaagaag acctggtccg tgtaagggtc tgagaaggtg ccctgggtcg 180
   ggggtgcgtc ttggccttgc cgtgccctca tcccccggct gaggcagcga cacagcaggt 240
   gcaccaactc cagcaggtta agcaccaggg agatgagtcc aaccaccaac atgaagatga 300
   tgaagatggt cttctccgtg gggcgagaga caaagcagtc cacgaggtag gggcagggtg 360
   ctcgctggca cacaaacacg ggctccatgg tccagccgta caggcgccac tggccataga 420
   ggaagcctgc ctctagcaca ctcttgcaga gcacactggc gacataggtg cccatcagtg 480
   ctccgcggat gcgcaggcga ccatcttctg ccaccgagat cttggccatc tgacgctcta 540
   cggccgccag cgcccgctcc acctgtgggt ccttggccgg cagtgcccgc agctccccct 600
   ccttctgccg cagccgctct tctcgccgag acaggtaaat gacatggccc aggtagacca 660
   gggtgggtgt gctgacgaag aggaactgca gcacccagta gcggatgtgg gagatgggga 720
   aggcctggtc atagcagacg ttggtgcagc ctggctgggc cgtgttacac tcgaaatctg 780
   actgctcgtc accccacact gactcgccgg ccaggcccag gatgaggatg cggaagatga 840
   agagcaccgt cagccagatc ttacccacca cggtcgagtg ctcctggacc tggtccagca 900
   acttctccac gaagccccag tcacccatgg ctcccgggcc tccgtcggca aggagacaga 960
   gcacgtcagt gtgtcagcat ggcatccttc tcgttcgccc agcaacaagc ctgcagggag 1020
   gtctgccacg cccgttctac cgcctgcctg ccgggcggcc caggtggagg tggggacgat 1080
   ggccggagtg acgcccgcg
<210> 5
   <211> 1015
   <212> DNA
   <213> Human
<400> 5
   gaggataggg agcctggggt caggagtgtg ggagacacag cgagactctg tctccaaaaa 60
   aaaaagtgct ttttgaaaat gttgaggttg aaatgatggg aaccaacatt ctttggattt 120
   agtggggagc ataatagcaa acaccccctt ggttcgcaca tgtacaggaa tgggacccag 180
   ttggggcaca gccatggact tccccgccct ggaatgtgtg gtgcaaagtg gggccagggc 240
   ccagacccaa gaggagaggg tggtccgcag acaccccggg atgtcagcat cccccgacct 300
   gccttctggc ggcacctccc gggtgctgtg ttgagtcagc aggcatgggg tgagagcctg 360
   gtatatgctg ggaacagggt gcaggggcca agcgttcctc cttcagcctt gacttgggcc 420
   atgcaccccc tctcccccaa acacaaacaa gcacttctcc agtatggtgc caggacaggt 480
   gtcccttcag tcctctggtt atgacctcaa gtcctacttg ggccctgcag cccagcctgt 540
   gttgtaacct ctgcgtcctc aagaccacac ctggaagatt cttcttccct ttgaaggaga 600
   atcatcattg ttgctttatc acttctaaga cattttgtac ggcacggaca agttaaacag 660
   aatgtgcttc cctccctggg gtctcacacg ctcccacgag aatgccacag gggccgtgca 720
   ctgggcaggc ttctctgtag aaccccaggg gcttcggccc agaccacagc gtcttgccct 780
   gagcctagag cagggagtcc cgaacttctg cattcacaga ccacctccac aattgttata 840
   accaaaggcc tcctgttctg ttatttcact taaatcaaca tgctattttg ttttcactca 900
   cttctgactt tagcctcgtg ctgagccgtg tatccatgca gtcatgttca cgtgctagtt 960
   acgtttttct tcttacacat gaaaataaat gcataagtgt tagaagaaaa aaaaa
<210> 6
   <211> 2313
   <212> DNA
   <213> Human
<400> 6
   ccagagcagg cctggtggtg agcagggacg gtgcaccgga cggcgggatc gagcaaatgg 60
   gtctggccat ggagcacgga gggtcctacg ctcgggcggg gggcagctct cggggctgct 120
   ggtattacct gcgctacttc ttcctcttcg tctccctcat ccaattcctc atcatcctgg 180
   ggctcgtgct cttcatggtc tatggcaacg tgcacgtgag cacagagtcc aacctgcagg 240
   ccaccgagcg ccgagccgag ggcctataca gtcagctcct agggctcacg gcctcccagt 300
   ccaacttgac caaggagctc aacttcacca cccgcgccaa ggatgccatc atgcagatgt 360
   ggctgaatgc tcgccgcgac ctggaccgca tcaatgccag cttccgccag tgccagggtg 420
   accgggtcat ctacacgaac aatcagaggt acatggctgc catcatcttg agtgagaagc 480
   aatgcagaga tcaattcaag gacatgaaca agagctgcga tgccttgctc ttcatgctga 540
   atcagaaggt gaagacgctg gaggtggaga tagccaagga gaagaccatt tgcactaagg 600
   ataaggaaag cgtgctgctg aacaaacgcg tggcggagga acagctggtt gaatgcgtga 660
   aaacccggga gctgcagcac caagagcgcc actggccaag gagcaactgc aaaaggtgca 720
   agccctctgc ctgcccctgg acaaggacaa gtttgagatg gaccttcgta acctgtggag 780
   ggactccatt atcccacgca gcctggacaa cctgggttac aacctctacc atcccctggg 840
   ctcggaattg gcctccatcc gcagagcctg cgaccacatg cccagcctca tgagctccaa 900
   ggtggaggag ctggcccgga gcctccgggc ggatatcgaa cgcgtggccc gcgagaactc 960
   agacctccaa cgccagaagc tggaagccca gcagggcctg cgggccagtc aggaggcgaa 1020
   acagaaggtg gagaaggagg ctcaggcccg ggaggccaag ctccaagctg aatgctcccg 1080
   gcagacccag ctagcgctgg aggagaaggc ggtgctgcgg aaggaacgag acaacctggc 1140
   caaggagctg gaagagaaga agagggaggc ggagcagctc aggatggagc tggccatcag 1200
   aaactcagcc ctggacacct gcatcaagac caagtcgcag ccgatgatgc cagtgtcaag 1260
   gcccatgggc cctgtcccca acccccagcc catcgaccca gctagcctgg aggagttcaa 1320
   gaggaagatc ctggagtccc agaggccccc tgcaggcatc cctgtagccc catccagtgg 1380
   ctgaggaggc tccaggcctg aggaccaagg gatggcccga ctcggcggtt tgcggaggat 1440
   gcagggatat gctcacagcg cccgacacaa ccccctcccg ccgcccccaa ccacccaggg 1500
   ccaccatcag acaactccct gcatgcaaac ccctagtacc ctctcacacc cgcacccgcg 1560
   cctcacgatc cctcacccag agcacacggc cgcggagatg acgtcacgca agcaacggcg 1620
   ctgacgtcac atatcaccgt ggtgatggcg tcacgtggcc atgtagacgt cacgaagaga 1680
   tatagcgatg gcgtcgtgca gatgcagcac gtcgcacaca gacatgggga acttggcatg 1740
   acgtcacacc gagatgcagc aacgacgtca cgggccatgt cgacgtcaca catattaatg 1800
   tcacacagac gcggcgatgg catcacacag acggtgatga tgtcacacac agacacagtg 1860
   acaacacaca ccatgacaac gacacctata gatatggcac caacatcaca tgcacgcatg 1920
   ccctttcaca cacactttct acccaattct cacctagtgt cacgttcccc cgaccctggc 1980
   acacgggcca aggtacccac aggatcccat cccctcccgc acagccctgg gccccagcac 2040
   ctcccctcct ccagcttcct ggcctcccag ccacttcctc acccccagtg cctggacccg 2100
   gaggtgagaa caggaagcca ttcacctccg ctccttgagc gtgagtgttt ccaggacccc 2160
   ctcggggccc tgagccgggg gtgagggtca cctgttgtcg ggaggggagc cactccttct 2220
   cccccaactc ccagccctgc ctgtggcccg ttgaaatgtt ggtggcactt aataaatatt 2280
   agtaaatcct taaaaaaaaa aaaaaaaaaa aaa
<210> 7
   <211> 389
   <212> DNA
   <213> Human
<400> 7
   gccaaaaaga tggcttcaaa agtaagaatg aaacatttga tccattcagc tttaggctat 60
   gccactggat tcatgtctag aaaagatagg ataatttctg taaagaaatg aagaccttgc 120
   tattctaaaa tcagatcctt acagatccag atttcaggaa acaaatacat aggggactaa 180
   ctttccttgt tcagattagt ttttctcctt tgcacccagc tatataatat gaggaagtat 240
   tgacttttta aaagtgtttt agttttccat ttctttgata tgaaaagtaa tatttcggga 300
   gaaccctgag ctattaataa tctatgtggc tagtgcgtat atattggtct gaatttgttc 360
   tccttttgtg gtgtccagtg ggtaacatc
<210> 8
   <211> 157
   <212> DNA
   <213> Human
<400> 8
   tgctttaaac agctgtgtca aaaactgaca tcagagagta aattgaattt ggttttgtag 60
   gaagcaggaa gcaagcccac tcaaacgtga aatttggcat gagggatcca gtaactttct 120
   cctcaatctg tgaactatat gtgagtttga tattttg
<210> 9
   <211> 561
   <212> DNA
   <213> Human
<400> 9
   aatagtcaaa acataaacaa aagctaatta actggcactg ttgtcacctg agactaagtg 60
   gatgttgttg gctgacatac aggctcagcc agcagagaaa gaattctgaa ttccccttgc 120
   tgaactgaac tattctgtta catatggttg acaaatctgt gtgttatttc ttttctacct 180
   accatattta aatttatgag tatcaaccga ggacatagtc aaaccttcga tgatgaacat 240
   tcctgatttt ttgcctgatt aatctctgtt gagctctact tgtggtcatt caagatttta 300
   tgatgttgaa aggaaaagtg aatatgacct ttaaaaattg tattttgggt gatgatagtc 360
   tcaccactat aaaactgtca attattgcct aatgttaaag atatccatca ttgtgattaa 420
   ttaaacctat aatgagtatt cttaatggag aattcttaat ggatggatta tcccctgatc 480
   ttttctttaa aatttctctg cacacacagg acttctcatt ttccaataaa tgggtgtact 540
   ctgccccaat ttctaggaaa a
<210> 10
   <211> 1508
   <212> DNA
   <213> Human
<400> 10
   cacaaacacg agagactcca cggtctgcct gagcaccgcc agcctcctag gctccagcac 60
   tcgcaggtcc attcttctgc acgagcctct ctgtccagat ccataagcac ggtcagctca 120
   gggtcgcgga gcagtacgag gacaagtacc agcagcagct cctctgaaca gagactgcta 180
   ggatcatcct tctcctccgg gcctgttgct gatggcataa tccgggtgca acccaaatct 240
   gagctcaagc caggtgagct taagccactg agcaaggaag atttgggcct gcacgcctac 300
   aggtgtgagg actgtggcaa gtgcaaatgt aaggagtgca cctacccaag gcctctgcca 360
   tcagactgga tctgcgacaa gcagtgcctt tgctcggccc agaacgtgat tgactatggg 420
   acttgtgtat gctgtgtgaa aggtctcttc tatcactgtt ctaatgatga tgaggacaac 480
   tgtgctgaca acccatgttc ttgcagccag tctcactgtt gtacacgatg gtcagccatg 540
   ggtgtcatgt ccctcttttt gccttgttta tggtgttacc ttccagccaa gggttgcctt 600
   aaattgtgcc aggggtgtta tgaccgggtt aacaggcctg gttgccgctg taaaaactca 660
   aacacagttt gctgcaaagt tcccactgtc ccccctagga actttgaaaa accaacatag 720
   catcattaat caggaatatt acagtaatga ggattttttc tttctttttt taatacacat 780
   atgcaaccaa ctaaacagtt ataatcttgg cactgttaat agaaagttgg gatagtcttt 840
   gctgtttgcg gtgaaatgct ttttgtccat gtgccgtttt aactgatatg cttgttagaa 900
   ctcagctaat ggagctcaaa gtatgagata cagaacttgg tgacccatgt attgcataag 960
   ctaaagcaac acagacactc ctaggcaaag tttttgtttg tgaatagtac ttgcaaaact 1020
   tgtaaattag cagatgactt ttttccattg ttttctccag agagaatgtg ctatattttt 1080
   gtatatacaa taatatttgc aactgtgaaa aacaagtggt gccatactac atggcacaga 1140
   cacaaaatat tatactaata tgttgtacat tcggaagaat gtgaatcaat cagtatgttt 1200
   ttagattgta ttttgcctta cagaaagcct ttattgtaag actctgattt ccctttggac 1260
   ttcatgtata ttgtacagtt acagtaaaat tcaaccttta ttttctaatt ttttcaacat 1320
   attgtttagt gtaaagaata tttatttgaa gttttattat tttataaaaa agaatattta 1380
   ttttaagagg catcttacaa attttgcccc ttttatgagg atgtgatagt tgctgcaaat 1440
   gaggggttac agatgcatat gtccaatata aaatagaaaa tatattaacg tttgaaatta 1500
   aaaaaaaa
<210> 11
   <211> 389
   <212> DNA
   <213> Human
<400> 11
   gggcaggtga tcagggcaca catttcccgt ccattgagac agtagcattc ccggcaccca 60
   tcgtgccagc tctcctcatt tttatgatga tgaccatcca cggtgagaca agtgcccgac 120
   aggatgggtg gcccagctga agcacaggcc gctctgcact tgcagataag acagccgtga 180
   ctgtcctgct ggaaacccaa ggggcagatc ttactgcatg agagctctgg acatttctta 240
   cagcgacaga tgtcacagcc gtgcttattc ttcagcaatc caagtggaca atacttgtca 300
   cagattatgg gtctgcactt cttgggcctt gggcggcact cacagatctc acagttttgg 360
   acctcggccg cgaccacgct gggtaccga
<210> 12
   <211> 981
   <212> DNA
   <213> Human
<400> 12
   tttttttttt ttggattgca aaaatttatt aaaattggag acactgtttt aatcttcttg 60
   tgccatgaga ctccatcagg cagtctacaa agaccactgg gaggctgagg atcacttgag 120
   cccagaagtt tgaggctgta gtaagcttca aaggccactg cactctagct tgggtgaggc 180
   aagacccttt caagcagtaa gctgcatgct tgcttgttgt ggtcattaaa aaccctagtt 240
   taggataaca acatattaat cagggcaaaa tacaaatgtg tgatgcttgt tagtagagta 300
   acctcagaat caaaatggaa cggttttaca gtgatatcat tatatttcat ttggcagaat 360
   cattacatca ttggttacac tgaaaatcat cacatgtacc aaaagctgac tcacctagtt 420
   taggataaca ggtctgcctg tttgaagatg aaaaataata cccatttaaa atttgcccta 480
   ctcaatttcc ttctcagtca cattttaact tttaaacagc taatcactcc catctacaga 540
   ttaaggtgta tatgccacca aaaccttttg ccaccttaaa aatttccttc aaagtttaaa 600
   ctaatgcctg catttcttca atcatgaatt ctgagtcctt tgcttcttta aaacttgctc 660
   cacacagtgt agtcaagccg actctccata cccaagcaag tcatccatgg ataaaaacgt 720
   taccaggagc agaaccatta agctggtcca ggcaagttgg actccaccat ttcaacttcc 780
   agctttctgt ctaatgcctg tgtgccaatg gcttgagtta ggcttgctct ttaggacttc 840
   agtagctatt ctcatccttc cttggggaca caactgtcca taaggtgcta tccagagcca 900
   cactgcatct gcacccagca ccatacctca caggagtcga ctcccacgag ccgcctgtat 960
   ataagagttc ttttgatgac g
<210> 13
   <211> 401
   <212> DNA
   <213> Human
<400> 13
   ataactacag cttcagcaga caactaaaga gactgcatta aggtgatttc tctggctata 60
   aagagagccc ggccgcagag catgtgactg ctgggacctc tgggataggc aacactgccc 120
   tctctccccc agagcgaccc cccgggcagg tcggggccca aggaatgacc cagcaactgc 180
   tccctaccca gcacactctc tttactgcca cctgcaatta tgctgtgaag atgactgggt 240
   gtggtcatca cgattcagag aaatcaagat ctatgaccat tttaggcaaa gagagaaact 300
   tggagaattg ctgaggacta ctgaaccttg ttttgctttt ttaaaaaata ctaaatcctc 360
   acttcagcat atttagttgt cattaaaatt aagctgatat t
<210> 14
   <211> 1002
   <212> DNA
   <213> Human
<400> 14
   gacaatataa aaagtggaaa caagcataaa ttgcagacat aaaataatct tctggtagaa 60
   acagttgtgg agaacaggtt gagtagagca acaacaacaa aagcttatgc agtcaccttc 120
   tttgaaaatg ttaaatacaa gtcctattct ctttgtccag ctgggtttag ctagaggtag 180
   ccaattactt ctcttaaggt ccatggcatt cgccaggatt ctataaaagc caagttaact 240
   gaagtaaata tctggggccc atcgcacccc cactaagtac tttgtcacca tgttgtatct 300
   taaaagtcat ttttcactgt ttgactcaga atttgggact tcagagtcaa acttcattgc 360
   ttactccaaa cccagtttaa ttccccactt ttttaagtag gcttagcttt gagtgatttt 420
   tggctataac cgaaatgtaa atccaccttc aaacaacaaa gtttgacaag actgaaatgt 480
   tactgaaaac aatggtgcca tatgctccaa agacatttcc ccaagataac tgccaaagag 540
   tttttgagga ggacaatgat catttattat gtaggagcct tgatatctct gcaaaataga 600
   attaatacag ctcaaatgga gtagtaacca agcttttctg cccaggaagt aacaaacatc 660
   actacgaaca tgagagtaca agaggaaact ttcataatgc attttttcat tcatacattc 720
   attcaataaa cattagccaa gctaatgtcc caagccactg tgccaggtat taacaatata 780
   acaacaataa aagacacagt ccttcctctc aaggtgttca gtctagtagg gaagatgatt 840
   attcattaaa atttttggtg catcagaatc atgaggagct tgtcaaaaat gtaaattcct 900
   gcctatgttc tcagatattc tggttaggtc aggagtggga acccaaaatc aattctttta 960
   acaaacacta aaggtgattc taacacaggc ggtgtgagga cc
<210> 15
   <211> 280
   <212> DNA
   <213> Human
<400> 15
   cgaggtgggc cacccgtgtc tggtctgaga tttttaaatg aggattacat tatcctattt 60
   ataatattcc tattctaatc tattgtattc ttacaattaa atgtatcaaa taattcttaa 120
   aaacattatt agaaacaaac tgcctaatac cttataagac taaaaaaatc accaagatga 180
   aactgtatta tgactctcaa tatttaaaca tttaaaaaaa tgttagtgtt tgttaagcac 240
   caatcttaac tatttcacct gcccgggcgg ccgctcgagg
<210> 16
   <211> 2041
   <212> DNA
   <213> Human
<400> 16
   ccccccgcag aactcccccc tggaatagga tttttaaaac ccttgacaat tagaaatcct 60
   atagaggtta gcatttttta ggtaaaaata tggttgcccc tacagggatc atgcaacttc 120
   cttaaaacca attcagcaca tatgtataaa gaaccctttt taaaaacatt tgtacttgaa 180
   atacagacac agtgatgctg aagacactaa acaaaaactg aaaagtacta taccttgata 240
   aattttgtta ttgccttctt tagagacttt ataatctcta gttgattttc aaggacttga 300
   atttaataat ggggtaatta cacaagacgt aaaggatttt ttaaaaacaa gtattttttt 360
   ttacctctag catcaattct tttataaaga atgctaaata aattacattt tttgttcagt 420
   aaaactgaag atagaccatt taaatgcttc taccaaattt aacgcagctt aattagggac 480
   caggtacata ttttcttctg aacatttttg gtcaagcatg tctaaccata aaagcaaatg 540
   gaattttaag aggtagattt tttttccatg atgcattttg ttaataaatg tgtcaagaaa 600
   ataaaaacaa gcactgagtg tgttctcttg aagtataagg gtctaatgaa aaataaaaga 660
   tagatatttg ttatagtctg acattttaac agtcatagta ttagacgttt cgtgaccagt 720
   gcattttgga ctctctcagg atcaaaatac gagtctgcca actgtattaa atcctcctcc 780
   accccctcca ccagttggtc cacagcttcc tggtgggtcg ttgtcatcaa atccattggg 840
   ccgaaatgaa catgaagcag atgcagcttg gagggcccgg gctcgagcat tcaactcttg 900
   ttcctgtaaa tatagtttat tgtcttttgt tatagcatcc ataagttctt tctgtagagg 960
   tgggtctcca tttatccaga gtccactggt tgggttatta ccacttaaac cattagtact 1020
   atgctgtttt ttatacaaaa gcacataagc tgtgtccttt ggaaacctgc tcgtaatttt 1080
   ctggactgac tgaaatgaag taaatgtcac tctactgtca ttaaataaaa acccattctt 1140
   ttgacatttc cttattttcc aaatcctgtt caaaaactgc actgggacta tctctcccta 1200
   gtaaatgact ctgggaggat gctaatgcca gagcctcaga ctggtggtac atctgatatg 1260
   aagagtctgt acttgtgata tttctggcat aagaatagta atgcccactt tcagaggata 1320
   taccagagtg aaccacaacg gaacttaata gatagggcac caattttgtg caggaagctt 1380
   catcagtccc tgaaggcttt aattttttag caaggttctc actaagatca gtgaagtcaa 1440
   catctacaga ccaactttct gacaatgaag agaaagaagt aattcttcta actggcaact 1500
   ccaaaaccag tggccagtga tacattgtct aaaattttcc ttctcacatg atacttctga 1560
   tcatatgaaa atctcaggag agtaagaata aggtattcag gttcctccgt gatttgcata 1620
   gttttctcag cattttgcag agaggcacag ttttcacaat aatattggtt atcaccagta 1680
   agaatctctg gagcccaaaa aataatttag taagtcagtt actgaaggtg tggtttcacc 1740
   tcccggtttc tgaggtacat ctttattaac aagaatcttg ttagattcgt tagggacaga 1800
   agtgttttca gaacagtaaa actcattagg aggactgcct atggtttttt cattcacaag 1860
   tgagtcacag atgaaggcag ctgttgttgg attataaact actggctctt ctgaaggacc 1920
   gggtacagac gcttgcatta gaccaccatc ttgtatactg ggtgatgatg ctggatcttg 1980
   gacagacatg ttttccaaag aagaggaagc acaaaacgca agcgaaagat ctgtaaaggc 2040
   t
<210> 17
   <211> 235
   <212> DNA
   <213> Human
<400> 17
   cgccccgggc aggtgtcagg ggttccaaac cagcctgggg aaacacagcg tagacccctc 60
   acctctacaa ataaaaaatt aaaaaattag ccaggtgtgg cagcgaacaa ctgtagtctc 120
   agatactcag gagactgagc tggaaaggat cacttgagcc caagaagttc aaggttacag 180
   tgggccacga tcatgtcatt acactccagc ttgggtgaca aaatgagact gtcta
<210> 18
   <211> 2732
   <212> DNA
   <213> Human
<400> 18
   gtgtggagtt tcagctgcta ttgactataa gagctatgga acagaaaaag cttgctggct 60
   tcatgttgat aactacttta tatggagctt cattggacct gttaccttca ttattctgct 120
   aaatattatc ttcttggtga tcacattgtg caaaatggtg aagcattcaa acactttgaa 180
   accagattct agcaggttgg aaaacattaa gtcttgggtg cttggcgctt tcgctcttct 240
   gtgtcttctt ggcctcacct ggtcctttgg gttgcttttt attaatgagg agactattgt 300
   gatggcatat ctcttcacta tatttaatgc tttccaggga gtgttcattt tcatctttca 360
   ctgtgctctc caaaagaaag tacgaaaaga atatggcaag tgcttcagac actcatactg 420
   ctgtggaggc ctcccaactg agagtcccca cagttcagtg aaggcatcaa ccaccagaac 480
   cagtgctcgc tattcctctg gcacacagag tcgtataaga agaatgtgga atgatactgt 540
   gagaaaacaa tcagaatctt cttttatctc aggtgacatc aatagcactt caacacttaa 600
   tcaaggtggc ataaatctta atatattatt acaggactga catcacatgg tctgagagcc 660
   catcttcaag atttatatca tttagaggac attcactgaa caatgccagg gatacaagtg 720
   ccatggatac tctaccgcta aatggtaatt ttaacaacag ctactcgctg cacaagggtg 780
   actataatga cagcgtgcaa gttgtggact gtggactaag tctgaatgat actgcttttg 840
   agaaaatgat catttcagaa ttagtgcaca acaacttacg gggcagcagc aagactcaca 900
   acctcgagct cacgctacca gtcaaacctg tgattggagg tagcagcagt gaagatgatg 960
   ctattgtggc agatgcttca tctttaatgc acagcgacaa cccagggctg gagctccatc 1020
   acaaagaact cgaggcacca cttattcctc agcggactca ctcccttctg taccaacccc 1080
   agaagaaagt gaagtccgag ggaactgaca gctatgtctc ccaactgaca gcagaggctg 1140
   aagatcacct acagtccccc aacagagact ctctttatac aagcatgccc aatcttagag 1200
   actctcccta tccggagagc agccctgaca tggaagaaga cctctctccc tccaggagga 1260
   gtgagaatga ggacatttac tataaaagca tgccaaatct tggagctggc catcagcttc 1320
   agatgtgcta ccagatcagc aggggcaata gtgatggtta tataatcccc attaacaaag 1380
   aagggtgtat tccagaagga gatgttagag aaggacaaat gcagctggtt acaagtcttt 1440
   aatcatacag ctaaggaatt ccaagggcca catgcgagta ttaataaata aagacaccat 1500
   tggcctgacg cagctccctc aaactctgct tgaagagatg actcttgacc tgtggttctc 1560
   tggtgtaaaa aagatgactg aaccttgcag ttctgtgaat ttttataaaa catacaaaaa 1620
   ctttgtatat acacagagta tactaaagtg aattatttgt tacaaagaaa agagatgcca 1680
   gccaggtatt ttaagattct gctgctgttt agagaaattg tgaaacaagc aaaacaaaac 1740
   tttccagcca ttttactgca gcagtctgtg aactaaattt gtaaatatgg ctgcaccatt 1800
   tttgtaggcc tgcattgtat tatatacaag acgtaggctt taaaatcctg tgggacaaat 1860
   ttactgtacc ttactattcc tgacaagact tggaaaagca ggagagatat tctgcatcag 1920
   tttgcagttc actgcaaatc ttttacatta aggcaaagat tgaaaacatg cttaaccact 1980
   agcaatcaag ccacaggcct tatttcatat gtttcctcaa ctgtacaatg aactattctc 2040
   atgaaaaatg gctaaagaaa ttatattttg ttctattgct agggtaaaat aaatacattt 2100
   gtgtccaact gaaatataat tgtcattaaa ataattttaa agagtgaaga aaatattgtg 2160
   aaaagctctt ggttgcacat gttatgaaat gttttttctt acactttgtc atggtaagtt 2220
   ctactcattt tcacttcttt tccactgtat acagtgttct gctttgacaa agttagtctt 2280
   tattacttac atttaaattt cttattgcca aaagaacgtg ttttatgggg agaaacaaac 2340
   tctttgaagc cagttatgtc atgccttgca caaaagtgat gaaatctaga aaagattgtg 2400
   tgtcacccct gtttattctt gaacagaggg caaagagggc actgggcact tctcacaaac 2460
   tttctagtga acaaaaggtg cctattcttt tttaaaaaaa taaaataaaa cataaatatt 2520
   actcttccat attccttctg cctatattta gtaattaatt tattttatga taaagttcta 2580
   atgaaatgta aattgtttca gcaaaattct gctttttttt catccctttg tgtaaacctg 2640
   ttaataatga gcccatcact aatatccagt gtaaagttta acacggtttg acagtaaata 2700
   aatgtgaatt ttttcaagtt aaaaaaaaaa aa
<210> 19
   <211> 276
   <212> DNA
   <213> Human
<400> 19
   ctccctaaat gattttaaaa taaattggat aaacatatga tataaagtgg gtactttaga 60
   aaccgccttt gcatattttt tatgtacaaa tctttgtata caattccgat gttccttata 120
   tattccctat atagcaaacc aaaaccagga cctcccaact gcatgcctca agtccctgtg 180
   gagcactctg gcaactggat ggccctactt gctttctgac aaaatagctg gaaaggagga 240
   gggaccaatt aaatacctcg gccgcgacca cgctgg
<210> 20
   <211> 2361
   <212> DNA
   <213> Human
<400> 20
   attgtaccag ccttgatgaa cgtgggccct gcttcgcttt tgagggccat aagctcattg 60
   cccactggtt tagaggctac cttatcattg tctcccgtga ccggaaggtt tctcccaagt 120
   cagagtttac cagcagggat tcacagagct ccgacaagca gattctaaac atctatgacc 180
   tgtgcaacaa gttcatagcc tatagcaccg tctttgagga tgtagtggat gtgcttgctg 240
   agtggggctc cctgtacgtg ctgacgcggg atgggcgggt ccacgcactg caggagaagg 300
   acacacagac caaactggag atgctgttta agaagaacct atttgagatg gcgattaacc 360
   ttgccaagag ccagcatctg gacagtgatg ggctggccca gattttcatg cagtatggag 420
   accatctcta cagcaagggc aaccacgatg ggctgtccca gcaatatatc cgaaccattg 480
   gaaagttgga gccatcctac gtgatccgca agtttctgga tgcccagcgc attcacaacc 540
   tgactgccta cctgcagacc ctgcaccgac aatccctggc caatgccgac cataccaccc 600
   tgctcctcaa ctgctatacc aagctcaagg acagctcgaa gctggaggag ttcatcaaga 660
   aaaagagtga gagtgaagtc cactttgatg tggagacagc catcaaggtc ctccggcagg 720
   ctggctacta ctcccatgcc ctgtatctgg cggagaacca tgcacatcat gagtggtacc 780
   tgaagatcca gctagaagac attaagaatt atcaggaagc ccttcgatac atcggcaagc 840
   tgccttttga gcaggcagag agcaacatga agcgctacgg caagatcctc atgcaccaca 900
   taccagagca gacaactcag ttgctgaagg gactttgtac tgattatcgg cccagcctcg 960
   aaggccgcag cgatagggag gccccaggct gcagggccaa ctctgaggag ttcatcccca 1020
   tctttgccaa taacccgcga gagctgaaag ccttcctaga gcacatgagt gaagtgcagc 1080
   cagactcacc ccaggggatc tacgacacac tccttgagct gcgactgcag aactgggccc 1140
   acgagaagga tccacaggtc aaagagaagc ttcacgcaga ggccatttcc ctgctgaaga 1200
   gtggtcgctt ctgcgacgtc tttgacaagg ccctggtcct gtgccagatg cacgacttcc 1260
   aggatggtgt cctttacctt tatgagcagg ggaagctgtt ccagcagatc atgcactacc 1320
   acatgcagca cgagcagtac cggcaggtca tcagcgtgtg tgagcgccat ggggagcagg 1380
   acccctcctt gtgggagcag gccctcagct acttcgctcg caaggaggag gactgcaagg 1440
   agtatgtggc agctgtcctc aagcatatcg agaacaagaa cctcatgcca cctcttctag 1500
   tggtgcagac cctggcccac aactccacag ccacactctc cgtcatcagg gactacctgg 1560
   tccaaaaact acagaaacag agccagcaga ttgcacagga tgagctgcgg gtgcggcggt 1620
   accgagagga gaccacccgt atccgccagg agatccaaga gctcaaggcc agtcctaaga 1680
   ttttccaaaa gaccaagtgc agcatctgta acagtgcctt ggagttgccc tcagtccact 1740
   tcctgtgtgg ccactccttc caccaacact gctttgagag ttactcggaa agtgatgctg 1800
   actgccccac ctgcctccct gaaaaccgga aggtcatgga tatgatccgg gcccaggaac 1860
   agaaacgaga tctccatgat caattccagc atcagctcaa gtgctccaat gacagctttt 1920
   ctgtgattgc tgactacttt ggcagaggtg ttttcaacaa attgactctg ctgaccgacc 1980
   ctcccacagc cagactgacc tccagcctgg aggctgggct gcaacgcgac ctactcatgc 2040
   actccaggag gggcacttaa gcagcctgga ggaagatgtg ggcaacagtg gaggacccag 2100
   agaacagaca caatgggacc tgggcgggcg ttacacagaa ggctggctga catgcccagg 2160
   gctccactct catctaatgt cacagccctc acaagactaa agcggaactt tttcttttcc 2220
   ctggccttcc ttaattttaa gtcaagcttg gcaatccctt cctctttaac taggcaggtg 2280
   ttagaatcat ttccagatta atggggggga aggggaacct caggcaaacc tcctgaagtt 2340
   ttggaaaaaa aagctggttt c
<210> 21
   <211> 179
   <212> DNA
   <213> Human
<400> 21
   aggtgttaga tgctcttgaa aaagaaactg catctaagct gtcagaaatg gattctttta 60
   acaatcaact aaaggaactg agagaaacct acaacacaca gcagttagcc cttgaacagc 120
   tttataagat caacgtgaca agttgaagga aattgaaagg aaaaaattag aactaatgc
<210> 22
   <211> 905
   <212> DNA
   <213> Human
<400> 22
   tttttttttt ttctttaacc gtgtggtctt tatttcagtg ccagtgttac agatacaaca 60
   caaatgttcc agttagaagg aattcaaacg gaatgccaag gtccaagcca ggctcaagaa 120
   ataaaaaggg aggtttggag taatagataa gatgactcca atactcactc ttcctaaggg 180
   caaaggtact tttgatacag agtctgatct ttgaaactgg tgaactcctc ttccacccat 240
   taccatagtt caaacaggca agttatgggc ttaggagcac tttaaaattt gtggtgggaa 300
   tagggtcatt aataactatg aatatatctt ttagaaggtg accattttgc actttaaagg 360
   gaatcaattt tgaaaatcat ggagactatt catgactaca gctaaagaat ggcgagaaag 420
   gggagctgga agagccttgg aagtttctat tacaaataga gcaccatatc cttcatgcca 480
   aatctcaaca aaagctcttt ttaactccat ctgtccagtg tttacaaata aactcgcaag 540
   gtctgaccag ttcttggtaa caaacataca tgtgtgtgtc tgtgtgtata cagcaatgca 600
   cagaaaaggc taccaggagc ctaatgcctc tttcaaacat tgggggaacc agtagaaaaa 660
   ggcagggctc cctaatgtcc attattacat ttccattccg aatgccagat gttaaaagtg 720
   cctgaagatg gtaacccagc tagtgaggaa taaatacccc accttgccca gtccacagag 780
   aaacaacagt agaaagaagg ggcaactctt tgctgcagag acaaagtgag tgttttttcg 840
   ccatggattg cagtcctctc ctccagacca gctgcttatt tcctcagggg cccagggaat 900
   gttga
<210> 23
   <211> 2134
   <212> DNA
   <213> Human
<400> 23
   ggtctcttct ttcctttttt tttttccaaa agtgttcttt tatttctagt aacatatatt 60
   gtataaatac tctattttat atgcacttcc acaaaagcga tataatttaa aagttttttt 120
   cattagaaat aaatgtataa aaataaatat gttattatag gcatttatta ctaactatag 180
   tccttcttgg aaggaacacc caaaccaata cttataaagt acatgtaatt tatagtaaca 240
   tattttacta tatacatatg gaaaaaatca tattctcaca gaagagctga acagacattc 300
   accaggatac gactgttgga ccagctgctg gagatggacc tgctacccct cagcagcctc 360
   cccaccacaa gacaagtgat ctcaatgtcc ccaaacctgt gggaccctgt tctacacacc 420
   tcatttttgt tccggcgttt catcctcctt gtgtgattgt actgattttc atgagacaca 480
   agttacttct ttacatccat attcccaaag cagggttaca tggtaggaaa gaaaggaagt 540
   tggaggtact aagctcattg tgtctcctct agcttttacc agcatctaat gcttcactgc 600
   tttttttcca ttgtagactt taatgcactt gaataaatac atggagttgt tttttcctca 660
   aaatgaatta cacaaataaa gactgagatg gtccaaaaaa ggaaagagga agccatttgc 720
   gttatttcac gttgctgagc ctttctctca tgttgaacaa tctgaagttt taattctcgg 780
   tagaaataat gtataaacat tctctgaaac catagcagcc ataaacagtg ctggtcaaag 840
   atcctatttg tactcctttc tccccccatt gttagtgagg taaagtaaaa caggtcttag 900
   taaaatctca cttttctcct acttttcatt tcccaacccc catgatacta agtatttgat 960
   aagtaccagg aaacaggggt tgtaatagtt ctaacttttt ttgacaattg ctttgttttt 1020
   tctaaacttg taatagatgt aacaaaagaa ataataataa taatgcccgg ggctttatta 1080
   tgctatatca ctgctcagag gttaataatc ctcactaact atcctatcaa atttgcaact 1140
   ggcagtttac tctgatgatt caactccttt tctatctacc cccataatcc caccttactg 1200
   atacacctca ctggttactg gcaagatacg ctggatccct ccagccttct tgctttccct 1260
   gcaccagccc ttcctcactt tgccttgccc tcaaagctaa caccacttaa accacttaac 1320
   tgcattctgc cattgtgcaa aagtctatga aatgtttagg tttctttaaa ggatcacagc 1380
   tctcatgaga taacacccct ccatcatggg acagacactt caagcttctt tttttgtaac 1440
   ccttcccaca ggtcttagaa catgatgacc actcccccag ctgccactgg gggcagggat 1500
   ggtctgcaca aggtctggtg ctggctggct tcacttcctt tgcacactcg gaagcaggct 1560
   gtccattaat gtctcggcat tctaccagtc ttctctgcca acccaattca catgacttag 1620
   aacattcgcc ccactcttca atgacccatg ctgaaaaagt ggggatagca ttgaaagatt 1680
   ccttcttctt ctttacgaag taggtgtatt taattttagg tcgaagggca ttgcccacag 1740
   taagaacctg gatggtcaag ggctctttga gagggctaaa gctgcgaatt ctttccaatg 1800
   ccgcagagga gccgctgtac ctcaagacaa cacctttgta cataatgtct tgctctaagg 1860
   tggacaaagt gtagtcacca ttaagaatat atgtgccatc agcagctttg atggcaagaa 1920
   agctgccatt gttcctggat cccctctggt tccgctgttt cacttcgatg ttggtggctc 1980
   cagttggaat tgtgatgata tcatgatatc caggttttgc actagtaact gatcctgata 2040
   tttttttaca agtagatcca tttcccccgc aaacaccaca tttatcaaac ttctttttgg 2100
   agtctatgat gcgatcacaa ccagctttta caca
<210> 24
   <211> 1626
   <212> DNA
   <213> Human
<400> 24
   ggacaatttc tagaatctat agtagtatca ggatatattt tgctttaaaa tatattttgg 60
   ttattttgaa tacagacatt ggctccaaat tttcatcttt gcacaatagt atgacttttc 120
   actagaactt ctcaacattt gggaactttg caaatatgag catcatatgt gttaaggctg 180
   tatcatttaa tgctatgaga tacattgttt tctccctatg ccaaacaggt gaacaaacgt 240
   agttgttttt tactgatact aaatgttggc tacctgtgat tttatagtat gcacatgtca 300
   gaaaaaggca agacaaatgg cctcttgtac tgaatacttc ggcaaactta ttgggtcttc 360
   attttctgac agacaggatt tgactcaata tttgtagagc ttgcgtagaa tggattacat 420
   ggtagtgatg cactggtaga aatggttttt agttattgac tcagaattca tctcaggatg 480
   aatcttttat gtctttttat tgtaagcata tctgaattta ctttataaag atggttttag 540
   aaagctttgt ctaaaaattt ggcctaggaa tggtaacttc attttcagtt gccaaggggt 600
   agaaaaataa tatgtgtgtt gttatgttta tgttaacata ttattaggta ctatctatga 660
   atgtatttaa atatttttca tattctgtga caagcattta taatttgcaa caagtggagt 720
   ccatttagcc cagtgggaaa gtcttggaac tcaggttacc cttgaaggat atgctggcag 780
   ccatctcttt gatctgtgct taaactgtaa tttatagacc agctaaatcc ctaacttgga 840
   tctggaatgc attagttatg ccttgtacca ttcccagaat ttcaggggca tcgtgggttt 900
   ggtctagtga ttgaaaacac aagaacagag agatccagct gaaaaagagt gatcctcaat 960
   atcctaacta actggtcctc aactcaagca gagtttcttc actctggcac tgtgatcatg 1020
   aaacttagta gaggggattg tgtgtatttt atacaaattt aatacaatgt cttacattga 1080
   taaaattctt aaagagcaaa actgcatttt atttctgcat ccacattcca atcatattag 1140
   aactaagata tttatctatg aagatataaa tggtgcagag agactttcat ctgtggattg 1200
   cgttgtttct tagggttcct agcactgatg cctgcacaag catgtgatat gtgaaataaa 1260
   atggattctt ctatagctaa atgagttccc tctggggaga gttctggtac tgcaatcaca 1320
   atgccagatg gtgtttatgg gctatttgtg taagtaagtg gtaagatgct atgaagtaag 1380
   tgtgtttgtt ttcatcttat ggaaactctt gatgcatgtg cttttgtatg gaataaattt 1440
   tggtgcaata tgatgtcatt caactttgca ttgaattgaa ttttggttgt atttatatgt 1500
   attatacctg tcacgcttct agttgcttca accattttat aaccattttt gtacatattt 1560
   tacttgaaaa tattttaaat ggaaatttaa ataaacattt gatagtttac ataataaaaa 1620
   aaaaaa
<210> 25
   <211> 1420
   <212> DNA
   <213> Human
<400> 25
   gttcagcatt gtttctgctt ctgaaatctg tatagtacac tggtttgtaa tcattatgtc 60
   ttcattgaaa tccttgctac ttctcttcct cctcaatgaa agacacgaga gacaagagcg 120
   acacaagctt aagaaaaacg agcaaggaag agtatcttca ttattctcat tttctctgag 180
   ttggaaacaa aaacatgaag gactccaact agaagacaga tatttacatt taaatagatt 240
   agtgggaaaa ctttaagagt ttccacatat tagttttcat tttttgagtc aagagactgc 300
   tccttgtact gggagacact agtagtatat gtttgtaatg ttactttaaa attatctttt 360
   tattttataa ggcccataaa tactggttaa actctgttaa aagtgggcct tctatcttgg 420
   atggtttcac tgccatcagc catgctgata tattagaaat ggcatcccta tctacttact 480
   ttaatgctta aaattataca taaaatgctt tatttagaaa acctacatga tacagtggtg 540
   tcagccttgc catgtatcag tttcacttga aatttgagac caattaaatt tcaactgttt 600
   agggtggaga aagaggtact ggaaaacatg cagatgagga tatcttttat gtgcaacagt 660
   atcctttgca tgggaggaga gttactcttg aaaggcaggc agcttaagtg gacaatgttt 720
   tgtatatagt tgagaatttt acgacacttt taaaaattgt gtaattgtta aatgtccagt 780
   tttgctctgt tttgcctgaa gttttagtat ttgttttcta ggtggacctc tgaaaaccaa 840
   accagtacct ggggaggtta gatgtgtgtt tcaggcttgg agtgtatgag tggttttgct 900
   tgtattttcc tccagagatt ttgaacttta ataattgcgt gtgtgttttt ttttttttaa 960
   gtggctttgt ttttttttct caagtaaaat tgtgaacata tttcctttat aggggcaggg 1020
   catgagttag ggagactgaa gagtattgta gactgtacat gtgccttctt aatgtgtttc 1080
   tcgacacatt ttttttcagt aacttgaaaa ttcaaaaggg acatttggtt aggttactgt 1140
   acatcaatct atgcataaat ggcagcttgt tttcttgagc cactgtctaa attttgtttt 1200
   tatagaaatt ttttatactg attggttcat agatggtcag ttttgtacac agactgaaca 1260
   atacagcact ttgccaaaaa tgagtgtagc attgtttaaa cattgtgtgt taacacctgt 1320
   tctttgtaat tgggttgtgg tgcattttgc actacctgga gttacagttt tcaatctgtc 1380
   agtaaataaa gtgtccttta acttcaaaaa aaaaaaaaaa
<210> 26
   <211> 689
   <212> DNA
   <213> Human
<400> 26
   aaacaaacaa aaaaaaagtt agtactgtat atgtaaatac tagcttttca atgtgctata 60
   caaacaatta tagcacatcc ttccttttac tctgtctcac ctcctttagg tgagtacttc 120
   cttaaataag tgctaaacat acatatacgg aacttgaaag ctttggttag ccttgcctta 180
   ggtaatcagc ctagtttaca ctgtttccag ggagtagttg aattactata aaccattagc 240
   cacttgtctc tgcaccattt atcacaccag gacagggtct ctcaacctgg gcgctactgt 300
   catttggggc caggtgattc ttccttgcaa gggctgtcct gtacctgccc gggcggccgc 360
   tcgaagcgtg gtcgcggccg aggtactgaa aggaccaagg agctctggct gccctcagga 420
   attccaaatg accgaaggaa caaagcttca gggctctggg tggtgtctcc cactattcag 480
   gaggtggtcg gaggtaacgc agcttcattt cgtccagtcc tttccagtat ttaaagttgt 540
   tgtcaagatg ctgcattaaa tcaggcaggt ctacaaaggc atcccaagca tcaaacatgt 600
   ctgtgatgaa gtaatcaatg aaacaccgga acctccgacc acctcctgaa tagtgggaga 660
   cacacccaga gcctgaagtt tgtccttcg
<210> 27
   <211> 471
   <212> DNA
   <213> Human
<400> 27
   tcccagcggc atgaagtttg agattggcca ggccctgtac ctgggcttca tctccttcgt 60
   ccctctcgct cattggtggc accctgcttt gcctgtcctg ccaggacgag gcaccctaca 120
   agccctaacc caggccccgc ccagggccac cacgaccact gcaaacaccg cacctgccta 180
   ccagccacca gctgcctaca aagacaatcg ggccccctca gtgacctcgg ccaccacagc 240
   gggtacaggc tgaacgacta cgtgtgagtc cccacagcct gcttctcccc tgggctgctg 300
   tgggctggtt cccggcggga ctgtcaatgg aggcaggggt tccagcacaa agtttacttc 360
   tgggcaattt ttgtatccaa ggaaataatg tgaatgcgag gaaatgtctt tagagcacag 420
   ggacagaggg ggaaataaga ggaggagaaa gctctctata ccaaagactg a
<210> 28
   <211> 929
   <212> DNA
   <213> Human
<400> 28
   ggtgaactca gtgcattggg ccaatggttc gacacaggct ctgccagcca caaccatcct 60
   gctgcttctg acggtttggc tgctggtggg ctttcccctc actgtcattg gaggcatctt 120
   tgggaagaac aacgccagcc cctttgatgc accctgtcgc accaagaaca tcgcccggga 180
   gattccaccc cagccctggt acaagtctac tgtcatccac atgactgttg gaggcttcct 240
   gcctttcagt gccatctctg tggagctgta ctacatcttt gccacagtat ggggtcggga 300
   gcagtacact ttgtacggca tcctcttctt tgtcttcgcc atcctgctga gtgtgggggc 360
   ttgcatctcc attgcactca cctacttcca gttgtctggg gaggattacc gctggtggtg 420
   gcgatctgtg ctgagtgttg gctccaccgg cctcttcatc ttcctctact cagttttcta 480
   ttatgcccgg cgctccaaca tgtctggggc agtacagaca gtagagttct tcggctactc 540
   cttactcact ggttatgtct tcttcctcat gctgggcacc atctcctttt tttcttccct 600
   aaagttcatc cggtatatct atgttaacct caagatggac tgagttctgt atggcagaac 660
   tattgctgtt ctctcccttt cttcatgccc tgttgaactc tcctaccagc ttctcttctg 720
   attgactgaa ttgtgtgatg gcattgttgc cttccctttt tccctttggg cattccttcc 780
   ccagagaggg cctggaaatt ataaatctct atcacataag gattatatat ttgaactttt 840
   taagttgcct ttagttttgg tcctgatttt tctttttaca attaccaaaa taaaatttat 900
   taagaaaaag aaaaaaaaaa aaaaaaaaa
<210> 29
   <211> 1775
   <212> DNA
   <213> Human
<400> 29
   gaacgtgatg ggaactttgg gaggatgtct gagaaaatgt ccgaagggat tttggccaac 60
   accagaaaac gccaatgtcc taggaattcc ctcccaaaat gcttcccaaa aaattactca 120
   ttgacaattc aaattgcact tggctggcgg cagcccgggc ggccttcagt ccgtgtgggg 180
   cgcccgcgtg gccttctcct cgtaggactc cccaaactcg ttcactctgc gtttatccac 240
   aggataaagc caccgctggt acaggtagac cagaaacacc acgtcgtccc ggaagcaggc 300
   cagccggtga gacgtgggca tggtgatgat gaaggcaaag acgtcatcaa tgaaggtgtt 360
   gaaagccttg taggtgaagg ccttccaggg cagatgtgcc actgacttca acttgtagtt 420
   cacaaagagc tggggcagca tgaagaggaa accaaaggca tagaccccgt tgacgaagct 480
   gttgattaac caggagtacc agctcttata tttgatattc aggagtgaat agacagcacc 540
   cccgacacag agagggtaca gcaggtatga caagtacttc atggcctgag tatcgtactc 600
   ctcggttttc ctctcagatt cgctgtaagt gccaaactga aattcgggca tcaggcctct 660
   ccaaaaaata gtcatcttca atgccttctt cactttccac agctcaatgg cggctccaac 720
   acccgccggg accagcacca gcaggctcgt ctgctcgtcc agcaggaaca gaaagatgac 780
   cacggtgctg aagcagcgcc agagcactgc cttggtggac atgccgatca tgctcttctt 840
   cttcttccag aaactgatgt catttttaaa ggccaggaaa tcaaagagaa gatggaacgc 900
   tgcgacaaag aaggtcagcg ccaggaagta taagttggta tctacaaaaa ttcctttcac 960
   ctcatcagca tctttctctg aaaacccgaa ctgctgcagg gagtacacgg cgtcctgcat 1020
   gtggatccag aagcgcagcc gccccagtga gaccttgtcg taggacacgg tgaggggcag 1080
   ctcggtggtg gagcggttta tgaccatcag gtccttcacg cggttgctga gctggtcgat 1140
   gaacaggatg ggcaggtaat gcacggtttt ccccagctgg atcatcttca tgtaccgatg 1200
   cacatcggca ggcagggagg acccgtcaaa gacaaagttg tccgccatca cgttcagcgc 1260
   cagccgcggt cgccagtggg acactggctc atccagggca ctcgtcggct tcttctccgc 1320
   ctcgatctgc tgtgtatcag actccccggt gagcaggttg atttcttctg gcttggggac 1380
   catgtaggtg gtcagaggac tgaccaggtg cacctgcttc ccgtcgtgcc acggcaggac 1440
   cccagcgtga tggaggaaga tgtaggcata cagcgtccca ttgtttctcg ttttctttgg 1500
   tacagaaaca ttaactgtcc tttcaaattt ggactccaca tcaaagtctt ccacattcaa 1560
   gaccaggtcg atgttgttct cagcacccag gtgggacctc gtcgtggtgt acacgctcag 1620
   ctgcagcttg ggccgccgcg ccaggtaggg ctggatgcag ttggcgtcgc cggagcacgg 1680
   gcgggtgtag acgatgccgt acatgaccca gcaggtgtgc accacgtaga ccacgaacac 1740
   gcccaccacc aagctggtga aggagctgcg gcccc
<210> 30
   <211> 1546
   <212> DNA
   <213> Human
<400> 30
   aaaataagta ggaatgggca gtgggtattc acattcacta caccttttcc atttgctaat 60
   aaggccctgc caggctggga gggaattgtc cctgcctgct tctggagaaa gaagatattg 120
   acaccatcta cgggcaccat ggaactgctt caagtgacca ttctttttct tctgcccagt 180
   atttgcagca gtaacagcac aggtgtttta gaggcagcta ataattcact tgttgttact 240
   acaacaaaac catctataac aacaccaaac acagaatcat tacagaaaaa tgttgtcaca 300
   ccaacaactg gaacaactcc taaaggaaca atcaccaatg aattacttaa aatgtctctg 360
   atgtcaacag ctactttttt aacaagtaaa gatgaaggat tgaaagccac aaccactgat 420
   gtcaggaaga atgactccat catttcaaac gtaacagtaa caagtgttac acttccaaat 480
   gctgtttcaa cattacaaag ttccaaaccc aagactgaaa ctcagagttc aattaaaaca 540
   acagaaatac caggtagtgt tctacaacca gatgcatcac cttctaaaac tggtacatta 600
   acctcaatac cagttacaat tccagaaaac acctcacagt ctcaagtaat aggcactgag 660
   ggtggaaaaa atgcaagcac ttcagcaacc agccggtctt attccagtat tattttgccg 720
   gtggttattg ctttgattgt aataacactt tcagtatttg ttctggtggg tttgtaccga 780
   atgtgctgga aggcagatcc gggcacacca gaaaatggaa atgatcaacc tcagtctgat 840
   aaagagagcg tgaagcttct taccgttaag acaatttctc atgagtctgg tgagcactct 900
   gcacaaggaa aaaccaagaa ctgacagctt gaggaattct ctccacacct aggcaataat 960
   tacgcttaat cttcagcttc tatgcaccaa gcgtggaaaa ggagaaagtc ctgcagaatc 1020
   aatcccgact tccatacctg ctgctggact gtaccagacg tctgtcccag taaagtgatg 1080
   tccagctgac atgcaataat ttgatggaat caaaaagaac cccggggctc tcctgttctc 1140
   tcacatttaa aaattccatt actccattta caggagcgtt cctaggaaaa ggaattttag 1200
   gaggagaatt tgtgagcagt gaatctgaca gcccaggagg tgggctcgct gataggcatg 1260
   actttcctta atgtttaaag ttttccgggc caagaatttt tatccatgaa gactttccta 1320
   cttttctcgg tgttcttata ttacctactg ttagtattta ttgtttacca ctatgttaat 1380
   gcagggaaaa gttgcacgtg tattattaaa tattaggtag aaatcatacc atgctacttt 1440
   gtacatataa gtattttatt cctgctttcg tgttactttt aataaataac tactgtactc 1500
   aatactctaa aaatactata acatgactgt gaaaatggca aaaaaa
<210> 31
   <211> 750
   <212> DNA
   <213> Human
<400> 31
   cacttgggca cccccatttt ctaaaaaaat ggaaatctgg agggcaaaaa aggtgtgctg 60
   aagggaagtg cctctgatgg cccaaaaacc ttcttccaaa ctagtgtagg aatggaatgg 120
   atagcaaatg gatccttttt ggcctccttt ggagcatgcc ttccctatct tatccttggc 180
   cccactaaag cagaacgtta cggatatttc tgtttttgcc attggatgcc tatctggcca 240
   aacagccttt ccctaattgg aaaatgcagt cctgtttaaa acctttgatt tacgactact 300
   tgtacatgct tgctcattac aattttgaca ttttttacat agtgaagacc ccaaacatat 360
   cagtgaaaca tgacaagatc ataaagaaca gtatcatatt attatttagt cgcttttaca 420
   gtggcaagcc aattttgaaa tatctcattt aaaactcaga cccaattcac tgagttatac 480
   ttttaatagc ttcctcagca cactatttcc catgcattaa atatgataaa ataatctatc 540
   actgcccatc ggtcttgtaa aaaggaagtc tgaatacaga gcccacaaca ctaaaattgt 600
   ttttctagct acaaagtata gcatcatcaa cacagacacg atttggactc cctgacaggt 660
   ggattggaaa acggtgttta aagagaagag aacattttaa cataaatgtc attaagaatc 720
   ccaaaggcct tatttgtcac caccgtcccg
<210> 32
   <211> 1620
   <212> DNA
   <213> Human
<400> 32
   gcaattcccc cctcccacta aacgactccc agtaattatg tttacaaccc attggatgca 60
   gtgcagccat tcataagaac cttggtgccc cagaaaaatc tgtccttttt ggtaccaaac 120
   ctgaggtctt ttggaagata atgtagaaaa ccactaccta ttgaaggcct gttttggcta 180
   atctgtgcaa actctgatga tacctgcctt atgtggattc ttttccacac tgctttcatt 240
   tttaagtata aagacttaga aaactagaat aatgctttta caaataatta aaagtatgtg 300
   atgttctggg ttttttcctt ctttttagaa ccccgcctcc atttaaaaaa ttaaaaaaaa 360
   aaaaaaaact tttaacattt aaaaaataaa aattaacaaa atttcactta ttccaggaca 420
   cgctggcatt tggactcaat gaaaagggca cctaaagaaa ataaggctga ctgaatgttt 480
   tccataattt tcacacaata acagtccctt tctatccagc ttgccttcca tttatctcta 540
   gggttagctt ttcaggcaac atccttggtc attgcccaga aagtacctga gctatcagtg 600
   attggaatgg cacaggaaac cgaatcacat gggtgccctc cccttggttt tcaagtatct 660
   tggagttgtg cacaaaaatt aggtcatgcc ttcagtgtct tgttctttaa acctaccctt 720
   tgacaatcag gtgctaatga ttgtatacta ttaaaaccag cacataagta ttgtaaatgt 780
   gtgttcctcc taggttggaa gaaatgtctt tccttctatc tgggtcctgt taaagcgggt 840
   gtcagttgtg tcttttcacc tcgatttgtg aattaataga attgggggga gaggaaatga 900
   tgatgtcaat taagtttcag gtttggcatg atcatcattc tcgatgatat tctcactttg 960
   tcgcaaatct gcccttatcg taagaacaag tttcagaatt ttccctccac tatacgactc 1020
   cagtattatg tttacaatcc attggatgag tgcagcatta taagaccttg gtgcccagaa 1080
   aaatctgtcc tttttggtac caaacctgag gtcttttgga agataatgta gaaaaccact 1140
   acctattgaa ggcctgtttt ggctaatctg tgcaaactct gatgatacct gcttatgtgg 1200
   attcttttcc acactgcttt catttttaag tataaagact tagaaaacta gaataatgct 1260
   tttacaaata attaaaagta tgtgatgttc tgggtttttt ccttcttttt agaaccctgt 1320
   atttaaacaa gccttctttt taagtcttgt ttgaaattta agtctcagat cttctggata 1380
   ccaaatcaaa aacccaacgc gtaaaacagg gcagtatttg tgttcctaat tttaaaaagc 1440
   tttatgtata ctctataaat atagatgcat aaacaacact tccccttgag tagcacatca 1500
   acatacagca ttgtacatta caatgaaaat gtgtaactta agggtattat atatataaat 1560
   acatatatac ctttgtaacc tttatactgt aaataaaaaa gttgctttag tcaaaaaaaa 1620
<210> 33
   <211> 2968
   <212> DNA
   <213> Human
<400> 33
   gaaaaagtag aaggaaacac agttcatata gaagtaaaag aaaaccctga agaggaggag 60
   gaggaggaag aagaggaaga agaagatgaa gaaagtgaag aggaggagga agaggaggga 120
   gaaagtgaag gcagtgaagg tgatgaggaa gatgaaaagg tgtcagatga gaaggattca 180
   gggaagacat tagataaaaa gccaagtaaa gaaatgagct cagattctga atatgactct 240
   gatgatgatc ggactaaaga agaaagggct tatgacaaag caaaacggag gattgagaaa 300
   cggcgacttg aacatagtaa aaatgtaaac accgaaaagc taagagcccc tattatctgc 360
   gtacttgggc atgtggacac agggaagaca aaaattctag ataagctccg tcacacacat 420
   gtacaagatg gtgaagcagg tggtatcaca caacaaattg gggccaccaa tgttcctctt 480
   gaagctatta atgaacagac taagatgatt aaaaattttg atagagagaa tgtacggatt 540
   ccaggaatgc taattattga tactcctggg catgaatctt tcagtaatct gagaaataga 600
   ggaagctctc tttgtgacat tgccatttta gttgttgata ttatgcatgg tttggagccc 660
   cagacaattg agtctatcaa ccttctcaaa tctaaaaaat gtcccttcat tgttgcactc 720
   aataagattg ataggttata tgattggaaa aagagtcctg actctgatgt ggctgctact 780
   ttaaagaagc agaaaaagaa tacaaaagat gaatttgagg agcgagcaaa ggctattatt 840
   gtagaatttg cacagcaggg tttgaatgct gctttgtttt atgagaataa agatccccgc 900
   acttttgtgt ctttggtacc tacctctgca catactggtg atggcatggg aagtctgatc 960
   taccttcttg tagagttaac tcagaccatg ttgagcaaga gacttgcaca ctgtgaagag 1020
   ctgagagcac aggtgatgga ggttaaagct ctcccgggga tgggcaccac tatagatgtc 1080
   atcttgatca atgggcgttt gaaggaagga gatacaatca ttgttcctgg agtagaaggg 1140
   cccattgtaa ctcagattcg aggcctcctg ttacctcctc ctatgaagga attacgagtg 1200
   aagaaccagt atgaaaagca taaagaagta gaagcagctc agggggtaaa gattcttgga 1260
   aaagacctgg agaaaacatt ggctggttta cccctccttg tggcttataa agaagatgaa 1320
   atccctgttc ttaaagatga attgatccat gagttaaagc agacactaaa tgctatcaaa 1380
   ttagaagaaa aaggagtcta tgtccaggca tctacactgg gttctttgga agctctactg 1440
   gaatttctga aaacatcaga agtgccctat gcaggaatta acattggccc agtgcataaa 1500
   aaagatgtta tgaaggcttc agtgatgttg gaacatgacc ctcagtatgc agtaattttg 1560
   gccttcgatg tgagaattga acgagatgca caagaaatgg ctgatagttt aggagttaga 1620
   atttttagtg cagaaattat ttatcattta tttgatgcct ttacaaaata tagacaagac 1680
   tacaagaaac agaaacaaga agaatttaag cacatagcag tatttccctg caagataaaa 1740
   atcctccctc agtacatttt taattctcga gatccgatag tgatgggggt gacggtggaa 1800
   gcaggtcagg tgaaacaggg gacacccatg tgtgtcccaa gcaaaaattt tgttgacatc 1860
   ggaatagtaa caagtattga aataaaccat aaacaagtgg atgttgcaaa aaaaggacaa 1920
   gaagtttgtg taaaaataga acctatccct ggtgagtcac ccaaaatgtt tggaagacat 1980
   tttgaagcta cagatattct tgttagtaag atcagccggc agtccattga tgcactcaaa 2040
   gactggttca gagatgaaat gcagaagagt gactggcagc ttattgtgga gctgaagaaa 2100
   gtatttgaaa tcatctaatt ttttcacatg gagcaggaac tggagtaaat gcaatactgt 2160
   gttgtaatat cccaacaaaa atcagacaaa aaatggaaca gacgtatttg gacactgatg 2220
   gacttaagta tggaaggaag aaaaataggt gtataaaatg ttttccatga gaaaccaaga 2280
   aacttacact ggtttgacag tggtcagtta catgtcccca cagttccaat gtgcctgttc 2340
   actcacctct cccttcccca acccttctct acttggctgc tgttttaaag tttgcccttc 2400
   cccaaatttg gatttttatt acagatctaa agctctttcg attttatact gattaaatca 2460
   gtactgcagt atttgattaa aaaaaaaaaa gcagattttg tgattcttgg gacttttttg 2520
   acgtaagaaa tacttcttta tttatgcata ttcttcccac agtgattttt ccagcattct 2580
   tctgccatat gcctttaggg cttttataaa atagaaaatt aggcattctg atatttcttt 2640
   agctgctttg tgtgaaacca tggtgtaaaa gcacagctgg ctgcttttta ctgcttgtgt 2700
   agtcacgagt ccattgtaat catcacaatt ctaaaccaaa ctaccaataa agaaaacaga 2760
   catccaccag taagcaagct ctgttaggct tccatggtta gtggtagctt ctctcccaca 2820
   agttgtcctc ctaggacaag gaattatctt aacaaactaa actatccatc acactacctt 2880
   ggtatgccag cacctgggta acagtaggag attttataca ttaatctgat ctgtttaatc 2940
   tgatcggttt agtagagatt ttatacat
<210> 34
   <211> 6011
   <212> DNA
   <213> Human
<400> 34
   acggggcgcc ggacgacccg cacatcttat cctccacgcc ccactcgcac tcggagcggg 60
   accgccccgg actccccctc gggccggcca ctcgaggagt gaggagagag gccgccggcc 120
   cggcttgagc cgagcgcagc accccccgcg ccccgcgcca gaagtttggt tgaaccgggc 180
   tgccgggaga aacttttttc tttttccccc ctctcccggg agagtctctg gaggaggagg 240
   ggaactcccc cggcccaagg ctcgtgggct cggggtcgcg cggccgcaga aggggcgggg 300
   tccgcccgcg aggggaggcg cccccgggga cccgagaggg gggtgaggac cgcgggctgc 360
   tggtgcggcg gcggcagcgt gtgccccgcg caggggaggc gccgccccgc tcccggcccg 420
   gctgcgagga ggaggcggcg gcggcgcagg aggatgtact tggtggcggg ggacaggggg 480
   ttggccggct gcgggcacct cctggtctcg ctgctggggc tgctgctgct gccggcgcgc 540
   tccggcaccc gggcgctggt ctgcctgccc tgtgacgagt ccaagtgcga ggagcccagg 600
   aaccgcccgg ggagcatcgt gcagggcgtc tgcggctgct gctacacgtg cgccagccag 660
   gggaacgaga gctgcggcgg caccttcggg atttacggaa cctgcgaccg ggggctgcgt 720
   tgtgtcatcc gccccccgct caatggcgac tccctcaccg agtacgaagc gggcgtttgc 780
   gaagatgaga actggactga tgaccaactg cttggtttta aaccatgcaa tgaaaacctt 840
   attgctggct gcaatataat caatgggaaa tgtgaatgta acaccattcg aacctgcagc 900
   aatccctttg agtttccaag tcaggatatg tgcctttcag ctttaaagag aattgaagaa 960
   gagaagccag attgctccaa ggcccgctgt gaagtccagt tctctccacg ttgtcctgaa 1020
   gattctgttc tgatcgaggg ttatgctcct cctggggagt gctgtccctt acccagccgc 1080
   tgcgtgtgca accccgcagg ctgtctgcgc aaagtctgcc agccgggaaa cctgaacata 1140
   ctagtgtcaa aagcctcagg gaagccggga gagtgctgtg acctctatga gtgcaaacca 1200
   gttttcggcg tggactgcag gactgtggaa tgccctactg ttcagcagac cgcgtgtccc 1260
   ccggacagct atgaaactca agtcagacta actgcagatg gttgctgtac tttgccaaca 1320
   agatgcgagt gtctctctgg cttatgtggt ttccccgtgt gtgaggtggg atccactccc 1380
   cgcatagtct ctcgtggcga tgggacacct ggaaagtgct gtgatgtctt tgaatgtgtt 1440
   aatgatacaa agccagcctg cgtatttaac aatgtggaat attatgatgg agacatgttt 1500
   cgaatggaca actgtcggtt ctgtcgatgc caagggggcg ttgccatctg cttcaccgcc 1560
   cagtgtggtg agataaactg cgagaggtac tacgtgcccg aaggagagtg ctgcccagtg 1620
   tgtgaagatc cagtgtatcc ttttaataat cccgctggct gctatgccaa tggcctgatc 1680
   cttgcccacg gagaccggtg gcgggaagac gactgcacat tctgccagtg cgtcaacggt 1740
   gaacgccact gcgttgcgac cgtctgcgga cagacctgca caaaccctgt gaaagtgcct 1800
   ggggagtgtt gccctgtgtg cgaagaacca accatcatca cagttgatcc acctgcatgt 1860
   ggggagttat caaactgcac tctgacacgg aaggactgca ttaatggttt caaacgcgat 1920
   cacaatggtt gtcggacctg tcagtgcata aacacccagg aactatgttc agaacgtaaa 1980
   caaggctgca ccttgaactg tcccttcggt ttccttactg atgcccaaaa ctgtgagatc 2040
   tgtgagtgcc gcccaaggcc caagaagtgc agacccataa tctgtgacaa gtattgtcca 2100
   cttggattgc tgaagaataa gcacggctgt gacatctgtc gctgtaagaa atgtccagag 2160
   ctctcatgca gtaagatctg ccccttgggt ttccagcagg acagtcacgg ctgtcttatc 2220
   tgcaagtgca gagaggcctc tgcttcagct gggccaccca tcctgtcggg cacttgtctc 2280
   accgtggatg gtcatcatca taaaaatgag gagagctggc acgatgggtg ccgggaatgc 2340
   tactgtctca atggacggga aatgtgtgcc ctgatcacct gcccggtgcc tgcctgtggc 2400
   aaccccacca ttcaccctgg acagtgctgc ccatcatgtg cagatgactt tgtggtgcag 2460
   aagccagagc tcagtactcc ctccatttgc cacgcccctg gaggagaata ctttgtggaa 2520
   ggagaaacgt ggaacattga ctcctgtact cagtgcacct gccacagcgg acgggtgctg 2580
   tgtgagacag aggtgtgccc accgctgctc tgccagaacc cctcacgcac ccaggattcc 2640
   tgctgcccac agtgtacaga tcaacctttt cggccttcct tgtcccgcaa taacagcgta 2700
   cctaattact gcaaaaatga tgaaggggat atattcctgg cagctgagtc ctggaagcct 2760
   gacgtttgta ccagctgcat ctgcattgat agcgtaatta gctgtttctc tgagtcctgc 2820
   ccttctgtat cctgtgaaag acctgtcttg agaaaaggcc agtgttgtcc ctactgcata 2880
   aaagacacaa ttccaaagaa ggtggtgtgc cacttcagtg ggaaggccta tgccgacgag 2940
   gagcggtggg accttgacag ctgcacccac tgctactgcc tgcagggcca gaccctctgc 3000
   tcgaccgtca gctgcccccc tctgccctgt gttgagccca tcaacgtgga aggaagttgc 3060
   tgcccaatgt gtccagaaat gtatgtccca gaaccaacca atatacccat tgagaagaca 3120
   aaccatcgag gagaggttga cctggaggtt cccctgtggc ccacgcctag tgaaaatgat 3180
   atcgtccatc tccctagaga tatgggtcac ctccaggtag attacagaga taacaggctg 3240
   cacccaagtg aagattcttc actggactcc attgcctcag ttgtggttcc cataattata 3300
   tgcctctcta ttataatagc attcctattc atcaatcaga agaaacagtg gataccactg 3360
   ctttgctggt atcgaacacc aactaagcct tcttccttaa ataatcagct agtatctgtg 3420
   gactgcaaga aaggaaccag agtccaggtg gacagttccc agagaatgct aagaattgca 3480
   gaaccagatg caagattcag tggcttctac agcatgcaaa aacagaacca tctacaggca 3540
   gacaatttct accaaacagt gtgaagaaag gcaactagga tgaggtttca aaagacggaa 3600
   gacgactaaa tctgctctaa aaagtaaact agaatttgtg cacttgctta gtggattgta 3660
   ttggattgtg acttgatgta cagcgctaag accttactgg gatgggctct gtctacagca 3720
   atgtgcagaa caagcattcc cacttttcct caagataact gaccaagtgt tttcttagaa 3780
   ccaaagtttt taaagttgct aagatatatt tgcctgtaag atagctgtag agatatttgg 3840
   ggtggggaca gtgagtttgg atggggaaag gggtgggagg gtggtgttgg gaagaaaaat 3900
   tggtcagctt ggctcgggga gaaacctggt aacataaaag cagttcagtg gcccagaggt 3960
   tatttttttc ctattgctct gaagactgca ctggttgctg caaagctcag gcctgaatga 4020
   gcaggaaaca aaaaaggcct tgcgacccag ctgccataac caccttagaa ctaccagacg 4080
   agcacatcag aaccctttga cagccatccc aggtctaaag ccacaagttt cttttctata 4140
   cagtcacaac tgcagtaggc agtgaggaag ccagagaaat gcgatagcgg catttctcta 4200
   aagcgggtta ttaaggatat atacagttac actttttgct gcttttattt tcttccaagc 4260
   caatcaatca gccagttcct agcagagtca gcacatgaac aagatctaag tcatttcttg 4320
   atgtgagcac tggagctttt tttttttaca acgtgacagg aagaggaggg agagggtgac 4380
   gaacaccagg catttccagg ggctatattt cactgtttgt tgttgctttg ttctgttata 4440
   ttgttggttg ttcatagttt ttgttgaagc tctagcttaa gaagaaactt tttttaaaaa 4500
   gactgtttgg ggattctttt tccttattat atactgattc tacaaaatag aaactacttc 4560
   attttaattg tatattattc aagcaccttt gttgaagctc aaaaaaaatg atgcctcttt 4620
   aaactttagc aattatagga gtatttatgt aactatctta tgcttcaaaa aacaaaagta 4680
   tttgtgtgca tgtgtatata atatatatat atacatatat atttatacac atacaattta 4740
   tgttttcctg ttgaatgtat ttttatgaga ttttaaccag aacaaaggca gataaacagg 4800
   cattccatag cagtgctttt gatcacttac aaattttttg aataacacaa aatctcattc 4860
   tacctgcagt ttaattggaa agatgtgtgt gtgagagtat gtatgtgtgt gtgtgtgtgt 4920
   gtgtgtgcgc gcgcacgcac gccttgagca gtcagcattg cacctgctat ggagaagggt 4980
   attcctttat taaaatcttc ctcatttgga tttgctttca gttggttttc aatttgctca 5040
   ctggccagag acattgatgg cagttcttat ctgcatcact aatcagctcc tggatttttt 5100
   tttttttttt tcaaacaatg gtttgaaaca actactggaa tattgtccac aataagctgg 5160
   aagtttgttg tagtatgcct caaatataac tgactgtata ctatagtggt aacttttcaa 5220
   acagccctta gcacttttat actaattaac ccatttgtgc attgagtttt cttttaaaaa 5280
   tgcttgttgt gaaagacaca gatacccagt atgcttaacg tgaaaagaaa atgtgttctg 5340
   ttttgtaaag gaactttcaa gtattgttgt aaatacttgg acagaggttg ctgaacttta 5400
   aaaaaaatta atttattatt ataatgacct aatttattaa tctgaagatt aaccattttt 5460
   ttgtcttaga atatcaaaaa gaaaaagaaa aaggtgttct agctgtttgc atcaaaggaa 5520
   aaaaagattt attatcaagg ggcaatattt ttatcttttc caaaataaat ttgttaatga 5580
   tacattacaa aaatagattg acatcagcct gattagtata aattttgttg gtaattaatc 5640
   cattcctggc ataaaaagtc tttatcaaaa aaaattgtag atgcttgctt tttgtttttt 5700
   caatcatggc catattatga aaatactaac aggatatagg acaaggtgta aattttttta 5760
   ttattatttt aaagatatga tttatcctga gtgctgtatc tattactctt ttactttggt 5820
   tcctgttgtg ctcttgtaaa agaaaaatat aatttcctga agaataaaat agatatatgg 5880
   cacttggagt gcatcatagt tctacagttt gtttttgttt tcttcaaaaa agctgtaaga 5940
   gaattatctg caacttgatt cttggcagga aataaacatt ttgagttgaa atcaaaaaaa 6000
   aaaaaaaaaa a
<210> 34a
   <211> 1036
   <212> DNA
   <213> Human
<400> 34a
<210> 35
   <211> 716
   <212> DNA
   <213> Human
<400> 35
<210> 36
   <211> 395
   <212> DNA
   <213> Human
<400> 36
<210> 37
   <211> 134
   <212> DNA
   <213> Human
<400> 37
   ccctcgagcg gccgcccggg caggtacttt taccaccgaa ttgttcactt gactttaaga 60
   aacccataaa gctgcctggc tttcagcaac aggcctatca acaccatggt gagtctccat 120
   aagggacacc gtgt
<210> 38
   <211> 644
   <212> DNA
   <213> Human
<400> 38
   aagcctgttg tcatggggga ggtggtggcg cttggtggcc actggcggcc gaggtagagg 60
   cagtggcgct tgagttggtc gggggcagcg gcagatttga ggcttaagca acttcttccg 120
   gggaagagtg ccagtgcagc cactgttaca attcaagatc ttgatctata tccatagatt 180
   ggaatattgg tgggccagca atcctcagac gcctcactta ggacaaatga ggaaactgag 240
   gcttggtgaa gttacgaaac ttgtccaaaa tcacacaact tgtaaagggc acagccaaga 300
   ttcagagcca ggctgtaaaa attaaaatga acaaattacg gcaaagtttt aggagaaaga 360
   aggatgttta tgttccagag gccagtcgtc cacatcagtg gcagacagat gaagaaggcg 420
   ttcgcaccgg aaaatgtagc ttcccggtta agtaccttgg ccatgtagaa gttgatgaat 480
   caagaggaat gcacatctgt gaagatgctg taaaaagatt gaaagctgaa aggaagttct 540
   tcaaaggctt ctttggaaaa actggaaaga aagcagttaa agcagtttct gtgggtctaa 600
   gcagatggac tcagaggttg tggatgaaaa actaaggacc tcat
<210> 39
   <211> 657
   <212> DNA
   <213> Human
<400> 39
   ctttttgttt gggttttcca atgtagatgt ctcagtgaaa tgtgcagata tactttgttc 60
   cttatatggt caccagtgtt aattatggac aaatacatta aaacaagggt tcctggccca 120
   gcctcccatc taatctcttt gatactcttg gaatctaagt ctgaggagcg atttctgaat 180
   tagccagtgt tgtaccaact ttctgttagg aattgtatta gaataacctt tctttttcag 240
   acctgctcag tgagacatct tggggaatga agtaggaaaa tagacatttg gtggaaaaac 300
   agcaaaatga gaacattaaa aagactcatt caagtatgag tataaagggc atggaaattc 360
   tggtcctttg agcaaaatga gaagaaaaaa ttctgctcag cagtattcac tgtgttaaga 420
   ttttttgttt tttacacgaa tggaaaaatg atgtgtaagt ggtatagatt ttaatcagct 480
   aacagtcact ccagagattt tgatcagcac caattcctat agtagtaagt atttaaaagt 540
   taagaaatac tactacattt aacattataa agtagagttc tggacataac tgaaaattag 600
   atgtttgctt caatagaaat ttgttcccac ttgtattttc aacaaaatta tcggaac
<210> 40
   <211> 1328
   <212> DNA
   <213> Human
<400> 40
   acaattttaa aataactagc aattaatcac agcatatcag gaaaaagtac acagtgagtt 60
   ctggttagtt tttgtaggct cattatggtt agggtcgtta agatgtatat aagaacctac 120
   ctatcatgct gtatgtatca ctcattccat tttcatgttc catgcatact cgggcatcat 180
   gctaatatgt atccttttaa gcactctcaa ggaaacaaaa gggcctttta tttttataaa 240
   ggtaaaaaaa attccccaaa tattttgcac tgaatgtacc aaaggtgaag ggacattaca 300
   atatgactaa cagcaactcc atcacttgag aagtataata gaaaatagct tctaaatcaa 360
   acttccttca cagtgccgtg tctaccacta caaggactgt gcatctaagt aataattttt 420
   taagattcac tatatgtgat agtatgatat gcatttattt aaaatgcatt agactctctt 480
   ccatccatca aatactttac aggatggcat ttaatacaga tatttcgtat ttcccccact 540
   gctttttatt tgtacagcat cattaaacac taagctcagt taaggagcca tcagcaacac 600
   tgaagagatc agtagtaaga attccatttt ccctcatcag tgaagacacc acaaattgaa 660
   actcagaact atatttctaa gcctgcattt tcactgatgc ataattttct tagtaatatt 720
   aagagacagt ttttctatgg catctccaaa actgcatgac atcactagtc ttacttctgc 780
   ttaattttat gagaaggtat tcttcatttt aattgctttt gggattactc cacatctttg 840
   tttatttctt gactaatcag attttcaata gagtgaagtt aaattggggg tcataaaagc 900
   attggattga catatggttt gccagcctat gggtttacag gcattgccca aacatttctt 960
   tgagatctat atttataagc agccatggaa ttcctattat gggatgttgg caatcttaca 1020
   ttttatagag gtcatatgca tagttttcat aggtgttttg taagaactga ttgctctcct 1080
   gtgagttaag ctatgtttac tactgggacc ctcaagagga ataccactta tgttacactc 1140
   ctgcactaaa ggcacgtact gcagtgtgaa gaaatgttct gaaaaagggt tatagaaatc 1200
   tggaaataag aaaggaagag ctctctgtat tctataattg gaagagaaaa aaagaaaaac 1260
   ttttaactgg aaatgttagt ttgtacttat tgatcatgaa tacaagtata tatttaattt 1320
   tgaaaaaa
<210> 41
   <211> 987
   <212> DNA
   <213> Human
<400> 41
   aacagagact ggcacaggac ctcttcattg caggaagatg gtagtgtagg caggtaacat 60
   tgagctcttt tcaaaaaagg agagctcttc ttcaagataa ggaagtggta gttatggtgg 120
   taacccccgg ctatcagtcc ggatggttgc cacccctcct gctgtaggat ggaagcagcc 180
   atggagtggg agggaggcgc aataagacac ccctccacag agcttggcat catgggaagc 240
   tggttctacc tcttcctggc tcctttgttt aaaggcctgg ctgggagcct tccttttggg 300
   tgtctttctc ttctccaacc aacagaaaag actgctcttc aaaggtggag ggtcttcatg 360
   aaacacagct gccaggagcc caggcacagg gctgggggcc tggaaaaagg agggcacaca 420
   ggaggaggga ggagctggta gggagatgct ggctttacct aaggtctcga aacaaggagg 480
   gcagaatagg cagaggcctc tccgtcccag gcccattttt gacagatggc gggacggaaa 540
   tgcaatagac cagcctgcaa gaaagacatg tgttttgatg acaggcagtg tggccgggtg 600
   gaacaagcac aggccttgga atccaatgga ctgaatcaga accctaggcc tgccatctgt 660
   cagccgggtg acctgggtca attttagcct ctaaaagcct cagtctcctt atctgcaaaa 720
   tgaggcttgt gatacctgtt ttgaagggtt gctgagaaaa ttaaagataa gggtatccaa 780
   aatagtctac ggccatacca ccctgaacgt gcctaatctc gtaagctaag cagggtcagg 840
   cctggttagt acctggatgg ggagagtatg gaaaacatac ctgcccgcag ttggagttgg 900
   actctgtctt aacagtagcg tggcacacag aaggcactca gtaaatactt gttgaataaa 960
   tgaagtagcg atttggtgtg aaaaaaa
<210> 42
   <211> 956
   <212> DNA
   <213> Human
<400> 42
   cggacggtgg ggcggacgcg tgggtgcagg agcagggcgg ctgccgactg ccccaaccaa 60
   ggaaggagcc cctgagtccg cctgcgcctc catccatctg tccggccaga gccggcatcc 120
   ttgcctgtct aaagccttaa ctaagactcc cgccccgggc tggccctgtg cagaccttac 180
   tcaggggatg tttacctggt gctcgggaag ggaggggaag gggccgggga gggggcacgg 240
   caggcgtgtg gcagccacac gcaggcggcc agggcggcca gggacccaaa gcaggatgac 300
   cacgcacctc cacgccactg cctcccccga atgcatttgg aaccaaagtc taaactgagc 360
   tcgcagcccc cgcgccctcc ctccgcctcc catcccgctt agcgctctgg acagatggac 420
   gcaggccctg tccagccccc agtgcgctcg ttccggtccc cacagactgc cccagccaac 480
   gagattgctg gaaaccaagt caggccaggt gggcggacaa aagggccagg tgcggcctgg 540
   ggggaacgga tgctccgagg actggactgt ttttttcaca catcgttgcc gcagcggtgg 600
   gaaggaaagg cagatgtaaa tgatgtgttg gtttacaggg tatatttttg ataccttcaa 660
   tgaattaatt cagatgtttt acgcaaggaa ggacttaccc agtattactg ctgctgtgct 720
   tttgatctct gcttaccgtt caagaggcgt gtgcaggccg acagtcggtg accccatcac 780
   tcgcaggacc aagggggcgg ggactgctgg ctcacgcccc gctgtgtcct ccctcccctc 840
   ccttccttgg gcagaatgaa ttcgatgcgt attctgtggc cgccatctgc gcagggtggt 900
   ggtattctgt catttacaca cgtcgttcta attaaaaagc gaattatact ccaaaa
<210> 43
   <211> 536
   <212> DNA
   <213> Human
<400> 43
   aaataaacac ttccataaca ttttgttttc gaagtctatt aatgcaatcc cacttttttc 60
   cccctagttt ctaaatgtta aagagagggg aaaaaaggct caggatagtt ttcacctcac 120
   agtgttagct gtcttttatt ttactcttgg aaatagagac tccattaggg ttttgacatt 180
   ttgggaaccc agttttacca ttgtgtcagt aaaacaataa gatagtttga gagcatatga 240
   tctaaataaa gacatttgaa gggttagttt gaattctaaa agtaggtaat agccaaatag 300
   cattctcatc ccttaacaga caaaaactta tttgtcaaaa gaattagaaa aggtgaaaat 360
   attttttcca gatgaaactt gtgccacttc caattgacta atgaaataca aggagacaga 420
   ctggaaaaag tgggttatgc cacctttaaa accctttctg gtaaatatta tggtagctaa 480
   agggtggttt ccccggcacc tggacctgga caggtagggt tccgtggtta accagt
<210> 44
   <211> 1630
   <212> DNA
   <213> Human
<400> 44
   ggggagggac gagtatggaa ccctgaaggt agcaagtcca ggcactggcc tgaccatccg 60
   gctccctggg caccaagtcc caggcaggag cagctgtttt ccatcccttc ccagacaagc 120
   tctattttta tcacaatgac ctttagagag gtctcccagg ccagctcaag gtgtcccact 180
   atcccctctg gagggaagag gcaggaaaat tctccccggg tccctgtcat gctactttct 240
   ccatcccagt tcagactgtc caggacatct tatctgcagc cataagagaa ttataaggca 300
   gtgatttccc ttaggcccag gacttgggcc tccagctcat ctgttccttc tgggcccatt 360
   catggcaggt tctgggctca aagctgaact ggggagagaa gagatacaga gctaccatgt 420
   gactttacct gattgccctc agtttggggt tgcttattgg gaaagagaga gacaaagagt 480
   tacttgttac gggaaatatg aaaagcatgg ccaggatgca tagaggagat tctagcaggg 540
   gacaggattg gctcagatga cccctgaggg ctcttccagt cttgaaatgc attccatgat 600
   attaggaagt cgggggtggg tggtggtggt gggctagttg ggtttgaatt taggggccga 660
   tgagcttggg tacgtgagca gggtgttaag ttagggtctg cctgtatttc tggtcccctt 720
   ggaaatgtcc ccttcttcag tgtcagacct cagtcccagt gtccatatcg tgcccagaaa 780
   agtagacatt atcctgcccc atcccttccc cagtgcactc tgacctagct agtgcctggt 840
   gcccagtgac ctgggggagc ctggctgcag gccctcactg gttccctaaa ccttggtggc 900
   tgtgattcag gtccccaggg gggactcagg gaggaatatg gctgagttct gtagtttcca 960
   gagttggctg gtagagcctt ctagaggttc agaatattag cttcaggatc agctgggggt 1020
   atggaattgg ctgaggatca aacgtatgta ggtgaaagga taccaggatg ttgctaaagg 1080
   tgagggacag tttgggtttg ggacttacca gggtgatgtt agatctggaa cccccaagtg 1140
   aggctggagg gagttaaggt cagtatggaa gatagggttg ggacagggtg ctttggaatg 1200
   aaagagtgac cttagagggc tccttgggcc tcaggaatgc tcctgctgct gtgaagatga 1260
   gaaggtgctc ttactcagtt aatgatgagt gactatattt accaaagccc ctacctgctg 1320
   ctgggtccct tgtagcacag gagactgggg ctaagggccc ctcccaggga agggacacca 1380
   tcaggcctct ggctgaggca gtagcataga ggatccattt ctacctgcat ttcccagagg 1440
   actagcagga ggcagccttg agaaaccggc agttcccaag ccagcgcctg gctgttctct 1500
   cattgtcact gccctctccc caacctctcc tctaacccac tagagattgc ctgtgtcctg 1560
   cctcttgcct cttgtagaat gcagctctgg ccctcaataa atgcttcctg cattcatctg 1620
   caaaaaaaaa
<210> 45
   <211> 169
   <212> DNA
   <213> Human
<400> 45
   tcttttgctt ttagcttttt atttttgtat taacaggagt cttattacac ataggtctga 60
   taaaactggt ttatgatctt cagtctgatt ccagtgctgc ataactagat aacgtatgaa 120
   ggaaaaacga cgacgaacaa aaaagtaagt gcttggaaga cttagttga
<210> 46
   <211> 769
   <212> DNA
   <213> Human
<400> 46
   tgcaggtcat atttactatc ggcaataaaa ggaagcaaag cagtattaag cagcggtgga 60
   atttgtcgct ttcacttttt ataaagtgct acataaaatg tcatatttcc aaatttaaaa 120
   acataactcc agttcttacc atgagaacag catggtgatc acgaaggatc ttcttgaaaa 180
   aaacaaaaac aaaaacaaaa aacaatgatc tcttctgggt atcacatcaa atgagataca 240
   aaggtgtact aggcaatctt agagatctgg caacttattt tatatataag gcatctgtga 300
   ccaagagacg ttatgaatta aatgtacaaa tgtattatgt ataaatgtat taaatgcaag 360
   cttcatataa tgacaccaat gtctctaagt tgctcagaga tcttgactgg ctgtggccct 420
   ggccagctcc tttcctgata gtctgattct gccttcatat ataggcagct cctgatcatc 480
   catgccagtg aatgagaaaa caagcatgga atatataaac tttaacatta aaaaatgttt 540
   tattttgtaa taaaatcaaa tttcccattg aaaccttcaa aaactttgca gaatgaggtt 600
   ttgatatatg tgtacaagta gtaccttctt agtgcaagaa aacatcatta tttctgtctg 660
   cctgcctttt tgtttttaaa aatgaagact atcattgaaa caagtttgtc ttcagtatca 720
   ggacatgttg acggagagga aaggtaggaa agggttaggg atagaagcc
<210> 47
   <211> 2529
   <212> DNA
   <213> Human
<400> 47
   tttagttcat agtaatgtaa aaccatttgt ttaattctaa atcaaatcac tttcacaaca 60
   gtgaaaatta gtgactggtt aaggtgtgcc actgtacata tcatcatttt ctgactgggg 120
   tcaggacctg gtcctagtcc acaagggtgg caggaggagg gtggaggcta agaacacaga 180
   aaacacacaa aagaaaggaa agctgccttg gcagaaggat gaggtggtga gcttgccgag 240
   ggatggtggg aagggggctc cctgttgggg ccgagccagg agtcccaagt cagctctcct 300
   gccttactta gctcctggca gagggtgagt ggggacctac gaggttcaaa atcaaatggc 360
   atttggccag cctggcttta ctaacaggtt cccagagtgc ctctgttggc tgagctctcc 420
   tgggctcact ccatttcatt gaagagtcca aatgattcat tttcctaccc acaacttttc 480
   attattcttc tggaaaccca tttctgttga gtccatctga cttaagtcct ctctccctcc 540
   actagttggg gccactgcac tgaggggggt cccaccaatt ctctctagag aagagacact 600
   ccagaggccc ctgcaacttt gcggatttcc agaaggtgat aaaaagagca ctcttgagtg 660
   ggtgcccagg aatgtttaaa atctatcagg cacactataa agctggtggt ttcttcctac 720
   caagtggatt cggcatatga accacctact caatacttta tattttgtct gtttaaacac 780
   tgaactctgg tgttgacagg tacaaaggag aagagatggg gactgtgaag aggggagggc 840
   ttccctcatc ttcctcaaga tctttgtttc cataaactat gcagtcataa ttgagaaaaa 900
   gcaatagatg gggcttccta ccatttgttg gttattgctg gggttagcca ggagcagtgt 960
   ggatggcaaa gtaggagaga ggcccagagg aaagcccatc tccctccagc tttggggtct 1020
   ccagaaagag gctggatttc tgggatgaag cctagaaggc agagcaagaa ctgttccacc 1080
   aggtgaacag tcctacctgc ttggtaccat agtccctcaa taagattcag aggaagaagc 1140
   ttatgaaact gaaaatcaaa tcaaggtatt gggaagaata atttcccctc gattccacag 1200
   gagggaagac cacacaatat cattgtgctg gggctcccca aggccctgcc acctggcttt 1260
   acaaatcatc aggggttgcc tgcttggcag tcacatgctt ccctggtttt agcacacata 1320
   caaggagttt tcagggaact ctatcaagcc ataccaaaat cagggtcaca tgtgggtttc 1380
   ccctttcctt gcctcttcat aaaagacaac ttggcttctg aggatggtgg tcttttgcat 1440
   gcagttgggc tgacctgaca aagcccccag tttcctgtgg caggttctgg gagaggatgc 1500
   attcaagctt ctgcagccta ggggacaggg ctgcttgttc agttattact gcctcggagc 1560
   tccaaatccc accaaagtcc tgactccagg tctttcctaa tgcacagtag tcagtctcag 1620
   cttcggcagt attctcggct gtatgttctc tggcagagag aggcagatga acatagtttt 1680
   agggagaaag ctgatgggaa acctgtgagt taagccacat gtctcaccag gaataattta 1740
   tgccaggaaa ccaggaagtc attcaagttg ttctctgagg ccaaagacac tgagcacagc 1800
   ccagagccaa taaaagatct ttgagtctct ggtgaattca cgaagtgacc ccagctttag 1860
   ctactgcaat tatgattttt atgggacagc aatttcttgc atctctacag aggaagaaga 1920
   gggggagtgg gaggggaagg aaagagaaca gagcggcact gggatttgaa aggggaacct 1980
   ctctatctga ggagccccca ctggcttcag aagcaactta ccaaggggta tttaaagaca 2040
   tgaaaatttc cagaaatacc atttggtgca tccctttgtt tctgtaatat taaactcagg 2100
   tgaaattata ctctgacagt ttctctcttt ctgcctcttc cctctgcaga gtcaggacct 2160
   gcagaactgg ctgaaacaag atttcatggt gtcacccatg agagatgact caatgccaag 2220
   gcctgaagtt atagagtgtt tacagcggtg gcgatattca ggggtcatcg ccaactggtc 2280
   tcgagttcca aagctctgat gaagaaacaa gactccttga tgtgttactg atcccactga 2340
   ttccaggagt caagattagc caggaagcca aacaccagga gttggggtgg cacgtcacca 2400
   gtccagagcc ctgccacgga tgtacgcagg agcccagcat taggcaatca ggagccagaa 2460
   catgatcacc agggccacaa ataggaagag gcgtgacagg aactgctcgt ccacatacct 2520
   ggggtgtcc
<210> 48
   <211> 1553
   <212> DNA
   <213> Human
<400> 48
<210> 49
   <211> 921
   <212> DNA
   <213> Human
<400> 49
   ctgtggtccc agctactcag gaggctgagg cgggaggatt gcttgagccc aggagttgga 60
   tgttgcagtg agccaagatc gcaccattgc cctccactct gggccacgga gcaataccct 120
   gtctcagaaa acaaacaaca aaaagcagaa acgctgaagg ggtcggttta cgggaaaacc 180
   gcctgtcaga acacttggct actcctaccc cagatcagtg gacctgggaa tgagggttgg 240
   tcccgggagg cttttctcca agctgttgcc accagacccg ccatgggaac cctggccaca 300
   gaagcctccc ggggagtgag ccagagcctg gaccgctgtg ctgatgtgtc tggggtggag 360
   ggagggtggg gagtgtgcaa gggtgtgtgt gtgcccgggg ggtgttcatg ggcaagcatg 420
   tgcgtgcctg tgtgtgtgcg tgcccctccc ctgcagccgt cggtggtatc tccctccagc 480
   cccttcgcca ccttctgagc attgtctgtc cacgtgagac tgcccagaga cagcagagct 540
   ccacgtggtt ttaaggggag acctttccct ggacctgggg gtctcgccgt atctcatgac 600
   caggtgctaa atgacccgac atgcatcacc tgcctttcga tgaccaacct ccctgtcccc 660
   gtcccgctga cctgcccccg tggcgtctca cggtgatgcc tgctcctgac attggtgttc 720
   actgtagcaa actacattct ggatgggaat tttcatgtac atgtgtggca tgtggaaaat 780
   ttcaaataaa atggacttga tttagaaagc caaaaagctg tgtggtcctt ccagcacgga 840
   tactttgacc tcttgcctac aaccccttcc ttgggtccga ggctggtagc tttgttcact 900
   tcagatggtt gggggcgggt g
<210> 50
   <211> 338
   <212> DNA
   <213> Human
<400> 50
   atgatctatc tagatgccct accgtaaaat caaaacacaa aaccctactg actcattccc 60
   tcccttccag atattacccc atttctctac ttcccattgt agccaaactt tccaaaaatt 120
   catgttctgt cttcatttcc tcatgttcaa cccaccctgt cttagctacc acccctcagt 180
   aacgacctag cctgggtaga aacaaatgtc agcatgatac catactcaat gatccttcgt 240
   cactgttgtc attgtcatca ttccatggcc ttactttccc tctcagcgcc atttgctaca 300
   gtaagaaact ttctttcttg aattcttggt tctcttgg
<210> 51
   <211> 1191
   <212> DNA
   <213> Human
<400> 51
   ctagcaagca ggtaaacgag ctttgtacaa acacacacag accaacacat ccggggatgg 60
   ctgtgtgttg ctagagcaga ggctgattaa acactcagtg tgttggctct ctgtgccact 120
   cctggaaaat aatgaattgg gtaaggaaca gttaataaga aaatgtgcct tgctaactgt 180
   gcacattaca acaaagagct ggcagctcct gaaggaaaag ggcttgtgcc gctgccgttc 240
   aaacttgtca gtcaactcat gccagcagcc tcagcgtctg cctccccagc acaccctcat 300
   tacatgtgtc tgtctggcct gatctgtgca tctgctcgga gacgctcctg acaagtcggg 360
   aatttctcta tttctccact ggtgcaaaga gcggatttct ccctgcttct cttctgtcac 420
   ccccgctcct ctcccccagg aggctccttg atttatggta gctttggact tgcttccccg 480
   tctgactgtc cttgacttct agaatggaag aagctgagct ggtgaaggga agactccagg 540
   ccatcacaga taaaagaaaa atacaggaag aaatctcaca gaagcgtctg aaaatagagg 600
   aagacaaact aaagcaccag catttgaaga aaaaggcctt gagggagaaa tggcttctag 660
   atggaatcag cagcggaaaa gaacaggaag agatgaagaa gcaaaatcaa caagaccagc 720
   accagatcca ggttctagaa caaagtatcc tcaggcttga gaaagagatc caagatcttg 780
   aaaaagctga actgcaaatc tcaacgaagg aagaggccat tttaaagaaa ctaaagtcaa 840
   ttgagcggac aacagaagac attataagat ctgtgaaagt ggaaagagaa gaaagagcag 900
   aagagtcaat tgaggacatc tatgctaata tccctgacct tccaaagtcc tacatacctt 960
   ctaggttaag gaaggagata aatgaagaaa aagaagatga tgaacaaaat aggaaagctt 1020
   tatatgccat ggaaattaaa gttgaaaaag acttgaagac tggagaaagt acagttctgt 1080
   cttccaatac ctctggccat cagatgactt taaaaggtac aggagtaaaa gtttaagatg 1140
   atgggcaaaa gtccagtgta ttcagtaaag tgctaatcac aagttggagg t
<210> 52
   <211> 1200
   <212> DNA
   <213> Human
<400> 52
   aacagggact ctcactctat caaccccagg ctggagtccg gtgcgcccac cctggctccc 60
   tgcaacctcc gcctcccagg ctcaagcaac tctcctgcct cagtcgctct agtagctggg 120
   actacaggca cacaccacca tgcccagcca atttttgcat tttttgtaga gacagggttt 180
   cgccttctgt ccaggccggc atcatatact ttaaatcatg cccagatgac tttaatacct 240
   aatacaatat atcaggttgg tttaaaaata attgcttttt tattattttt gcatttttgc 300
   accaacctta atgctatgta aatagttgtt atactgttgc ttaacaacag tatgacaatt 360
   ttggcttttt ctttgtatta ttttgtattt ttttttttta ttgtgtggtc tttttttttt 420
   ttctcagtgt tttcaattcc tccttggttg aatccatgga tgcaaaaccc acagatatga 480
   agggctggct atatatgcat tgatgattgt cctattatat tagttataaa gtgtcattta 540
   atatgtagtg aaagttatgg tacagtggaa agagtagttg aaaacataaa catttggacc 600
   tttcaagaaa ggtagcttgg tgaagttttt caccttcaaa ctatgtccca gtcagggctc 660
   tgctactaat tagctataat ctttgcacaa attacatcac ctttgagtct cagttgcctc 720
   acctgtaaaa tgaaagaact ggatactctc taaggtcact tccagccctg tcattctata 780
   actctgttat gctgaggaag aaattcacat tgtgttaact gtatgagtca aactgaaaat 840
   gattattaaa gtgggaaaaa gccaattgct tctcttagaa agctcaacta aatttgagaa 900
   gaataatctt ttcaattttt taagaattta aatattttta agggtttgac ctatttattt 960
   agagatgggg tctcactctg tcacccagac tggagtacag tggcacaatc atagctcact 1020
   gctgcctcaa attcatgggc tcaagtgatc ctcctgcctc tgcctccaga gtagctgcga 1080
   ctatgggcat gtgccaccac gcctggctaa catttgtatt gacctattta tttattgtga 1140
   tttatatctt tttttttttt tctttttttt ttttttacaa aatcagaaat acttattttg 1200
<210> 53
   <211> 989
   <212> DNA
   <213> Human
<400> 53
   aagccaccac tcaaaacttc ctatacattt tcacagcaga gacaagtgaa catttatttt 60
   tatgcctttc ttcctatgtg tatttcaagt ctttttcaaa acaaggcccc aggactctcc 120
   gattcaatta gtccttgggc tggtcgactg tgcaggagtc cagggagcct ctacaaatgc 180
   agagtgactc tttaccaaca taaaccctag atacatgcaa aaagcaggac ccttcctcca 240
   ggaatgtgcc atttcagatg cacagcaccc atgcagaaaa gctggaattt tccttggaac 300
   cgactgtgat agaggtgctt acatgaacat tgctactgtc tttctttttt tttgagacag 360
   gtttcgcttg tgcccaggct gagtgcaatg cgtgatctca ctcactgcaa ttccacctcc 420
   aggttcaagc attctcctgc tcagcctcct agtagctggg ttacaggcac tgccaccatg 480
   ccggctaatt ttgtattttt gtagagatgg atttctccat ttggtcaggc ggtctcgaac 540
   cccaacctca gtgatctgcc acctcagcct cctaagtgtt ggattacagg atgagccacc 600
   cgaccggcca ctactgtctt tctttgaccc ttccagtttc gaagataaag aggaaataat 660
   ttctctgaag tacttgataa aatttccaaa caaaacacat gtccacttca ctgataaaaa 720
   atttaccgca gtttggcacc taagagtatg acaacagcaa taaaaagtaa tttcaaagag 780
   ttaagatttc ttcagcaaaa tagatgattc acatcttcaa gtcctttttg aaatcagtta 840
   ttaatattat tctttcctca tttccatctg aatgactgca gcaatagttt tttttttttt 900
   tttttttttt ttgcgagatg gaatctcgct ctgtcgccca gcgggagtgc actggcgcaa 960
   gcccggctca ccgcaatctc tgccacccg
<210> 54
   <211> 250
   <212> DNA
   <213> Human
<400> 54
   catttcccca ttggtcctga tgttgaagat ttagttaaag aggctgtaag tcaggttcga 60
   gcagaggcta ctacaagaag tagggaatca agtccctcac atgggctatt aaaactaggt 120
   agtggtggag tagtgaaaaa gaaatctgag caacttcata acgtaactgc ctttcaggga 180
   aaagggcatt ctttaggaac tgcatctggt aacccacacc ttgatccaag agctagggaa 240
   acttcagttg
<210> 55
   <211> 2270
   <212> DNA
   <213> Human
<400> 55
   gcgcccccga gcagcgcccg cgccctccgc gccttctccg ccgggacctc gagcgaaaga 60
   ggcccgcgcg ccgcccagcc ctcgcctccc tgcccaccgg gcacaccgcg ccgccacccc 120
   gaccccgctg cgcacggcct gtccgctgca caccagcttg ttggcgtctt cgtcgccgcg 180
   ctcgccccgg gctactcctg cgcgccacaa tgagctcccg catcgccagg gcgctcgcct 240
   tagtcgtcac ccttctccac ttgaccaggc tggcgctctc cacctgcccc gctgcctgcc 300
   actgccccct ggaggcgccc aagtgcgcgc cgggagtcgg gctggtccgg gacggctgcg 360
   gctgctgtaa ggtctgcgcc aagcagctca acgaggactg cagcaaaacg cagccctgcg 420
   accacaccaa ggggctggaa tgcaacttcg gcgccaagtc caccgctctg aaggggatct 480
   gcagagctca gtcagagggc agaccctgtg aatataactc cagaatctac caaaacgggg 540
   aaagtttcca gcccaactgt aaacatcagt gcacatgtat tgatggcgcc gtgggctgca 600
   ttcctctgtg tccccaagaa ctatctctcc ccaacttggg ctgtcccaac cctcggctgg 660
   tcaaagttac cgggcagtgc tgcgaggagt gggtctgtga cgaggatagt atcaaggacc 720
   ccatggagga ccaggacggc ctccttggca aggagctggg attcgatgcc tccgaggtgg 780
   agttgacgag aaacaatgaa ttgattgcag ttggaaaagg cagctcactg aagcggctcc 840
   ctgtttttgg aatggagcct cgcatcctat acaacccttt acaaggccag aaatgtattg 900
   ttcaaacaac ttcatggtcc cagtgctcaa agacctgtgg aactggtatc tccacacgag 960
   ttaccaatga caaccctgag tgccgccttg tgaaagaaac ccggatttgt gaggtgcggc 1020
   cttgtggaca gccagtgtac agcagcctga aaaagggcaa gaaatgcagc aagaccaaga 1080
   aatcccccga accagtcagg tttacttacg ctggatgttt gagtgtgaag aaataccggc 1140
   ccaagtactg cggttcctgc gtggacggcc gatgctgcac gccccagctg accaggactg 1200
   tgaagatgcg gttccgctgc gaagatgggg agacattttc caagaacgtc atgatgatcc 1260
   agtcctgcaa atgcaactac aactgcccgc atgccaatga agcagcgttt cccttctaca 1320
   ggctgttcaa tgacattcac aaatttaggg actaaatgct acctgggttt ccagggcaca 1380
   cctagacaaa caagggagaa gagtgtcaga atcagaatca tggagaaaat gggcgggggt 1440
   ggtgtgggtg atgggactca ttgtagaaag gaagccttgc tcattcttga ggagcattaa 1500
   ggtatttcga aactgccaag ggtgctggtg cggatggaca ctaatgcagc cacgattgga 1560
   gaatactttg cttcatagta ttggagcaca tgttactgct tcattttgga gcttgtggag 1620
   ttgatgactt tctgttttct gtttgtaaat tatttgctaa gcatattttc tctaggcttt 1680
   tttccttttg gggttctaca gtcgtaaaag agataataag attagttgga cagtttaaag 1740
   cttttattcg tcctttgaca aaagtaaatg ggagggcatt ccatcccttc ctgaaggggg 1800
   acactccatg agtgtctgtg agaggcagct atctgcactc taaactgcaa acagaaatca 1860
   ggtgttttaa gactgaatgt tttatttatc aaaatgtagc ttttggggag ggaggggaaa 1920
   tgtaatactg gaataatttg taaatgattt taattttata ttcagtgaaa agattttatt 1980
   tatggaatta accatttaat aaagaaatat ttacctaata tctgagtgta tgccattcgg 2040
   tatttttaga ggtgctccaa agtcattagg aacaacctag ctcacgtact caattattca 2100
   aacaggactt attgggatac agcagtgaat taagctatta aaataagata atgattgctt 2160
   ttataccttc agtagagaaa agtctttgca tataaagtaa tgtttaaaaa acatgtattg 2220
   aacacgacat tgtatgaagc acaataaaga ttctgaagct aaaaaaaaaa
<210> 56
   <211> 1636
   <212> DNA
   <213> Human
<400> 56
<210> 57
   <211> 460
   <212> DNA
   <213> Human
<400> 57
   ccatgtgtgt atgagagaga gagagattgg gagggagagg gagctcacta gcgcatatgt 60
   gcctccaggg ggctgcagat gtgtctgagg gtgagcctgg tgaaagagaa gacaaaagaa 120
   tggaatgagc taaagcagcc gcctggggtg ggaggccgag cccatttgta tgcagcaggg 180
   ggcaggagcc cagcaaggga gcctccattc ccaggactct ggagggagct gagaccatcc 240
   atgcccgcag agccctccct cacactccat cctgtccagc cctaattgtg caggtgggga 300
   aactgaggct gggaagtcac atagcaagtg actggcagag ctgggactgg aacccaacca 360
   gcctcctaga ccacggttct tcccatcaat ggaatgctag agactccagc caggtgggta 420
   ccgagctcga attcgtaatc atggtcatag ctgtttcctg
<210> 58
   <211> 1049
   <212> DNA
   <213> Human
<400> 58
   atctgatcaa gaatacctgc cctggtcact ctgcggatgt ttctgtccac ttgttcacat 60
   tgaggaccaa gatatccttt tttacagagg cacttgttcg gtctaacaca gacacctcca 120
   tgacgacatg ctggctcaca ttttgcagtt ctgcagaagt ccccctccca gcctggacta 180
   cagcagcact ttcccgtggg ggtgcagtag ccgtttcgac agagcctgga gcactctgaa 240
   gtcagtgtct gtgcaggttg taccgtggct ctgcattcct caggcattaa aggtcttttg 300
   ggatctacaa ttttgtagag ttttccattg tgagtctggg tcatactttt actgcttgat 360
   aaaatgtaaa cttcacctag ttcatcttct ccaaatccca agatgtgacc ggaaaagtag 420
   cctctacagg acccactagt gccgacacag agtggttttt cttgccactg ctttgtcaca 480
   ggactttgct ggagagttag gaaattccca ttacgatctc caaacacgta gcttccatac 540
   aatctttctg actggcagcc ccggtataca aatccaccaa ccaaaggacc attactgaat 600
   ggcttgaatt ctaaaagtga tggctcactt tcataatctt tcccctttat tatctgtaga 660
   attctggctg atgatctgtt ttttccattg gagtctgaac acagtatcgt taaattgatg 720
   tttatatcag tgggatgtct atccacagca catctgcctg gatcgtggag cccatgagca 780
   aacacttcgg ggggctggtt ggtgctgttg aagtgtgggt tgctccttgg tatggaataa 840
   ggcacgttgc acatgtctgt gtccacatcc agccgtagca ctgagcctgt gaaatcactt 900
   aacccatcca tttcttccat atcatccagt gtaatcatcc catcaccaag aatgatgtac 960
   aaaaacccgt cagggccaaa gagcagttgc cctcccagat gctttctgtg gagttctgca 1020
   acttcaagaa agactctggc tgttctcaa
<210> 59
   <211> 747
   <212> DNA
   <213> Human
<400> 59

### Sequence Identifier

<110> Schering Aktiengesellschaft
<120> Combinations and compositions which interfere with VEGF/ VEGF and angiopoietin/ Tie receptor function and their use II
<130> 51867AEPM1XX00-P
<140>
   <141>
<160> 59
<210> 1
   <211> 1835
   <212> DNA
   <213> Human
<400> 1
   ttttacagtt ttccttttct tcagagttta ttttgaattt tcatttttgg ataaccaagc 60
   agctctttaa gaagaatgca cagaagagtc attctggcac ttttggatag tacataagat 120
   tttctttttt ttttttaaat tttttttaat agtcacattc agctcgcttg ctcaaaccag 180
   actcccacat tgggtgagca agatgagccc ataggattcc agagttaata cgtaaccgta 240
   tatacaaaca gccaaaaaac cataatggtg ccacagggat ggagcaggga agggcatctc 300
   taacgtgtcc tctagtctat cttcgctaaa cagaacccac gttacacatg ataactagag 360
   agcacactgt gttgaaacga ggatgctgac cccaaatggc acttggcagc atgcagttta 420
   aagcaaaaga gacatccttt aataactgta taaaatccag gcagttccat taaaggggtt 480
   aagaaaacca acaacaacaa aaagcgaggg actgtctgtt gtcactgtca aaaaggcact 540
   tggagttaat gggaccagga ttggaggact cttagctgat acagatttca gtacgatttc 600
   attaaaaggc ttggatgtta agagaggaca ctcagcggtt cctgaaggga gacgctgaga 660
   tggaccgctg agaagcggaa cagatgaaca caaaggaatc aaatctttac aaccaaattg 720
   catttaagcg acaacaaaaa aaggcaaacc ccaaaacgca acctaaccaa agcaaaatct 780
   aagcaaaatc agacaacgaa gcagcgatgc atagctttcc tttgagagaa cgcatacctt 840
   gagacgctac gtgccaacct aagttctcaa cgacagcttc acagtaggat tattgtgata 900
   aaaatgactc aagcgatgca aaaagtttca tctgttccca gaatccgagg gagaactgag 960
   gtgatcgtta gagcatagcg acatcacgtg cggtttctta atgtccctgg tggcggatac 1020
   gccgagtcct cggaaggaca tctggacacc actttcagcc acctccttgc aggggcgaca 1080
   tccgccaaag tcatccttta ttccgagtaa taactttaat tcctttctaa catttacacg 1140
   gcaaacagga atgcagtaaa cgtccacgtc cgtcccacgg ctgggctgcc gttccgtttc 1200
   ctccacgaac gggtacgcgc ttccatgaga aaggatattt ggcaatttta tattccacag 1260
   tcaggtgggt ctgcgatagc tcatttaatg ttaaacgcca tcaggggcct ctcctcccgt 1320
   ttctgccagg ggcttttctt gtcttctcct tggcgagctc gtgggcagat cttctctggt 1380
   gggggctggc tgctggctcc gagggggcat ccgcagtccg tctggtcgtc tcctcctgca 1440
   ggctgggcag ctggccacca cttctccgac tcgacccctc caacaagcat cgcagggcac 1500
   tgtcctcggg ggtacagacc gtggtcccac attcgctacc actctgttcc acgtcatcca 1560
   ggtacacgag ctgcgtgtag gccgtgctgt ctggggctcg aggctctttc tgctggtgct 1620
   cttggacggg cgggtagttc tgctgcagag acaaagcatc tccccttccc ttccgggctg 1680
   attttggttc attcatatct acgccagagt ccaaactggc atcattactt ccgttccttc 1740
   cagctctttg gagaatcaat gtatgaatgt ctaacctgac cgttggacct gccatccaag 1800
   gagacgaacc acgcccgggg gtgcggaagc ggcct
<210> 2
   <211> 581
   <212> DNA
   <213> Human
<400> 2
   gttctagatt gttttattca gtaattagct cttaagaccc ctggggcctg tgctacccag 60
   acactaacaa cagtctctat ccagttgctg gttctgggtg acgtgatctc cccatcatga 120
   tcaacttact tcctgtggcc cattagggaa gtggtgacct cgggagctat ttgcctgttg 180
   agtgcacaca cctggaaaca tactgctctc attttttcat ccacatcagt gagaaatgag 240
   tggcccgtta gcaagatata actatgcaat catgcaacaa agctgcctaa taacatttca 300
   tttattacag gactaaaagt tcattattgt ttgtaaagga tgaattcata acctctgcag 360
   agttatagtt catacacagt tgatttccat ttataaaggc agaaagtcct tgttttctct 420
   aaatgtcaag ctttgactga aaactcccgt ttttccagtc actggagtgt gtgcgtatga 480
   aagaaaatct ttagcaatta gatgggagag aagggaaata gtacttgaaa tgtaggccct 540
   cacctcccca tgacatcctc catgagcctc ctgatgtagt g
<210> 3
   <211> 516
   <212> DNA
   <213> Human
<400> 3
   tagagatgtt ggttgatgac ccccgggatc tggagcagat gaatgaagag tctctggaag 60
   tcagcccaga catgtgcatc tacatcacag aggacatgct catgtcgcgg aacctgaatg 120
   gacactctgg gttgattgtg aaagaaattg ggtcttccac ctcgagctct tcagaaacag 180
   ttgttaagct tcgtggccag agtactgatt ctcttccaca gactatatgt cggaaaccaa 240
   agacctccac tgatcgacac agcttgagcc tcgatgacat cagactttac cagaaagact 300
   tcctgcgcat tgcaggtctg tgtcaggaca ctgctcagag ttacaccttt ggatgtggcc 360
   atgaactgga tgaggaaggc ctctattgca acagttgctt ggcccagcag tgcatcaaca 420
   tccaagatgc ttttccagtc aaaagaacca gcaaatactt ttctctggat ctcactcatg 480
   atgaagttcc agagtttgtt gtgtaaagtc cgtctg
<210> 4
   <211> 1099
   <212> DNA
   <213> Human
<400> 4
   cccacaacac aggggccctg aaacacgcca gcctctcctc tgtggtcagc ttggcccagt 60
   cctgctcact ggatcacagc ccattgtagg tggggcatgg tggggatcag ggcccctggc 120
   ccacggggag gtagaagaag acctggtccg tgtaagggtc tgagaaggtg ccctgggtcg 180
   ggggtgcgtc ttggccttgc cgtgccctca tcccccggct gaggcagcga cacagcaggt 240
   gcaccaactc cagcaggtta agcaccaggg agatgagtcc aaccaccaac atgaagatga 300
   tgaagatggt cttctccgtg gggcgagaga caaagcagtc cacgaggtag gggcagggtg 360
   ctcgctggca cacaaacacg ggctccatgg tccagccgta caggcgccac tggccataga 420
   ggaagcctgc ctctagcaca ctcttgcaga gcacactggc gacataggtg cccatcagtg 480
   ctccgcggat gcgcaggcga ccatcttctg ccaccgagat cttggccatc tgacgctcta 540
   cggccgccag cgcccgctcc acctgtgggt ccttggccgg cagtgcccgc agctccccct 600
   ccttctgccg cagccgctct tctcgccgag acaggtaaat gacatggccc aggtagacca 660
   gggtgggtgt gctgacgaag aggaactgca gcacccagta gcggatgtgg gagatgggga 720
   aggcctggtc atagcagacg ttggtgcagc ctggctgggc cgtgttacac tcgaaatctg 780
   actgctcgtc accccacact gactcgccgg ccaggcccag gatgaggatg cggaagatga 840
   agagcaccgt cagccagatc ttacccacca cggtcgagtg ctcctggacc tggtccagca 900
   acttctccac gaagccccag tcacccatgg ctcccgggcc tccgtcggca aggagacaga 960
   gcacgtcagt gtgtcagcat ggcatccttc tcgttcgccc agcaacaagc ctgcagggag 1020
   gtctgccacg cccgttctac cgcctgcctg ccgggcggcc caggtggagg tggggacgat 1080
   ggccggagtg acgcccgcg
<210> 5
   <211> 1015
   <212> DNA
   <213> Human
<400> 5
   gaggataggg agcctggggt caggagtgtg ggagacacag cgagactctg tctccaaaaa 60
   aaaaagtgct ttttgaaaat gttgaggttg aaatgatggg aaccaacatt ctttggattt 120
   agtggggagc ataatagcaa acaccccctt ggttcgcaca tgtacaggaa tgggacccag 180
   ttggggcaca gccatggact tccccgccct ggaatgtgtg gtgcaaagtg gggccagggc 240
   ccagacccaa gaggagaggg tggtccgcag acaccccggg atgtcagcat cccccgacct 300
   gccttctggc ggcacctccc gggtgctgtg ttgagtcagc aggcatgggg tgagagcctg 360
   gtatatgctg ggaacagggt gcaggggcca agcgttcctc cttcagcctt gacttgggcc 420
   atgcaccccc tctcccccaa acacaaacaa gcacttctcc agtatggtgc caggacaggt 480
   gtcccttcag tcctctggtt atgacctcaa gtcctacttg ggccctgcag cccagcctgt 540
   gttgtaacct ctgcgtcctc aagaccacac ctggaagatt cttcttccct ttgaaggaga 600
   atcatcattg ttgctttatc acttctaaga cattttgtac ggcacggaca agttaaacag 660
   aatgtgcttc cctccctggg gtctcacacg ctcccacgag aatgccacag gggccgtgca 720
   ctgggcaggc ttctctgtag aaccccaggg gcttcggccc agaccacagc gtcttgccct 780
   gagcctagag cagggagtcc cgaacttctg cattcacaga ccacctccac aattgttata 840
   accaaaggcc tcctgttctg ttatttcact taaatcaaca tgctattttg ttttcactca 900
   cttctgactt tagcctcgtg ctgagccgtg tatccatgca gtcatgttca cgtgctagtt 960
   acgtttttct tcttacacat gaaaataaat gcataagtgt tagaagaaaa aaaaa
<210> 6
   <211> 2313
   <212> DNA
   <213> Human
<400> 6
   ccagagcagg cctggtggtg agcagggacg gtgcaccgga cggcgggatc gagcaaatgg 60
   gtctggccat ggagcacgga gggtcctacg ctcgggcggg gggcagctct cggggctgct 120
   ggtattacct gcgctacttc ttcctcttcg tctccctcat ccaattcctc atcatcctgg 180
   ggctcgtgct cttcatggtc tatggcaacg tgcacgtgag cacagagtcc aacctgcagg 240
   ccaccgagcg ccgagccgag ggcctataca gtcagctcct agggctcacg gcctcccagt 300
   ccaacttgac caaggagctc aacttcacca cccgcgccaa ggatgccatc atgcagatgt 360
   ggctgaatgc tcgccgcgac ctggaccgca tcaatgccag cttccgccag tgccagggtg 420
   accgggtcat ctacacgaac aatcagaggt acatggctgc catcatcttg agtgagaagc 480
   aatgcagaga tcaattcaag gacatgaaca agagctgcga tgccttgctc ttcatgctga 540
   atcagaaggt gaagacgctg gaggtggaga tagccaagga gaagaccatt tgcactaagg 600
   ataaggaaag cgtgctgctg aacaaacgcg tggcggagga acagctggtt gaatgcgtga 660
   aaacccggga gctgcagcac caagagcgcc actggccaag gagcaactgc aaaaggtgca 720
   agccctctgc ctgcccctgg acaaggacaa gtttgagatg gaccttcgta acctgtggag 780
   ggactccatt atcccacgca gcctggacaa cctgggttac aacctctacc atcccctggg 840
   ctcggaattg gcctccatcc gcagagcctg cgaccacatg cccagcctca tgagctccaa 900
   ggtggaggag ctggcccgga gcctccgggc ggatatcgaa cgcgtggccc gcgagaactc 960
   agacctccaa cgccagaagc tggaagccca gcagggcctg cgggccagtc aggaggcgaa 1020
   acagaaggtg gagaaggagg ctcaggcccg ggaggccaag ctccaagctg aatgctcccg 1080
   gcagacccag ctagcgctgg aggagaaggc ggtgctgcgg aaggaacgag acaacctggc 1140
   caaggagctg gaagagaaga agagggaggc ggagcagctc aggatggagc tggccatcag 1200
   aaactcagcc ctggacacct gcatcaagac caagtcgcag ccgatgatgc cagtgtcaag 1260
   gcccatgggc cctgtcccca acccccagcc catcgaccca gctagcctgg aggagttcaa 1320
   gaggaagatc ctggagtccc agaggccccc tgcaggcatc cctgtagccc catccagtgg 1380
   ctgaggaggc tccaggcctg aggaccaagg gatggcccga ctcggcggtt tgcggaggat 1440
   gcagggatat gctcacagcg cccgacacaa ccccctcccg ccgcccccaa ccacccaggg 1500
   ccaccatcag acaactccct gcatgcaaac ccctagtacc ctctcacacc cgcacccgcg 1560
   cctcacgatc cctcacccag agcacacggc cgcggagatg acgtcacgca agcaacggcg 1620
   ctgacgtcac atatcaccgt ggtgatggcg tcacgtggcc atgtagacgt cacgaagaga 1680
   tatagcgatg gcgtcgtgca gatgcagcac gtcgcacaca gacatgggga acttggcatg 1740
   acgtcacacc gagatgcagc aacgacgtca cgggccatgt cgacgtcaca catattaatg 1800
   tcacacagac gcggcgatgg catcacacag acggtgatga tgtcacacac agacacagtg 1860
   acaacacaca ccatgacaac gacacctata gatatggcac caacatcaca tgcacgcatg 1920
   ccctttcaca cacactttct acccaattct cacctagtgt cacgttcccc cgaccctggc 1980
   acacgggcca aggtacccac aggatcccat cccctcccgc acagccctgg gccccagcac 2040
   ctcccctcct ccagcttcct ggcctcccag ccacttcctc acccccagtg cctggacccg 2100
   gaggtgagaa caggaagcca ttcacctccg ctccttgagc gtgagtgttt ccaggacccc 2160
   ctcggggccc tgagccgggg gtgagggtca cctgttgtcg ggaggggagc cactccttct 2220
   cccccaactc ccagccctgc ctgtggcccg ttgaaatgtt ggtggcactt aataaatatt 2280
   agtaaatcct taaaaaaaaa aaaaaaaaaa aaa
<210> 7
   <211> 389
   <212> DNA
   <213> Human
<400> 7
   gccaaaaaga tggcttcaaa agtaagaatg aaacatttga tccattcagc tttaggctat 60
   gccactggat tcatgtctag aaaagatagg ataatttctg taaagaaatg aagaccttgc 120
   tattctaaaa tcagatcctt acagatccag atttcaggaa acaaatacat aggggactaa 180
   ctttccttgt tcagattagt ttttctcctt tgcacccagc tatataatat gaggaagtat 240
   tgacttttta aaagtgtttt agttttccat ttctttgata tgaaaagtaa tatttcggga 300
   gaaccctgag ctattaataa tctatgtggc tagtgcgtat atattggtct gaatttgttc 360
   tccttttgtg gtgtccagtg ggtaacatc
<210> 8
   <211> 157
   <212> DNA
   <213> Human
<400> 8
   tgctttaaac agctgtgtca aaaactgaca tcagagagta aattgaattt ggttttgtag 60
   gaagcaggaa gcaagcccac tcaaacgtga aatttggcat gagggatcca gtaactttct 120
   cctcaatctg tgaactatat gtgagtttga tattttg
<210> 9
   <211> 561
   <212> DNA
   <213> Human
<400> 9
   aatagtcaaa acataaacaa aagctaatta actggcactg ttgtcacctg agactaagtg 60
   gatgttgttg gctgacatac aggctcagcc agcagagaaa gaattctgaa ttccccttgc 120
   tgaactgaac tattctgtta catatggttg acaaatctgt gtgttatttc ttttctacct 180
   accatattta aatttatgag tatcaaccga ggacatagtc aaaccttcga tgatgaacat 240
   tcctgatttt ttgcctgatt aatctctgtt gagctctact tgtggtcatt caagatttta 300
   tgatgttgaa aggaaaagtg aatatgacct ttaaaaattg tattttgggt gatgatagtc 360
   tcaccactat aaaactgtca attattgcct aatgttaaag atatccatca ttgtgattaa 420
   ttaaacctat aatgagtatt cttaatggag aattcttaat ggatggatta tcccctgatc 480
   ttttctttaa aatttctctg cacacacagg acttctcatt ttccaataaa tgggtgtact 540
   ctgccccaat ttctaggaaa a
<210> 10
   <211> 1508
   <212> DNA
   <213> Human
<400> 10
   cacaaacacg agagactcca cggtctgcct gagcaccgcc agcctcctag gctccagcac 60
   tcgcaggtcc attcttctgc acgagcctct ctgtccagat ccataagcac ggtcagctca 120
   gggtcgcgga gcagtacgag gacaagtacc agcagcagct cctctgaaca gagactgcta 180
   ggatcatcct tctcctccgg gcctgttgct gatggcataa tccgggtgca acccaaatct 240
   gagctcaagc caggtgagct taagccactg agcaaggaag atttgggcct gcacgcctac 300
   aggtgtgagg actgtggcaa gtgcaaatgt aaggagtgca cctacccaag gcctctgcca 360
   tcagactgga tctgcgacaa gcagtgcctt tgctcggccc agaacgtgat tgactatggg 420
   acttgtgtat gctgtgtgaa aggtctcttc tatcactgtt ctaatgatga tgaggacaac 480
   tgtgctgaca acccatgttc ttgcagccag tctcactgtt gtacacgatg gtcagccatg 540
   ggtgtcatgt ccctcttttt gccttgttta tggtgttacc ttccagccaa gggttgcctt 600
   aaattgtgcc aggggtgtta tgaccgggtt aacaggcctg gttgccgctg taaaaactca 660
   aacacagttt gctgcaaagt tcccactgtc ccccctagga actttgaaaa accaacatag 720
   catcattaat caggaatatt acagtaatga ggattttttc tttctttttt taatacacat 780
   atgcaaccaa ctaaacagtt ataatcttgg cactgttaat agaaagttgg gatagtcttt 840
   gctgtttgcg gtgaaatgct ttttgtccat gtgccgtttt aactgatatg cttgttagaa 900
   ctcagctaat ggagctcaaa gtatgagata cagaacttgg tgacccatgt attgcataag 960
   ctaaagcaac acagacactc ctaggcaaag tttttgtttg tgaatagtac ttgcaaaact 1020
   tgtaaattag cagatgactt ttttccattg ttttctccag agagaatgtg ctatattttt 1080
   gtatatacaa taatatttgc aactgtgaaa aacaagtggt gccatactac atggcacaga 1140
   cacaaaatat tatactaata tgttgtacat tcggaagaat gtgaatcaat cagtatgttt 1200
   ttagattgta ttttgcctta cagaaagcct ttattgtaag actctgattt ccctttggac 1260
   ttcatgtata ttgtacagtt acagtaaaat tcaaccttta ttttctaatt ttttcaacat 1320
   attgtttagt gtaaagaata tttatttgaa gttttattat tttataaaaa agaatattta 1380
   ttttaagagg catcttacaa attttgcccc ttttatgagg atgtgatagt tgctgcaaat 1440
   gaggggttac agatgcatat gtccaatata aaatagaaaa tatattaacg tttgaaatta 1500
   aaaaaaaa
<210> 11
   <211> 389
   <212> DNA
   <213> Human
<400> 11
   gggcaggtga tcagggcaca catttcccgt ccattgagac agtagcattc ccggcaccca 60
   tcgtgccagc tctcctcatt tttatgatga tgaccatcca cggtgagaca agtgcccgac 120
   aggatgggtg gcccagctga agcacaggcc gctctgcact tgcagataag acagccgtga 180
   ctgtcctgct ggaaacccaa ggggcagatc ttactgcatg agagctctgg acatttctta 240
   cagcgacaga tgtcacagcc gtgcttattc ttcagcaatc caagtggaca atacttgtca 300
   cagattatgg gtctgcactt cttgggcctt gggcggcact cacagatctc acagttttgg 360
   acctcggccg cgaccacgct gggtaccga
<210> 12
   <211> 981
   <212> DNA
   <213> Human
<400> 12
   tttttttttt ttggattgca aaaatttatt aaaattggag acactgtttt aatcttcttg 60
   tgccatgaga ctccatcagg cagtctacaa agaccactgg gaggctgagg atcacttgag 120
   cccagaagtt tgaggctgta gtaagcttca aaggccactg cactctagct tgggtgaggc 180
   aagacccttt caagcagtaa gctgcatgct tgcttgttgt ggtcattaaa aaccctagtt 240
   taggataaca acatattaat cagggcaaaa tacaaatgtg tgatgcttgt tagtagagta 300
   acctcagaat caaaatggaa cggttttaca gtgatatcat tatatttcat ttggcagaat 360
   cattacatca ttggttacac tgaaaatcat cacatgtacc aaaagctgac tcacctagtt 420
   taggataaca ggtctgcctg tttgaagatg aaaaataata cccatttaaa atttgcccta 480
   ctcaatttcc ttctcagtca cattttaact tttaaacagc taatcactcc catctacaga 540
   ttaaggtgta tatgccacca aaaccttttg ccaccttaaa aatttccttc aaagtttaaa 600
   ctaatgcctg catttcttca atcatgaatt ctgagtcctt tgcttcttta aaacttgctc 660
   cacacagtgt agtcaagccg actctccata cccaagcaag tcatccatgg ataaaaacgt 720
   taccaggagc agaaccatta agctggtcca ggcaagttgg actccaccat ttcaacttcc 780
   agctttctgt ctaatgcctg tgtgccaatg gcttgagtta ggcttgctct ttaggacttc 840
   agtagctatt ctcatccttc cttggggaca caactgtcca taaggtgcta tccagagcca 900
   cactgcatct gcacccagca ccatacctca caggagtcga ctcccacgag ccgcctgtat 960
   ataagagttc ttttgatgac g
<210> 13
   <211> 401
   <212> DNA
   <213> Human
<400> 13
   ataactacag cttcagcaga caactaaaga gactgcatta aggtgatttc tctggctata 60
   aagagagccc ggccgcagag catgtgactg ctgggacctc tgggataggc aacactgccc 120
   tctctccccc agagcgaccc cccgggcagg tcggggccca aggaatgacc cagcaactgc 180
   tccctaccca gcacactctc tttactgcca cctgcaatta tgctgtgaag atgactgggt 240
   gtggtcatca cgattcagag aaatcaagat ctatgaccat tttaggcaaa gagagaaact 300
   tggagaattg ctgaggacta ctgaaccttg ttttgctttt ttaaaaaata ctaaatcctc 360
   acttcagcat atttagttgt cattaaaatt aagctgatat t
<210> 14
   <211> 1002
   <212> DNA
   <213> Human
<400> 14
   gacaatataa aaagtggaaa caagcataaa ttgcagacat aaaataatct tctggtagaa 60
   acagttgtgg agaacaggtt gagtagagca acaacaacaa aagcttatgc agtcaccttc 120
   tttgaaaatg ttaaatacaa gtcctattct ctttgtccag ctgggtttag ctagaggtag 180
   ccaattactt ctcttaaggt ccatggcatt cgccaggatt ctataaaagc caagttaact 240
   gaagtaaata tctggggccc atcgcacccc cactaagtac tttgtcacca tgttgtatct 300
   taaaagtcat ttttcactgt ttgactcaga atttgggact tcagagtcaa acttcattgc 360
   ttactccaaa cccagtttaa ttccccactt ttttaagtag gcttagcttt gagtgatttt 420
   tggctataac cgaaatgtaa atccaccttc aaacaacaaa gtttgacaag actgaaatgt 480
   tactgaaaac aatggtgcca tatgctccaa agacatttcc ccaagataac tgccaaagag 540
   tttttgagga ggacaatgat catttattat gtaggagcct tgatatctct gcaaaataga 600
   attaatacag ctcaaatgga gtagtaacca agcttttctg cccaggaagt aacaaacatc 660
   actacgaaca tgagagtaca agaggaaact ttcataatgc attttttcat tcatacattc 720
   attcaataaa cattagccaa gctaatgtcc caagccactg tgccaggtat taacaatata 780
   acaacaataa aagacacagt ccttcctctc aaggtgttca gtctagtagg gaagatgatt 840
   attcattaaa atttttggtg catcagaatc atgaggagct tgtcaaaaat gtaaattcct 900
   gcctatgttc tcagatattc tggttaggtc aggagtggga acccaaaatc aattctttta 960
   acaaacacta aaggtgattc taacacaggc ggtgtgagga cc
<210> 15
   <211> 280
   <212> DNA
   <213> Human
<400> 15
   cgaggtgggc cacccgtgtc tggtctgaga tttttaaatg aggattacat tatcctattt 60
   ataatattcc tattctaatc tattgtattc ttacaattaa atgtatcaaa taattcttaa 120
   aaacattatt agaaacaaac tgcctaatac cttataagac taaaaaaatc accaagatga 180
   aactgtatta tgactctcaa tatttaaaca tttaaaaaaa tgttagtgtt tgttaagcac 240
   caatcttaac tatttcacct gcccgggcgg ccgctcgagg
<210> 16
   <211> 2041
   <212> DNA
   <213> Human
<400> 16
   ccccccgcag aactcccccc tggaatagga tttttaaaac ccttgacaat tagaaatcct 60
   atagaggtta gcatttttta ggtaaaaata tggttgcccc tacagggatc atgcaacttc 120
   cttaaaacca attcagcaca tatgtataaa gaaccctttt taaaaacatt tgtacttgaa 180
   atacagacac agtgatgctg aagacactaa acaaaaactg aaaagtacta taccttgata 240
   aattttgtta ttgccttctt tagagacttt ataatctcta gttgattttc aaggacttga 300
   atttaataat ggggtaatta cacaagacgt aaaggatttt ttaaaaacaa gtattttttt 360
   ttacctctag catcaattct tttataaaga atgctaaata aattacattt tttgttcagt 420
   aaaactgaag atagaccatt taaatgcttc taccaaattt aacgcagctt aattagggac 480
   caggtacata ttttcttctg aacatttttg gtcaagcatg tctaaccata aaagcaaatg 540
   gaattttaag aggtagattt tttttccatg atgcattttg ttaataaatg tgtcaagaaa 600
   ataaaaacaa gcactgagtg tgttctcttg aagtataagg gtctaatgaa aaataaaaga 660
   tagatatttg ttatagtctg acattttaac agtcatagta ttagacgttt cgtgaccagt 720
   gcattttgga ctctctcagg atcaaaatac gagtctgcca actgtattaa atcctcctcc 780
   accccctcca ccagttggtc cacagcttcc tggtgggtcg ttgtcatcaa atccattggg 840
   ccgaaatgaa catgaagcag atgcagcttg gagggcccgg gctcgagcat tcaactcttg 900
   ttcctgtaaa tatagtttat tgtcttttgt tatagcatcc ataagttctt tctgtagagg 960
   tgggtctcca tttatccaga gtccactggt tgggttatta ccacttaaac cattagtact 1020
   atgctgtttt ttatacaaaa gcacataagc tgtgtccttt ggaaacctgc tcgtaatttt 1080
   ctggactgac tgaaatgaag taaatgtcac tctactgtca ttaaataaaa acccattctt 1140
   ttgacatttc cttattttcc aaatcctgtt caaaaactgc actgggacta tctctcccta 1200
   gtaaatgact ctgggaggat gctaatgcca gagcctcaga ctggtggtac atctgatatg 1260
   aagagtctgt acttgtgata tttctggcat aagaatagta atgcccactt tcagaggata 1320
   taccagagtg aaccacaacg gaacttaata gatagggcac caattttgtg caggaagctt 1380
   catcagtccc tgaaggcttt aattttttag caaggttctc actaagatca gtgaagtcaa 1440
   catctacaga ccaactttct gacaatgaag agaaagaagt aattcttcta actggcaact 1500
   ccaaaaccag tggccagtga tacattgtct aaaattttcc ttctcacatg atacttctga 1560
   tcatatgaaa atctcaggag agtaagaata aggtattcag gttcctccgt gatttgcata 1620
   gttttctcag cattttgcag agaggcacag ttttcacaat aatattggtt atcaccagta 1680
   agaatctctg gagcccaaaa aataatttag taagtcagtt actgaaggtg tggtttcacc 1740
   tcccggtttc tgaggtacat ctttattaac aagaatcttg ttagattcgt tagggacaga 1800
   agtgttttca gaacagtaaa actcattagg aggactgcct atggtttttt cattcacaag 1860
   tgagtcacag atgaaggcag ctgttgttgg attataaact actggctctt ctgaaggacc 1920
   gggtacagac gcttgcatta gaccaccatc ttgtatactg ggtgatgatg ctggatcttg 1980
   gacagacatg ttttccaaag aagaggaagc acaaaacgca agcgaaagat ctgtaaaggc 2040
   t
<210> 17
   <211> 235
   <212> DNA
   <213> Human
<400> 17
   cgccccgggc aggtgtcagg ggttccaaac cagcctgggg aaacacagcg tagacccctc 60
   acctctacaa ataaaaaatt aaaaaattag ccaggtgtgg cagcgaacaa ctgtagtctc 120
   agatactcag gagactgagc tggaaaggat cacttgagcc caagaagttc aaggttacag 180
   tgggccacga tcatgtcatt acactccagc ttgggtgaca aaatgagact gtcta
<210> 18
   <211> 2732
   <212> DNA
   <213> Human
<400> 18
   gtgtggagtt tcagctgcta ttgactataa gagctatgga acagaaaaag cttgctggct 60
   tcatgttgat aactacttta tatggagctt cattggacct gttaccttca ttattctgct 120
   aaatattatc ttcttggtga tcacattgtg caaaatggtg aagcattcaa acactttgaa 180
   accagattct agcaggttgg aaaacattaa gtcttgggtg cttggcgctt tcgctcttct 240
   gtgtcttctt ggcctcacct ggtcctttgg gttgcttttt attaatgagg agactattgt 300
   gatggcatat ctcttcacta tatttaatgc tttccaggga gtgttcattt tcatctttca 360
   ctgtgctctc caaaagaaag tacgaaaaga atatggcaag tgcttcagac actcatactg 420
   ctgtggaggc ctcccaactg agagtcccca cagttcagtg aaggcatcaa ccaccagaac 480
   cagtgctcgc tattcctctg gcacacagag tcgtataaga agaatgtgga atgatactgt 540
   gagaaaacaa tcagaatctt cttttatctc aggtgacatc aatagcactt caacacttaa 600
   tcaaggtggc ataaatctta atatattatt acaggactga catcacatgg tctgagagcc 660
   catcttcaag atttatatca tttagaggac attcactgaa caatgccagg gatacaagtg 720
   ccatggatac tctaccgcta aatggtaatt ttaacaacag ctactcgctg cacaagggtg 780
   actataatga cagcgtgcaa gttgtggact gtggactaag tctgaatgat actgcttttg 840
   agaaaatgat catttcagaa ttagtgcaca acaacttacg gggcagcagc aagactcaca 900
   acctcgagct cacgctacca gtcaaacctg tgattggagg tagcagcagt gaagatgatg 960
   ctattgtggc agatgcttca tctttaatgc acagcgacaa cccagggctg gagctccatc 1020
   acaaagaact cgaggcacca cttattcctc agcggactca ctcccttctg taccaacccc 1080
   agaagaaagt gaagtccgag ggaactgaca gctatgtctc ccaactgaca gcagaggctg 1140
   aagatcacct acagtccccc aacagagact ctctttatac aagcatgccc aatcttagag 1200
   actctcccta tccggagagc agccctgaca tggaagaaga cctctctccc tccaggagga 1260
   gtgagaatga ggacatttac tataaaagca tgccaaatct tggagctggc catcagcttc 1320
   agatgtgcta ccagatcagc aggggcaata gtgatggtta tataatcccc attaacaaag 1380
   aagggtgtat tccagaagga gatgttagag aaggacaaat gcagctggtt acaagtcttt 1440
   aatcatacag ctaaggaatt ccaagggcca catgcgagta ttaataaata aagacaccat 1500
   tggcctgacg cagctccctc aaactctgct tgaagagatg actcttgacc tgtggttctc 1560
   tggtgtaaaa aagatgactg aaccttgcag ttctgtgaat ttttataaaa catacaaaaa 1620
   ctttgtatat acacagagta tactaaagtg aattatttgt tacaaagaaa agagatgcca 1680
   gccaggtatt ttaagattct gctgctgttt agagaaattg tgaaacaagc aaaacaaaac 1740
   tttccagcca ttttactgca gcagtctgtg aactaaattt gtaaatatgg ctgcaccatt 1800
   tttgtaggcc tgcattgtat tatatacaag acgtaggctt taaaatcctg tgggacaaat 1860
   ttactgtacc ttactattcc tgacaagact tggaaaagca ggagagatat tctgcatcag 1920
   tttgcagttc actgcaaatc ttttacatta aggcaaagat tgaaaacatg cttaaccact 1980
   agcaatcaag ccacaggcct tatttcatat gtttcctcaa ctgtacaatg aactattctc 2040
   atgaaaaatg gctaaagaaa ttatattttg ttctattgct agggtaaaat aaatacattt 2100
   gtgtccaact gaaatataat tgtcattaaa ataattttaa agagtgaaga aaatattgtg 2160
   aaaagctctt ggttgcacat gttatgaaat gttttttctt acactttgtc atggtaagtt 2220
   ctactcattt tcacttcttt tccactgtat acagtgttct gctttgacaa agttagtctt 2280
   tattacttac atttaaattt cttattgcca aaagaacgtg ttttatgggg agaaacaaac 2340
   tctttgaagc cagttatgtc atgccttgca caaaagtgat gaaatctaga aaagattgtg 2400
   tgtcacccct gtttattctt gaacagaggg caaagagggc actgggcact tctcacaaac 2460
   tttctagtga acaaaaggtg cctattcttt tttaaaaaaa taaaataaaa cataaatatt 2520
   actcttccat attccttctg cctatattta gtaattaatt tattttatga taaagttcta 2580
   atgaaatgta aattgtttca gcaaaattct gctttttttt catccctttg tgtaaacctg 2640
   ttaataatga gcccatcact aatatccagt gtaaagttta acacggtttg acagtaaata 2700
   aatgtgaatt ttttcaagtt aaaaaaaaaa aa
<210> 19
   <211> 276
   <212> DNA
   <213> Human
<400> 19
   ctccctaaat gattttaaaa taaattggat aaacatatga tataaagtgg gtactttaga 60
   aaccgccttt gcatattttt tatgtacaaa tctttgtata caattccgat gttccttata 120
   tattccctat atagcaaacc aaaaccagga cctcccaact gcatgcctca agtccctgtg 180
   gagcactctg gcaactggat ggccctactt gctttctgac aaaatagctg gaaaggagga 240
   gggaccaatt aaatacctcg gccgcgacca cgctgg
<210> 20
   <211> 2361
   <212> DNA
   <213> Human
<400> 20
   attgtaccag ccttgatgaa cgtgggccct gcttcgcttt tgagggccat aagctcattg 60
   cccactggtt tagaggctac cttatcattg tctcccgtga ccggaaggtt tctcccaagt 120
   cagagtttac cagcagggat tcacagagct ccgacaagca gattctaaac atctatgacc 180
   tgtgcaacaa gttcatagcc tatagcaccg tctttgagga tgtagtggat gtgcttgctg 240
   agtggggctc cctgtacgtg ctgacgcggg atgggcgggt ccacgcactg caggagaagg 300
   acacacagac caaactggag atgctgttta agaagaacct atttgagatg gcgattaacc 360
   ttgccaagag ccagcatctg gacagtgatg ggctggccca gattttcatg cagtatggag 420
   accatctcta cagcaagggc aaccacgatg gggctgtcca gcaatatatc cgaaccattg 480
   gaaagttgga gccatcctac gtgatccgca agtttctgga tgcccagcgc attcacaacc 540
   tgactgccta cctgcagacc ctgcaccgac aatccctggc caatgccgac cataccaccc 600
   tgctcctcaa ctgctatacc aagctcaagg acagctcgaa gctggaggag ttcatcaaga 660
   aaaagagtga gagtgaagtc cactttgatg tggagacagc catcaaggtc ctccggcagg 720
   ctggctacta ctcccatgcc ctgtatctgg cggagaacca tgcacatcat gagtggtacc 780
   tgaagatcca gctagaagac attaagaatt atcaggaagc ccttcgatac atcggcaagc 840
   tgccttttga gcaggcagag agcaacatga agcgctacgg caagatcctc atgcaccaca 900
   taccagagca gacaactcag ttgctgaagg gactttgtac tgattatcgg cccagcctcg 960
   aaggccgcag cgatagggag gccccaggct gcagggccaa ctctgaggag ttcatcccca 1020
   tctttgccaa taacccgcga gagctgaaag ccttcctaga gcacatgagt gaagtgcagc 1080
   cagactcacc ccaggggatc tacgacacac tccttgagct gcgactgcag aactgggccc 1140
   acgagaagga tccacaggtc aaagagaagc ttcacgcaga ggccatttcc ctgctgaaga 1200
   gtggtcgctt ctgcgacgtc tttgacaagg ccctggtcct gtgccagatg cacgacttcc 1260
   aggatggtgt cctttacctt tatgagcagg ggaagctgtt ccagcagatc atgcactacc 1320
   acatgcagca cgagcagtac cggcaggtca tcagcgtgtg tgagcgccat ggggagcagg 1380
   acccctcctt gtgggagcag gccctcagct acttcgctcg caaggaggag gactgcaagg 1440
   agtatgtggc agctgtcctc aagcatatcg agaacaagaa cctcatgcca cctcttctag 1500
   tggtgcagac cctggcccac aactccacag ccacactctc cgtcatcagg gactacctgg 1560
   tccaaaaact acagaaacag agccagcaga ttgcacagga tgagctgcgg gtgcggcggt 1620
   accgagagga gaccacccgt atccgccagg agatccaaga gctcaaggcc agtcctaaga 1680
   ttttccaaaa gaccaagtgc agcatctgta acagtgcctt ggagttgccc tcagtccact 1740
   tcctgtgtgg ccactccttc caccaacact gctttgagag ttactcggaa agtgatgctg 1800
   actgccccac ctgcctccct gaaaaccgga aggtcatgga tatgatccgg gcccaggaac 1860
   agaaacgaga tctccatgat caattccagc atcagctcaa gtgctccaat gacagctttt 1920
   ctgtgattgc tgactacttt ggcagaggtg ttttcaacaa attgactctg ctgaccgacc 1980
   ctcccacagc cagactgacc tccagcctgg aggctgggct gcaacgcgac ctactcatgc 2040
   actccaggag gggcacttaa gcagcctgga ggaagatgtg ggcaacagtg gaggaccaag 2100
   agaacagaca caatgggacc tgggcgggcg ttacacagaa ggctggctga catgcccagg 2160
   gctccactct catctaatgt cacagccctc acaagactaa agcggaactt tttcttttcc 2220
   ctggccttcc ttaattttaa gtcaagcttg gcaatccctt cctctttaac taggcaggtg 2280
   ttagaatcat ttccagatta atggggggga aggggaacct caggcaaacc tcctgaagtt 2340
   ttggaaaaaa aagctggttt c
<210> 21
   <211> 179
   <212> DNA
   <213> Human
<400> 21
   aggtgttaga tgctcttgaa aaagaaactg catctaagct gtcagaaatg gattctttta 60
   acaatcaact aaaggaactg agagaaacct acaacacaca gcagttagcc cttgaacagc 120
   tttataagat caacgtgaca agttgaagga aattgaaagg aaaaaattag aactaatgc
<210> 22
   <211> 905
   <212> DNA
   <213> Human
<400> 22
   tttttttttt ttctttaacc gtgtggtctt tatttcagtg ccagtgttac agatacaaca 60
   caaatgttcc agttagaagg aattcaaacg gaatgccaag gtccaagcca ggctcaagaa 120
   ataaaaaggg aggtttggag taatagataa gatgactcca atactcactc ttcctaaggg 180
   caaaggtact tttgatacag agtctgatct ttgaaactgg tgaactcctc ttccacccat 240
   taccatagtt caaacaggca agttatgggc ttaggagcac tttaaaattt gtggtgggaa 300
   tagggtcatt aataactatg aatatatctt ttagaaggtg accattttgc actttaaagg 360
   gaatcaattt tgaaaatcat ggagactatt catgactaca gctaaagaat ggcgagaaag 420
   gggagctgga agagccttgg aagtttctat tacaaataga gcaccatatc cttcatgcca 480
   aatctcaaca aaagctcttt ttaactccat ctgtccagtg tttacaaata aactcgcaag 540
   gtctgaccag ttcttggtaa caaacataca tgtgtgtgtc tgtgtgtata cagcaatgca 600
   cagaaaaggc taccaggagc ctaatgcctc tttcaaacat tgggggaacc agtagaaaaa 660
   ggcagggctc cctaatgtcc attattacat ttccattccg aatgccagat gttaaaagtg 720
   cctgaagatg gtaacccagc tagtgaggaa taaatacccc accttgccca gtccacagag 780
   aaacaacagt agaaagaagg ggcaactctt tgctgcagag acaaagtgag tgttttttcg 840
   ccatggattg cagtcctctc ctccagacca gctgcttatt tcctcagggg cccagggaat 900
   gttga
<210> 23
   <211> 2134
   <212> DNA
   <213> Human
<400> 23
   ggtctcttct ttcctttttt tttttccaaa agtgttcttt tatttctagt aacatatatt 60
   gtataaatac tctattttat atgcacttcc acaaaagcga tataatttaa aagttttttt 120
   cattagaaat aaatgtataa aaataaatat gttattatag gcatttatta ctaactatag 180
   tccttcttgg aaggaacacc caaaccaata cttataaagt acatgtaatt tatagtaaca 240
   tattttacta tatacatatg gaaaaaatca tattctcaca gaagagctga acagacattc 300
   accaggatac gactgttgga ccagctgctg gagatggacc tgctacccct cagcagcctc 360
   cccaccacaa gacaagtgat ctcaatgtcc ccaaacctgt gggaccctgt tctacacacc 420
   tcatttttgt tccggcgttt catcctcctt gtgtgattgt actgattttc atgagacaca 480
   agttacttct ttacatccat attcccaaag cagggttaca tggtaggaaa gaaaggaagt 540
   tggaggtact aagctcattg tgtctcctct agcttttacc agcatctaat gcttcactgc 600
   tttttttcca ttgtagactt taatgcactt gaataaatac atggagttgt tttttcctca 660
   aaatgaatta cacaaataaa gactgagatg gtccaaaaaa ggaaagagga agccatttgc 720
   gttatttcac gttgctgagc ctttctctca tgttgaacaa tctgaagttt taattctcgg 780
   tagaaataat gtataaacat tctctgaaac catagcagcc ataaacagtg ctggtcaaag 840
   atcctatttg tactcctttc tccccccatt gttagtgagg taaagtaaaa caggtcttag 900
   taaaatctca cttttctcct acttttcatt tcccaacccc catgatacta agtatttgat 960
   aagtaccagg aaacaggggt tgtaatagtt ctaacttttt ttgacaattg ctttgttttt 1020
   tctaaacttg taatagatgt aacaaaagaa ataataataa taatgcccgg ggctttatta 1080
   tgctatatca ctgctcagag gttaataatc ctcactaact atcctatcaa atttgcaact 1140
   ggcagtttac tctgatgatt caactccttt tctatctacc cccataatcc caccttactg 1200
   atacacctca ctggttactg gcaagatacg ctggatccct ccagccttct tgctttccct 1260
   gcaccagccc ttcctcactt tgccttgccc tcaaagctaa caccacttaa accacttaac 1320
   tgcattctgc cattgtgcaa aagtctatga aatgtttagg tttctttaaa ggatcacagc 1380
   tctcatgaga taacacccct ccatcatggg acagacactt caagcttctt tttttgtaac 1440
   ccttcccaca ggtcttagaa catgatgacc actcccccag ctgccactgg gggcagggat 1500
   ggtctgcaca aggtctggtg ctggctggct tcacttcctt tgcacactcg gaagcaggct 1560
   gtccattaat gtctcggcat tctaccagtc ttctctgcca acccaattca catgacttag 1620
   aacattcgcc ccactcttca atgacccatg ctgaaaaagt ggggatagca ttgaaagatt 1680
   ccttcttctt ctttacgaag taggtgtatt taattttagg tcgaagggca ttgcccacag 1740
   taagaacctg gatggtcaag ggctctttga gagggctaaa gctgcgaatt ctttccaatg 1800
   ccgcagagga gccgctgtac ctcaagacaa cacctttgta cataatgtct tgctctaagg 1860
   tggacaaagt gtagtcacca ttaagaatat atgtgccatc agcagctttg atggcaagaa 1920
   agctgccatt gttcctggat cccctctggt tccgctgttt cacttcgatg ttggtggctc 1980
   cagttggaat tgtgatgata tcatgatatc caggttttgc actagtaact gatcctgata 2040
   tttttttaca agtagatcca tttcccccgc aaacaccaca tttatcaaac ttctttttgg 2100
   agtctatgat gcgatcacaa ccagctttta caca
<210> 24
   <211> 1626
   <212> DNA
   <213> Human
<400> 24
   ggacaatttc tagaatctat agtagtatca ggatatattt tgctttaaaa tatattttgg 60
   ttattttgaa tacagacatt ggctccaaat tttcatcttt gcacaatagt atgacttttc 120
   actagaactt ctcaacattt gggaactttg caaatatgag catcatatgt gttaaggctg 180
   tatcatttaa tgctatgaga tacattgttt tctccctatg ccaaacaggt gaacaaacgt 240
   agttgttttt tactgatact aaatgttggc tacctgtgat tttatagtat gcacatgtca 300
   gaaaaaggca agacaaatgg cctcttgtac tgaatacttc ggcaaactta ttgggtcttc 360
   attttctgac agacaggatt tgactcaata tttgtagagc ttgcgtagaa tggattacat 420
   ggtagtgatg cactggtaga aatggttttt agttattgac tcagaattca tctcaggatg 480
   aatcttttat gtctttttat tgtaagcata tctgaattta ctttataaag atggttttag 540
   aaagctttgt ctaaaaattt ggcctaggaa tggtaacttc attttcagtt gccaaggggt 600
   agaaaaataa tatgtgtgtt gttatgttta tgttaacata ttattaggta ctatctatga 660
   atgtatttaa atatttttca tattctgtga caagcattta taatttgcaa caagtggagt 720
   ccatttagcc cagtgggaaa gtcttggaac tcaggttacc cttgaaggat atgctggcag 780
   ccatctcttt gatctgtgct taaactgtaa tttatagacc agctaaatcc ctaacttgga 840
   tctggaatgc attagttatg ccttgtacca ttcccagaat ttcaggggca tcgtgggttt 900
   ggtctagtga ttgaaaacac aagaacagag agatccagct gaaaaagagt gatcctcaat 960
   atcctaacta actggtcctc aactcaagca gagtttcttc actctggcac tgtgatcatg 1020
   aaacttagta gaggggattg tgtgtatttt atacaaattt aatacaatgt cttacattga 1080
   taaaattctt aaagagcaaa actgcatttt atttctgcat ccacattcca atcatattag 1140
   aactaagata tttatctatg aagatataaa tggtgcagag agactttcat ctgtggattg 1200
   cgttgtttct tagggttcct agcactgatg cctgcacaag catgtgatat gtgaaataaa 1260
   atggattctt ctatagctaa atgagttccc tctggggaga gttctggtac tgcaatcaca 1320
   atgccagatg gtgtttatgg gctatttgtg taagtaagtg gtaagatgct atgaagtaag 1380
   tgtgtttgtt ttcatcttat ggaaactctt gatgcatgtg cttttgtatg gaataaattt 1440
   tggtgcaata tgatgtcatt caactttgca ttgaattgaa ttttggttgt atttatatgt 1500
   attatacctg tcacgcttct agttgcttca accattttat aaccattttt gtacatattt 1560
   tacttgaaaa tattttaaat ggaaatttaa ataaacattt gatagtttac ataataaaaa 1620
   aaaaaa
<210> 25
   <211> 1420
   <212> DNA
   <213> Human
<400> 25
   gttcagcatt gtttctgctt ctgaaatctg tatagtacac tggtttgtaa tcattatgtc 60
   ttcattgaaa tccttgctac ttctcttcct cctcaatgaa agacacgaga gacaagagcg 120
   acacaagctt aagaaaaacg agcaaggaag agtatcttca ttattctcat tttctctgag 180
   ttggaaacaa aaacatgaag gactccaact agaagacaga tatttacatt taaatagatt 240
   agtgggaaaa ctttaagagt ttccacatat tagttttcat tttttgagtc aagagactgc 300
   tccttgtact gggagacact agtagtatat gtttgtaatg ttactttaaa attatctttt 360
   tattttataa ggcccataaa tactggttaa actctgttaa aagtgggcct tctatcttgg 420
   atggtttcac tgccatcagc catgctgata tattagaaat ggcatcccta tctacttact 480
   ttaatgctta aaattataca taaaatgctt tatttagaaa acctacatga tacagtggtg 540
   tcagccttgc catgtatcag tttcacttga aatttgagac caattaaatt tcaactgttt 600
   agggtggaga aagaggtact ggaaaacatg cagatgagga tatcttttat gtgcaacagt 660
   atcctttgca tgggaggaga gttactcttg aaaggcaggc agcttaagtg gacaatgttt 720
   tgtatatagt tgagaatttt acgacacttt taaaaattgt gtaattgtta aatgtccagt 780
   tttgctctgt tttgcctgaa gttttagtat ttgttttcta ggtggacctc tgaaaaccaa 840
   accagtacct ggggaggtta gatgtgtgtt tcaggcttgg agtgtatgag tggttttgct 900
   tgtattttcc tccagagatt ttgaacttta ataattgcgt gtgtgttttt ttttttttaa 960
   gtggctttgt ttttttttct caagtaaaat tgtgaacata tttcctttat aggggcaggg 1020
   catgagttag ggagactgaa gagtattgta gactgtacat gtgccttctt aatgtgtttc 1080
   tcgacacatt ttttttcagt aacttgaaaa ttcaaaaggg acatttggtt aggttactgt 1140
   acatcaatct atgcataaat ggcagcttgt tttcttgagc cactgtctaa attttgtttt 1200
   tatagaaatt ttttatactg attggttcat agatggtcag ttttgtacac agactgaaca 1260
   atacagcact ttgccaaaaa tgagtgtagc attgtttaaa cattgtgtgt taacacctgt 1320
   tctttgtaat tgggttgtgg tgcattttgc actacctgga gttacagttt tcaatctgtc 1380
   agtaaataaa gtgtccttta acttcaaaaa aaaaaaaaaa
<210> 26
   <211> 689
   <212> DNA
   <213> Human
<400> 26
   aaacaaacaa aaaaaaagtt agtactgtat atgtaaatac tagcttttca atgtgctata 60
   caaacaatta tagcacatcc ttccttttac tctgtctcac ctcctttagg tgagtacttc 120
   cttaaataag tgctaaacat acatatacgg aacttgaaag ctttggttag ccttgcctta 180
   ggtaatcagc ctagtttaca ctgtttccag ggagtagttg aattactata aaccattagc 240
   cacttgtctc tgcaccattt atcacaccag gacagggtct ctcaacctgg gcgctactgt 300
   catttggggc caggtgattc ttccttgcaa gggctgtcct gtacctgccc gggcggccgc 360
   tcgaagcgtg gtcgcggccg aggtactgaa aggaccaagg agctctggct gccctcagga 420
   attccaaatg accgaaggaa caaagcttca gggctctggg tggtgtctcc cactattcag 480
   gaggtggtcg gaggtaacgc agcttcattt cgtccagtcc tttccagtat ttaaagttgt 540
   tgtcaagatg ctgcattaaa tcaggcaggt ctacaaaggc atcccaagca tcaaacatgt 600
   ctgtgatgaa gtaatcaatg aaacaccgga acctccgacc acctcctgaa tagtgggaga 660
   cacacccaga gcctgaagtt tgtccttcg
<210> 27
   <211> 471
   <212> DNA
   <213> Human
<400> 27
   tcccagcggc atgaagtttg agattggcca ggccctgtac ctgggcttca tctccttcgt 60
   ccctctcgct cattggtggc accctgcttt gcctgtcctg ccaggacgag gcaccctaca 120
   agccctaacc caggccccgc ccagggccac cacgaccact gcaaacaccg cacctgccta 180
   ccagccacca gctgcctaca aagacaatcg ggccccctca gtgacctcgg ccaccacagc 240
   gggtacaggc tgaacgacta cgtgtgagtc cccacagcct gcttctcccc tgggctgctg 300
   tgggctggtt cccggcggga ctgtcaatgg aggcaggggt tccagcacaa agtttacttc 360
   tgggcaattt ttgtatccaa ggaaataatg tgaatgcgag gaaatgtctt tagagcacag 420
   ggacagaggg ggaaataaga ggaggagaaa gctctctata ccaaagactg a
<210> 28
   <211> 929
   <212> DNA
   <213> Human
<400> 28
   ggtgaactca gtgcattggg ccaatggttc gacacaggct ctgccagcca caaccatcct 60
   gctgcttctg acggtttggc tgctggtggg ctttcccctc actgtcattg gaggcatctt 120
   tgggaagaac aacgccagcc cctttgatgc accctgtcgc accaagaaca tcgcccggga 180
   gattccaccc cagccctggt acaagtctac tgtcatccac atgactgttg gaggcttcct 240
   gcctttcagt gccatctctg tggagctgta ctacatcttt gccacagtat ggggtcggga 300
   gcagtacact ttgtacggca tcctcttctt tgtcttcgcc atcctgctga gtgtgggggc 360
   ttgcatctcc attgcactca cctacttcca gttgtctggg gaggattacc gctggtggtg 420
   gcgatctgtg ctgagtgttg gctccaccgg cctcttcatc ttcctctact cagttttcta 480
   ttatgcccgg cgctccaaca tgtctggggc agtacagaca gtagagttct tcggctactc 540
   cttactcact ggttatgtct tcttcctcat gctgggcacc atctcctttt tttcttccct 600
   aaagttcatc cggtatatct atgttaacct caagatggac tgagttctgt atggcagaac 660
   tattgctgtt ctctcccttt cttcatgccc tgttgaactc tcctaccagc ttctcttctg 720
   attgactgaa ttgtgtgatg gcattgttgc cttccctttt tccctttggg cattccttcc 780
   ccagagaggg cctggaaatt ataaatctct atcacataag gattatatat ttgaactttt 840
   taagttgcct ttagttttgg tcctgatttt tctttttaca attaccaaaa taaaatttat 900
   taagaaaaag aaaaaaaaaa aaaaaaaaa
<210> 29
   <211> 1775
   <212> DNA
   <213> Human
<400> 29
   gaacgtgatg ggaactttgg gaggatgtct gagaaaatgt ccgaagggat tttggccaac 60
   accagaaaac gccaatgtcc taggaattcc ctcccaaaat gcttcccaaa aaattactca 120
   ttgacaattc aaattgcact tggctggcgg cagcccgggc ggccttcagt ccgtgtgggg 180
   cgcccgcgtg gccttctcct cgtaggactc cccaaactcg ttcactctgc gtttatccac 240
   aggataaagc caccgctggt acaggtagac cagaaacacc acgtcgtccc ggaagcaggc 300
   cagccggtga gacgtgggca tggtgatgat gaaggcaaag acgtcatcaa tgaaggtgtt 360
   gaaagccttg taggtgaagg ccttccaggg cagatgtgcc actgacttca acttgtagtt 420
   cacaaagagc tggggcagca tgaagaggaa accaaaggca tagaccccgt tgacgaagct 480
   gttgattaac caggagtacc agctcttata tttgatattc aggagtgaat agacagcacc 540
   cccgacacag agagggtaca gcaggtatga caagtacttc atggcctgag tatcgtactc 600
   ctcggttttc ctctcagatt cgctgtaagt gccaaactga aattcgggca tcaggcctct 660
   ccaaaaaata gtcatcttca atgccttctt cactttccac agctcaatgg cggctccaac 720
   acccgccggg accagcacca gcaggctcgt ctgctcgtcc agcaggaaca gaaagatgac 780
   cacggtgctg aagcagcgcc agagcactgc cttggtggac atgccgatca tgctcttctt 840
   cttcttccag aaactgatgt catttttaaa ggccaggaaa tcaaagagaa gatggaacgc 900
   tgcgacaaag aaggtcagcg ccaggaagta taagttggta tctacaaaaa ttcctttcac 960
   ctcatcagca tctttctctg aaaacccgaa ctgctgcagg gagtacacgg cgtcctgcat 1020
   gtggatccag aagcgcagcc gccccagtga gaccttgtcg taggacacgg tgaggggcag 1080
   ctcggtggtg gagcggttta tgaccatcag gtccttcacg cggttgctga gctggtcgat 1140
   gaacaggatg ggcaggtaat gcacggtttt ccccagctgg atcatcttca tgtaccgatg 1200
   cacatcggca ggcagggagg acccgtcaaa gacaaagttg tccgccatca cgttcagcgc 1260
   cagccgcggt cgccagtggg acactggctc atccagggca ctcgtcggct tcttctccgc 1320
   ctcgatctgc tgtgtatcag actccccggt gagcaggttg atttcttctg gcttggggac 1380
   catgtaggtg gtcagaggac tgaccaggtg cacctgcttc ccgtcgtgcc acggcaggac 1440
   cccagcgtga tggaggaaga tgtaggcata cagcgtccca ttgtttctcg ttttctttgg 1500
   tacagaaaca ttaactgtcc tttcaaattt ggactccaca tcaaagtctt ccacattcaa 1560
   gaccaggtcg atgttgttct cagcacccag gtgggacctc gtcgtggtgt acacgctcag 1620
   ctgcagcttg ggccgccgcg ccaggtaggg ctggatgcag ttggcgtcgc cggagcacgg 1680
   gcgggtgtag acgatgccgt acatgaccca gcaggtgtgc accacgtaga ccacgaacac 1740
   gcccaccacc aagctggtga aggagctgcg gcccc
<210> 30
   <211> 1546
   <212> DNA
   <213> Human
<400> 30
   aaaataagta ggaatgggca gtgggtattc acattcacta caccttttcc atttgctaat 60
   aaggccctgc caggctggga gggaattgtc cctgcctgct tctggagaaa gaagatattg 120
   acaccatcta cgggcaccat ggaactgctt caagtgacca ttctttttct tctgcccagt 180
   atttgcagca gtaacagcac aggtgtttta gaggcagcta ataattcact tgttgttact 240
   acaacaaaac catctataac aacaccaaac acagaatcat tacagaaaaa tgttgtcaca 300
   ccaacaactg gaacaactcc taaaggaaca atcaccaatg aattacttaa aatgtctctg 360
   atgtcaacag ctactttttt aacaagtaaa gatgaaggat tgaaagccac aaccactgat 420
   gtcaggaaga atgactccat catttcaaac gtaacagtaa caagtgttac acttccaaat 480
   gctgtttcaa cattacaaag ttccaaaccc aagactgaaa ctcagagttc aattaaaaca 540
   acagaaatac caggtagtgt tctacaacca gatgcatcac cttctaaaac tggtacatta 600
   acctcaatac cagttacaat tccagaaaac acctcacagt ctcaagtaat aggcactgag 660
   ggtggaaaaa atgcaagcac ttcagcaacc agccggtctt attccagtat tattttgccg 720
   gtggttattg ctttgattgt aataacactt tcagtatttg ttctggtggg tttgtaccga 780
   atgtgctgga aggcagatcc gggcacacca gaaaatggaa atgatcaacc tcagtctgat 840
   aaagagagcg tgaagcttct taccgttaag acaatttctc atgagtctgg tgagcactct 900
   gcacaaggaa aaaccaagaa ctgacagctt gaggaattct ctccacacct aggcaataat 960
   tacgcttaat cttcagcttc tatgcaccaa gcgtggaaaa ggagaaagtc ctgcagaatc 1020
   aatcccgact tccatacctg ctgctggact gtaccagacg tctgtcccag taaagtgatg 1080
   tccagctgac atgcaataat ttgatggaat caaaaagaac cccggggctc tcctgttctc 1140
   tcacatttaa aaattccatt actccattta caggagcgtt cctaggaaaa ggaattttag 1200
   gaggagaatt tgtgagcagt gaatctgaca gcccaggagg tgggctcgct gataggcatg 1260
   actttcctta atgtttaaag ttttccgggc caagaatttt tatccatgaa gactttccta 1320
   cttttctcgg tgttcttata ttacctactg ttagtattta ttgtttacca ctatgttaat 1380
   gcagggaaaa gttgcacgtg tattattaaa tattaggtag aaatcatacc atgctacttt 1440
   gtacatataa gtattttatt cctgctttcg tgttactttt aataaataac tactgtactc 1500
   aatactctaa aaatactata acatgactgt gaaaatggca aaaaaa
<210> 31
   <211> 750
   <212> DNA
   <213> Human
<400> 31
   cacttgggca cccccatttt ctaaaaaaat ggaaatctgg agggcaaaaa aggtgtgctg 60
   aagggaagtg cctctgatgg cccaaaaacc ttcttccaaa ctagtgtagg aatggaatgg 120
   atagcaaatg gatccttttt ggcctccttt ggagcatgcc ttccctatct tatccttggc 180
   cccactaaag cagaacgtta cggatatttc tgtttttgcc attggatgcc tatctggcca 240
   aacagccttt ccctaattgg aaaatgcagt cctgtttaaa acctttgatt tacgactact 300
   tgtacatgct tgctcattac aattttgaca ttttttacat agtgaagacc ccaaacatat 360
   cagtgaaaca tgacaagatc ataaagaaca gtatcatatt attatttagt cgcttttaca 420
   gtggcaagcc aattttgaaa tatctcattt aaaactcaga cccaattcac tgagttatac 480
   ttttaatagc ttcctcagca cactatttcc catgcattaa atatgataaa ataatctatc 540
   actgcccatc ggtcttgtaa aaaggaagtc tgaatacaga gcccacaaca ctaaaattgt 600
   ttttctagct acaaagtata gcatcatcaa cacagacacg atttggactc cctgacaggt 660
   ggattggaaa acggtgttta aagagaagag aacattttaa cataaatgtc attaagaatc 720
   ccaaaggcct tatttgtcac caccgtcccg
<210> 32
   <211> 1620
   <212> DNA
   <213> Human
<400> 32
   gcaattcccc cctcccacta aacgactccc agtaattatg tttacaaccc attggatgca 60
   gtgcagccat tcataagaac cttggtgccc cagaaaaatc tgtccttttt ggtaccaaac 120
   ctgaggtctt ttggaagata atgtagaaaa ccactaccta ttgaaggcct gttttggcta 180
   atctgtgcaa actctgatga tacctgcctt atgtggattc ttttccacac tgctttcatt 240
   tttaagtata aagacttaga aaactagaat aatgctttta caaataatta aaagtatgtg 300
   atgttctggg ttttttcctt ctttttagaa ccccgcctcc atttaaaaaa ttaaaaaaaa 360
   aaaaaaaact tttaacattt aaaaaataaa aattaacaaa atttcactta ttccaggaca 420
   cgctggcatt tggactcaat gaaaagggca cctaaagaaa ataaggctga ctgaatgttt 480
   tccataattt tcacacaata acagtccctt tctatccagc ttgccttcca tttatctcta 540
   gggttagctt ttcaggcaac atccttggtc attgcccaga aagtacctga gctatcagtg 600
   attggaatgg cacaggaaac cgaatcacat gggtgccctc cccttggttt tcaagtatct 660
   tggagttgtg cacaaaaatt aggtcatgcc ttcagtgtct tgttctttaa acctaccctt 720
   tgacaatcag gtgctaatga ttgtatacta ttaaaaccag cacataagta ttgtaaatgt 780
   gtgttcctcc taggttggaa gaaatgtctt tccttctatc tgggtcctgt taaagcgggt 840
   gtcagttgtg tcttttcacc tcgatttgtg aattaataga attgggggga gaggaaatga 900
   tgatgtcaat taagtttcag gtttggcatg atcatcattc tcgatgatat tctcactttg 960
   tcgcaaatct gcccttatcg taagaacaag tttcagaatt ttccctccac tatacgactc 1020
   cagtattatg tttacaatcc attggatgag tgcagcatta taagaccttg gtgcccagaa 1080
   aaatctgtcc tttttggtac caaacctgag gtcttttgga agataatgta gaaaaccact 1140
   acctattgaa ggcctgtttt ggctaatctg tgcaaactct gatgatacct gcttatgtgg 1200
   attcttttcc acactgcttt catttttaag tataaagact tagaaaacta gaataatgct 1260
   tttacaaata attaaaagta tgtgatgttc tgggtttttt ccttcttttt agaaccctgt 1320
   atttaaacaa gccttctttt taagtcttgt ttgaaattta agtctcagat cttctggata 1380
   ccaaatcaaa aacccaacgc gtaaaacagg gcagtatttg tgttcctaat tttaaaaagc 1440
   tttatgtata ctctataaat atagatgcat aaacaacact tccccttgag tagcacatca 1500
   acatacagca ttgtacatta caatgaaaat gtgtaactta agggtattat atatataaat 1560
   acatatatac ctttgtaacc tttatactgt aaataaaaaa gttgctttag tcaaaaaaaa 1620
<210> 33
   <211> 2968
   <212> DNA
   <213> Human
<400> 33
   gaaaaagtag aaggaaacac agttcatata gaagtaaaag aaaaccctga agaggaggag 60
   gaggaggaag aagaggaaga agaagatgaa gaaagtgaag aggaggagga agaggaggga 120
   gaaagtgaag gcagtgaagg tgatgaggaa gatgaaaagg tgtcagatga gaaggattca 180
   gggaagacat tagataaaaa gccaagtaaa gaaatgagct cagattctga atatgactct 240
   gatgatgatc ggactaaaga agaaagggct tatgacaaag caaaacggag gattgagaaa 300
   cggcgacttg aacatagtaa aaatgtaaac accgaaaagc taagagcccc tattatctgc 360
   gtacttgggc atgtggacac agggaagaca aaaattctag ataagctccg tcacacacat 420
   gtacaagatg gtgaagcagg tggtatcaca caacaaattg gggccaccaa tgttcctctt 480
   gaagctatta atgaacagac taagatgatt aaaaattttg atagagagaa tgtacggatt 540
   ccaggaatgc taattattga tactcctggg catgaatctt tcagtaatct gagaaataga 600
   ggaagctctc tttgtgacat tgccatttta gttgttgata ttatgcatgg tttggagccc 660
   cagacaattg agtctatcaa ccttctcaaa tctaaaaaat gtcccttcat tgttgcactc 720
   aataagattg ataggttata tgattggaaa aagagtcctg actctgatgt ggctgctact 780
   ttaaagaagc agaaaaagaa tacaaaagat gaatttgagg agcgagcaaa ggctattatt 840
   gtagaatttg cacagcaggg tttgaatgct gctttgtttt atgagaataa agatccccgc 900
   acttttgtgt ctttggtacc tacctctgca catactggtg atggcatggg aagtctgatc 960
   taccttcttg tagagttaac tcagaccatg ttgagcaaga gacttgcaca ctgtgaagag 1020
   ctgagagcac aggtgatgga ggttaaagct ctcccgggga tgggcaccac tatagatgtc 1080
   atcttgatca atgggcgttt gaaggaagga gatacaatca ttgttcctgg agtagaaggg 1140
   cccattgtaa ctcagattcg aggcctcctg ttacctcctc ctatgaagga attacgagtg 1200
   aagaaccagt atgaaaagca taaagaagta gaagcagctc agggggtaaa gattcttgga 1260
   aaagacctgg agaaaacatt ggctggttta cccctccttg tggcttataa agaagatgaa 1320
   atccctgttc ttaaagatga attgatccat gagttaaagc agacactaaa tgctatcaaa 1380
   ttagaagaaa aaggagtcta tgtccaggca tctacactgg gttctttgga agctctactg 1440
   gaatttctga aaacatcaga agtgccctat gcaggaatta acattggccc agtgcataaa 1500
   aaagatgtta tgaaggcttc agtgatgttg gaacatgacc ctcagtatgc agtaattttg 1560
   gccttcgatg tgagaattga acgagatgca caagaaatgg ctgatagttt aggagttaga 1620
   atttttagtg cagaaattat ttatcattta tttgatgcct ttacaaaata tagacaagac 1680
   tacaagaaac agaaacaaga agaatttaag cacatagcag tatttccctg caagataaaa 1740
   atcctccctc agtacatttt taattctcga gatccgatag tgatgggggt gacggtggaa 1800
   gcaggtcagg tgaaacaggg gacacccatg tgtgtcccaa gcaaaaattt tgttgacatc 1860
   ggaatagtaa caagtattga aataaaccat aaacaagtgg atgttgcaaa aaaaggacaa 1920
   gaagtttgtg taaaaataga acctatccct ggtgagtcac ccaaaatgtt tggaagacat 1980
   tttgaagcta cagatattct tgttagtaag atcagccggc agtccattga tgcactcaaa 2040
   gactggttca gagatgaaat gcagaagagt gactggcagc ttattgtgga gctgaagaaa 2100
   gtatttgaaa tcatctaatt ttttcacatg gagcaggaac tggagtaaat gcaatactgt 2160
   gttgtaatat cccaacaaaa atcagacaaa aaatggaaca gacgtatttg gacactgatg 2220
   gacttaagta tggaaggaag aaaaataggt gtataaaatg ttttccatga gaaaccaaga 2280
   aacttacact ggtttgacag tggtcagtta catgtcccca cagttccaat gtgcctgttc 2340
   actcacctct cccttcccca acccttctct acttggctgc tgttttaaag tttgcccttc 2400
   cccaaatttg gatttttatt acagatctaa agctctttcg attttatact gattaaatca 2460
   gtactgcagt atttgattaa aaaaaaaaaa gcagattttg tgattcttgg gacttttttg 2520
   acgtaagaaa tacttcttta tttatgcata ttcttcccac agtgattttt ccagcattct 2580
   tctgccatat gcctttaggg cttttataaa atagaaaatt aggcattctg atatttcttt 2640
   agctgctttg tgtgaaacca tggtgtaaaa gcacagctgg ctgcttttta ctgcttgtgt 2700
   agtcacgagt ccattgtaat catcacaatt ctaaaccaaa ctaccaataa agaaaacaga 2760
   catccaccag taagcaagct ctgttaggct tccatggtta gtggtagctt ctctcccaca 2820
   agttgtcctc ctaggacaag gaattatctt aacaaactaa actatccatc acactacctt 2880
   ggtatgccag cacctgggta acagtaggag attttataca ttaatctgat ctgtttaatc 2940
   tgatcggttt agtagagatt ttatacat
<210> 34
   <211> 6011
   <212> DNA
   <213> Human
<400> 34
   acggggcgcc ggacgacccg cacatcttat cctccacgcc ccactcgcac tcggagcggg 60
   accgccccgg actccccctc gggccggcca ctcgaggagt gaggagagag gccgccggcc 120
   cggcttgagc cgagcgcagc accccccgcg ccccgcgcca gaagtttggt tgaaccgggc 180
   tgccgggaga aacttttttc ttttttcccc ctctcccggg agagtctctg gaggaggagg 240
   ggaactcccc cggcccaagg ctcgtgggct cggggtcgcg cggccgcaga aggggcgggg 300
   tccgcccgcg aggggaggcg cccccgggga cccgagaggg gggtgaggac cgcgggctgc 360
   tggtgcggcg gcggcagcgt gtgccccgcg caggggaggc gccgccccgc tcccggcccg 420
   gctgcgagga ggaggcggcg gcggcgcagg aggatgtact tggtggcggg ggacaggggg 480
   ttggccggct gcgggcacct cctggtctcg ctgctggggc tgctgctgct gccggcgcgc 540
   tccggcaccc gggcgctggt ctgcctgccc tgtgacgagt ccaagtgcga ggagcccagg 600
   aaccgcccgg ggagcatcgt gcagggcgtc tgcggctgct gctacacgtg cgccagccag 660
   gggaacgaga gctgcggcgg caccttcggg atttacggaa cctgcgaccg ggggctgcgt 720
   tgtgtcatcc gccccccgct caatggcgac tccctcaccg agtacgaagc gggcgtttgc 780
   gaagatgaga actggactga tgaccaactg cttggtttta aaccatgcaa tgaaaacctt 840
   attgctggct gcaatataat caatgggaaa tgtgaatgta acaccattcg aacctgcagc 900
   aatccctttg agtttccaag tcaggatatg tgcctttcag ctttaaagag aattgaagaa 960
   gagaagccag attgctccaa ggcccgctgt gaagtccagt tctctccacg ttgtcctgaa 1020
   gattctgttc tgatcgaggg ttatgctcct cctggggagt gctgtccctt acccagccgc 1080
   tgcgtgtgca accccgcagg ctgtctgcgc aaagtctgcc agccgggaaa cctgaacata 1140
   ctagtgtcaa aagcctcagg gaagccggga gagtgctgtg acctctatga gtgcaaacca 1200
   gttttcggcg tggactgcag gactgtggaa tgccctactg ttcagcagac cgcgtgtccc 1260
   ccggacagct atgaaactca agtcagacta actgcagatg gttgctgtac tttgccaaca 1320
   agatgcgagt gtctctctgg cttatgtggt ttccccgtgt gtgaggtggg atccactccc 1380
   cgcatagtct ctcgtggcga tgggacacct ggaaagtgct gtgatgtctt tgaatgtgtt 1440
   aatgatacaa agccagcctg cgtatttaac aatgtggaat attatgatgg agacatgttt 1500
   cgaatggaca actgtcggtt ctgtcgatgc caagggggcg ttgccatctg cttcaccgcc 1560
   cagtgtggtg agataaactg cgagaggtac tacgtgcccg aaggagagtg ctgcccagtg 1620
   tgtgaagatc cagtgtatcc ttttaataat cccgctggct gctatgccaa tggcctgatc 1680
   cttgcccacg gagaccggtg gcgggaagac gactgcacat tctgccagtg cgtcaacggt 1740
   gaacgccact gcgttgcgac cgtctgcgga cagacctgca caaaccctgt gaaagtgcct 1800
   ggggagtgtt gccctgtgtg cgaagaacca accatcatca cagttgatcc acctgcatgt 1860
   ggggagttat caaactgcac tctgacacgg aaggactgca ttaatggttt caaacgcgat 1920
   cacaatggtt gtcggacctg tcagtgcata aacacccagg aactatgttc agaacgtaaa 1980
   caaggctgca ccttgaactg tcccttcggt ttccttactg atgcccaaaa ctgtgagatc 2040
   tgtgagtgcc gcccaaggcc caagaagtgc agacccataa tctgtgacaa gtattgtcca 2100
   cttggattgc tgaagaataa gcacggctgt gacatctgtc gctgtaagaa atgtccagag 2160
   ctctcatgca gtaagatctg ccccttgggt ttccagcagg acagtcacgg ctgtcttatc 2220
   tgcaagtgca gagaggcctc tgcttcagct gggccaccca tcctgtcggg cacttgtctc 2280
   accgtggatg gtcatcatca taaaaatgag gagagctggc acgatgggtg ccgggaatgc 2340
   tactgtctca atggacggga aatgtgtgcc ctgatcacct gcccggtgcc tgcctgtggc 2400
   aaccccacca ttcaccctgg acagtgctgc ccatcatgtg cagatgactt tgtggtgcag 2460
   aagccagagc tcagtactcc ctccatttgc cacgcccctg gaggagaata ctttgtggaa 2520
   ggagaaacgt ggaacattga ctcctgtact cagtgcacct gccacagcgg acgggtgctg 2580
   tgtgagacag aggtgtgccc accgctgctc tgccagaacc cctcacgcac ccaggattcc 2640
   tgctgcccac agtgtacaga tcaacctttt cggccttcct tgtcccgcaa taacagcgta 2700
   cctaattact gcaaaaatga tgaaggggat atattcctgg cagctgagtc ctggaagcct 2760
   gacgtttgta ccagctgcat ctgcattgat agcgtaatta gctgtttctc tgagtcctgc 2820
   ccttctgtat cctgtgaaag acctgtcttg agaaaaggcc agtgttgtcc ctactgcata 2880
   aaagacacaa ttccaaagaa ggtggtgtgc cacttcagtg ggaaggccta tgccgacgag 2940
   gagcggtggg accttgacag ctgcacccac tgctactgcc tgcagggcca gaccctctgc 3000
   tcgaccgtca gctgcccccc tctgccctgt gttgagccca tcaacgtgga aggaagttgc 3060
   tgcccaatgt gtccagaaat gtatgtccca gaaccaacca atatacccat tgagaagaca 3120
   aaccatcgag gagaggttga cctggaggtt cccctgtggc ccacgcctag tgaaaatgat 3180
   atcgtccatc tccctagaga tatgggtcac ctccaggtag attacagaga taacaggctg 3240
   cacccaagtg aagattcttc actggactcc attgcctcag ttgtggttcc cataattata 3300
   tgcctctcta ttataatagc attcctattc atcaatcaga agaaacagtg gataccactg 3360
   ctttgctggt atcgaacacc aactaagcct tcttccttaa ataatcagct agtatctgtg 3420
   gactgcaaga aaggaaccag agtccaggtg gacagttccc agagaatgct aagaattgca 3480
   gaaccagatg caagattcag tggcttctac agcatgcaaa aacagaacca tctacaggca 3540
   gacaatttct accaaacagt gtgaagaaag gcaactagga tgaggtttca aaagacggaa 3600
   gacgactaaa tctgctctaa aaagtaaact agaatttgtg cacttgctta gtggattgta 3660
   ttggattgtg acttgatgta cagcgctaag accttactgg gatgggctct gtctacagca 3720
   atgtgcagaa caagcattcc cacttttcct caagataact gaccaagtgt tttcttagaa 3780
   ccaaagtttt taaagttgct aagatatatt tgcctgtaag atagctgtag agatatttgg 3840
   ggtggggaca gtgagtttgg atggggaaag gggtgggagg gtggtgttgg gaagaaaaat 3900
   tggtcagctt ggctcgggga gaaacctggt aacataaaag cagttcagtg gcccagaggt 3960
   tatttttttc ctattgctct gaagactgca ctggttgctg caaagctcag gcctgaatga 4020
   gcaggaaaca aaaaaggcct tgcgacccag ctgccataac caccttagaa ctaccagacg 4080
   agcacatcag aaccctttga cagccatccc aggtctaaag ccacaagttt cttttctata 4140
   cagtcacaac tgcagtaggc agtgaggaag ccagagaaat gcgatagcgg catttctcta 4200
   aagcgggtta ttaaggatat atacagttac actttttgct gcttttattt tcttccaagc 4260
   caatcaatca gccagttcct agcagagtca gcacatgaac aagatctaag tcatttcttg 4320
   atgtgagcac tggagctttt tttttttaca acgtgacagg aagaggaggg agagggtgac 4380
   gaacaccagg catttccagg ggctatattt cactgtttgt tgttgctttg ttctgttata 4440
   ttgttggttg ttcatagttt ttgttgaagc tctagcttaa gaagaaactt tttttaaaaa 4500
   gactgtttgg ggattctttt tccttattat atactgattc tacaaaatag aaactacttc 4560
   attttaattg tatattattc aagcaccttt gttgaagctc aaaaaaaatg atgcctcttt 4620
   aaactttagc aattatagga gtatttatgt aactatctta tgcttcaaaa aacaaaagta 4680
   tttgtgtgca tgtgtatata atatatatat atacatatat atttatacac atacaattta 4740
   tgttttcctg ttgaatgtat ttttatgaga ttttaaccag aacaaaggca gataaacagg 4800
   cattccatag cagtgctttt gatcacttac aaattttttg aataacacaa aatctcattc 4860
   tacctgcagt ttaattggaa agatgtgtgt gtgagagtat gtatgtgtgt gtgtgtgtgt 4920
   gtgtgtgcgc gcgcacgcac gccttgagca gtcagcattg cacctgctat ggagaagggt 4980
   attcctttat taaaatcttc ctcatttgga tttgctttca gttggttttc aatttgctca 5040
   ctggccagag acattgatgg cagttcttat ctgcatcact aatcagctcc tggatttttt 5100
   tttttttttt tcaaacaatg gtttgaaaca actactggaa tattgtccac aataagctgg 5160
   aagtttgttg tagtatgcct caaatataac tgactgtata ctatagtggt aacttttcaa 5220
   acagccctta gcacttttat actaattaac ccatttgtgc attgagtttt cttttaaaaa 5280
   tgcttgttgt gaaagacaca gatacccagt atgcttaacg tgaaaagaaa atgtgttctg 5340
   ttttgtaaag gaactttcaa gtattgttgt aaatacttgg acagaggttg ctgaacttta 5400
   aaaaaaatta atttattatt ataatgacct aatttattaa tctgaagatt aaccattttt 5460
   ttgtcttaga atatcaaaaa gaaaaagaaa aaggtgttct agctgtttgc atcaaaggaa 5520
   aaaaagattt attatcaagg ggcaatattt ttatcttttc caaaataaat ttgttaatga 5580
   tacattacaa aaatagattg acatcagcct gattagtata aattttgttg gtaattaatc 5640
   cattcctggc ataaaaagtc tttatcaaaa aaaattgtag atgcttgctt tttgtttttt 5700
   caatcatggc catattatga aaatactaac aggatatagg acaaggtgta aattttttta 5760
   ttattatttt aaagatatga tttatcctga gtgctgtatc tattactctt ttactttggt 5820
   tcctgttgtg ctcttgtaaa agaaaaatat aatttcctga agaataaaat agatatatgg 5880
   cacttggagt gcatcatagt tctacagttt gtttttgttt tcttcaaaaa agctgtaaga 5940
   gaattatctg caacttgatt cttggcagga aataaacatt ttgagttgaa atcaaaaaaa 6000
   aaaaaaaaaa a
<210> 34a
   <211> 1036
   <212> DNA
   <213> Human
<400> 34a
<210> 35
   <211> 716
   <212> DNA
   <213> Human
<400> 35
<210> 36
   <211> 395
   <212> DNA
   <213> Human
<400> 36
   ccaatacttc attcttcatt ggtggagaag attgtagact tctaagcatt ttccaaataa 60
   aaaagctatg atttgatttc caacttttaa acattgcatg tcctttgcca tttactacat 120
   tctccaaaaa aaccttgaaa tgaagaaggc cacccttaaa atacttcaga ggctgaaaat 180
   atgattatta cattggaatc ctttagccta tgtgatattt ctttaacttt gcactttcac 240
   gcccagtaaa accaaagtca gggtaaccaa tgtcatttta caaaatgtta aaaccctaat 300
   tgcagttcct tttttaaatt attttaaaga ttacttaaca acattagaca gtgcaaaaaa 360
   agaagcaagg aaagcattct taattctacc atcct
<210> 37
   <211> 134
   <212> DNA
   <213> Human
<400> 37
   ccctcgagcg gccgcccggg caggtacttt taccaccgaa ttgttcactt gactttaaga 60
   aacccataaa gctgcctggc tttcagcaac aggcctatca acaccatggt gagtctccat 120
   aagggacacc gtgt
<210> 38
   <211> 644
   <212> DNA
   <213> Human
<400> 38
   aagcctgttg tcatggggga ggtggtggcg cttggtggcc actggcggcc gaggtagagg 60
   cagtggcgct tgagttggtc gggggcagcg gcagatttga ggcttaagca acttcttccg 120
   gggaagagtg ccagtgcagc cactgttaca attcaagatc ttgatctata tccatagatt 180
   ggaatattgg tgggccagca atcctcagac gcctcactta ggacaaatga ggaaactgag 240
   gcttggtgaa gttacgaaac ttgtccaaaa tcacacaact tgtaaagggc acagccaaga 300
   ttcagagcca ggctgtaaaa attaaaatga acaaattacg gcaaagtttt aggagaaaga 360
   aggatgttta tgttccagag gccagtcgtc cacatcagtg gcagacagat gaagaaggcg 420
   ttcgcaccgg aaaatgtagc ttcccggtta agtaccttgg ccatgtagaa gttgatgaat 480
   caagaggaat gcacatctgt gaagatgctg taaaaagatt gaaagctgaa aggaagttct 540
   tcaaaggctt ctttggaaaa actggaaaga aagcagttaa agcagtttct gtgggtctaa 600
   gcagatggac tcagaggttg tggatgaaaa actaaggacc tcat
<210> 39
   <211> 657
   <212> DNA
   <213> Human
<400> 39
   ctttttgttt gggttttcca atgtagatgt ctcagtgaaa tgtgcagata tactttgttc 60
   cttatatggt caccagtgtt aattatggac aaatacatta aaacaagggt tcctggccca 120
   gcctcccatc taatctcttt gatactcttg gaatctaagt ctgaggagcg atttctgaat 180
   tagccagtgt tgtaccaact ttctgttagg aattgtatta gaataacctt tctttttcag 240
   acctgctcag tgagacatct tggggaatga agtaggaaaa tagacatttg gtggaaaaac 300
   agcaaaatga gaacattaaa aagactcatt caagtatgag tataaagggc atggaaattc 360
   tggtcctttg agcaaaatga gaagaaaaaa ttctgctcag cagtattcac tgtgttaaga 420
   ttttttgttt tttacacgaa tggaaaaatg atgtgtaagt ggtatagatt ttaatcagct 480
   aacagtcact ccagagattt tgatcagcac caattcctat agtagtaagt atttaaaagt 540
   taagaaatac tactacattt aacattataa agtagagttc tggacataac tgaaaattag 600
   atgtttgctt caatagaaat ttgttcccac ttgtattttc aacaaaatta tcggaac
<210> 40
   <211> 1328
   <212> DNA
   <213> Human
<400> 40
   acaattttaa aataactagc aattaatcac agcatatcag gaaaaagtac acagtgagtt 60
   ctggttagtt tttgtaggct cattatggtt agggtcgtta agatgtatat aagaacctac 120
   ctatcatgct gtatgtatca ctcattccat tttcatgttc catgcatact cgggcatcat 180
   gctaatatgt atccttttaa gcactctcaa ggaaacaaaa gggcctttta tttttataaa 240
   ggtaaaaaaa attccccaaa tattttgcac tgaatgtacc aaaggtgaag ggacattaca 300
   atatgactaa cagcaactcc atcacttgag aagtataata gaaaatagct tctaaatcaa 360
   acttccttca cagtgccgtg tctaccacta caaggactgt gcatctaagt aataattttt 420
   taagattcac tatatgtgat agtatgatat gcatttattt aaaatgcatt agactctctt 480
   ccatccatca aatactttac aggatggcat ttaatacaga tatttcgtat ttcccccact 540
   gctttttatt tgtacagcat cattaaacac taagctcagt taaggagcca tcagcaacac 600
   tgaagagatc agtagtaaga attccatttt ccctcatcag tgaagacacc acaaattgaa 660
   actcagaact atatttctaa gcctgcattt tcactgatgc ataattttct tagtaatatt 720
   aagagacagt ttttctatgg catctccaaa actgcatgac atcactagtc ttacttctgc 780
   ttaattttat gagaaggtat tcttcatttt aattgctttt gggattactc cacatctttg 840
   tttatttctt gactaatcag attttcaata gagtgaagtt aaattggggg tcataaaagc 900
   attggattga catatggttt gccagcctat gggtttacag gcattgccca aacatttctt 960
   tgagatctat atttataagc agccatggaa ttcctattat gggatgttgg caatcttaca 1020
   ttttatagag gtcatatgca tagttttcat aggtgttttg taagaactga ttgctctcct 1080
   gtgagttaag ctatgtttac tactgggacc ctcaagagga ataccactta tgttacactc 1140
   ctgcactaaa ggcacgtact gcagtgtgaa gaaatgttct gaaaaagggt tatagaaatc 1200
   tggaaataag aaaggaagag ctctctgtat tctataattg gaagagaaaa aaagaaaaac 1260
   ttttaactgg aaatgttagt ttgtacttat tgatcatgaa tacaagtata tatttaattt 1320
   tgaaaaaa
<210> 41
   <211> 987
   <212> DNA
   <213> Human
<400> 41
   aacagagact ggcacaggac ctcttcattg caggaagatg gtagtgtagg caggtaacat 60
   tgagctcttt tcaaaaaagg agagctcttc ttcaagataa ggaagtggta gttatggtgg 120
   taacccccgg ctatcagtcc ggatggttgc cacccctcct gctgtaggat ggaagcagcc 180
   atggagtggg agggaggcgc aataagacac ccctccacag agcttggcat catgggaagc 240
   tggttctacc tcttcctggc tcctttgttt aaaggcctgg ctgggagcct tccttttggg 300
   tgtctttctc ttctccaacc aacagaaaag actgctcttc aaaggtggag ggtcttcatg 360
   aaacacagct gccaggagcc caggcacagg gctgggggcc tggaaaaagg agggcacaca 420
   ggaggaggga ggagctggta gggagatgct ggctttacct aaggtctcga aacaaggagg 480
   gcagaatagg cagaggcctc tccgtcccag gcccattttt gacagatggc gggacggaaa 540
   tgcaatagac cagcctgcaa gaaagacatg tgttttgatg acaggcagtg tggccgggtg 600
   gaacaagcac aggccttgga atccaatgga ctgaatcaga accctaggcc tgccatctgt 660
   cagccgggtg acctgggtca attttagcct ctaaaagcct cagtctcctt atctgcaaaa 720
   tgaggcttgt gatacctgtt ttgaagggtt gctgagaaaa ttaaagataa gggtatccaa 780
   aatagtctac ggccatacca ccctgaacgt gcctaatctc gtaagctaag cagggtcagg 840
   cctggttagt acctggatgg ggagagtatg gaaaacatac ctgcccgcag ttggagttgg 900
   actctgtctt aacagtagcg tggcacacag aaggcactca gtaaatactt gttgaataaa 960
   tgaagtagcg atttggtgtg aaaaaaa
<210> 42
   <211> 956
   <212> DNA
   <213> Human
<400> 42
   cggacggtgg ggcggacgcg tgggtgcagg agcagggcgg ctgccgactg ccccaaccaa 60
   ggaaggagcc cctgagtccg cctgcgcctc catccatctg tccggccaga gccggcatcc 120
   ttgcctgtct aaagccttaa ctaagactcc cgccccgggc tggccctgtg cagaccttac 180
   tcaggggatg tttacctggt gctcgggaag ggaggggaag gggccgggga gggggcacgg 240
   caggcgtgtg gcagccacac gcaggcggcc agggcggcca gggacccaaa gcaggatgac 300
   cacgcacctc cacgccactg cctcccccga atgcatttgg aaccaaagtc taaactgagc 360
   tcgcagcccc cgcgccctcc ctccgcctcc catcccgctt agcgctctgg acagatggac 420
   gcaggccctg tccagccccc agtgcgctcg ttccggtccc cacagactgc cccagccaac 480
   gagattgctg gaaaccaagt caggccaggt gggcggacaa aagggccagg tgcggcctgg 540
   ggggaacgga tgctccgagg actggactgt ttttttcaca catcgttgcc gcagcggtgg 600
   gaaggaaagg cagatgtaaa tgatgtgttg gtttacaggg tatatttttg ataccttcaa 660
   tgaattaatt cagatgtttt acgcaaggaa ggacttaccc agtattactg ctgctgtgct 720
   tttgatctct gcttaccgtt caagaggcgt gtgcaggccg acagtcggtg accccatcac 780
   tcgcaggacc aagggggcgg ggactgctgg ctcacgcccc gctgtgtcct ccctcccctc 840
   ccttccttgg gcagaatgaa ttcgatgcgt attctgtggc cgccatctgc gcagggtggt 900
   ggtattctgt catttacaca cgtcgttcta attaaaaagc gaattatact ccaaaa
<210> 43
   <211> 536
   <212> DNA
   <213> Human
<400> 43
   aaataaacac ttccataaca ttttgttttc gaagtctatt aatgcaatcc cacttttttc 60
   cccctagttt ctaaatgtta aagagagggg aaaaaaggct caggatagtt ttcacctcac 120
   agtgttagct gtcttttatt ttactcttgg aaatagagac tccattaggg ttttgacatt 180
   ttgggaaccc agttttacca ttgtgtcagt aaaacaataa gatagtttga gagcatatga 240
   tctaaataaa gacatttgaa gggttagttt gaattctaaa agtaggtaat agccaaatag 300
   cattctcatc ccttaacaga caaaaactta tttgtcaaaa gaattagaaa aggtgaaaat 360
   attttttcca gatgaaactt gtgccacttc caattgacta atgaaataca aggagacaga 420
   ctggaaaaag tgggttatgc cacctttaaa accctttctg gtaaatatta tggtagctaa 480
   agggtggttt ccccggcacc tggacctgga caggtagggt tccgtggtta accagt
<210> 44
   <211> 1630
   <212> DNA
   <213> Human
<400> 44
   ggggagggac gagtatggaa ccctgaaggt agcaagtcca ggcactggcc tgaccatccg 60
   gctccctggg caccaagtcc caggcaggag cagctgtttt ccatcccttc ccagacaagc 120
   tctattttta tcacaatgac ctttagagag gtctcccagg ccagctcaag gtgtcccact 180
   atcccctctg gagggaagag gcaggaaaat tctccccggg tccctgtcat gctactttct 240
   ccatcccagt tcagactgtc caggacatct tatctgcagc cataagagaa ttataaggca 300
   gtgatttccc ttaggcccag gacttgggcc tccagctcat ctgttccttc tgggcccatt 360
   catggcaggt tctgggctca aagctgaact ggggagagaa gagatacaga gctaccatgt 420
   gactttacct gattgccctc agtttggggt tgcttattgg gaaagagaga gacaaagagt 480
   tacttgttac gggaaatatg aaaagcatgg ccaggatgca tagaggagat tctagcaggg 540
   gacaggattg gctcagatga cccctgaggg ctcttccagt cttgaaatgc attccatgat 600
   attaggaagt cgggggtggg tggtggtggt gggctagttg ggtttgaatt taggggccga 660
   tgagcttggg tacgtgagca gggtgttaag ttagggtctg cctgtatttc tggtcccctt 720
   ggaaatgtcc ccttcttcag tgtcagacct cagtcccagt gtccatatcg tgcccagaaa 780
   agtagacatt atcctgcccc atcccttccc cagtgcactc tgacctagct agtgcctggt 840
   gcccagtgac ctgggggagc ctggctgcag gccctcactg gttccctaaa ccttggtggc 900
   tgtgattcag gtccccaggg gggactcagg gaggaatatg gctgagttct gtagtttcca 960
   gagttggctg gtagagcctt ctagaggttc agaatattag cttcaggatc agctgggggt 1020
   atggaattgg ctgaggatca aacgtatgta ggtgaaagga taccaggatg ttgctaaagg 1080
   tgagggacag tttgggtttg ggacttacca gggtgatgtt agatctggaa cccccaagtg 1140
   aggctggagg gagttaaggt cagtatggaa gatagggttg ggacagggtg ctttggaatg 1200
   aaagagtgac cttagagggc tccttgggcc tcaggaatgc tcctgctgct gtgaagatga 1260
   gaaggtgctc ttactcagtt aatgatgagt gactatattt accaaagccc ctacctgctg 1320
   ctgggtccct tgtagcacag gagactgggg ctaagggccc ctcccaggga agggacacca 1380
   tcaggcctct ggctgaggca gtagcataga ggatccattt ctacctgcat ttcccagagg 1440
   actagcagga ggcagccttg agaaaccggc agttcccaag ccagcgcctg gctgttctct 1500
   cattgtcact gccctctccc caacctctcc tctaacccac tagagattgc ctgtgtcctg 1560
   cctcttgcct cttgtagaat gcagctctgg ccctcaataa atgcttcctg cattcatctg 1620
   caaaaaaaaa
<210> 45
   <211> 169
   <212> DNA
   <213> Human
<400> 45
   tcttttgctt ttagcttttt atttttgtat taacaggagt cttattacac ataggtctga 60
   taaaactggt ttatgatctt cagtctgatt ccagtgctgc ataactagat aacgtatgaa 120
   ggaaaaacga cgacgaacaa aaaagtaagt gcttggaaga cttagttga
<210> 46
   <211> 769
   <212> DNA
   <213> Human
<400> 46
   tgcaggtcat atttactatc ggcaataaaa ggaagcaaag cagtattaag cagcggtgga 60
   atttgtcgct ttcacttttt ataaagtgct acataaaatg tcatatttcc aaatttaaaa 120
   acataactcc agttcttacc atgagaacag catggtgatc acgaaggatc ttcttgaaaa 180
   aaacaaaaac aaaaacaaaa aacaatgatc tcttctgggt atcacatcaa atgagataca 240
   aaggtgtact aggcaatctt agagatctgg caacttattt tatatataag gcatctgtga 300
   ccaagagacg ttatgaatta aatgtacaaa tgtattatgt ataaatgtat taaatgcaag 360
   cttcatataa tgacaccaat gtctctaagt tgctcagaga tcttgactgg ctgtggccct 420
   ggccagctcc tttcctgata gtctgattct gccttcatat ataggcagct cctgatcatc 480
   catgccagtg aatgagaaaa caagcatgga atatataaac tttaacatta aaaaatgttt 540
   tattttgtaa taaaatcaaa tttcccattg aaaccttcaa aaactttgca gaatgaggtt 600
   ttgatatatg tgtacaagta gtaccttctt agtgcaagaa aacatcatta tttctgtctg 660
   cctgcctttt tgtttttaaa aatgaagact atcattgaaa caagtttgtc ttcagtatca 720
   ggacatgttg acggagagga aaggtaggaa agggttaggg atagaagcc
<210> 47
   <211> 2529
   <212> DNA
   <213> Human
<400> 47
   tttagttcat agtaatgtaa aaccatttgt ttaattctaa atcaaatcac tttcacaaca 60
   gtgaaaatta gtgactggtt aaggtgtgcc actgtacata tcatcatttt ctgactgggg 120
   tcaggacctg gtcctagtcc acaagggtgg caggaggagg gtggaggcta agaacacaga 180
   aaacacacaa aagaaaggaa agctgccttg gcagaaggat gaggtggtga gcttgccgag 240
   ggatggtggg aagggggctc cctgttgggg ccgagccagg agtcccaagt cagctctcct 300
   gccttactta gctcctggca gagggtgagt ggggacctac gaggttcaaa atcaaatggc 360
   atttggccag cctggcttta ctaacaggtt cccagagtgc ctctgttggc tgagctctcc 420
   tgggctcact ccatttcatt gaagagtcca aatgattcat tttcctaccc acaacttttc 480
   attattcttc tggaaaccca tttctgttga gtccatctga cttaagtcct ctctccctcc 540
   actagttggg gccactgcac tgaggggggt cccaccaatt ctctctagag aagagacact 600
   ccagaggccc ctgcaacttt gcggatttcc agaaggtgat aaaaagagca ctcttgagtg 660
   ggtgcccagg aatgtttaaa atctatcagg cacactataa agctggtggt ttcttcctac 720
   caagtggatt cggcatatga accacctact caatacttta tattttgtct gtttaaacac 780
   tgaactctgg tgttgacagg tacaaaggag aagagatggg gactgtgaag aggggagggc 840
   ttccctcatc ttcctcaaga tctttgtttc cataaactat gcagtcataa ttgagaaaaa 900
   gcaatagatg gggcttccta ccatttgttg gttattgctg gggttagcca ggagcagtgt 960
   ggatggcaaa gtaggagaga ggcccagagg aaagcccatc tccctccagc tttggggtct 1020
   ccagaaagag gctggatttc tgggatgaag cctagaaggc agagcaagaa ctgttccacc 1080
   aggtgaacag tcctacctgc ttggtaccat agtccctcaa taagattcag aggaagaagc 1140
   ttatgaaact gaaaatcaaa tcaaggtatt gggaagaata atttcccctc gattccacag 1200
   gagggaagac cacacaatat cattgtgctg gggctcccca aggccctgcc acctggcttt 1260
   acaaatcatc aggggttgcc tgcttggcag tcacatgctt ccctggtttt agcacacata 1320
   caaggagttt tcagggaact ctatcaagcc ataccaaaat cagggtcaca tgtgggtttc 1380
   ccctttcctt gcctcttcat aaaagacaac ttggcttctg aggatggtgg tcttttgcat 1440
   gcagttgggc tgacctgaca aagcccccag tttcctgtgg caggttctgg gagaggatgc 1500
   attcaagctt ctgcagccta ggggacaggg ctgcttgttc agttattact gcctcggagc 1560
   tccaaatccc accaaagtcc tgactccagg tctttcctaa tgcacagtag tcagtctcag 1620
   cttcggcagt attctcggct gtatgttctc tggcagagag aggcagatga acatagtttt 1680
   agggagaaag ctgatgggaa acctgtgagt taagccacat gtctcaccag gaataattta 1740
   tgccaggaaa ccaggaagtc attcaagttg ttctctgagg ccaaagacac tgagcacagc 1800
   ccagagccaa taaaagatct ttgagtctct ggtgaattca cgaagtgacc ccagctttag 1860
   ctactgcaat tatgattttt atgggacagc aatttcttgc atctctacag aggaagaaga 1920
   gggggagtgg gaggggaagg aaagagaaca gagcggcact gggatttgaa aggggaacct 1980
   ctctatctga ggagccccca ctggcttcag aagcaactta ccaaggggta tttaaagaca 2040
   tgaaaatttc cagaaatacc atttggtgca tccctttgtt tctgtaatat taaactcagg 2100
   tgaaattata ctctgacagt ttctctcttt ctgcctcttc cctctgcaga gtcaggacct 2160
   gcagaactgg ctgaaacaag atttcatggt gtcacccatg agagatgact caatgccaag 2220
   gcctgaagtt atagagtgtt tacagcggtg gcgatattca ggggtcatcg ccaactggtc 2280
   tcgagttcca aagctctgat gaagaaacaa gactccttga tgtgttactg atcccactga 2340
   ttccaggagt caagattagc caggaagcca aacaccagga gttggggtgg cacgtcacca 2400
   gtccagagcc ctgccacgga tgtacgcagg agcccagcat taggcaatca ggagccagaa 2460
   catgatcacc agggccacaa ataggaagag gcgtgacagg aactgctcgt ccacatacct 2520
   ggggtgtcc
<210> 48
   <211> 1553
   <212> DNA
   <213> Human
<400> 48
<210> 49
   <211> 921
   <212> DNA
   <213> Human
<400> 49
   ctgtggtccc agctactcag gaggctgagg cgggaggatt gcttgagccc aggagttgga 60
   tgttgcagtg agccaagatc gcaccattgc cctccactct gggccacgga gcaataccct 120
   gtctcagaaa acaaacaaca aaaagcagaa acgctgaagg ggtcggttta cgggaaaacc 180
   gcctgtcaga acacttggct actcctaccc cagatcagtg gacctgggaa tgagggttgg 240
   tcccgggagg cttttctcca agctgttgcc accagacccg ccatgggaac cctggccaca 300
   gaagcctccc ggggagtgag ccagagcctg gaccgctgtg ctgatgtgtc tggggtggag 360
   ggagggtggg gagtgtgcaa gggtgtgtgt gtgcccgggg ggtgttcatg ggcaagcatg 420
   tgcgtgcctg tgtgtgtgcg tgcccctccc ctgcagccgt cggtggtatc tccctccagc 480
   cccttcgcca ccttctgagc attgtctgtc cacgtgagac tgcccagaga cagcagagct 540
   ccacgtggtt ttaaggggag acctttccct ggacctgggg gtctcgccgt atctcatgac 600
   caggtgctaa atgacccgac atgcatcacc tgcctttcga tgaccaacct ccctgtcccc 660
   gtcccgctga cctgcccccg tggcgtctca cggtgatgcc tgctcctgac attggtgttc 720
   actgtagcaa actacattct ggatgggaat tttcatgtac atgtgtggca tgtggaaaat 780
   ttcaaataaa atggacttga tttagaaagc caaaaagctg tgtggtcctt ccagcacgga 840
   tactttgacc tcttgcctac aaccccttcc ttgggtccga ggctggtagc tttgttcact 900
   tcagatggtt gggggcgggt g
<210> 50
   <211> 338
   <212> DNA
   <213> Human
<400> 50
   atgatctatc tagatgccct accgtaaaat caaaacacaa aaccctactg actcattccc 60
   tcccttccag atattacccc atttctctac ttcccattgt agccaaactt tccaaaaatt 120
   catgttctgt cttcatttcc tcatgttcaa cccaccctgt cttagctacc acccctcagt 180
   aacgacctag cctgggtaga aacaaatgtc agcatgatac catactcaat gatccttcgt 240
   cactgttgtc attgtcatca ttccatggcc ttactttccc tctcagcgcc atttgctaca 300
   gtaagaaact ttctttcttg aattcttggt tctcttgg
<210> 51
   <211> 1191
   <212> DNA
   <213> Human
<400> 51
   ctagcaagca ggtaaacgag ctttgtacaa acacacacag accaacacat ccggggatgg 60
   ctgtgtgttg ctagagcaga ggctgattaa acactcagtg tgttggctct ctgtgccact 120
   cctggaaaat aatgaattgg gtaaggaaca gttaataaga aaatgtgcct tgctaactgt 180
   gcacattaca acaaagagct ggcagctcct gaaggaaaag ggcttgtgcc gctgccgttc 240
   aaacttgtca gtcaactcat gccagcagcc tcagcgtctg cctccccagc acaccctcat 300
   tacatgtgtc tgtctggcct gatctgtgca tctgctcgga gacgctcctg acaagtcggg 360
   aatttctcta tttctccact ggtgcaaaga gcggatttct ccctgcttct cttctgtcac 420
   ccccgctcct ctcccccagg aggctccttg atttatggta gctttggact tgcttccccg 480
   tctgactgtc cttgacttct agaatggaag aagctgagct ggtgaaggga agactccagg 540
   ccatcacaga taaaagaaaa atacaggaag aaatctcaca gaagcgtctg aaaatagagg 600
   aagacaaact aaagcaccag catttgaaga aaaaggcctt gagggagaaa tggcttctag 660
   atggaatcag cagcggaaaa gaacaggaag agatgaagaa gcaaaatcaa caagaccagc 720
   accagatcca ggttctagaa caaagtatcc tcaggcttga gaaagagatc caagatcttg 780
   aaaaagctga actgcaaatc tcaacgaagg aagaggccat tttaaagaaa ctaaagtcaa 840
   ttgagcggac aacagaagac attataagat ctgtgaaagt ggaaagagaa gaaagagcag 900
   aagagtcaat tgaggacatc tatgctaata tccctgacct tccaaagtcc tacatacctt 960
   ctaggttaag gaaggagata aatgaagaaa aagaagatga tgaacaaaat aggaaagctt 1020
   tatatgccat ggaaattaaa gttgaaaaag acttgaagac tggagaaagt acagttctgt 1080
   cttccaatac ctctggccat cagatgactt taaaaggtac aggagtaaaa gtttaagatg 1140
   atgggcaaaa gtccagtgta ttcagtaaag tgctaatcac aagttggagg t
<210> 52
   <211> 1200
   <212> DNA
   <213> Human
<400> 52
   aacagggact ctcactctat caaccccagg ctggagtccg gtgcgcccac cctggctccc 60
   tgcaacctcc gcctcccagg ctcaagcaac tctcctgcct cagtcgctct agtagctggg 120
   actacaggca cacaccacca tgcccagcca atttttgcat tttttgtaga gacagggttt 180
   cgccttctgt ccaggccggc atcatatact ttaaatcatg cccagatgac tttaatacct 240
   aatacaatat atcaggttgg tttaaaaata attgcttttt tattattttt gcatttttgc 300
   accaacctta atgctatgta aatagttgtt atactgttgc ttaacaacag tatgacaatt 360
   ttggcttttt ctttgtatta ttttgtattt ttttttttta ttgtgtggtc tttttttttt 420
   ttctcagtgt tttcaattcc tccttggttg aatccatgga tgcaaaaccc acagatatga 480
   agggctggct atatatgcat tgatgattgt cctattatat tagttataaa gtgtcattta 540
   atatgtagtg aaagttatgg tacagtggaa agagtagttg aaaacataaa catttggacc 600
   tttcaagaaa ggtagcttgg tgaagttttt caccttcaaa ctatgtccca gtcagggctc 660
   tgctactaat tagctataat ctttgcacaa attacatcac ctttgagtct cagttgcctc 720
   acctgtaaaa tgaaagaact ggatactctc taaggtcact tccagccctg tcattctata 780
   actctgttat gctgaggaag aaattcacat tgtgttaact gtatgagtca aactgaaaat 840
   gattattaaa gtgggaaaaa gccaattgct tctcttagaa agctcaacta aatttgagaa 900
   gaataatctt ttcaattttt taagaattta aatattttta agggtttgac ctatttattt 960
   agagatgggg tctcactctg tcacccagac tggagtacag tggcacaatc atagctcact 1020
   gctgcctcaa attcatgggc tcaagtgatc ctcctgcctc tgcctccaga gtagctgcga 1080
   ctatgggcat gtgccaccac gcctggctaa catttgtatt gacctattta tttattgtga 1140
   tttatatctt tttttttttt tctttttttt ttttttacaa aatcagaaat acttattttg 1200
<210> 53
   <211> 989
   <212> DNA
   <213> Human
<400> 53
   aagccaccac tcaaaacttc ctatacattt tcacagcaga gacaagtgaa catttatttt 60
   tatgcctttc ttcctatgtg tatttcaagt ctttttcaaa acaaggcccc aggactctcc 120
   gattcaatta gtccttgggc tggtcgactg tgcaggagtc cagggagcct ctacaaatgc 180
   agagtgactc tttaccaaca taaaccctag atacatgcaa aaagcaggac ccttcctcca 240
   ggaatgtgcc atttcagatg cacagcaccc atgcagaaaa gctggaattt tccttggaac 300
   cgactgtgat agaggtgctt acatgaacat tgctactgtc tttctttttt tttgagacag 360
   gtttcgcttg tgcccaggct gagtgcaatg cgtgatctca ctcactgcaa ttccacctcc 420
   aggttcaagc attctcctgc tcagcctcct agtagctggg ttacaggcac tgccaccatg 480
   ccggctaatt ttgtattttt gtagagatgg atttctccat ttggtcaggc ggtctcgaac 540
   cccaacctca gtgatctgcc acctcagcct cctaagtgtt ggattacagg atgagccacc 600
   cgaccggcca ctactgtctt tctttgaccc ttccagtttc gaagataaag aggaaataat 660
   ttctctgaag tacttgataa aatttccaaa caaaacacat gtccacttca ctgataaaaa 720
   atttaccgca gtttggcacc taagagtatg acaacagcaa taaaaagtaa tttcaaagag 780
   ttaagatttc ttcagcaaaa tagatgattc acatcttcaa gtcctttttg aaatcagtta 840
   ttaatattat tctttcctca tttccatctg aatgactgca gcaatagttt tttttttttt 900
   tttttttttt ttgcgagatg gaatctcgct ctgtcgccca gcgggagtgc actggcgcaa 960
   gcccggctca ccgcaatctc tgccacccg
<210> 54
   <211> 250
   <212> DNA
   <213> Human
<400> 54
   catttcccca ttggtcctga tgttgaagat ttagttaaag aggctgtaag tcaggttcga 60
   gcagaggcta ctacaagaag tagggaatca agtccctcac atgggctatt aaaactaggt 120
   agtggtggag tagtgaaaaa gaaatctgag caacttcata acgtaactgc ctttcaggga 180
   aaagggcatt ctttaggaac tgcatctggt aacccacacc ttgatccaag agctagggaa 240
   acttcagttg
<210> 55
   <211> 2270
   <212> DNA
   <213> Human
<400> 55
   gcgcccccga gcagcgcccg cgccctccgc gccttctccg ccgggacctc gagcgaaaga 60
   ggcccgcgcg ccgcccagcc ctcgcctccc tgcccaccgg gcacaccgcg ccgccacccc 120
   gaccccgctg cgcacggcct gtccgctgca caccagcttg ttggcgtctt cgtcgccgcg 180
   ctcgccccgg gctactcctg cgcgccacaa tgagctcccg catcgccagg gcgctcgcct 240
   tagtcgtcac ccttctccac ttgaccaggc tggcgctctc cacctgcccc gctgcctgcc 300
   actgccccct ggaggcgccc aagtgcgcgc cgggagtcgg gctggtccgg gacggctgcg 360
   gctgctgtaa ggtctgcgcc aagcagctca acgaggactg cagcaaaacg cagccctgcg 420
   accacaccaa ggggctggaa tgcaacttcg gcgccaagtc caccgctctg aaggggatct 480
   gcagagctca gtcagagggc agaccctgtg aatataactc cagaatctac caaaacgggg 540
   aaagtttcca gcccaactgt aaacatcagt gcacatgtat tgatggcgcc gtgggctgca 600
   ttcctctgtg tccccaagaa ctatctctcc ccaacttggg ctgtcccaac cctcggctgg 660
   tcaaagttac cgggcagtgc tgcgaggagt gggtctgtga cgaggatagt atcaaggacc 720
   ccatggagga ccaggacggc ctccttggca aggagctggg attcgatgcc tccgaggtgg 780
   agttgacgag aaacaatgaa ttgattgcag ttggaaaagg cagctcactg aagcggctcc 840
   ctgtttttgg aatggagcct cgcatcctat acaacccttt acaaggccag aaatgtattg 900
   ttcaaacaac ttcatggtcc cagtgctcaa agacctgtgg aactggtatc tccacacgag 960
   ttaccaatga caaccctgag tgccgccttg tgaaagaaac ccggatttgt gaggtgcggc 1020
   cttgtggaca gccagtgtac agcagcctga aaaagggcaa gaaatgcagc aagaccaaga 1080
   aatcccccga accagtcagg tttacttacg ctggatgttt gagtgtgaag aaataccggc 1140
   ccaagtactg cggttcctgc gtggacggcc gatgctgcac gccccagctg accaggactg 1200
   tgaagatgcg gttccgctgc gaagatgggg agacattttc caagaacgtc atgatgatcc 1260
   agtcctgcaa atgcaactac aactgcccgc atgccaatga agcagcgttt cccttctaca 1320
   ggctgttcaa tgacattcac aaatttaggg actaaatgct acctgggttt ccagggcaca 1380
   cctagacaaa caagggagaa gagtgtcaga atcagaatca tggagaaaat gggcgggggt 1440
   ggtgtgggtg atgggactca ttgtagaaag gaagccttgc tcattcttga ggagcattaa 1500
   ggtatttcga aactgccaag ggtgctggtg cggatggaca ctaatgcagc cacgattgga 1560
   gaatactttg cttcatagta ttggagcaca tgttactgct tcattttgga gcttgtggag 1620
   ttgatgactt tctgttttct gtttgtaaat tatttgctaa gcatattttc tctaggcttt 1680
   tttccttttg gggttctaca gtcgtaaaag agataataag attagttgga cagtttaaag 1740
   cttttattcg tcctttgaca aaagtaaatg ggagggcatt ccatcccttc ctgaaggggg 1800
   acactccatg agtgtctgtg agaggcagct atctgcactc taaactgcaa acagaaatca 1860
   ggtgttttaa gactgaatgt tttatttatc aaaatgtagc ttttggggag ggaggggaaa 1920
   tgtaatactg gaataatttg taaatgattt taattttata ttcagtgaaa agattttatt 1980
   tatggaatta accatttaat aaagaaatat ttacctaata tctgagtgta tgccattcgg 2040
   tatttttaga ggtgctccaa agtcattagg aacaacctag ctcacgtact caattattca 2100
   aacaggactt attgggatac agcagtgaat taagctatta aaataagata atgattgctt 2160
   ttataccttc agtagagaaa agtctttgca tataaagtaa tgtttaaaaa acatgtattg 2220
   aacacgacat tgtatgaagc acaataaaga ttctgaagct aaaaaaaaaa
<210> 56
   <211> 1636
   <212> DNA
   <213> Human
<400> 56
   cttgaatgaa gctgacacca agaaccgcgg gaagagcttg ggcccaaagc aggaaaggga 60
   agcgctcgag ttggaaagga accgctgctg ctggccgaac tcaagcccgg gcgcccccac 120
   cagtttgatt ggaagtccag ctgtgaaacc tggagcgtcg ccttctcccc agatggctcc 180
   tggtttgctt ggtctcaagg acactgcatc gtcaaactga tcccctggcc gttggaggag 240
   cagttcatcc ctaaagggtt tgaagccaaa agccgaagta gcaaaaatga gacgaaaggg 300
   cggggcagcc caaaagagaa gacgctggac tgtggtcaga ttgtctgggg gctggccttc 360
   agcccgtggc cttccccacc cagcaggaag ctctgggcac gccaccaccc ccaagtgccc 420
   gatgtctctt gcctggttct tgctacggga ctcaacgatg ggcagatcaa gatctgggag 480
   gtgcagacag ggctcctgct tttgaatctt tccggccacc aagatgtcgt gagagatctg 540
   agcttcacac ccagtggcag tttgattttg gtctccgcgt cacgggataa gactcttcgc 600
   atctgggacc tgaataaaca cggtaaacag attcaagtgt tatcgggcca cctgcagtgg 660
   gtttactgct gttccatctc cccagactgc agcatgctgt gctctgcagc tggagagaag 720
   tcggtctttc tatggagcat gaggtcctac acgttaattc ggaagctaga gggccatcaa 780
   agcagtgttg tctcttgtga cttctccccc gactctgccc tgcttgtcac ggcttcttac 840
   gataccaatg tgattatgtg ggacccctac accggcgaaa ggctgaggtc actccaccac 900
   acccaggttg accccgccat ggatgacagt gacgtccaca ttagctcact gagatctgtg 960
   tgcttctctc cagaaggctt gtaccttgcc acggtggcag atgacagact cctcaggatc 1020
   tgggccctgg aactgaaaac tcccattgca tttgctccta tgaccaatgg gctttgctgc 1080
   acattttttc cacatggtgg agtcattgcc acagggacaa gagatggcca cgtccagttc 1140
   tggacagctc ctagggtcct gtcctcactg aagcacttat gccggaaagc ccttcgaagt 1200
   ttcctaacaa cttaccaagt cctagcactg ccaatcccca agaaaatgaa agagttcctc 1260
   acatacagga ctttttaagc aacaccacat cttgtgcttc tttgtagcag ggtaaatcgt 1320
   cctgtcaaag ggagttgctg gaataatggg ccaaacatct ggtcttgcat tgaaatagca 1380
   tttctttggg attgtgaata gaatgtagca aaaccagatt ccagtgtaca taaaagaatt 1440
   tttttgtctt taaatagata caaatgtcta tcaactttaa tcaagttgta acttatattg 1500
   aagacaattt gatacataat aaaaaattat gacaatgtcc tgggaaaaaa aaaatgtaga 1560
   aagatggtga agggtgggat ggatgaggag cgtggtgacg ggggcctgca gcgggttggg 1620
   gaccctgtgc tgcgtt
<210> 57
   <211> 460
   <212> DNA
   <213> Human
<400> 57
   ccatgtgtgt atgagagaga gagagattgg gagggagagg gagctcacta gcgcatatgt 60
   gcctccaggg ggctgcagat gtgtctgagg gtgagcctgg tgaaagagaa gacaaaagaa 120
   tggaatgagc taaagcagcc gcctggggtg ggaggccgag cccatttgta tgcagcaggg 180
   ggcaggagcc cagcaaggga gcctccattc ccaggactct ggagggagct gagaccatcc 240
   atgcccgcag agccctccct cacactccat cctgtccagc cctaattgtg caggtgggga 300
   aactgaggct gggaagtcac atagcaagtg actggcagag ctgggactgg aacccaacca 360
   gcctcctaga ccacggttct tcccatcaat ggaatgctag agactccagc caggtgggta 420
   ccgagctcga attcgtaatc atggtcatag ctgtttcctg
<210> 58
   <211> 1049
   <212> DNA
   <213> Human
<400> 58
   atctgatcaa gaatacctgc cctggtcact ctgcggatgt ttctgtccac ttgttcacat 60
   tgaggaccaa gatatccttt tttacagagg cacttgttcg gtctaacaca gacacctcca 120
   tgacgacatg ctggctcaca ttttgcagtt ctgcagaagt ccccctccca gcctggacta 180
   cagcagcact ttcccgtggg ggtgcagtag ccgtttcgac agagcctgga gcactctgaa 240
   gtcagtgtct gtgcaggttg taccgtggct ctgcattcct caggcattaa aggtcttttg 300
   ggatctacaa ttttgtagag ttttccattg tgagtctggg tcatactttt actgcttgat 360
   aaaatgtaaa cttcacctag ttcatcttct ccaaatccca agatgtgacc ggaaaagtag 420
   cctctacagg acccactagt gccgacacag agtggttttt cttgccactg ctttgtcaca 480
   ggactttgct ggagagttag gaaattccca ttacgatctc caaacacgta gcttccatac 540
   aatctttctg actggcagcc ccggtataca aatccaccaa ccaaaggacc attactgaat 600
   ggcttgaatt ctaaaagtga tggctcactt tcataatctt tcccctttat tatctgtaga 660
   attctggctg atgatctgtt ttttccattg gagtctgaac acagtatcgt taaattgatg 720
   tttatatcag tgggatgtct atccacagca catctgcctg gatcgtggag cccatgagca 780
   aacacttcgg ggggctggtt ggtgctgttg aagtgtgggt tgctccttgg tatggaataa 840
   ggcacgttgc acatgtctgt gtccacatcc agccgtagca ctgagcctgt gaaatcactt 900
   aacccatcca tttcttccat atcatccagt gtaatcatcc catcaccaag aatgatgtac 960
   aaaaacccgt cagggccaaa gagcagttgc cctcccagat gctttctgtg gagttctgca 1020
   acttcaagaa agactctggc tgttctcaa
<210> 59
   <211> 747
   <212> DNA
   <213> Human
<400> 59
   tttttcaaat cacatatggc ttctttgacc ccatcaaata actttattca cacaaacgtc 60
   ccttaattta caaagcctca gtcattcata cacattaggg gatccacagt gttcaaggaa 120
   cttaaatata atgtatcata ccaacccaag taaaccaagt acaaaaaata ttcatataaa 180
   gttgttcaca cgtaggtcct agattaccag cttctgtgca aaaaaaggaa atgaagaaaa 240
   atagatttat taactagtat tggaaactaa ctttgtgcct ggcttaaaac ctccctcacg 300
   ctcgtctgtc ccacacaaat gtttaagaag tcactgcaat gtactccccg gctctgatga 360
   aaagaagccc ctggcacaaa agattccagt gcccctgaag aggctccctt cctcctgtgg 420
   gctctcctag aaaaccagcg ggacggcctc cctgctgata ccgtctataa ccttaggggg 480
   ccctcgggca ggcaacggca gtggactcat ctcggtgatg gctgtagatg ctaacactgg 540
   ccaattcaat gccacaccta ctggttaccc tttgagggca tttctccaga cagaagcccc 600
   ttgaagccta ggtagggcag gatcagagat acacccgtgt ttgtctcgaa gggctccaca 660
   gcccagtacg acatgcttgc agaagtagta tctctggact tctgcctcca gtcgaccggc 720
   cgcgaattta gtagtaatag cggccgc

## Claims

1. Pharmaceutical compositions comprising a combination of Compound I and Compound II, wherein Compound I and Compound II comprise at least one of (i) a compound of general formula I 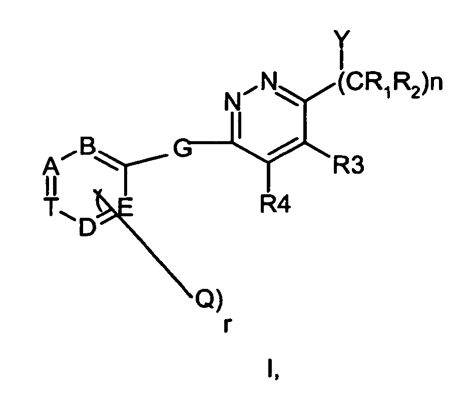 in which
r has the meaning of 0 to 2,
n has the meaning of 0 to 2;
R₃ und R₄
a) each independently from eaxh other have the meaning of lower alkyl,
b) together form a bridge of general partial formula II, 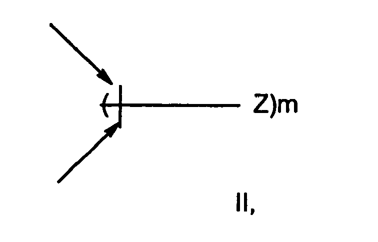 wherein the binding is via the two terminal C- atoms, and
m has the meaning of 0 to 4; or
c) together form a bridge of partial formula III 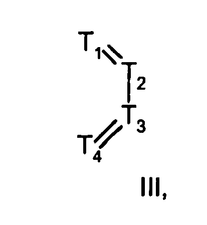 wherein one or two of the ring members T₁,T₂,T₃,T₄ has the meaning of nitrogen, and each others have the meaning of CH, and the binding is via the atoms T₁ and T₄;
G has the meaning of C₁ -C₆ - alkyl, C₂ - C₆ - alkylene or C₂ - C₆ - alkenylene; or C₂ - C₆ - alkylene or C₃ **-**C₆-alkenylene, which are substituted with acyloxy or hydroxy; -CH₂-O-, -CH₂ -S-, -CH₂NH-, -CH₂-O-CH₂-, -CH₂-S-CH₂, -CH₂-NH-CH₂, oxa (-O-), thia (-S-) or imino (-NH-),
A, B, D, E and T independently from each other have the meaning of N or CH , with the provisio that not more than three of these Substituents have the meaning of N,
Q has the meaning of lower alkyl, lower alkyloxy or halogene,
R₁ and R₂ independently from each other have the meaning of H or lower alkyl,
X has the meaning of imino, oxa or thia;
Y has the meaning of hydrogene, unsubstituted or substituted aryl, heteroaryl, or unsubstituted or substituted cycloalkyl; and
Z has the meaning of amino, mono- or disubstituted amino, halogen, alkyl, substituted alkyl, hydroxy, etherificated or esterificated hydroxy, nitro, cyano, carboxy, esterificated carboxy, alkanoyl, carbamoyl, N-mono- or N, N- disubstituted carbamoyl, amidino, guanidino, mercapto, sulfo, phenylthio, phenyl-lower-alkyl-thio, alkyl-phenyl-thio, phenylsulfinyl, phenyl-lower-alkyl-sulfinyl, alkylphenylsulfinyl, phenylsulfonyl, phenyl-lower-alkan-sulfonyl, or alkylphenylsulfonyl, whereas, if more than one rest Z is present (m≥2), the substituents Z are equal or different from each other, and wherein the bonds marked with an arrow are single or double bonds; or an N-oxide of said compound, wherein one ore more N-atoms carry an oxygene atom, or a salt thereof;
and/or (ii) a compound of general formula IV 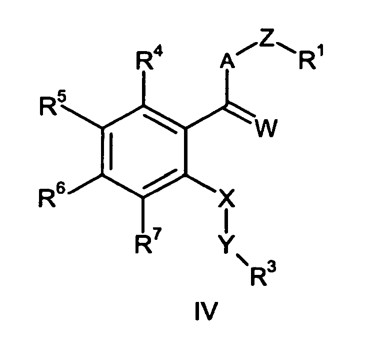 in which
A has the meaning of group =NR²,
W has the meaning of oxygen, sulfur, two hydrogen atoms or the group =NR⁸,
Z has the meaning of the group =NR¹⁰ or =N-, -N(R¹⁰)-(CH₂)_{q}-, branched or unbranched C₁₋₆-Alkyl or is the group 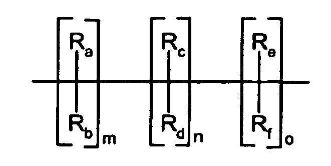 or A, Z and R¹ together form the group 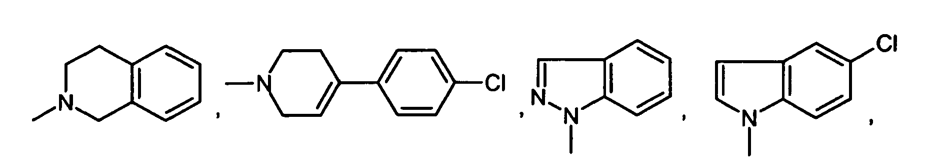 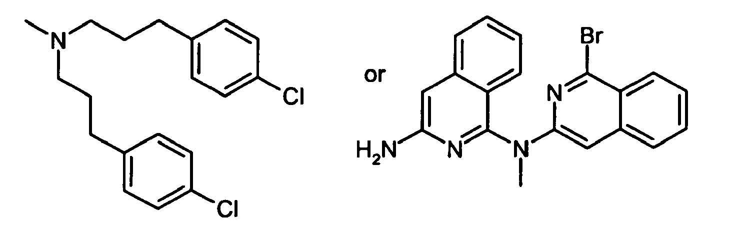
m, n and o has the meaning of 0-3,
q has the meaning of 1-6,
Rₐ, R_{b}, R_{c}, R_{d}, Rₑ, R_{f} independently from each other have the meaning of hydrogen, C₁₋₄ alkyl or the group =NR¹⁰, and/ or Rₐ and/ or R_{b} together with R_{c} and or R_{d} or R_{c} together with Rₑ and/ or R_{f} form a bound, or up to two of the groups Rₐ-R_{f} form a bridge with each up to 3 C-atoms with R¹ or R²,
X has the meaning of group =NR⁹ or =N-,
Y has the meaning of group -(CH₂)ₚ,
p has the meaning of integer 1-4,
R¹ has the meaning of unsubstituted or optionally substituted with one or more of halogene, C₁₋₆-alkyl, or C₁₋₆-alkyl or C₁₋₆-alkoxy, which is optionally substituted by one or more of halogen, or is unsubstituted or substituted aryl or heteroaryl,
R² has the meaning of hydrogen or C₁₋₆-alkyl, or form a bridge with up to 3 ring atoms with Rₐ-R_{f} together with Z or R₁,
R³ has the meaning of monocyclic or bicyclic aryl or heteroaryl which is unsubstituted or optionally substituted with one or more of für halogen, C₁₋₆-alkyl, C₁₋₆-alkoxy or hydroxy,
R⁴ ,R⁵, R⁶ and R⁷ independently from each other have the meaning of hydrogen, halogene or C₁₋₆-alkoxy, C₁₋₆-alkyl or C₁₋₆-carboxyalkyl, which are unsubstituted or optionally substituted with one or more of halogene, or R⁵ and R⁶ together form the group 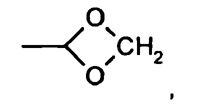
R⁸, R⁹ and R¹⁰ independently from each other have the meaning of hydrogen or C₁₋₆-alkyl,
as well as their isomers and salts;
and/or (iii) a compound of general formula V 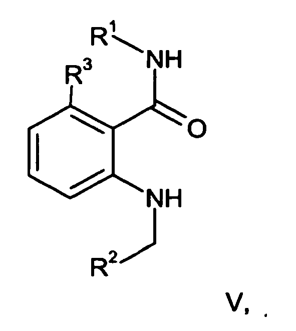 in which
R¹ has the meaning of group 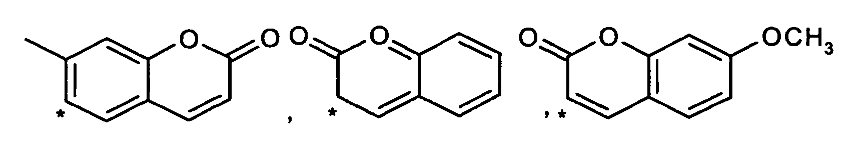 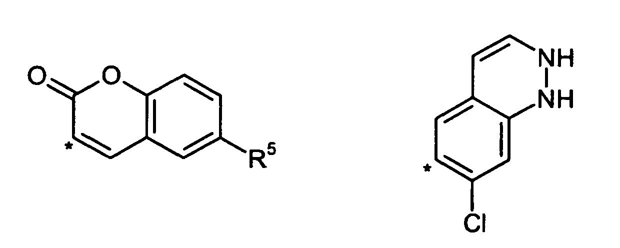 in which R⁵ is chloro, bromo or the group -OCH₃, 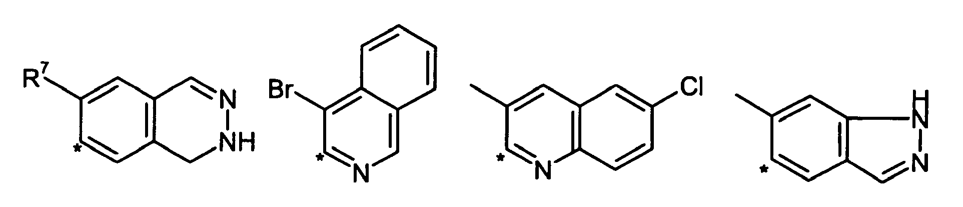 in which R⁷ is -CH₃ or chloro, 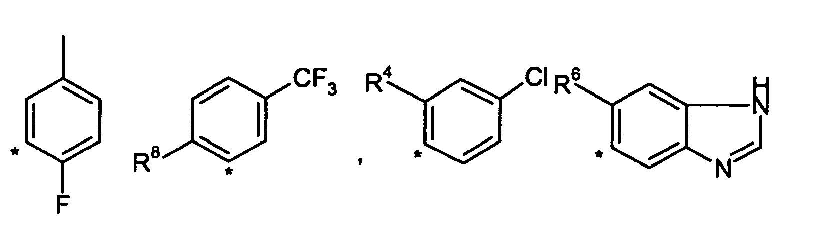
| | | |
|---|---|---|
| in which R⁸ is -CH₃, fluoro, chloro or -CF₃ | in which R⁴ is fluoro, chloro, bromo, -CF₃, -N=C, -CH₃,-OCF₃ or -CH₂OH | in which R⁶ is -CH₃ or chloro |
R² has the meaning of pyridyl or the group 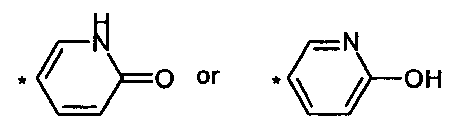 and
R³ has the meaning of hydrogen or fluoro, as well as their isomers and salts; and/or (iv) Seq. ID Nos. 1-59, sTie2, mAB 4301-42-35, scFv-tTF and/or L19 scFv-tTF conjugate, with the provisio that Compound I is not identical to Compound II.

2. Pharmaceutical compositions according to claim 1 which comprise as Compound I and/or II at least one of Seq. ID Nos. 1-59.

3. Pharmaceutical compositions according to claims 1-2 which comprise as Compound I and/ or II Seq. ID Nos. 34a.

4. Pharmaceutical compositions according to claims 1-3 which comprise as Compound I and/ or II at least one of sTie2, mAB 4301-42-35, scFv-tTF and/ or L19 scFv-tTFconjugate.

5. Pharmaceutical compositions according to claims 1-4 which comprise as Compound I and/ or II at least one compound of general formula I 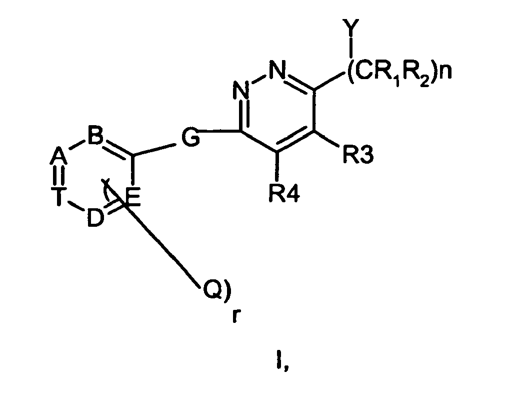 in which
r has the meaning of 0 to 2,
n has the meaning of 0 to 2;
R₃ und R₄
a) each independently from each other have the meaning of lower alkyl,
b) together form a bridge of general partial formula II, 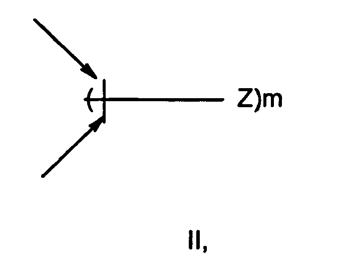 wherein the binding is via the two terminal C- atoms, and
m has the meaning of 0 to 4; or
c) together form a bridge of partial formula III 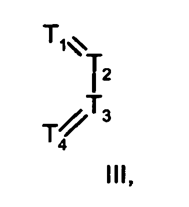
has wherein one or two of the ring members T₁,T₂,T₃,T₄ the meaning of nitrogen, and each others have the meaning of CH, and the binding is via the atoms T₁ and T₄;
G has the meaning of C₁ -C₆- alkyl, C₂- C₆- alkylene or C₂- C₆- alkenylene; or C₂- C₆- alkylene or C₃ -C₆-alkenylene, which are substituted with acyloxy or hydroxy; -CH₂-O-, -CH₂ -S-, -CH₂-NH-, -CH₂-O-CH₂-, -CH₂-S-CH₂-, -CH₂-NH-CH₂, oxa (-O-), thia (-S-) or imino (-NH-),
A, B, D, E and T independently from each other have the meaning of N or CH , with the provisio that not more than three of these Substituents have the meaning of N,
Q has the meaning of lower alkyl, lower alkyloxy or halogene,
R₁ and R₂ independently from each other have the meaning of H or lower alkyl,
X has the meaning of imino, oxa or thia;
Y has the meaning of hydrogene, unsubstituted or substituted aryl, heteroaryl, or unsubstituted or substituted cycloalkyl; and
Z has the meaning of amino, mono- or disubstituted amino, halogen, alkyl, substituted alkyl, hydroxy, etherificated or esterificated hydroxy, nitro, cyano, carboxy, esterificated carboxy, alkanoyl, carbamoyl, N-mono- or N, N- disubstituted carbamoyl, amidino, guanidino, mercapto, sulfo, phenylthio, phenyl-lower-alkyl-thio, alkyl-phenyl-thio, phenylsulfinyl, phenyl-lower-alkyl-sulfinyl, alkylphenylsulfinyl, phenylsulfonyl, phenyl-lower-alkan-sulfonyl, or alkylphenylsulfonyl, whereas, if more than one rest Z is present (m≥2), the substituents Z are equal or different from each other, and wherein the bonds marked with an arrow are single or double bonds; or an N-oxide of said compound, wherein one ore more N-atoms carry an oxygene atom, or a salt thereof,
and/or a compound of general formula IV 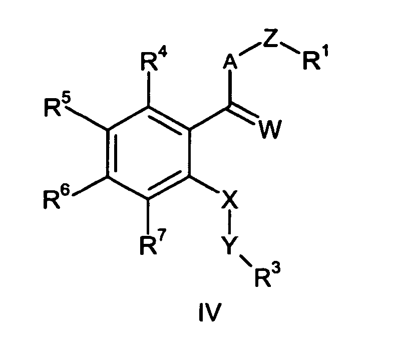 in which
A has the meaning of group =NR² ,
W has the meaning of oxygen, sulfur, two hydrogen atoms or the group =NR⁸,
Z has the meaning of the group =NR¹⁰ or =N-, -N(R¹⁰)-(CH₂)_{q}-, branched or unbranched C₁-₆-Alkyl or is the group 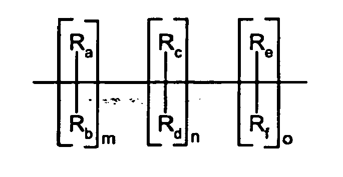 or A, Z and R¹ together form the group 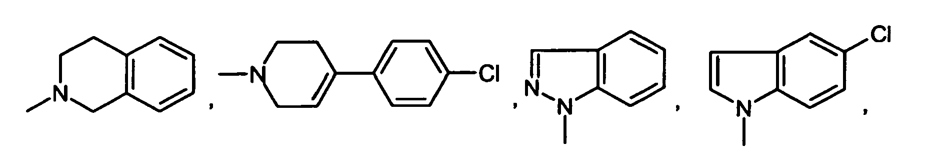 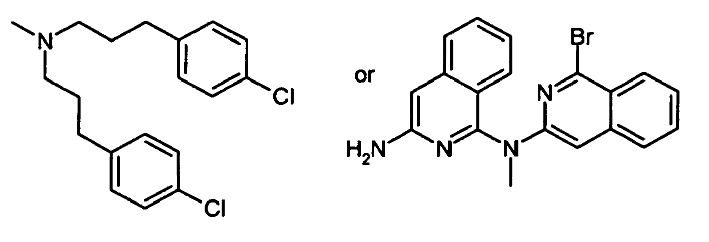
m, n and o has the meaning of 0 - 3,
q has the meaning of 1 - 6,
Rₐ, R_{b}, R_{c}, R_{d}, Rₑ, R_{f} independently from each other have the meaning of hydrogen, C₁₋₄ alkyl or the group =NR¹⁰, and/ or Rₐ and/ or R_{b} together with R_{c} and or R_{d} or R_{c} together with Rₑ and/ or R_{f} form a bound, or up to two of the groups Rₐ-R_{f} form a bridge with each up to 3 C-atoms with R¹ or R²,
X has the meaning of group =NR⁹ or =N-,
Y has the meaning of group -(CH₂)ₚ,
p has the meaning of integer 1-4,
R¹ has the meaning of unsubstituted or optionally substituted with one or more of halogene, C₁-₆-alkyl, or C₁₋₆-alkyl or C₁₋₆-alkoxy, which is optionally substituted by one or more of halogen, or is unsubstituted or substituted aryl or heteroaryl,
R² has the meaning of hydrogen or C₁-₆-alkyl, or form a bridge with up to 3 ring atoms with Rₐ-R_{f} together with Z or R₁,
R³ has the meaning of monocyclic or bicyclic aryl or heteroaryl which is unsubstituted or optionally substituted with one or more of für halogen, C₁₋₆-alkyl, C₁₋₆-alkoxy or hydroxy,
R⁴ ,R⁵, R⁶ and R⁷ independently from each other have the meaning of hydrogen, halogene or C₁₋₆-alkoxy, C₁₋₆-alkyl or C₁₋₆-carboxyalkyl, which are unsubstituted or optionally substituted with one or more of halogene, or R⁵ and R⁶ together form the group 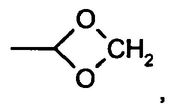
R⁸, R⁹ and R¹⁰ independently from each other have the meaning of hydrogen or C₁₋₆-alkyl,
as well as their isomers and salts,
and/ or a compound of general formula V 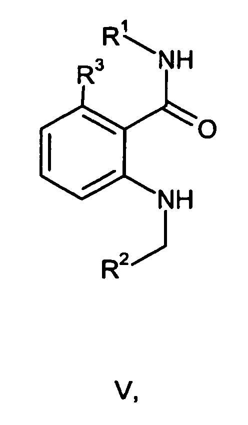 in which
R¹ has the meaning of group 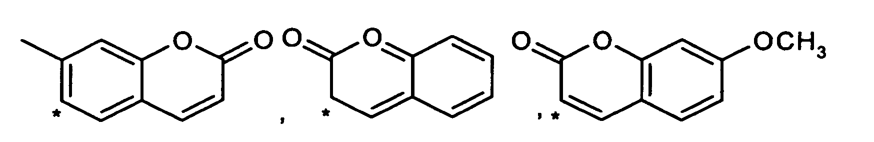 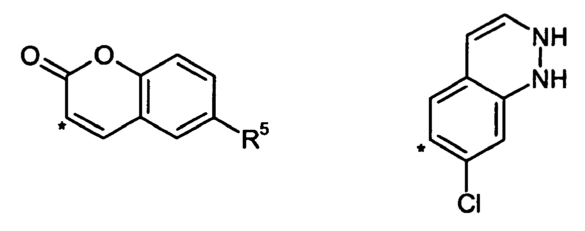 in which R⁵ is chloro, bromo or the group -OCH₃, 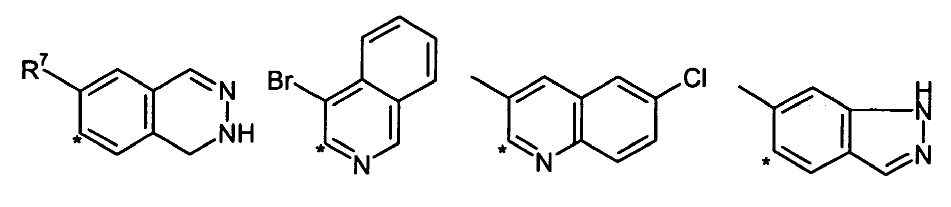 in which R⁷ is -CH₃ or chloro, 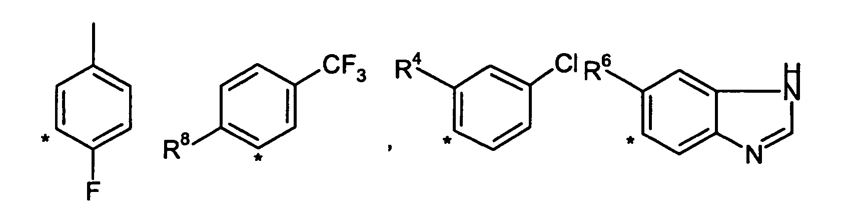
| | | |
|---|---|---|
| in which R⁸ is -CH₃, fluoro, chloro or -CF₃ | in which R⁴ is fluoro, chloro, bromo, -CF₃, -N=C, -CH₃,-OCF₃ or -CH₂OH | in which R⁶ is -CH₃ or chloro |
R² has the meaning of pyridyl or the group 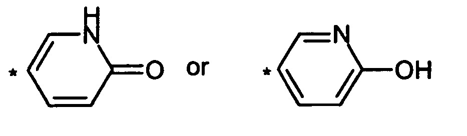 and
R³ has the meaning of hydrogen or fluoro, as well as their isomers and salts.

6. Pharmaceutical compositions according to claim 1-5 which comprise as Compound I and/ or II (4-Chlorophenyl)[4-(4-pyridylmethyl)-phthalazin-1-yl]ammonium hydrogen succinate.

7. Pharmaceutical compositions according to claims 1-6 which comprise as Compound I (4-Chlorophenyl)[4-(4-pyridylmethyl)-phthalazin-1-yl]ammonium hydrogen succinate, Seq. ID Nos. 1-59, sTie2, mAB 4301-42-35, scFv-tTF and/ or L19 scFv-tTF conjugate, and as Compound II (4-Chlorophenyl)[4-(4-pyridylmethyl)-phthalazin-1-yl]ammonium hydrogen succinate, Seq. ID Nos. 1-59, sTie2, mAB 4301-42-35, scFv-tTF and/ or L19 scFv-tTF conjugate, with the provisio that Compound I is not identically to Compound II.

8. Pharmaceutical compositions according to claims 1-7 which comprise as Compound I (4-Chlorophenyl)[4-(4-pyridylmethyl)-phthalazin-1-yl]ammonium hydrogen succinate and as Compound II Seq. ID Nos. 1-59, sTie2, mAB 4301-42-35, scFv-tTF and/ or L19 scFv-tTF conjugate.

9. Pharmaceutical compositions according to claims 1-7 which comprise as Compound I mAB 4301-42-35 and as Compound II sTie2, and/ or scFv-tTF conjugate.

10. Pharmaceutical compositions according to claims 1-7 which comprise as Compound I scFv-tTF conjugate and as Compound II sTie2 and/ or mAB 4301-42-35.

11. Pharmaceutical compositions according to claims 1-7 which comprise as Compound I L19 scFv-tTF conjugate and as Compound II sTie2.

12. Use of pharmaceutical compositions according to claims 1-11, for the production of a medicament for the treatment of tumors, cancers, psoriasis, arthritis, such as rheumatoide arthritis, hemangioma, angiofribroma, eye diseases, such as diabetic retinopathy, neovascular glaukoma, kidney diseases, such as glomerulonephritis, diabetic nephropathie, maligneous nephrosclerosis, thrombic microangiopatic syndrome, transplantation rejections and glomerulopathy, fibrotic diseases, such as cirrhotic liver, mesangial cell proliferative diseases, artheriosclerosis, damage of nerve tissues, suppression of the ascites formation in patients and suppression of VEGF oedemas.

## Patentansprüche

1. Pharmazeutische Zusammensetzungen, enthaltend eine Kombination von Verbindung I und Verbindung II, wobei Verbindung I und Verbindung II wenigstens eine der folgenden umfassen
(i) eine Verbindung der allgemeinen Formel I 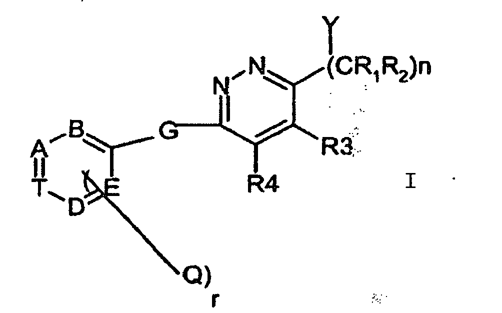 in welcher
r für 0 bis 2 steht,
n für 0 bis 2 steht;
R₃ und R₄
a) jeweils unabhängig voneinander für Niederalkyl stehen,
b) zusammen eine Brücke der Formel II bilden, 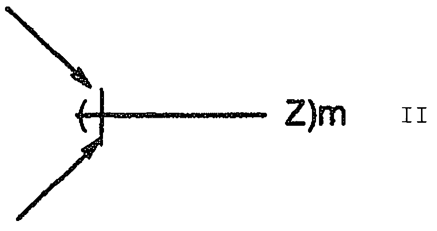 wobei die Bindung über die beiden terminalen C-Atome erfolgt und
m für 0 bis 4 steht; oder
c) zusammen eine Brücke der Teilformel III bilden 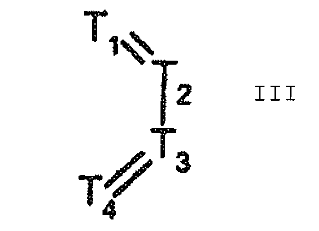 wobei eines oder zwei der Ringglieder T₁, T₂, T₃, T₄ für Stickstoff steht/stehen und die anderen für CH stehen und die Bindung über die Atome T₁ und T₄ erfolgt;
G für C₁-C₆-Alkyl, C₂-C₆-Alkylen oder C₂-C₆-Alkenylen; oder durch Acyloxy oder Hydroxy substituiertes C₂-C₆-Alkylen oder C₃-C₆-Alkenylen; -CH₂-O-, -CH₂-S-, -CH₂-NH-, -CH₂-O-CH₂-, -CH₂-S-CH₂-, -CH₂-NH-CH₂, Oxa (-O-), Thia (-S-) oder Imino (-NH-) steht,
A, B, D, E und T unabhängig voneinander für N oder CH stehen, mit der Maßgabe, daß nicht mehr als drei dieser Substituenten für N stehen,
Q für Niederalkyl, Niederalkyloxy oder Halogen steht,
R₁ und R₂ unabhängig voneinander für H oder Niederalkyl stehen,
X für Imino, Oxa oder Thia steht;
Y für Wasserstoff, unsubstituiertes oder substituiertes Aryl, Heteroaryl oder unsubstituiertes oder substituiertes Cycloalkyl steht; und
Z für Amino, mono- oder disubstituiertes Amino, Halogen, Alkyl, substituiertes Alkyl, Hydroxy, verethertes oder verestertes Hydroxy, Nitro, Cyano, Carboxy, verestertes Carboxy, Alkanoyl, Carbamoyl, N-mono- oder N,N-disubstituiertes Carbamoyl, Amidino, Guanidino, Mercapto, Sulfo, Phenylthio, Phenyl-niederalkylthio, Alkyl-phenyl-thio, Phenylsulfinyl, Phenyl-niederalkyl-sulfinyl, Alkylphenylsulfinyl, Phenylsulfonyl, Phenyl-niederalkan-sulfonyl oder Alkylphenylsulfonyl steht,
wobei, wenn mehr als ein Rest Z vorhanden ist (m ≥ 2) , die Substituenten Z gleich oder verschieden voneinander sind, und wobei die mit einem Pfeil **gekennzeichnet**en Bindungen Einfachbindungen oder Doppelbindungen sind; oder ein N-Oxid der Verbindung, wobei ein oder mehrere N-Atome ein Sauerstoffatom tragen, oder ein Salz davon;
und/oder (ii) eine Verbindung der allgemeinen Formel IV 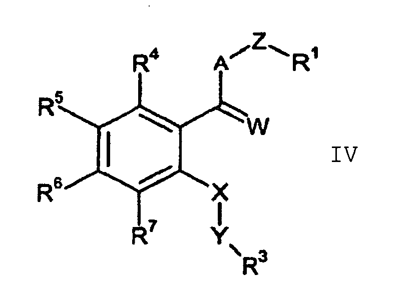 in welcher
A für eine Gruppe =NR² steht,
W für Sauerstoff, Schwefel, zwei Wasserstoffatome oder die Gruppe =NR⁸ steht,
Z für die Gruppe =NR¹⁰ oder =N-, -N (R¹⁰)-(CH₂)_{q}-, geradkettiges oder verzweigtes C₁-C₆-Alkyl steht oder für die Gruppe 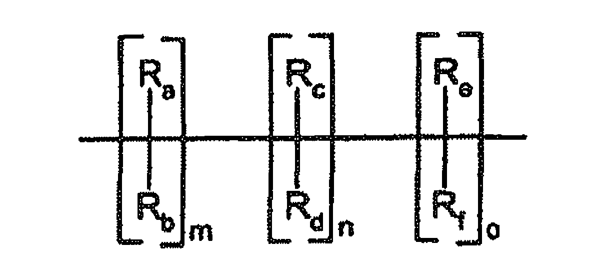 steht oder A, Z und R¹ zusammen die Gruppe 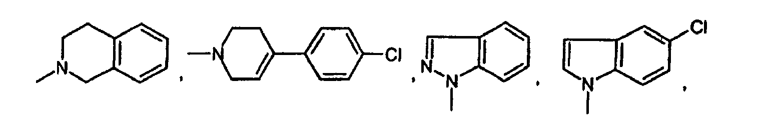 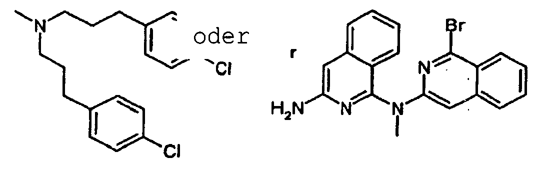 bilden,
m, n und o für 0 bis 3 stehen,
q für 1 bis 6 steht,
Rₐ, R_{b}, R_{c}, R_{d}, Rₑ, R_{f} unabhängig voneinander für Wasserstoff, C₁-C₄-Alkyl oder die Gruppe =NR¹⁰ stehen, und/oder Rₐ und/oder R_{b} zusammen mit R_{c} und/oder R_{d} oder R_{c} zusammen mit Rₑ und/oder R_{f} eine Bindung bilden, oder bis zu zwei Gruppen Rₐ-R_{f} eine Brücke mit jeweils bis zu 3 C-Atomen mit R¹ oder R² bilden,
X für eine Gruppe =NR⁹ oder =N- steht,
Y für eine Gruppe -(CH₂)ₚ steht,
p für eine ganze Zahl 1 bis 4 steht,
R¹ für unsubstituiertes oder gegebenenfalls durch ein oder mehrere Halogene substituiertes, C₁-C₆-Alkyl oder C₁-C₆-Alkyl oder C₁-C₆-Alkoxy, das gegebenfalls durch ein oder mehrere Halogene substituiert ist, oder für unsubstituiertes oder substituiertes Aryl oder Heteroaryl steht,
R² für Wasserstoff oder C₁-C₆-Alkyl steht oder mit Rₐ-R_{f} zusammen mit Z oder R₁ eine Brücke mit bis zu 3 Ringatomen bildet,
R³ für monocyclisches oder bicyclisches, unsubstituiertes oder gegebenenfalls durch ein oder mehrere Halogene, C₁-C₆-Alkyl, C₁-C₆-Alkoxy oder Hydroxy substituiertes Aryl oder Heteroaryl steht,
R⁴, R⁵, R⁶ und R⁷ unabhängig voneinander für Wasserstoff, Halogen oder unsubstituiertes oder gegebenenfalls durch ein oder mehrere Halogene substituiertes C₁-C₆-Alkoxy, C₁-C₆-Alkyl oder C₁-C₆-Carboxyalkyl steht, oder R⁵ und R⁶ zusammen die Gruppe 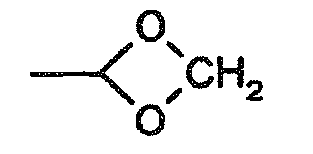 bilden,
R⁸, R⁹ und R¹⁰ unabhängig voneinander für Wasserstoff oder C₁-C₆-Alkyl stehen, sowie ihre Isomere und Salze;
und/oder (iii) eine Verbindung der allgemeinen Formel V 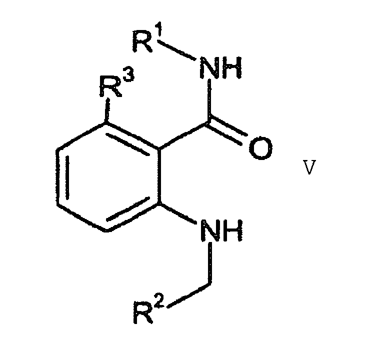
in welcher
R¹ für eine Gruppe 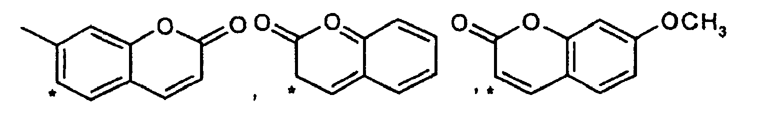 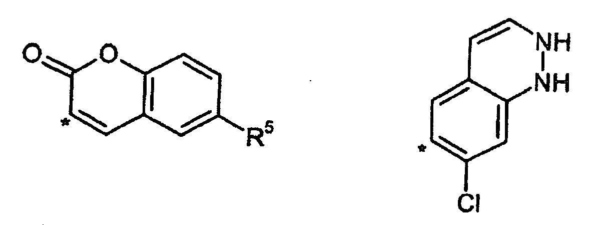 steht
in welcher R⁵ für Chlor, Brom oder die Gruppe -OCH₃ steht, 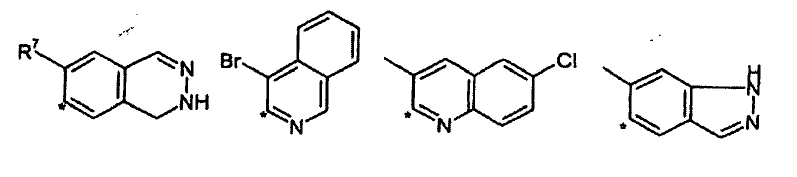 in welcher R⁷ für -CH₃ oder Chlor steht, 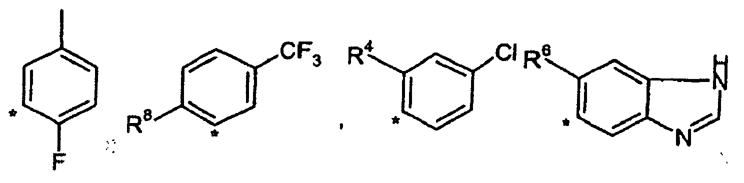 in welcher R⁸ für -CH₃, Fluor, Chlor oder -CF₃ steht,
in welcher R⁴ für Fluor, Chlor, Brom, -CF₃, -N=C, -CH₃, -OCF₃ oder -CH₂OH steht,
in welcher R⁶ für -CH₃ oder Chlor steht,
R² für Pyridyl oder die Gruppe 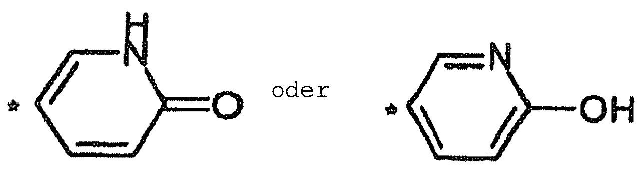 steht
und
R³ für Wasserstoff oder Fluor steht, sowie ihre Isomere und Salze;
und/oder (iv) SEQ ID Nr. 1-59, sTie2, mAB 4301-42-35, ein scFv-tTF-Konjugat und/oder ein L19-scFv-tTF-Konjugat, mit der Maßgabe, daß Verbindung I nicht mit Verbindung II identisch ist.

2. Pharmazeutische Zusammensetzungen nach Anspruch 1, enthaltend als Verbindung I und/oder II wenigstens eine der SEQ ID Nr. 1-59.

3. Pharmazeutische Zusammensetzungen nach Anspruch 1 oder 2, enthaltend als Verbindung I und/oder II SEQ ID Nr. 34a.

4. Pharmazeutische Zusammensetzungen nach einem der Ansprüche 1 bis 3, enthaltend als Verbindung I und/oder II wenigstens eine der folgenden: sTie2, mAB 4301-42-35, ein scFv-tTF-Konjugat und/oder ein L19-scFv-tTF-Konjugat.

5. Pharmazeutische Zusammensetzungen nach einem der Ansprüche 1 bis 4, enthaltend als Verbindung I und/oder II wenigstens eine Verbindung der allgemeinen Formel I 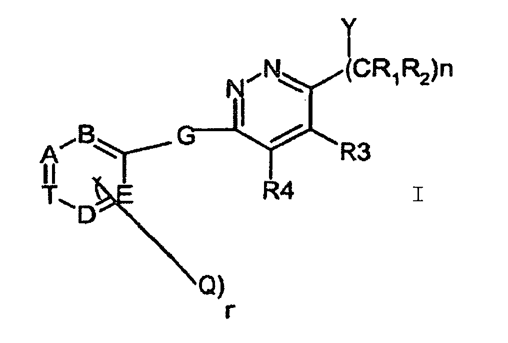 in welcher
r für 0 bis 2 steht,
n für 0 bis 2 steht;
R₃ und R₄
a) jeweils unabhängig voneinander für Niederalkyl stehen,
b) zusammen eine Brücke der Formel II bilden, 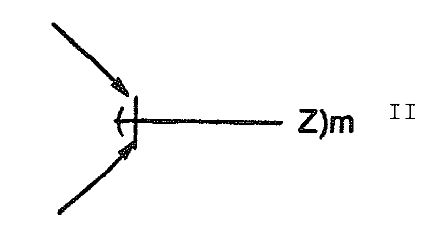 wobei die Bindung über die beiden terminalen C-Atome erfolgt und
m für 0 bis 4 steht; oder
c) zusammen eine Brücke der Teilformel III bilden 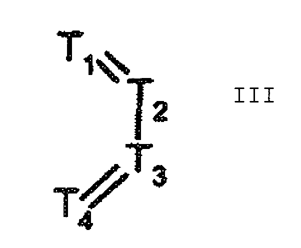 wobei eines oder zwei der Ringglieder T₁, T₂, T₃, T₄ für Stickstoff steht/stehen und die anderen für CH stehen und die Bindung über die Atome T₁ und T₄ erfolgt;
G für C₁-C₆-Alkyl, C₂-C₆-Alkylen oder C₂-C₆-Alkenylen; oder durch Acyloxy oder Hydroxy substituiertes C₂-C₆-Alkylen oder C₃-C₆-Alkenylen; -CH₂-O-, -CH₂-S-, -CH₂-NH-, -CH₂-O-CH₂-, -CH₂-S-CH₂-, -CH₂-NH-CH₂, Oxa (-O-), Thia (-S-) oder Imino (-NH-) steht,
A, B, D, E und T unabhängig voneinander für N oder CH stehen, mit der Maßgabe, daß nicht mehr als drei dieser Substituenten für N stehen,
Q für Niederalkyl, Niederalkyloxy oder Halogen steht,
R₁ und R₂ unabhängig voneinander für H oder Niederalkyl stehen,
X für Imino, Oxa oder Thia steht;
Y für Wasserstoff, unsubstituiertes oder substituiertes Aryl, Heteroaryl oder unsubstituiertes oder substituiertes Cycloalkyl steht; und
Z für Amino, mono- oder disubstituiertes Amino, Halogen, Alkyl, substituiertes Alkyl, Hydroxy, verethertes oder verestertes Hydroxy, Nitro, Cyano, Carboxy, verestertes Carboxy, Alkanoyl, Carbamoyl, N-mono- oder N,N-disubstituiertes Carbamoyl, Amidino, Guanidino, Mercapto, Sulfo, Phenylthio, Phenyl-niederalkylthio, Alkyl-phenyl-thio, Phenylsulfinyl, Phenyl-niederalkyl-sulfinyl, Alkylphenylsulfinyl, Phenylsulfonyl, Phenyl-niederalkan-sulfonyl oder Alkylphenylsulfonyl steht,
wobei, wenn mehr als ein Rest Z vorhanden ist (m ≥ 2), die Substituenten Z gleich oder verschieden voneinander sind, und wobei die mit einem Pfeil **gekennzeichnet**en Bindungen Einfachbindungen oder Doppelbindungen sind; oder ein N-Oxid der Verbindung, wobei ein oder mehrere N-Atome ein Sauerstoffatom tragen, oder ein Salz davon; und/oder eine Verbindung der allgemeinen Formel IV 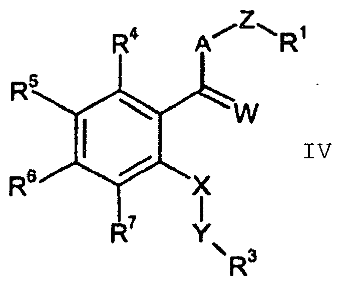 in welcher
A für eine Gruppe =NR² steht,
W für Sauerstoff, Schwefel, zwei Wasserstoffatome oder die Gruppe =NR⁸ steht,
Z für die Gruppe =NR¹⁰ oder =N-, -N (R¹⁰)-(CH₂)_{q}-, geradkettiges oder verzweigtes C₁-C₆-Alkyl steht oder für die Gruppe 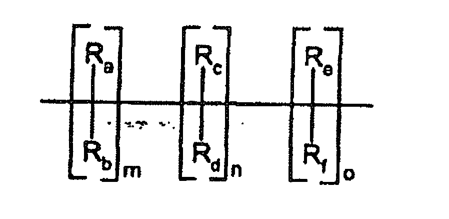 steht oder A, Z und R¹ zusammen die Gruppe 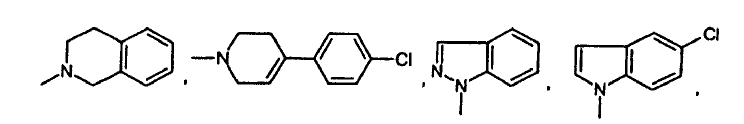 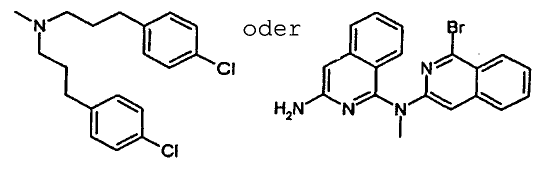 bilden,
m, n und o für 0 bis 3 stehen,
q für 1 bis 6 steht,
Rₐ, R_{b}, R_{c}, R_{d}, Rₑ, R_{f} unabhängig voneinander für Wasserstoff, C₁-C₄-Alkyl oder die Gruppe =NR¹⁰ stehen, und/oder Rₐ und/oder R_{b} zusammen mit R_{c} und/oder R_{d} oder R_{c} zusammen mit Rₑ und/oder R_{f} eine Bindung bilden, oder bis zu zwei Gruppen Rₐ-R_{f} eine Brücke mit jeweils bis zu 3 C-Atomen mit R¹ oder R² bilden,
X für eine Gruppe =NR⁹ oder =N- steht,
Y für eine Gruppe -(CH₂)ₚ steht,
p für eine ganze Zahl 1 bis 4 steht,
R¹ für unsubstituiertes oder gegebenenfalls durch ein oder mehrere Halogene substituiertes, C₁-C₆-Alkyl oder C₁-C₆-Alkyl oder C₁-C₆-Alkoxy, das gegebenfalls durch ein oder mehrere Halogene substituiert ist, oder für unsubstituiertes oder substituiertes Aryl oder Heteroaryl steht,
R² für Wasserstoff oder C₁-C₆-Alkyl steht oder mit Rₐ-R_{f} zusammen mit Z oder R₁ eine Brücke mit bis zu 3 Ringatomen bildet,
R³ für monocyclisches oder bicyclisches, unsubstituiertes oder gegebenenfalls durch ein oder mehrere Halogene, C₁-C₆-Alkyl, C₁-C₆-Alkoxy oder Hydroxy substituiertes Aryl oder Heteroaryl steht,
R⁴, R⁵, R⁶ und R⁷ unabhängig voneinander für Wasserstoff, Halogen oder unsubstituiertes oder gegebenenfalls durch ein oder mehrere Halogene substituiertes C₁-C₆-Alkoxy, C₁-C₆-Alkyl oder C₁-C₆-Carboxyalkyl steht, oder R⁵ und R⁶ zusammen die Gruppe 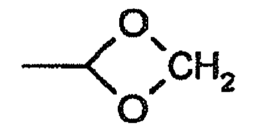 bilden,
R⁸, R⁹ und R¹⁰ unabhängig voneinander für Wasserstoff oder C₁-C₆-Alkyl stehen, sowie ihre Isomere und Salze; und/oder eine Verbindung der allgemeinen Formel V 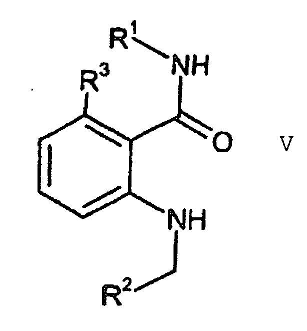 in welcher
R¹ für eine Gruppe 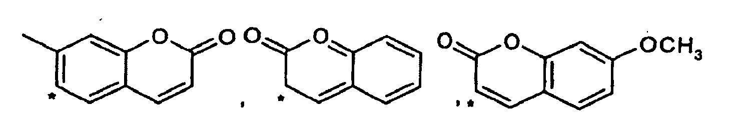 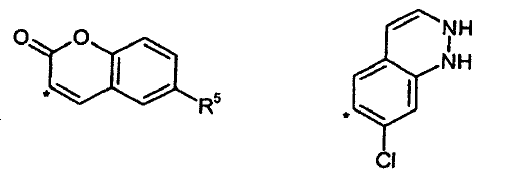 steht
wobei R⁵ für Chloro, Brom oder die Gruppe -OCH₃ steht, 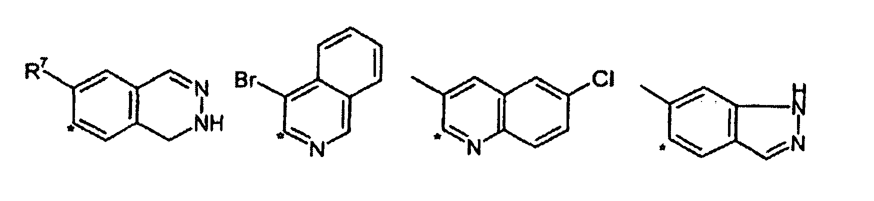 wobei R⁷ für -CH₃ oder Chlor steht, 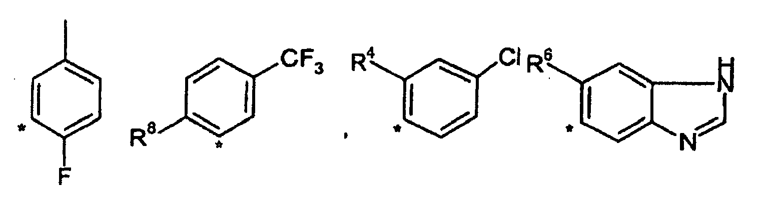
wobei R⁸ für -CH₃, Fluor, Chlor oder -CF₃ steht,
wobei R⁴ für Fluor, Chlor, Brom, -CF₃, -N=C, -CH₃, -OCF₃ oder -CH₂OH steht,
wobei R⁶ für -CH₃ oder Chlor steht,
R² für Pyridyl oder die Gruppe 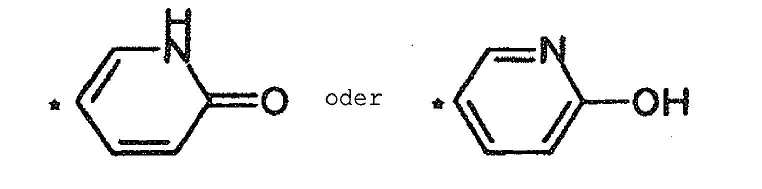 steht
und
R³ für Isomere Wasserstoff oder Fluor steht, sowie ihre und Salze.

6. Pharmazeutische Zusammensetzungen nach einem der Ansprüche 1 bis 5, enthaltend als Verbindung I und/oder II (4-Chlorphenyl)[4-(4-pyridylmethyl)-phthalazin-1-yl]ammoniumhydrogensuccinat.

7. Pharmazeutische Zusammensetzungen nach einem der Ansprüche 1 bis 6, enthaltend als Verbindung I (4-Chlorphenyl)[4-(4-pyridylmethyl)phthalazin-1-yl]-ammoniumhydrogensuccinat, SEQ ID Nr. 1-59, sTie2, mAB 4301-42-35, ein scFv-tTF-Konjugat und/oder ein L19-scFv-tTF-Konjugat und als Verbindung II (4-Chlorphenyl) [4-(4-pyridylmethyl) phthalazin-1-yl]-ammoniumhydrogensuccinat, SEQ ID Nr. 1-59, sTie2, mAB 4301-42-35, ein scFv-tTF-Konjugat und/oder ein L19-scFv-tTF-Konjugat, mit der Maßgabe, daß Verbindung I nicht mit Verbindung II identisch ist.

8. Pharmazeutische Zusammensetzungen nach einem der Ansprüche 1 bis 7, enthaltend als Verbindung I (4-Chlorphenyl)[4-(4-pyridylmethyl)phthalazin-1-yl]-ammoniumhydrogensuccinat und als Verbindung II SEQ ID Nr. 1-59, sTie2, mAB 4301-42-35, ein scFv-tTF-Konjugat und/oder ein L19-scFv-tTF-Konjugat.

9. Pharmazeutische Zusammensetzungen nach einem der Ansprüche 1 bis 7, enthaltend als Verbindung I mAB 4301-42-35 und als Verbindung II sTie2 und/oder ein scFv-tTF-Konjugat.

10. Pharmazeutische Zusammensetzungen nach einem der Ansprüche 1 bis 7, enthaltend als Verbindung I ein scFv-tTF-Konjugat und als Verbindung II- sTie2 und/oder mAB 4301-42-35.

11. Pharmazeutische Zusammensetzungen nach einem der Ansprüche 1 bis 7, enthaltend als Verbindung I ein L19-scFv-tTF-Konjugat und als Verbindung II sTie2.

12. Verwendung von pharmazeutischen Zusammensetzungen nach einem der Ansprüche 1 bis 11 zur Herstellung eines Medikaments zur Behandlung von Tumoren, Krebs, Psoriasis, Arthritis wie z.B. rheumatoider Arthritis, Hämangiomen, Angiofibromen, Augenkrankheiten wie z.B. diabetischer Retinopathie, neovaskulärem Glaukom, Nierenkrankheiten wie z.B. Glomerulonephritis, diabetischer Nephropathie, maligner Nephrosklerose, thrombotische Mikroangiopathie, Transplantatabstoßungen und Glomerulopathie, fibrotischen Krankheiten wie z.B. Leberzirrhose, proliferativen Krankheiten von Mesangialzellen, Arteriosklerose, Schädigungen von Nervengewebe, Unterdrückung der Entstehung von Aszites in Patienten und Unterdrückung von VEGF-Ödemen.

## Revendications

1. Compositions pharmaceutiques comprenant une combinaison d'un composé I et d'un composé II, **caractérisées en ce que** le composé I et le composé II comprennent au moins l'un parmi
(i) un composé de formule générale I 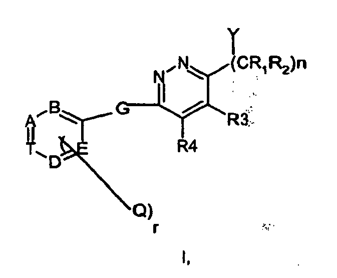 dans laquelle
r vaut 0 à 2,
n vaut 0 à 2 ;
R₃ et R₄ a)
chacun indépendamment l'un de l'autre représentent un alkyle inférieur,
b) ensemble forment un pont de formule générale partielle II, 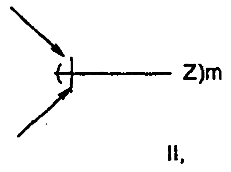 dans laquelle la liaison se fait via les deux atomes de C terminaux, et
m vaut 0 à 4 ; ou
c) ensemble forment un pont de formule partielle III 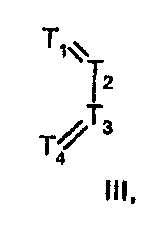 dans laquelle un ou deux des chaînons T₁, T₂, T₃, T₄ représentent un azote et chacun des autres représente CH, et la liaison se fait via les atomes T₁ et T₄;
G représente un alkyle en C₁-C₆, un alkylène en C₂-C₆ ou un alcénylène en C₂-C₆ ; ou un alkylène en C₂-C₆ ou un alcénylène en C₃-C₆ substitués par un groupe acyloxy ou hydroxy ; -CH₂-O-, -CH₂-S-, -CH₂-NH-, -CH₂-O-CH₂-, -CH₂-S-CH₂-, -CH₂-NH-CH₂-, oxa (-O-) , thia (-S-) ou imino(-NH-),
A, B, D, E et T indépendamment les uns des autres représentent N ou CH, à la condition que pas plus de trois de ces substituants ne représentent N,
Q représente un alkyle inférieur, un alkyloxy inférieur ou un halogène,
R₁ et R₂ chacun indépendamment l' un de l'autre représentent H ou un alkyle inférieur,
X représente imino, oxa ou thia ;
Y représente un hydrogène, un aryle non substitué ou substitué, un hétéroaryle non substitué ou substitué, ou un cycloalkyle non substitué ou substitué ; et
Z représente : amino, amino mono- ou disubstitué, halogène, alkyle, alkyle substitué, hydroxy, hydroxy étherifié ou estérifié, nitro, cyano, carboxy, carboxy estérifié, alcanoyle, carbamoyle, carbamoyle N-mono- ou N,N-disubstitué, amidino, guanidino, mercapto, sulfo, phénylthio, phényl-alkylthio inférieur, alkyl-phényl-thio, phénylsulfinyle, phényl-alkylsulfinyle inférieur, alkylphénylsulfinyle, phénylsulfonyle, phényl-alcanesulfonyle inférieur, ou alkylphénylsulfonyle, alors que si plus d'un Z restant est présent (m > 2), les substituants Z sont identiques ou différents les uns des autres, et où les liaisons marquées d'une flèche sont des liaisons simples ou doubles ; ou un N-oxyde dudit composé, où un ou plusieurs atomes de N portent un atome d'oxygène, ou un sel de celui-ci ;
et/ou (ii) un composé de formule générale IV 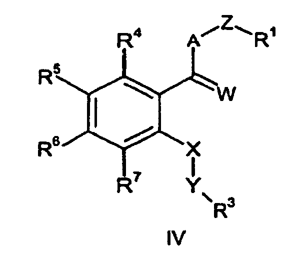 dans laquelle
A représente un groupe =NR²,
W représente oxygène, soufre, deux atomes d'hydrogène ou le groupe =NR⁸,
Z représente le groupe =NR¹⁰ ou =N-, -N (R¹⁰)-(CH₂)_{q}-, un alkyle en C₁-C₆ ramifié ou non ramifié ou est le groupe 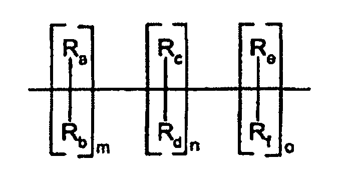 ou A, Z et R¹ ensemble forment le groupe 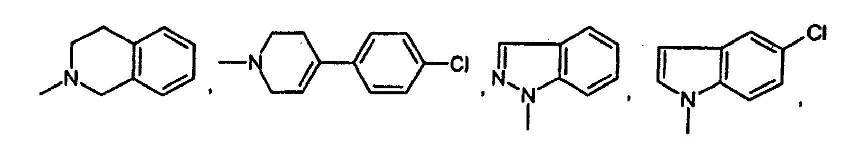 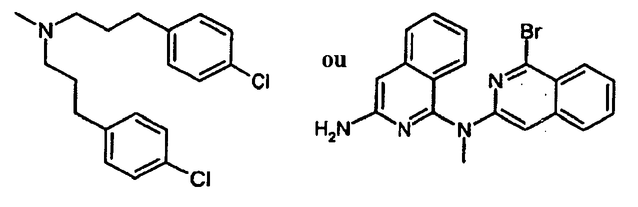
m, n et o valent 0 à 3,
q vaut 1 à 6,
Rₐ, R_{b}, R_{c}, R_{d}, Rₑ, R_{f} indépendamment les uns des autres représentent un hydrogène, un alkyle en C₁-C₄ ou le groupe =NR¹⁰, et/ou Rₐ et/ou R_{b} conjointement avec R_{c} et/ou R_{d} ou R_{c} conjointement avec Rₑ et/ou R_{f} forment une liaison, ou jusqu'à deux des groupes Rₐ-R_{f} forment un pont ayant chacun jusqu'à 3 atomes de C avec R¹ ou R²,
X représente un groupe =NR⁹ou =N-,
Y représente un groupe -(CH₂)ₚ,
p représente un entier de 1 à 4,
R¹ représente non substitué ou éventuellement substitué par un ou plusieurs parmi un halogène ou un alkyle en C₁-C₆, ou un alkyle en C₁-C₆ ou un alcoxy en C₁-C₆ éventuellement substitué par un ou plusieurs halogènes, ou est un aryle ou hétéroaryle non subtitué ou substitué,
R² représente hydrogène ou un alkyle en C₁-C₆, ou forme un pont ayant jusqu'à 3 atomes du cycle avec Rₐ-R_{f} conjointement avec Z ou R₁,
R³ représente un aryle ou un hétéroaryle monocyclique ou bicyclique non substitué ou éventuellement substitué par un ou plusieurs parmi un halogène, un alkyle en C₁-C₆, un alcoxy en C₁-C₆ ou un hydroxy,
R⁴, R⁵ R⁶ et R⁷ indépendamment les uns des autres représentent un hydrogène, un halogène
ou un alcoxy en C₁-C₆, un alkyle en C₁-C₆
ou un carboxyalkyle en C₁-C₆, non substitués ou éventuellement substitués par un ou plusieurs halogènes, ou R⁵ et R⁶ ensemble forment le groupe 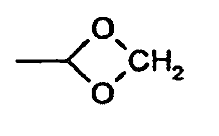
R⁸, R⁹ et R¹⁰ indépendamment les uns des autres représentent un hydrogène ou un alkyle en C₁-C₆,
ainsi que ses isomères et sels ;
et/ou (iii) un composé de formule générale V 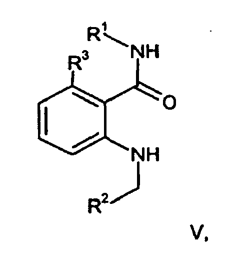 dans laquelle
R¹ représente un groupe 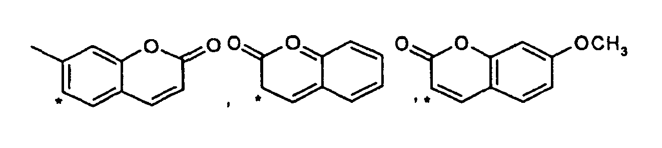 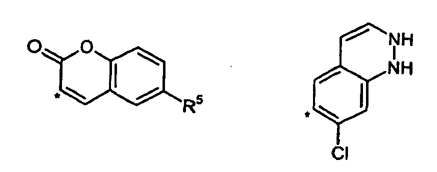 où R⁵ est chloro, bromo ou le groupe -OCH₃, 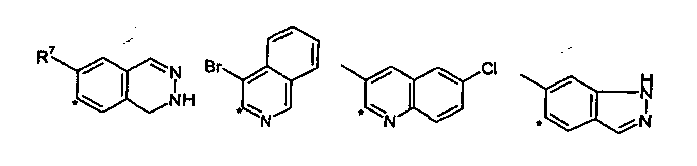 où R⁷ est -CH₃ ou chloro, 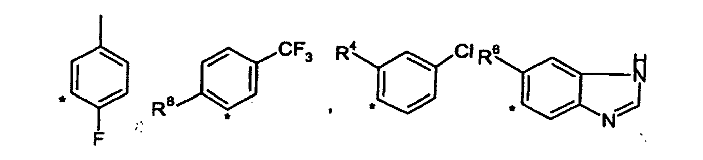
où R⁸ est -CH₃, fluoro, chloro ou -CF₃,
où R⁴ est fluoro, chloro, bromo, -CF₃, -N=C, -CH₃, -OCF₃, ou -CH₂OH
où R⁶ est -CH₃ ou chloro,
R² représente pyridyle ou le groupe 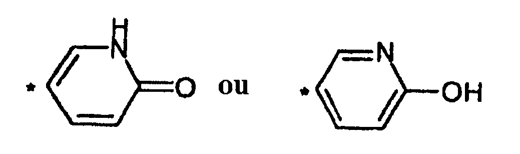 et
R³ représente un hydrogène ou fluoro, ainsi que ses isomères et sels ;
et/ou (iv) SEQ ID NO : 1 à 59, sTie2, l'AcM 4301-42-35, un conjugué scFv-tTF et/ou un conjugué L19 scFv-tTF, à la condition que le composé I ne soit pas identique au composé II.

2. Compositions pharmaceutiques selon la revendication 1, qui comprennent à titre de composé I et/ou II au moins l'une des SEQ ID NO : 1 à 59.

3. Compositions pharmaceutiques selon les revendications 1-2, qui comprennent à titre de composé I et/ou II la SEQ ID NO : 34a.

4. Compositions pharmaceutiques selon les revendications 1-3, qui comprennent à titre de composé I et/ou II au moins l'un parmi sTie2, l'AcM 4301-42-35, un conjugué scFv-tTF et/ou un conjugué L19 scFv-tTF.

5. Compositions pharmaceutiques selon les revendications 1-4, qui comprennent à titre de composé I et/ou II au moins un composé de formule générale I 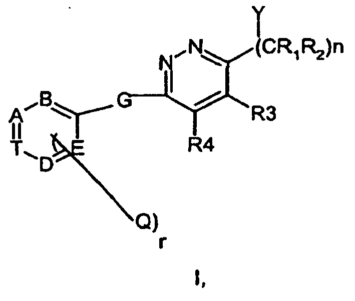 dans laquelle
r vaut 0 à 2,
n vaut 0 à 2 ;
R₃ et R₄
a) chacun indépendamment l'un de l'autre représentent un alkyle inférieur,
b) ensemble forment un pont de formule générale partielle II, 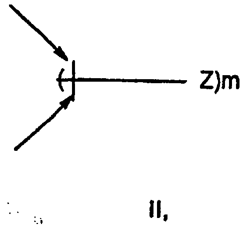 dans laquelle la liaison se fait via les deux atomes de C terminaux, et
m vaut 0 à 4 ; ou
c) ensemble forment un pont de formule partielle III 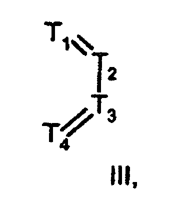 dans laquelle un ou deux des chaînons T₁, T₂, T₃, T₄ représentent un azote et chacun des autres représente CH, et la liaison se fait via les atomes T₁ et T₄ ;
G représente un alkyle en C₁-C₆, un alkylène en C₂-C₆ ou un alcénylène en C₂-C₆ ; ou un alkylène en C₂-C₆ ou un alcénylène en C₃-C₆ substitués par un groupe acyloxy ou hydroxy ; -CH₂-O-, -CH₂-S-, -CH₂-NH-, -CH₂-O-CH₂-, -CH₂-S-CH₂-, -CH₂-NH-CH₂-, oxa(-O-), thia(-S-) ou imino(-NH-),
A, B, D, E et T indépendamment les uns des autres représentent N ou CH, à la condition que pas plus de trois de ces substituants ne représentent N,
Q représente un alkyle inférieur, un alkyloxy inférieur ou un halogène,
R₁ et R₂ chacun indépendamment l'un de l'autre représentent H ou un alkyle inférieur,
X représente imino, oxa ou thia ;
Y représente un hydrogène, un aryle non substitué ou substitué, un hétéroaryle non substitué ou substitué, ou un cycloalkyle non substitué ou substitué; et
Z représente : amino, amino mono- ou disubstitué, halogène, alkyle, alkyle substitué, hydroxy, hydroxy étherifié ou estérifié, nitro, cyano, carboxy, carboxy estérifié, alcanoyle, carbamoyle, carbamoyle N-mono- ou N,N-disubstitué, amidino, guanidino, mercapto, sulfo, phénylthio, phényl-alkylthio inférieur, alkyl-phényl-thio, phénylsulfinyle, phényl-alkylsulfinyle inférieur, alkylphénylsulfinyle, phénylsulfonyle, phényl-alcanesulfonyle inférieur, ou alkylphénylsulfonyle, alors que si plus d'un Z restant est présent (m ≥ 2), les substituants Z sont identiques ou différents les uns des autres, et où les liaisons marquées d'une flèche sont des liaisons simples ou doubles ; ou un N-oxyde dudit composé, où un ou plusieurs atomes de N portent un atome d'oxygène, ou un sel de celui-ci ;
et/ou un composé de formule générale IV 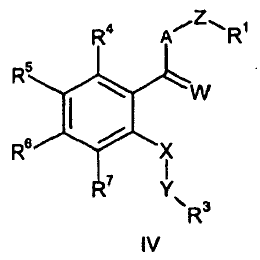 dans laquelle
A représente un groupe =NR²,
W représente oxygène, soufre, deux atomes d'hydrogène ou le groupe =NR⁸,
Z représente le groupe =NR¹⁰ ou =N-, -N(R¹⁰)-(CH₂)_{q}-, un alkyle en C₁-C₆ ramifié ou non ramifié ou est le groupe 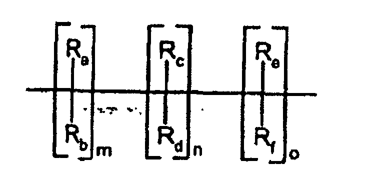 ou A, Z et R¹ ensemble forment le groupe 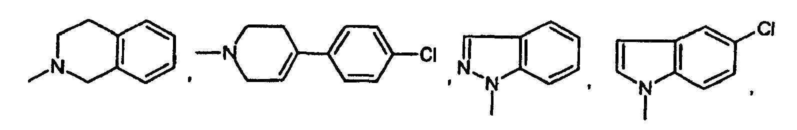 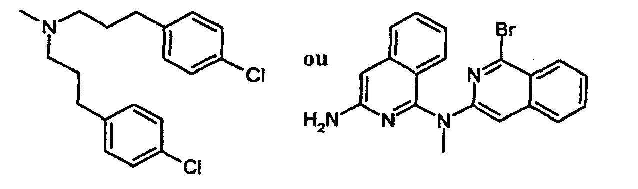
m, n et o valent 0 à 3,
q vaut 1 à 6,
Rₐ, R_{b}, R_{c}, R_{d}, Rₑ, R_{f} indépendamment les uns des autres représentent un hydrogène, un alkyle en C₁-C₄ ou le groupe =NR¹⁰ , et/ou Rₐ et/ou R_{b} conjointement avec R_{c} et/ou R_{d} ou R_{c} conjointement avec Rₑ et/ou R_{f} forment une liaison, ou jusqu'à deux des groupes Rₐ-R_{f} forment un pont ayant chacun jusqu'à 3 atomes de C avec R¹ ou R²,
X représente un groupe =NR⁹ou =N-,
Y représente un groupe -(CH₂)ₚ,
p représente un entier de 1 à 4,
R¹ représente non substitué ou éventuellement substitué par un ou plusieurs parmi un halogène ou un alkyle en C₁-C₆, ou un alkyle en C₁-C₆ ou un alcoxy en C₁-C₆ éventuellement substitué par un ou plusieurs halogènes, ou est un aryle ou hétéroaryle non subtitué ou substitué,
R² représente un hydrogène ou un alkyle en C₁-C₆, ou forme un pont ayant jusqu'à 3 atomes du cycle avec Rₐ-R_{f} conjointement avec Z ou R₁,
R³ représente un aryle ou un hétéroaryle monocyclique ou bicyclique non substitué ou éventuellement substitué par un ou plusieurs parmi un halogène, un alkyle en C₁-C₆, un alcoxy en C₁-C₆ ou un hydroxy,
R⁴ R⁵ R⁶ et R⁷ indépendamment les uns des autres représentent un hydrogène, un halogène ou un alcoxy en C₁-C₆, un alkyle en C₁-C₆ ou un carboxyalkyle en C₁-C₆, non substitués ou éventuellement substitués par un ou plusieurs halogènes, ou R⁵ et R⁶ ensemble forment le groupe 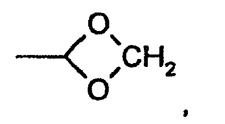
R⁸, R⁹ et R¹⁰ indépendamment les uns des autres représentent un hydrogène ou un alkyle en C₁-C₆,
ainsi que ses isomères et sels ;
et/ou un composé de formule générale V 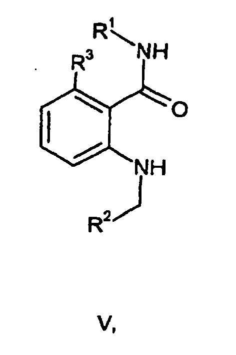 dans laquelle
R¹ représente un groupe 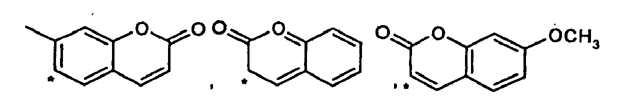 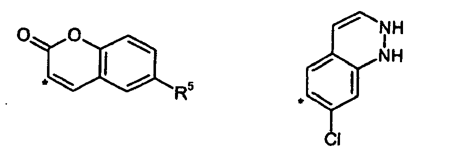 où R⁵ est chloro, bromo ou le groupe -OCH₃, 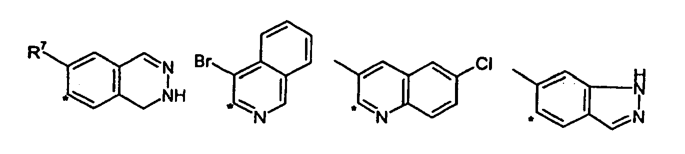 où R⁷ est -CH₃ ou chloro, 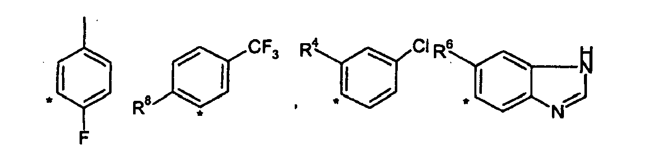 où R⁸ est -CH₃, fluoro, chloro ou -CF₃,
où R⁴ est fluoro, chloro, bromo, -CF₃, -N=C, -CH₃, -OCF₃, ou -CH₂OH
où R⁶ est -CH₃ ou chloro,
R² représente pyridyle ou le groupe 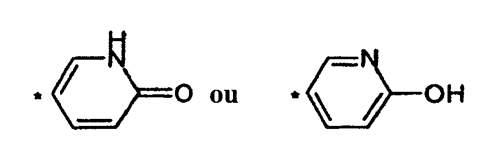 et
R³ représente un hydrogène ou fluoro, ainsi que ses isomères et sels.

6. Compositions pharmaceutiques selon les revendications 1-5, qui comprennent à titre de composé I et/ou II de l'hydrogénosuccinate de (4-chlorophényl)[4-(4-pyridylméthyl)-phtalazin-1-yl]-ammonium.

7. Compositions pharmaceutiques selon les revendications 1-6, qui comprennent à titre de composé I de l'hydrogénosuccinate de (4-chlorophényl)[4-(4-pyridylméthyl)-phtalazin-1-yl]-ammonium, SEQ ID N0 _{:} 1 à 59, sTie2, l'AcM 4301-42-35, un conjugué scFv-tT F et/ou un conjugué L19 scFv-tTF, et à titre de composé II, de l'hydrogénosuccinate de (4-chlorophényl)[4-(4-pyridylméthyl)-phtalazin-1-yl]ammonium, SEQ ID NO : 1-59, sTie2, l'AcM 4301-42-35, un conjugué scFv-tTF et/ou un conjugué L19 scFv-tTF, à la condition que le composé I ne soit pas identique au composé II.

8. Compositions pharmaceutiques selon les revendications 1-7, qui comprennent à titre de composé I de l'hydrogénosuccinate de (4-chlorophényl) [4-(4-pyridylméthyl)-phtalazin-1-yl]-ammonium, et à titre de composé II, SEQ ID NO : 1 à 59, sTie2, l'AcM 4301-42-35, un conjugué scFv-tTF et/ou un conjugué L19 scFv-tTF.

9. Compositions pharmaceutiques selon les revendications 1-7, qui comprennent à titre de composé I l'AcM 4301-42-35 et à titre de composé II sTie2 et/ou un conjugué scFv-tTF.

10. Compositions pharmaceutiques selon les revendications 1-7, qui comprennent à titre de composé I un conjugué scFv-tTF et à titre de composé II sTie2 et/ou l'AcM 4301-42-35.

11. Compositions pharmaceutiques selon les revendications 1-7, qui comprennent à titre de composé I un conjugué L19 scFv-tTF et à titre de composé II sTie2.

12. Utilisation de compositions pharmaceutiques selon les revendications 1-11, pour la production d'un médicament destiné au traitement de tumeurs, de cancers, du psoriasis, de l'arthrite, telle que la polyarthrite rhumatoïde, d'un hémangiome, d'un angiofibrome, de maladies de l'oeil, telles que la rétinopathie diabétique, le glaucome néovasculaire, de maladies du rein, telles que la glomérulonéphrite, la néphropathie diabétique, la néphrosclérose maligne, le syndrome microangiopathique thrombotique, les rejets de greffe et la glomérulopathie, les maladies fibrotiques, telles que le foie cirrhotique, les maladies à prolifération des cellules mésangiales, l'artériosclérose, un endommagement des tissus nerveux, la suppression de la formation d'ascite chez des patients et la suppression des oedèmes liés au VEGF.
